# EUROPÄISCHE PATENTSCHRIFT

(11) **EP 1 425 283 B1**
(45) Veröffentlichungstag und Bekanntmachung des Hinweises auf die Patenterteilung: **16.03.2005**
(21) Anmeldenummer: 02796211.7
(22) Anmeldetag: 09.08.2002
(51) Int. Cl.: C07D 491/04, A61K 31/519, A61P 9/00

(54) **NEUE 4-AMINOFUROPYRIMIDINE UND IHRE VERWENDUNG**
NOVEL 4-AMINOFUROPYRIMIDINES AND THE USE THEREOF
NOUVELLES 4-AMINOFUROPYRIMIDINES ET LEUR UTILISATION

(30) Priorität: 22.08.2001 DE 10141212
(43) Veröffentlichungstag der Anmeldung: 09.06.2004
(73) Patentinhaber: Bayer HealthCare AG, 51368 Leverkusen (DE)
(72) Erfinder: BISCHOFF, Erwin, 42115 Wuppertal (DE); HAUSWALD, Markus, 50674 Köln (DE); NELL, Peter, 42115 Wuppertal (DE); RÖHRIG, Susanne, 40724 Hilden (DE); SCHLEMMER, Karl-Heinz, 42113 Wuppertal (DE); STEINHAGEN, Henning, 65843 Sulzbach (DE); STOLTEFUSS, Jürgen, 42781 Haan (DE); WEIGAND, Stefan, 42115 Wuppertal (DE)
(86) Internationale Anmeldenummer: PCT/EP2002/008906
(87) Internationale Veröffentlichungsnummer: WO 2003/018589

(56) Entgegenhaltungen:
- DE-A- 1 817 146
- JP-A- 48 081 894
- SAIKACHI, H. ET AL.: "Synthesis of the Furan Derivatives. XLVIII On the Synthesis of Difurylfuro[2,3-d]pyrimidines and Difurylfuro-[3,2-d]-s-triazolopyrimidines" YAKUGAKU ZASSHI, Bd. 89, Nr. 10, 1969, Seiten 1434-1439, XP001120484
- JOHANNSEN, F. ET AL.: "Phosphorous Pentoxide in Organic Synthesis XXVI. Preparation and alkylation of furo[2,3-d]pyrimidin-4-amines" CHEMICA SCRIPTA, Bd. 26, 1986, Seiten 337-342, XP001120482

## Beschreibung

Die Erfindung betrifft neue 4-Aminofuro[2,3-*d*]pyrimidin-Derivate, Verfahren zu ihrer Herstellung sowie ihre Verwendung in Arzneimitteln, insbesondere zur Prävention und/oder Behandlung von kardiovaskulären Erkrankungen.

Adenosin ist ein endogener cardioprotektiver und neuroprotectiver Effektor (Olafsson et al., *Circulation* 1987, 76:1135-1145; Dragunow and Faull, *Trends in Pharmacol. Sci.* 1988, 9:193; Marangos, *Medical Hypothesis* 1990, 32:45). Es wird unter hypoxischen Bedingungen z.B. bei kardialen oder peripheren Verschlusskrankheiten freigesetzt (W. Makarewicz, *"Purine and Pyrimidine Metabolism in Man",* Plenum Press, New York, 11, 1998, 351-357). Daher ist dieser Effekt unter zellschädigenden Bedingungen mit begrenzter Sauerstoffversorgung wie z.B. bei Ischämie besonders ausgeprägt. Adenosin ist ein stark wirksamer Vasodilatator und ist in die metabolische Regulation des Blutflusses involviert. Es verstärkt das ischämische "preconditioning" (R. Strasser, A. Vogt, W. Scharper, *Z. Kardiologie* 85, 1996, 79-89; Schrader, *Circulation* 1990, 81:389-391) und kann das Wachstum von Kollateralgefäßen fördern. Daher schützt Adenosin als natürlicher Abwehrmechanismus vor den Folgen einer Reihe pathophysiologischer ischaemie-bedingter Situationen, wie cerebrale und kardiale Ischaemien, z.B. indem es die koronare oder cerebrale Durchblutung durch Vasodilatation steigert, die Thombozytenaggregation inhibiert und die Angiogenese stimuliert. Die vielen pharmakologischen Effekte von Adenosin umfassen auch Wirkungen auf die Nierendurchblutung, die Atmung, auf Schmerz, auf Entzündungen, auf den Gastrointestinaltrakt, auf Blutzellen und auf Adipocyten.

Systemisch verabreichtes Adenosin hat jedoch eine sehr kurze Halbwertszeit (Moser et al., *Am. J .Physiol.* 1989, 256:C799-C806) und führt zu einer starken systemischen Blutdrucksenkung, welche unerwünscht ist, da der Blutfluss in die ischämischen Gebiete noch weiter reduziert werden kann ("steal phenomenon", L.C. Becker, *Circulation* 57, 1978, 1103-1110). Hohe Dosen von Adenosin sind daher toxisch und der therapeutische Wert von systemisch verabreichtem Adenosin ist limitiert.

Adenosin ist ein Purin-Nucleosid und ein Zwischenprodukt auf dem Weg des Purin-Nucleotid-Abbaus bzw. der Purin-Nucleotid-Neusynthese. Adenosin-Kinase (ATP: adenosin 5'-phosphotransferase, EC 2.7.1.20) ist eines der Schlüsselenzyme der Regulation der intrazellulären Adenosin-Konzentration (Arch und Newsholm, *Essays Biochem.* 1978, 14:82-123). Adenosin-Kinase ist ein ubiquitär vorkommendes Enzym, das im Cytosol die Phosphorylierung von Adenosin zu AMP katalysiert, wobei ATP als Phosphatdonor benützt wird und Mg²⁺ vermutlich als MgATP²⁺-Komplex für die Reaktion notwendig ist (Palella et al., *J. Biol. Chem.* 1980, 255:5264-5269). Die Regulation der Adenosinkonzentration wird aber auch in Abhängigkeit von der jeweiligen Stoffwechsellage von anderen Enzymen wie der Adenosin-Desaminase und der S-Adenosylhomocystein-Hydrolase reguliert.

Der Vorteil der Adenosinkinase-Inhibition gegenüber systemisch verabreichtem Adenosin liegt in der Ischämieselektivität. Ein Adenosinkinase-Inhibitor kann die Konzentration von lokal durch die Ischämie entstandenem Adenosin erhöhen und auf diese Weise nur in den ischaemischen Gebieten die Gefäße maximal dilatieren, die Durchblutung verbessern und die zellprotektiven Effekte von Adenosin lang-anhaltend wirksam werden lassen. Somit können Adenosinkinase-Inhibitoren durch orale oder intravenöse Applikation zur Prävention und/oder Behandlung von ischämischen Erkrankungen eingesetzt werden.

Verschiedene Hinweise deuten darüber hinaus auf ein neuroprotektives, antikonvulsives, analgetisches und Schlaf-induzierendes Potential von Adenosinkinase-Inhibitoren hin, da sie die Eigeneffekte von Adenosin durch eine Hemmung seiner zellulären Rückaufnahme verstärken (K.A. Rudolphi et al., *Cerebrovascular and Brain Metabolism Reviews* 4, 1992, 364-369; T.F. Murray et al., *Drug Dev. Res.* 28, 1993, 410-415; T. Porkka-Heiskanen et al., *Science* 276, 1997, 1265-1268; *"Adenosine in the Nervous System ",* Ed.: Trevor Stone, Academic Press Ltd., 1991, 217-227; M.P. DeNinno, *Annual Reports in Medicinal Chemistry* 33, 1998, 111-120). Adenosinkinase-Inhibitoren können daher auch zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994) neuropathischen Schmerzen, wie zum Beispiel, bei diabetischer Neuropathie, postherpetischer Neuralgie, peripheren Nervenbeschädigungen, zentralem Schmerz und trigeminaler Neuralgie eingesetzt werden.

4-Aminofuro[2,3-*d*]pyrimidin-Derivate mit Glattmuskel-relaxierender Wirkung sind in der Offenlegungsschrift DE 1 817 146 beschrieben.

Aufgabe der vorliegenden Erfindung ist die Bereitstellung neuer Verbindungen mit verbesserten pharmazeutischen Eigenschaften. Diese Aufgabe wird durch die erfindungsgemäßen Verbindungen der Formel (I) gelöst, die als Adenosinkinase-Inhibitoren wirken.

Gegenstand der vorliegenden Erfindung sind Verbindungen der Formel (I) 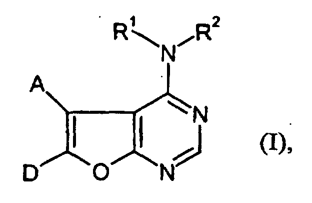 in welcher
- A: für Phenyl oder 5- oder 6-gliedriges Heteroaryl mit bis zu drei Heteroatomen aus der Reihe N, O und/oder S, die jeweils bis zu dreifach, unabhängig voneinander, durch Substituenten aus der Gruppe Halogen, Hydroxy, (C₁-C₆)-Alkoxy, Trifluormethyl, Trifluormethoxy, Amino, Carboxyl und (C₁-C₆)-Alkoxycarbonyl substituiert sein können,
oder für eine Gruppe der Formel 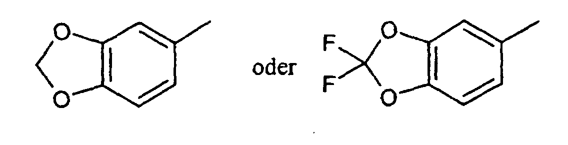 steht,
- D: für eine Gruppe der Formel

R³-E-G-

steht, worin
G Phenylen oder 5- oder 6-gliedriges Heteroarylen mit bis zu drei Heteroatomen aus der Reihe N, O und/oder S bedeutet, die jeweils bis zu zweifach, unabhängig voneinander, durch Substituenten aus der Gruppe Halogen, Trifluormethyl, Trifluormethoxy, (C₁-C₆)-Alkoxy, Amino, Nitro und Carboxyl substituiert sein können,
E für eine Bindung, eine Carbonylgruppe, eine Sulfonylgruppe oder für eine Gruppe der Formel *-C(O)-NR⁴- oder *-SO₂-NR⁴- steht,
worin * die Anknüpfungsstelle zur Gruppe R³ und R⁴ Wasserstoff oder (C₁-C₆)-Alkyl bedeutet, und
R³ für Halogen, Trifluormethyl, Hydroxy, gegebenenfalls durch Hydroxy oder Amino substituiertes (C₁-C₆)-Alkoxy, Trifluormeth-oxy, Nitro, Carboxyl oder eine Gruppe der Formel H-C(O)-NR⁴-,
worin R⁴ die oben angegebene Bedeutung hat,
für (C₁-C₆)-Alkyl, das ein- bis zweifach, unabhängig voneinander, durch Substituenten ausgewählt aus der Gruppe Halogen, Trifluormethyl, Hydroxy, (C₁-C₆)-Alkoxy, Amino, Mono- oder Di-(C₁-C₆)-alkylamino, (C₁-C₆)-Acylamino, (C₁-C₆)-Alkoxycarbonylamino, Amidino, Guanidino, Carboxyl, (C₁-C₆)-Alkoxycarbonyl, (C₆-C₁₀)-Aryl, (C₆-C₁₀)-Aryloxy und 5- bis 10-gliedriges Heteroaryl mit bis zu drei Heteroatomen aus der Reihe N, O und/oder S substituiert sein kann, wobei Aryl, Aryloxy und Heteroaryl ihrerseits jeweils ein- bis zweifach, unabhängig voneinander, durch Halogen, Hydroxy, Amino, (C₁-C₄)-Alkyl, (C₁-C₆)-Alkoxy, Cyano oder Nitro substituiert sein können,
für (C₃-C₇)-Cycloalkyl, das durch Phenyl oder bis zu vierfach durch (C₁-C₄)-Alkyl substituiert sein kann,
für (C₆-C₁₀)-Aryl, das ein- bis zweifach, unabhängig voneinander, durch Substituenten ausgewählt aus der Gruppe von Halogen, Trifluormethyl, (C₁-C₆)-Alkyl, Hydroxy, (C₁-C₆)-Alkoxy, (C₁-C₆)-Alkanoyl, Cyano, Nitro, Amino, Mono- und Di-(C₁-C₆)-alkylamino, (C₁-C₆)-Acylamino, Carboxyl, (C₁-C₆)-Alkoxycarbonyl und 5- bis 6-gliedriges Heteroaryl mit bis zu zwei Heteroatomen aus der Reihe N, O und/oder S substituiert sein kann,
für 4- bis 7-gliedriges, gesättigtes oder partiell ungesättigtes, über ein Ringkohlenstoffatom oder über ein Ringstickstoffatom gebundenes Heterocyclyl mit bis zu drei Heteroatomen aus der Reihe N, O und/oder S, das bis zu dreifach, unabhängig voneinander, durch (C₁-C₆)-Alkyl, welches seinerseits durch Hydroxy, (C₁-C₄)-Alkoxy oder Phenyl substituiert sein kann, (C₁-C₆)-Alkoxycarbonyl, (C₁-C₆)-Alkoxycarbonylamino, (C₁-C₆)-Alkanoyl, (C₁-C₆)-Alkylcarbonylamino, 1,2-Dioxyethylen, Carboxyl, Amino, Hydroxy, (C₁-C₆)-Alkoxy oder eine Oxo-Gruppe substituiert sein kann,
für 5- bis 10-gliedriges, über ein Ringkohlenstoffatom oder über ein Ringstickstoffatom gebundenes Heteroaryl mit bis zu drei Heteroatomen aus der Reihe N, O und/oder S, das seinerseits gegebenenfalls ein- bis zweifach, unabhängig voneinander, durch Substituenten ausgewählt aus der Gruppe von Halogen, Nitro, Amino, Hydroxy, (C₁-C₆)-Alkyl, (C₃-C₆)-Cycloalkyl, Phenyl, Benzyl und 5-gliedriges Heteroaryl mit bis zu zwei Heteroatomen aus der Reihe N, O und/oder S substituiert ist,
oder
für eine Gruppe der Formel -NR⁵R⁶ steht,
worin
R⁵ und R⁶ unabhängig voneinander für Wasserstoff, (C₁-C₆)-Alkyl, welches durch Hydroxy, Amino, (C₁-C₄)-Alkoxy, Mono- oder Di-(C₁-C₄)-alkylamino oder Phenyl substituiert sein kann, für (C₃-C₇)-Cycloalkyl, welches ein- bis zweifach, unabhängig voneinander, durch Hydroxy, Amino, (C₁-C₄)-Alkoxy, Monooder Di-(C₁-C₄)-alkylamino oder (C₁-C₄)-Alkyl substituiert sein kann, für (C₆-C₁₀)-Aryl, welches ein- bis zweifach, unabhängig voneinander, durch Hydroxy, Halogen, Amino, (C₁-C₄)-Alkoxy oder Nitro substituiert sein kann, oder für 5- bis 6-gliedriges Heterocyclyl oder 5- bis 6-gliedriges Heteroaryl mit jeweils bis zu zwei Heteroatomen aus der Reihe N, O und/oder S stehen,
oder
- D: für eine Gruppe der Formel 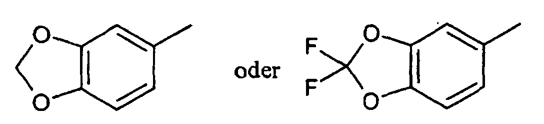 steht,
- R¹: für Wasserstoff, (C₃-C₆)-Cycloalkyl oder für (C₁-C₆)-Alkyl steht, das ein- bis zweifach, unabhängig voneinander, durch Hydroxy, (C₁-C₆)-Alkoxy, Amino, Mono- oder Di-(C₁-C₆)-alkylamino substituiert sein kann,
und
- R²: für (C₁-C₆)-Alkyl, das ein- bis zweifach, unabhängig voneinander, durch Substituenten ausgewählt aus der Gruppe von Trifluormethyl, Hydroxy, (C₁-C₆)-Alkoxy, Amino, Mono- oder Di-(C₁-C₆)-alkylamino, Phenylamino, Carboxyl, (C₁-C₆)-Alkoxycarbonyl, (C₃-C₇)-Cycloalkyl, Phenyl, welches seinerseits gegebenenfalls ein- bis zweifach durch (C₁-C₄)-Alkoxy substituiert ist, 5- bis 6-gliedriges Heterocyclyl mit bis zu zwei Heteroatomen aus der Reihe N, O und/oder S und 5- bis 6-gliedriges Heteroaryl mit bis zu zwei Heteroatomen aus der Reihe N, O und/oder S, welches seinerseits gegebenenfalls durch (C₁-C₄)-Alkyl oder Hydroxy-(C₁-C₄)-alkyl substituiert ist, substituiert sein kann,
für (C₆-C₁₀)-Aryl, das durch Hydroxy, (C₁-C₆)-Alkoxy, Amino, Mono- oder Di-(C₁-C₆)-alkylamino substituiert sein kann,
für 5- bis 6-gliedriges Heteroaryl mit bis zu drei Heteroatomen aus der Reihe N, O und/oder S, das durch (C₁-C₆)-Alkyl, Trifluormethyl, Halogen, Hydroxy, (C₁-C₆)-Alkoxy, Trifluormethoxy, Amino, Mono- oder Di-(C₁-C₆)-alkyl-amino substituiert sein kann,
für 5- bis 6-gliedriges Heterocyclyl mit bis zu zwei Heteroatomen aus der Reihe N, O und/oder S, das durch Benzyl oder bis zu vierfach durch (C₁-C₄)-Alkyl substituiert sein kann,
oder
für (C₄-C₈)-Cycloalkyl steht, das ein- bis zweifach, unabhängig voneinander, durch Hydroxy, Amino, (C₁-C₆)-Alkyl, (C₁-C₆)-Alkoxy, Mono- oder Di-(C₁-C₆)-alkyl-amino oder eine Gruppe der Formel R⁷-C(O)-NH- oder R⁷-SO₂-NH- substituiert sein kann,
worin
R⁷ für (C₁-C₆)-Alkoxy, Phenyl, Benzyl, Phenylamino, Mono- oder Di-(C₁-C₆)-alkylamino, welche ihrerseits gegebenenfalls in der Alkylgruppe durch (C₁-C₄)-Alkoxycarbonyl oder Carboxyl substituiert sind, oder für (C₄-C₇)-Cycloalkylamino, welches seinerseits gegebenenfalls in der Cycloalkylgruppe durch (C₁-C₄)-Allcyl substituiert ist,
für (C₁-C₆)-Alkyl, das durch Hydroxy, (C₁-C₆)-Alkoxy, Amino, Mono- oder Di-(C₁-C₆)-alkylamino, (C₁-C₆)-Acylamino oder durch 5-oder 6-gliedriges Heterocyclyl mit bis zu zwei Heteroatomen aus der Reihe N, O und/oder S substituiert sein kann,
für 5- oder 6-gliedriges, über ein Ringkohlenstoffatom oder über ein Ringstickstoffatom gebundenes Heterocyclyl mit bis zu zwei Heteroatomen aus der Reihe N, O und/oder S, das gegebenenfalls durch eine Oxo-Gruppe substituiert ist,
oder
für 5- oder 6-gliedriges Heteroaryl mit bis zu drei Heteroatomen aus der Reihe N, O und/oder S, das gegebenenfalls ein- bis zweifach durch (C₁-C₄)-Alkyl substituiert ist, steht,
oder
- R¹ und R²: zusammen mit dem Stickstoffatom, an das sie gebunden sind, einen 4- bis 11-gliedrigen, mono-, bi- oder spiro-cyclischen Heterocyclus bilden, der bis zu zwei weitere Heteroatome aus der Reihe N, O und/oder S enthalten und ein- bis vierfach, unabhängig voneinander, durch Substituenten ausgewählt aus der Gruppe Amino, Mono- oder Di-(C₁-C₆)-alkylamino, Hydroxy, (C₁-C₆)-Alkoxy, Oxo, Carboxyl, Carbamoyl, (C₁-C₆)-Alkoxycarbonyl, (C₁-C₆)-Alkoxycarbonylamino, (C₁-C₆)-Alkanoyl, (C₁-C₆)-Alkylcarbonylamino, (C₁-C₆)-Alkyl, welches seinerseits gegebenenfalls durch Hydroxy, (C₁-C₆)-Alkoxy, Amino, Mono- oder Di-(C₁-C₆)-alkylamino, Phenyl oder durch 5-oder 6-gliedriges Heterocyclyl mit bis zu zwei Heteroatomen aus der Reihe N, O und/oder S substituiert ist, Phenyl, welches seinerseits gegebenenfalls durch Halogen substituiert ist, (C₃-C₇)-Cycloalkyl, Pyridyl, Thienyl und 5-oder 6-gliedriges Heterocyclyl mit bis zu zwei Heteroatomen aus der Reihe N, O und/oder S substituiert sein kann,
sowie deren pharmazeutisch verträgliche Salze, Solvate, Hydrate und Hydrate der Salze.

Alkyl steht im Rahmen der Erfindung für einen geradkettigen oder verzweigten Alkylrest mit vorzugsweise 1 bis 6, 1 bis 4 bzw. 1 bis 2 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-, i-, s- oder t-Butyl, n-Pentyl und n-Hexyl.

Aryl steht im Rahmen der Erfindung für einen aromatischen Rest mit vorzugsweise 6 bis 10 Kohlenstoffatomen. Bevorzugte Arylreste sind Phenyl und Naphthyl.

Aryloxy steht im Rahmen der Erfindung für einen aromatischen Rest mit vorzugsweise 6 bis 10 Kohlenstoffatomen, der über ein Sauerstoffatom verknüpft ist. Bevorzugte Aryloxyreste sind Phenoxy und Naphthoxy.

Cycloalkyl steht im Rahmen der Erfindung für eine Cycloalkylgruppe mit vorzugsweise 3 bis 8, 4 bis 8, 3 bis 7 bzw. 3 bis 5 Kohlenstoffatomen. Beispielhaft und vorzugsweise seien genannt: Cyclopropyl, Cyclobutyl, Cyclopentyl, Cyclohexyl und Cycloheptyl.

Alkoxy steht im Rahmen der Erfindung vorzugsweise für einen geradkettigen oder verzweigten Alkoxyrest mit 1 bis 6, 1 bis 4 bzw. 1 bis 2 Kohlenstoffatomen. Bevorzugt ist ein geradkettiger oder verzweigter Alkoxyrest mit 1 bis 2 Kohlenstoffatomen. Beispielhaft und vorzugsweise seien genannt: Methoxy, Ethoxy, n-Propoxy, Isopropoxy, t-Butoxy, n-Pentoxy und n-Hexoxy.

Alkoxycarbonyl steht im Rahmen der Erfindung vorzugsweise für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen. Beispielhaft und vorzugsweise seien genannt: Methoxycarbonyl, Ethoxycarbonyl, n-Propoxycarbonyl, Isopropoxycarbonyl und t-Butoxycarbonyl.

Alkanoyl steht im Rahmen der Erfindung vorzugsweise für einen geradkettigen oder verzweigten Alkylrest mit 1 bis 6 bzw. 1 bis 4 Kohlenstoffatomen, der in der 1-Position ein doppelt gebundenes Sauerstoffatom trägt und über die 1-Position verknüpft ist. Bevorzugt ist ein geradkettiger oder verzweigter Alkanoyl-Rest mit 1 bis 4 Kohlenstoffatomen. Beispielhaft und vorzugsweise seien genannt: Formyl, Acetyl, Propionyl, n-Butyryl, i-Butyryl, Pivaloyl und n-Hexanoyl.

Alkanoyloxy steht im Rahmen der Erfindung vorzugsweise für einen geradkettigen oder verzweigten Alkylrest mit 1 bis 6, 1 bis 4 bzw. 1 bis 2 Kohlenstoffatomen, der in der 1-Position ein doppelt gebundenes Sauerstoffatom trägt und in der 1-Position über ein weiteres Sauerstoffatom verknüpft ist. Bevorzugt ist ein geradkettiger oder verzweigter Alkanoyloxy-Rest mit 1 bis 2 Kohlenstoffatomen. Beispielhaft und vorzugsweise seien genannt: Acetoxy, Propionoxy, n-Butyroxy, i-Butyroxy, Pivaloyloxy und n-Hexanoyloxy.

Monoalkylamino steht im Rahmen der Erfindung für eine Amino-Gruppe mit einem geradkettigen oder verzweigten Alkylsubstituenten, der vorzugsweise 1 bis 6, 1 bis 4 bzw. 1 bis 2 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, t-Butylamino, n-Pentylamino und n-Hexylamino.

Dialkylamino steht im Rahmen der Erfindung für eine Amino-Gruppe mit zwei gleichen oder verschiedenen geradkettigen oder verzweigten Alkylsubstituenten, die vorzugsweise jeweils 1 bis 6, 1 bis 4 bzw. 1 bis 2 Kohlenstoffatome aufweisen. Bevorzugt sind geradkettige oder verzweigte Dialkylamine-Reste mit jeweils 1 bis 4 Kohlenstoffatomen. Beispielhaft und vorzugsweise seien genannt: *N,N* Dimethylamino, *N*,*N*-Diethylamino, *N*-Ethyl-*N*-methylamino, *N*-Methyl-*N*-n-propylamino, *N*-Isopropyl-*N*-n-propylamino, *N*-t-Butyl*-N*-methylamino, *N*-Ethyl-*N*-n-pentylamino und *N*-n-Hexyl-*N*-methylamino.

Cycloalkylamino steht im Rahmen der Erfindung für eine Amino-Gruppe mit einem Cycloalkylsubstituenten, der vorzugsweise 4 bis 7, 4 bis 6 bzw. 5 bis 6 Kohlenstoffatome aufweist. Beispielhaft und vorzugsweise seien genannt: Cyclobutylamino, Cyclopentylamino, Cyclohexylamino und Cyloheptylamino.

Acylamino steht im Rahmen der Erfindung für eine Amino-Gruppe mit einem geradkettigen oder verzweigten Alkanoylsubstituenten, der vorzugsweise 1 bis 6, 1 bis 4 bzw. 1 bis 2 Kohlenstoffatome aufweist und über die Carbonylgruppe verknüpft ist. Bevorzugt ist ein Acylamino-Rest mit 1 bis 2 Kohlenstoffatomen. Beispielhaft und vorzugsweise seien genannt: Formamido, Acetamido, Propionamido, n-Butyramido und Pivaloylamido.

Alkylcarbonylamino steht im Rahmen der Erfindung für eine Amino-Gruppe mit einem geradkettigen oder verzweigten Alkylcarbonylsubstituenten (= Alkanoylsubstituent), der vorzugsweise im Alkylrest 1 bis 6 bzw. 1 bis 4 Kohlenstoffatome aufweist und über die Carbonylgruppe verknüpft ist. Bevorzugt ist ein Alkylcarbonylamino-Rest mit 1 bis 4 Kohlenstoffatomen. Beispielhaft und vorzugsweise seien genannt: Methylcarbonylamino, Ethylcarbonylamino, n-Propylcarbonylamino und t-Butylcarbonylamino.

Alkoxycarbonylamino steht im Rahmen der Erfindung für eine Amino-Gruppe mit einem geradkettigen oder verzweigten Alkoxycarbonylsubstituenten, der vorzugsweise im Alkoxyrest 1 bis 6 bzw. 1 bis 4 Kohlenstoffatome aufweist und über die Carbonylgruppe verknüpft ist. Bevorzugt ist ein Alkoxycarbonylamino-Rest mit 1 bis 4 Kohlenstoffatomen. Beispielhaft und vorzugsweise seien genannt: Methoxycarbonylamino, Ethoxycarbonylamino, n-Propoxycarbonylamino und t-Butoxycarbonylamino.

5- bis 6-gliedriges bzw. 5- bis 10-gliedriges Heteroaryl mit bis zu drei gleichen oder verschiedenen Heteroatomen aus der Reihe N, O und/oder S steht im Rahmen der Erfindung vorzugsweise für einen mono- bzw. bicyclischen aromatischen Heterocyclus, der über ein Ringkohlenstoffatom des Heteroaromaten oder gegebenenfalls über ein Ringstickstoffatom des Heteroaromaten verknüpft ist. Beispielhaft seien genannt: Furyl, Pyrrolyl, Thienyl, Thiazolyl, Oxazolyl, Imidazolyl, Triazolyl, Pyridyl, Pyrimidinyl, Pyridazinyl. Bevorzugt sind Pyridyl, Pyrimidinyl, Pyridazinyl, Furyl und Thiazolyl.

Ein 4- bis 7-gliedriger gesättigter oder teilweise ungesättigter Heterocyclus mit bis zu drei gleichen oder verschiedenen Heteroatomen aus der Reihe N, O und/oder S steht im Rahmen der Erfindung vorzugsweise für einen nicht-aromatischen Heterocyclus, der eine oder gegebenenfalls zwei Doppelbindungen enthalten kann und der über ein Ringkohlenstoffatom oder gegebenenfalls über ein Ringstickstoffatom verknüpft ist. Bevorzugt ist ein 5- bis 6-gliedriger gesättigter Heterocyclus mit bis zu zwei gleichen oder verschiedenen Heteroatomen aus der Reihe N, O und/oder S. Beispielhaft seien genannt: Tetrahydrofur-2-yl, Tetrahydrofur-3-yl, Pyrrolidin-1-yl, Pyrrolidin-2-yl, Pyrrolidin-3-yl, Pyrrolin-1-yl, Piperidin-1-yl, Piperidin-4-yl, 1,2-Dihydropyridin-1-yl, 1,4-Dihydropyridin-1-yl, Piperazin-1-yl, Morpholin-4-yl, Thiomorpholin-4-yl. Bevorzugt sind Piperidinyl, Piperazinyl, Morpholinyl und Pyrrolidinyl.

Halogen schließt im Rahmen der Erfindung Fluor, Chlor, Brom und Iod ein. Bevorzugt sind Fluor, Chlor oder Brom.

Die erfindungsgemäßen Verbindungen können in Abhängigkeit von dem Substitutionsmuster in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen in tautomeren Formen vorliegen. Dies ist dem Fachmann bekannt, und derartige Verbindungen sind ebenfalls vom Umfang der Erfindung umfasst.

Die erfindungsgemäßen Verbindungen können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbonoder Sulfonsäuren wie beispielsweise Essigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Physiologisch unbedenkliche Salze können ebenso Salze der erfindungsgemäßen Verbindungen mit Basen sein, wie beispielsweise Metall- oder Ammoniumsalze. Bevorzugte Beispiele sind Alkalimetallsalze (z.B. Natrium- oder Kaliumsalze), Erdalkalisalze (z.B. Magnesium- oder Calciumsalze), sowie Ammoniumsalze, die abgeleitet sind von Ammoniak oder organischen Aminen, wie beispielsweise Ethylamin, Di- bzw. Triethylamin, Ethyldiisopropylamin, Monoethanolamin, Di- bzw. Triethanolamin, Dicyclohexylamin, Dimethylaminoethanol, Dibenzylamin, N-Methylmorpholin, Dihydroabietylamin, 1-Ephenamin, Methylpiperidin, Arginin, Lysin, Ethylendiamin oder 2-Phenylethylamin.

Die erfindungsgemäßen Verbindungen können auch in Form ihrer Solvate, insbesondere in Form ihrer Hydrate vorliegen.

Außerdem umfasst die Erfindung auch Prodrugs der erfindungsgemäßen Verbindungen. Als "Prodrugs" werden erfindungsgemäß solche Derivate der Verbindungen der allgemeinen Formel (I) bezeichnet, welche selbst biologisch weniger aktiv oder auch inaktiv sein können, jedoch nach Applikation unter physiologischen Bedingungen in die entsprechende biologisch aktive Form überführt werden (beispielsweise metabolisch, solvolytisch oder auf andere Weise).

Bevorzugt sind Verbindungen der Formel (I), in welcher
- A: für Phenyl, das durch Fluor, Chlor, Brom oder Methoxy substituiert sein kann,
und
- G: für 1,3- oder 1,4-Phenylen, die ein- oder zweifach durch Methoxy substituiert sein können, oder für eine Gruppe der Formel 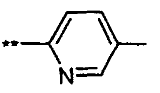 stehen,
worin ** die Anknüpfungsstelle zur Gruppe E bedeutet.

Bevorzugt sind gleichfalls Verbindungen der Formel (I), in welcher
- E: für eine Bindung, für eine Carbonylgruppe oder für eine Gruppe der Formel *-C(O)-NH- steht,
worin * die Anknüpfungsstelle zur Gruppe R³ bedeutet.

Bevorzugt sind gleichfalls Verbindungen der Formel (I), in welcher
- R¹: für Wasserstoff, Methyl oder Ethyl, welches durch Hydroxy substituiert sein kann,
und
- R²: für (C₁-C₄)-Alkyl, das ein- oder zweifach, unabhängig voneinander, durch Hydroxy, Amino, (C₁-C₄)-Alkoxy, Mono- oder Di-(C₁-C₄)-alkylamino substituiert sein kann, oder für (C₅-C₇)-Cycloalkyl steht,
oder
- R¹ und R²: zusammen mit dem Stickstoffatom, an das sie gebunden sind, einen Pyrrolidin-, Piperidin-, Piperazin- oder Morpholinring bilden, der ein- oder zweifach, unabhängig voneinander, durch (C₁-C₄)-Alkyl, welches seinerseits gegebenenfalls durch Hydroxy oder Amino substituiert ist, durch Amino, Mono- oder Di-(C₁-C₄)-alkylamino, Hydroxy, (C₁-C₄)-Alkoxy, Oxo, Carboxyl, Carbamoyl oder durch (C₁-C₄)-Alkoxycarbonyl substituiert sein kann.

Besonders bevorzugt sind Verbindungen der allgemeinen Formel (Ia) 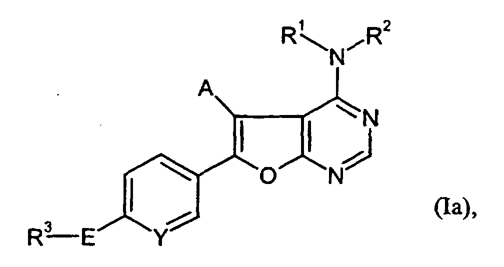 in welcher
- A: für Phenyl steht, das durch Fluor, Chlor, Brom oder Methoxy substituiert sein kann,
- Y: für CH oder N steht,
- E: für eine Bindung, für eine Carbonylgruppe oder für eine Gruppe der Formel *-C(O)-NH- steht,
worin * die Anknüpfungsstelle zur Gruppe R³ bedeutet,
- R³: für Hydroxy, Methoxy, Amino oder Mono-(C₁-C₄)-alkylamino, das in der Alkylgruppe durch Hydroxy oder Amino substituiert sein kann,
für (C₁-C₄)-Alkyl, das gegebenenfalls durch Hydroxy, Methoxy, Ethoxy, Amino, Mono- oder Dimethylarnino, (C₁-C₄)-Alkoxycarbonylamino, Acetamido, Carboxyl oder (C₁-C₄)-Alkoxycarbonyl substituiert ist,
oder
für einen 4- bis 6-gliedrigen gesättigten, über ein Ringkohlenstoffatom oder über ein Ringstickstoffatom gebundenen Heterocyclus mit bis zu zwei Heteroatomen aus der Reihe N und/oder O steht, der bis zu zweifach, unabhängig voneinander, durch (C₁-C₄)-Alkyl, welches seinerseits durch Hydroxy, Methoxy oder Ethoxy substituiert sein kann, durch Carboxyl, Amino, Hydroxy, Methoxy, Ethoxy oder eine Oxo-Gruppe substituiert sein kann,
- R¹: für Wasserstoff, Methyl oder für Ethyl, welches durch Hydroxy oder Amino substituiert sein kann, steht,
und
- R²: für (C₁-C₄)-Alkyl, das ein- oder zweifach, unabhängig voneinander, durch Hydroxy, Amino, (C₁-C₄)-Alkoxy, Mono- oder Di-(C₁-C₄)-alkylamino substituiert sein kann, oder für (C₅-C₇)-Cycloalkyl steht,
oder
- R¹ und R²: zusammen mit dem Stickstoffatom, an das sie gebunden sind, einen Pyrrolidin-, Piperidin-, Piperazin- oder Morpholinring bilden, der ein- oder zweifach, unabhängig voneinander, durch (C₁-C₄)-Alkyl, welches seinerseits gegebenenfalls durch Hydroxy oder Amino substituiert ist, durch Amino, Mono- oder Di-(C₁-C₄)-alkylamino, Hydroxy, (C₁-C₄)-Alkoxy, Oxo, Carboxyl, Carbamoyl oder durch (C₁-C₄)-Alkoxycarbonyl substituiert sein kann,
sowie deren pharmazeutisch verträgliche Salze, Solvate, Hydrate und Hydrate der Salze.

Ganz besonders bevorzugt sind Verbindungen der allgemeinen Formel (Ia),
in welcher
- A: für Phenyl steht, das durch Fluor substituiert sein kann,
- Y: für CH oder N steht,
- E: für eine Bindung oder für eine Gruppe der Formel *-C(O)-NH- steht,
worin * die Anknüpfungsstelle zur Gruppe R³ bedeutet,
- R³: für Amino oder Mono-(C₁-C₄)-alkylamino, das in der Alkylgruppe durch Hydroxy oder Amino substituiert sein kann,
für (C₁-C₄)-Alkyl, das gegebenenfalls durch Hydroxy, Amino, Mono- oder Dimethylamino substituiert ist,
oder
für Pyrrolidin, Piperazin oder Piperidin steht, die bis zu zweifach, unabhängig voneinander, durch Methyl, Ethyl, n-Propyl, iso-Propyl, n-Butyl, sec-Butyl, iso-Butyl oder tert-Butyl, welche ihrerseits durch Hydroxy substituiert sein können, durch Amino oder Hydroxy substituiert sein können,
- R¹ und R²: zusammen mit dem Stickstoffatom, an das sie gebunden sind, einen Piperidin-, Piperazin- oder Morpholinring bilden, der ein- oder zweifach, unabhängig voneinander, durch Methyl, Ethyl, n-Propyl oder iso-Propyl, welche ihrerseits gegebenenfalls durch Hydroxy oder Amino substituiert sind, durch Amino, Hydroxy oder Oxo substituiert sein können,
sowie deren pharmazeutisch verträgliche Salze, Solvate, Hydrate und Hydrate der Salze.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen angegebenen Restedefinitionen werden unabhängig von den jeweilig angegebenen Kombinationen der Reste beliebig auch durch Restedefinitionen anderer Kombinationen ersetzt.

Es wurde nun gefunden, dass Verbindungen der allgemeinen Formel (I) 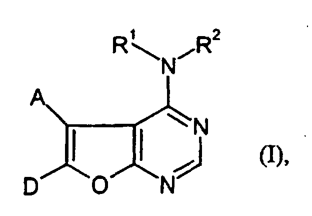 in welcher
- A: für Phenyl oder 5- oder 6-gliedriges Heteroaryl mit bis zu drei Heteroatomen aus der Reihe N, O und/oder S, die jeweils bis zu dreifach, unabhängig voneinander, durch Substituenten aus der Gruppe Halogen, Hydroxy, (C₁-C₆)-Alkoxy, Trifluormethyl, Trifluormethoxy, Amino, Carboxyl und (C₁-C₆)-Alkoxycarbonyl substituiert sein können,
oder für eine Gruppe der Formel 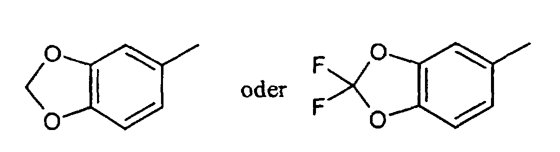 steht,
- D: für eine Gruppe der Formel

R³-E-G-

steht, worin
G Phenylen oder 5- oder 6-gliedriges Heteroarylen mit bis zu drei Heteroatomen aus der Reihe N, O und/oder S bedeutet, die jeweils bis zu zweifach, unabhängig voneinander, durch Substituenten aus der Gruppe Halogen, Trifluormethyl, Trifluormethoxy, (C₁-C₆)-Alkoxy, Amino, Nitro und Carboxyl substituiert sein können,
E für eine Bindung, eine Carbonylgruppe, eine Sulfonylgruppe oder für eine Gruppe der Formel *-C(O)-NR⁴- oder *-SO₂-NR⁴- steht,
worin * die Anknüpfungsstelle zur Gruppe R³ und R⁴ Wasserstoff oder (C₁-C₆)-Alkyl bedeutet, und
R³ für Halogen, Trifluormethyl, Hydroxy, (C₁-C₆)-Alkoxy, Trifluormethoxy, Nitro, Carboxyl oder eine Gruppe der Formel H-C(O)-NR⁴-,
worin R⁴ die oben angegebene Bedeutung hat,
fiir (C₁-C₆)-Alkyl, das ein- bis zweifach, unabhängig voneinander, durch Substituenten ausgewählt aus der Gruppe Halogen, Trifluormethyl, Hydroxy, (C₁-C₆)-Alkoxy, Amino, Mono- oder Di-(C₁-C₆)-alhylamino, (C₁-C₆)-Acylamino, (C₁-C₆)-Alkoxycarbonylamino, Amidino, Guanidino, Carboxyl, (C₁-C₆)-Alkoxycarbonyl, (C₆-C₁₀)-Aryl, (C₆-C₁₀)-Aryloxy und 5- bis 10-gliedriges Heteroaryl mit bis zu drei Heteroatomen aus der Reihe N, O und/oder S substituiert sein kann, wobei Aryl, Aryloxy und Heteroaryl ihrerseits jeweils ein- bis zweifach, unabhängig voneinander, durch Halogen, Hydroxy, Amino, (C₁-C₄)-Alkyl, (C₁-C₆)-Alkoxy, Cyano oder Nitro substituiert sein können,
für (C₃-C₇)-Cycloalkyl, das durch Phenyl oder bis zu vierfach durch (C₁-C₄)-Alkyl substituiert sein kann,
für (C₆-C₁₀)-Aryl, das ein- bis zweifach, unabhängig voneinander, durch Substituenten ausgewählt aus der Gruppe von Halogen, Trifluormethyl, (C₁-C₆)-Alkyl, Hydroxy, (C₁-C₆)-Alkoxy, (C₁-C₆)-Alkanoyl, Cyano, Nitro, Amino, Mono- und Di-(C₁-C₆)-alkylamino, (C₁-C₆)-Acylamino, Carboxyl, (C₁-C₆)-Alkoxycarbonyl und 5- bis 6-gliedriges Heteroaryl mit bis zu zwei Heteroatomen aus der Reihe N, O und/oder S substituiert sein kann,
für 4- bis 7-gliedriges, gesättigtes oder partiell ungesättigtes, über ein Ringkohlenstoffatom oder über ein Ringstickstoffatom gebundenes Heterocyclyl mit bis zu drei Heteroatomen aus der Reihe N, O und/oder S, das bis zu dreifach, unabhängig voneinander, durch (C₁-C₆)-Alkyl, welches seinerseits durch Hydroxy, (C₁-C₄)-Alkoxy oder Phenyl substituiert sein kann, (C₁-C₆)-Alkoxycarbonyl, Carboxyl, Amino, Hydroxy, (C₁-C₆)-Alkoxy oder eine Oxo-Gruppe substituiert sein kann,
für 5- bis 10-gliedriges, über ein Ringkohlenstoffatom oder über ein Ringstickstoffatom gebundenes Heteroaryl mit bis zu drei Heteroatomen aus der Reihe N, O und/oder S, das seinerseits gegebenenfalls ein- bis zweifach, unabhängig voneinander, durch Substituenten ausgewählt aus der Gruppe von Halogen, Nitro, Amino, Hydroxy, (C₁-C₆)-Alkyl, (C₃-C₆)-Cycloalkyl, Phenyl, Benzyl und 5-gliedriges Heteroaryl mit bis zu zwei Heteroatomen aus der Reihe N, O und/oder S substituiert ist,
oder
für eine Gruppe der Formel -NR⁵R⁶ steht,
worin
R⁵ und R⁶ unabhängig voneinander für Wasserstoff, (C₁-C₆)-Alkyl, welches durch Hydroxy, Amino, (C₁-C₄)-Alkoxy, Mono- oder Di-(C₁-C₄)-alkylamino, oder Phenyl substituiert sein kann, für (C₃-C₇)-Cycloalkyl, welches ein- bis zweifach, unabhängig voneinander, durch Hydroxy, Amino, (C₁-C₄)-Alkoxy, Mono- oder Di-(C₁-C₄)-alkylamino oder (C₁-C₄)-Alkyl substituiert sein kann, für (C₆-C₁₀)-Aryl, welches ein- bis zweifach, unabhängig voneinander, durch Hydroxy, Amino, (C₁-C₄)-Alkoxy oder Nitro substituiert sein kann, oder für 5- bis 6-gliedriges Heterocyclyl oder 5- bis 6-gliedriges Heteroaryl mit jeweils bis zu zwei Heteroatomen aus der Reihe N, O und/oder S stehen,
oder
- D: für eine Gruppe der Formel 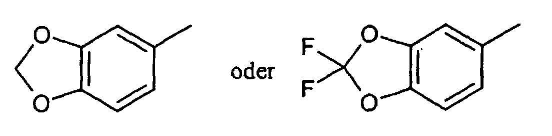 steht,
- R¹: für Wasserstoff, (C₃-C₆)-Cycloalkyl oder für (C₁-C₆)-Alkyl steht, das ein- bis zweifach, unabhängig voneinander, durch Hydroxy, (C₁-C₆)-Alkoxy, Amino, Mono- oder Di-(C₁-C₆)-alkylamino substituiert sein kann,
und
- R²: für (C₁-C₆)-Alkyl, das ein- bis zweifach, unabhängig voneinander, durch Substituenten ausgewählt aus der Gruppe von Trifluormethyl, Hydroxy, (C₁-C₆)-Alkoxy, Amino, Mono- oder Di-(C₁-C₆)-alkylamino, Phenylamino, Carboxyl, (C₁-C₆)-Alkoxycarbonyl, (C₃-C₇)-Cycloalkyl, Phenyl, welches seinerseits gegebenenfalls ein- bis zweifach durch (C₁-C₄)-Alkoxy substituiert ist, 5- bis 6-gliedriges Heterocyclyl mit bis zu zwei Heteroatomen aus der Reihe N, O und/oder S und 5- bis 6-gliedriges Heteroaryl mit bis zu zwei Heteroatomen aus der Reihe N, O und/oder S, welches seinerseits gegebenenfalls durch (C₁-C₄)-Alkyl oder Hydroxy-(C₁-C₄)-alkyl substituiert ist, substituiert sein kann,
für (C₆-C₁₀)-Aryl, das durch Hydroxy, (C₁-C₆)-Alkoxy, Amino, Mono- oder Di-(C₁-C₆)-alkylamino substituiert sein kann,
für 5- bis 6-gliedriges Heteroaryl mit bis zu drei Heteroatomen aus der Reihe N, O und/oder S, das durch (C₁-C₆)-Alkyl, Trifluormethyl, Halogen, Hydroxy, (C₁-C₆)-Alkoxy, Trifluormethoxy, Amino, Mono- oder Di-(C₁-C₆)-alkyl-amino substituiert sein kann,
für 5- bis 6-gliedriges Heterocyclyl mit bis zu zwei Heteroatomen aus der Reihe N, O und/oder S, das durch Benzyl oder bis zu vierfach durch (C₁-C₄)-Alkyl substituiert sein kann,
oder
für (C₄-C₈)-Cycloalkyl steht, das ein- bis zweifach, unabhängig voneinander, durch Hydroxy, Amino, (C₁-C₆)-Alkyl, (C₁-C₆)-Alkoxy, Mono- oder Di-(C₁-C₆)-alkyl-amino oder eine Gruppe der Formel R⁷-C(O)-NH- oder R⁷-SO₂-NH- substituiert sein kann,
worin
R⁷ für (C₁-C₆)-Alkoxy, Phenyl, Benzyl, Phenylamino, Mono- oder Di-(C₁-C₆)-alkylamino, welche ihrerseits gegebenenfalls in der Alkylgruppe durch (C₁-C₄)-Alkoxycarbonyl oder Carboxyl substituiert sind, oder für (C₄-C₇)-Cycloalkylamino, welches seinerseits gegebenenfalls in der Cycloalkylgruppe durch (C₁-C₄)-Alkyl substituiert ist,
für (C₁-C₆)-Alkyl, das durch Hydroxy, (C₁-C₆)-Alkoxy, Amino, Mono- oder Di-(C₁-C₆)-alkylamino, (C₁-C₆)-Acylamino oder durch 5- oder 6-gliedriges Heterocyclyl mit bis zu zwei Heteroatomen aus der Reihe N, O und/oder S substituiert sein kann,
für 5- oder 6-gliedriges, über ein Ringkohlenstoffatom oder über ein Ringstickstoffatom gebundenes Heterocyclyl mit bis zu zwei Heteroatomen aus der Reihe N, O und/oder S, das gegebenenfalls durch eine Oxo-Gruppe substituiert ist,
oder
für 5- oder 6-gliedriges Heteroaryl mit bis zu drei Heteroatomen aus der Reihe N, O und/oder S, das gegebenenfalls ein- bis zweifach durch (C₁-C₄)-Alkyl substituiert ist, steht,
oder
- R¹ und R²: zusammen mit dem Stickstoffatom, an das sie gebunden sind, einen 4- bis 11-gliedrigen, mono-, bi- oder spiro-cyclischen Heterocyclus bilden, der bis zu zwei weitere Heteroatome aus der Reihe N, O und/oder S enthalten und ein- bis vierfach, unabhängig voneinander, durch Substituenten ausgewählt aus der Gruppe Amino, Mono- oder Di-(C₁-C₆)-alkylamino, Hydroxy, (C₁-C₆)-Alkoxy, Oxo, Carboxyl, Carbamoyl, (C₁-C₆)-Allcoxycarbonyl, (C₁-C₆)-Alkyl, welches seinerseits gegebenenfalls durch Hydroxy, (C₁-C₆)-Alkoxy, Amino, Mono- oder Di-(C₁-C₆)-alkylamino oder durch 5- oder 6-gliedriges Heterocyclyl mit bis zu zwei Heteroatomen aus der Reihe N, O und/oder S substituiert ist, Phenyl, welches seinerseits gegebenenfalls durch Halogen substituiert ist, (C₃-C₇)-Cycloalkyl, Pyridyl und 5- oder 6-gliedriges Heterocyclyl mit bis zu zwei Heteroatomen aus der Reihe N, O und/oder S substituiert sein kann,
sowie deren pharmazeutisch verträgliche Salze, Solvate, Hydrate und Hydrate der Salze,
eine pharmakologische Wirkung zeigen und als Arzneimittel oder zur Herstellung von Arzneimittel-Formulierungen verwendet werden können.

Gegenstand der vorliegenden Erfindung ist auch ein Verfahren zur Herstellung der Verbindungen der Formel (I), dadurch gekennzeichnet, dass man Verbindungen der Formel (II) 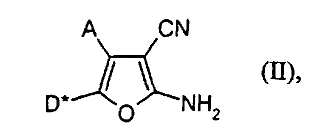 worin D* für D oder für einen unsubstituierten Phenylring steht und A und D die oben angegebene Bedeutung haben,
entweder
[A] zunächst mit Ameisensäure in Acetanhydrid zu Verbindungen der Formel (III) 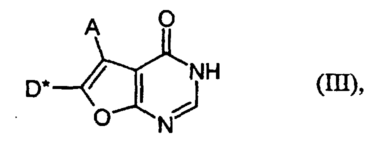 worin D* für D oder für einen unsubstituierten Phenylring steht und A und D die oben angegebene Bedeutung haben,
   umsetzt, dann mit Phosphorylchlorid in Verbindungen der Formel (IV) 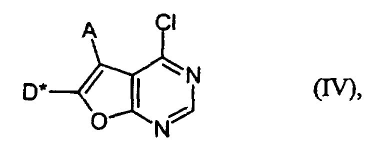 worin D* für D oder für einen unsubstituierten Phenylring steht und A und D die oben angegebene Bedeutung haben, überführt, anschließend für den Fall, dass D* für einen unsubstituierten Phenylring steht, diesen Phenylring nitriert, und schließlich mit Verbindungen der Formel (V) 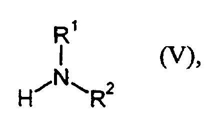 worin R¹ und R² die oben angegebene Bedeutung haben,
   zu Verbindungen der Formel (I) umsetzt
   oder
[B] zunächst mit Orthoameisensäuretriethylester in Verbindungen der Formel (VI) 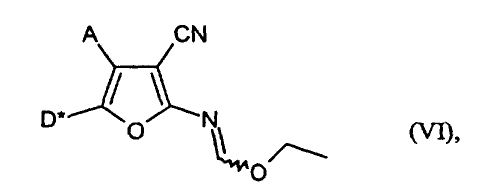 worin D* für D oder für einen unsubstituierten Phenylring steht und A und D die oben angegebene Bedeutung haben,
   überführt und dann mit Verbindungen der Formel (VII) 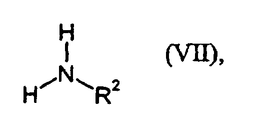 worin R² die oben angegebene Bedeutung hat,
   und anschließend mit einer Base direkt oder für den Fall, dass D* für einen unsubstituierten Phenylring steht, durch anschließende Nitrierung dieses Phenylringes, zu Verbindungen der Formel (I) umsetzt,
wobei sich bei den so erhaltenen Verbindungen der Formel (I) gegebenenfalls weitere Derivatisierungen, die nach üblichen Methoden durchgeführt werden können, anschließen können.

Das erfindungsgemäße Verfahren kann durch folgende Formelschemata beispielhaft erläutert werden 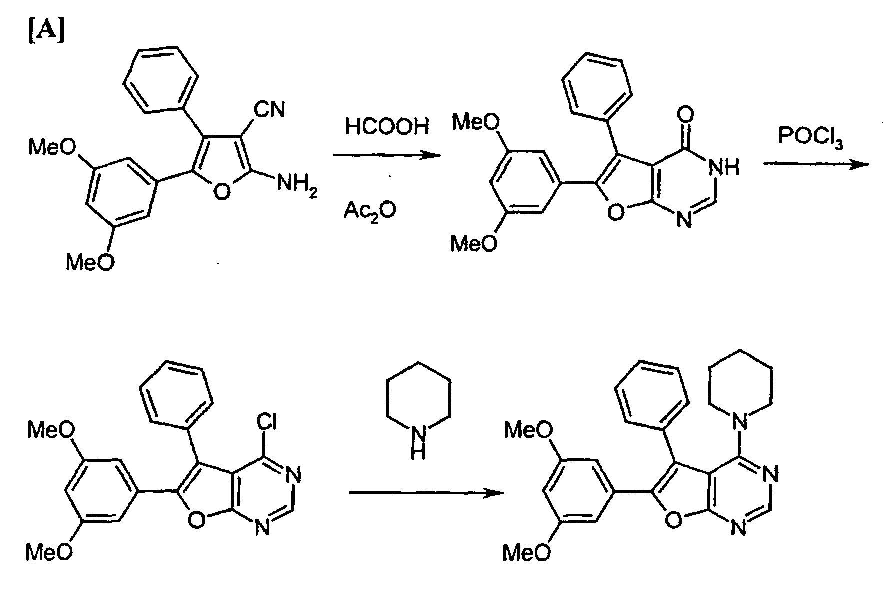 für den Fall, dass D* für einen unsubstituierten Phenylring steht: 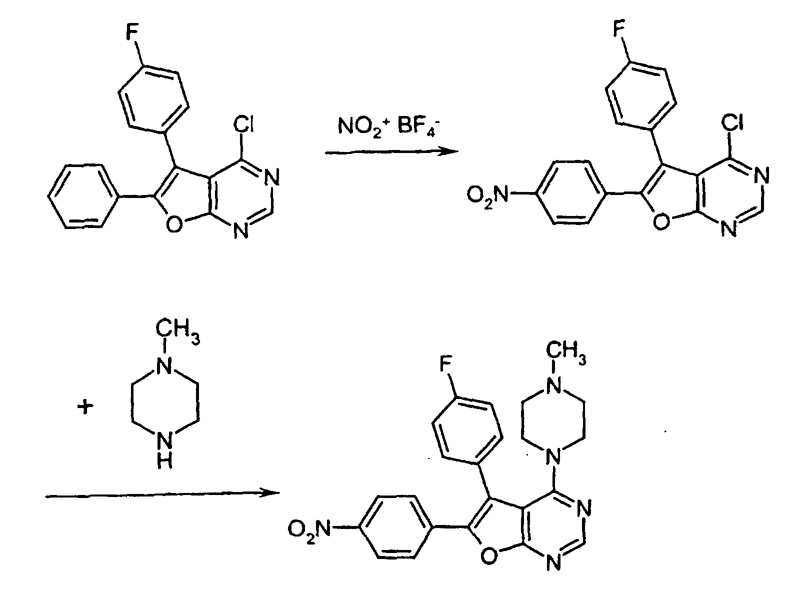 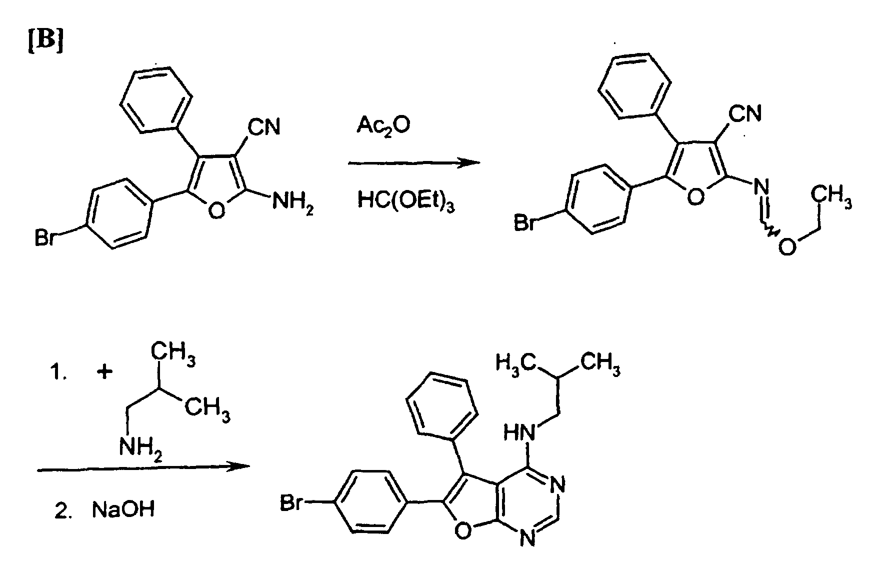 für den Fall, dass D* für einen unsubstituierten Phenylring steht:

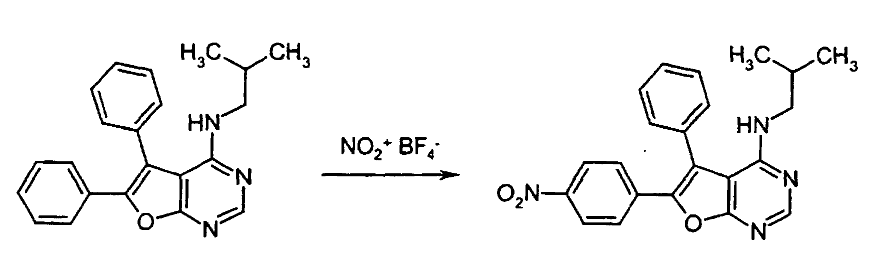

Als Lösungsmittel für das zuvor beschriebene Verfahren eignen sich hierbei organische Lösungsmittel, die unter den Reaktionsbedingungen inert sind, oder Wasser. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Ether wie Diethylether, Dioxan, Tetrahydrofuran, Glykoldimethylether oder Diethylenglykoldimethylether, Alkohole wie Methanol, Ethanol, Propanol, Isopropanol oder Butanol, Kohlenwasserstoffe wie Benzol, Xylol, Toluol, Hexan oder Cyclohexan, oder sonstige Lösungsmittel wie Dimethylformamid, Dimethylsulfoxid, N-Methylpyrrolidon, Acetonitril oder Pyridin oder deren Mischungen.

Die Reaktionen erfolgen im Allgemeinen in einem Temperaturbereich von -78°C bis 150°C.

Die Umsetzungen können bei normalem, erhöhtem oder erniedrigtem Druck durchgeführt werden (z.B. im Bereich von 0,5 bis 5 bar). Im Allgemeinen arbeitet man bei Normaldruck.

Als Basen eignen sich die üblichen anorganischen oder organischen Basen. Hierzu gehören bevorzugt Alkali- und Erdalkalihydroxide wie beispielsweise Lithium, Natrium- oder Kaliumhydroxid oder Alkali- und Erdalkalicarbonate wie Natriumoder Kaliumcarbonat oder Natrium- oder Kaliummethanolat oder Natrium- oder Kaliumethanolat oder Kalium-tert.-butylat oder Amide wie Natriumamid, Lithiumbis-(trimethylsilyl)amid oder Lithiumdiisopropylamid oder Amine wie Triethylamin, Diisopropylethylamin, Diisopropylamin, N-Methylmorpholin, 4-Dimethylaminopyridin oder Pyridin.

Der Reaktionsschritt [A] (II) -> (III) erfolgt vorzugsweise in einem Gemisch von Ameisensäure und Acetanhydrid im Verhältnis 2:1 (v:v) als Lösungsmittel. Der Temperaturbereich für diese Reaktion liegt vorzugsweise zwischen 50°C und 100°C, insbesondere bei Rückflusstemperatur.

Der Reaktionsschritt [A] (III) -> (IV) erfolgt vorzugsweise in einem Überschuss Phosphorylchlorid, beispielsweise 20- bis 50-fach, als Lösungsmittel. Der Temperaturbereich für diese Reaktion liegt vorzugsweise zwischen 80°C und 120°C, insbesondere bei Rückflusstemperatur.

Der Reaktionsschritt [A] (IV) + (V) -> (I) erfolgt vorzugsweise in Substanz oder in Ethanol als Lösungsmittel mit einem Überschuss, beispielsweise 4-fach, des Amins (V). Der Temperaturbereich für diese Reaktion liegt vorzugsweise zwischen 60°C und 90°C, insbesondere bei Rückflusstemperatur.

Die für den Fall, dass D* für einen unsubstituierten Phenylring steht, stattfindende Nitrierung des Phenylringes in Verbindungen der Formel (IV) oder den Reaktionsprodukten der Umsetzung (VI) + (VII) erfolgt vorzugsweise in Acetonitril oder Dichlormethan als Lösungsmittel. Der Temperaturbereich für diese Reaktion liegt vorzugsweise zwischen 0°C und 30°C, insbesondere bei 5°C. Als Nitrierungsreagenz wird bevorzugt Nitroniumtetrafluorborat verwendet.

Der Reaktionsschritt [B] (II) -> (VI) erfolgt vorzugsweise in einem Überschuss Orthoameisensäureethylester als Lösungsmittel in Gegenwart von Essigsäureanhydrid als Kondensationsmittel. Der Temperaturbereich für diese Reaktion liegt vorzugsweise zwischen 100°C und 150°C, insbesondere bei Rückflusstemperatur.

Der Reaktionsschritt [B] (VI) + (VII) -> (I) erfolgt vorzugsweise in Substanz oder in Ethanol als Lösungsmittel mit einem Überschuss, beispielsweise 4-fach, des Amins (V). Der Temperaturbereich für diese Reaktion liegt vorzugsweise zwischen 20°C und 60°C, insbesondere bei 40°C. Als Base im zweiten Teilschritt dieser Reaktion wird vorzugsweise wässrige Natronlauge verwendet bei einer Temperatur vorzugsweise zwischen 60°C und 120°C, insbesondere bei 90°C.

Die Verbindungen der Formel (II) sind teilweise literaturbekannt und können beispielsweise hergestellt werden,
indem man Verbindungen der Formel (VIII)

A-CHO (VIII),

worin
- A: die oben angegebene Bedeutung hat,
mit Triethylphosphit und Chlortrimethylsilan in Verbindungen der Formel (IX) 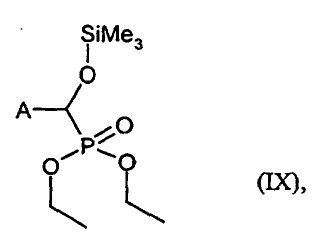 worin
- A: die oben angegebene Bedeutung hat,
überführt, anschließend in Gegenwart einer Base mit Verbindungen der Formel (X)

D*-CHO (X),

worin
D* für D oder für einen unsubstituierten Phenylring steht und D die oben angegebene Bedeutung hat,
zu Verbindungen der Formel (XI) 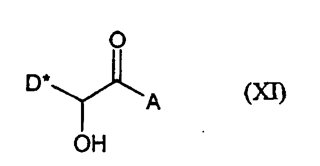 worin D* für D oder für einen unsubstituierten Phenylring steht und A und D die oben angegebene Bedeutung haben,
umsetzt, die dann in Gegenwart einer Base mit Malonsäuredinitril in Verbindungen der Formel (II) überführt werden.

Das folgende Reaktionsschema verdeutlicht die Reaktionssequenz:

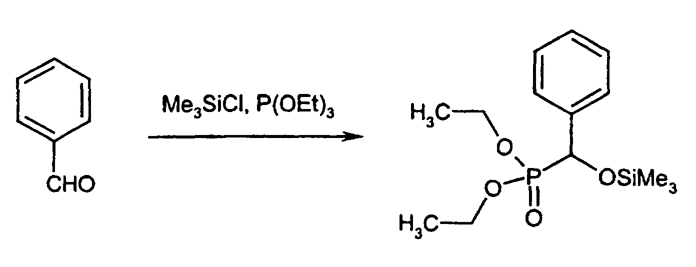

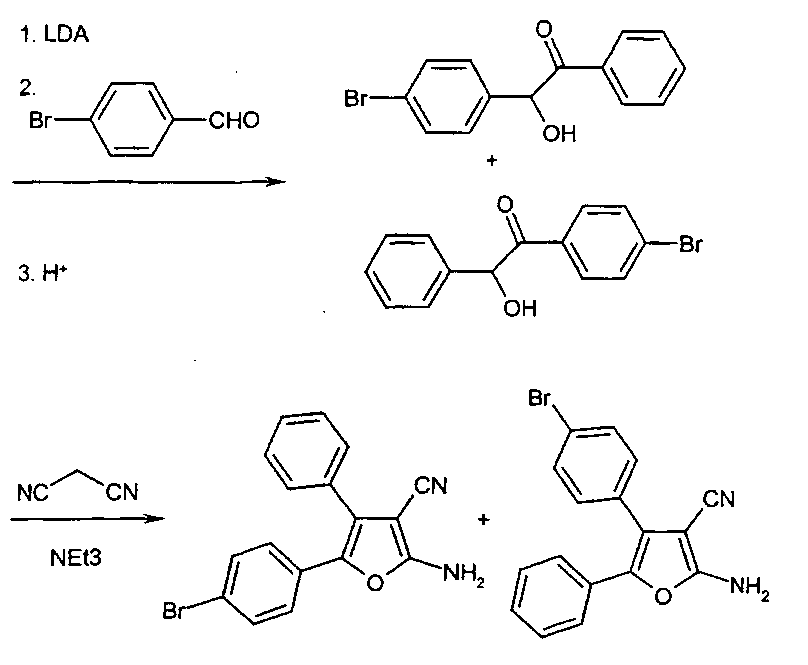

Die Verbindungen der Formel (XI) fallen in der Regel als Isomerengemisch an. Die Trennung entstandener Isomere kann in Abhängigkeit der Substituenten auch auf verschiedenen weiteren Stufen der Synthese erfolgen, da für die folgenden Umsetzungen auch das Isomerengemisch eingesetzt werden kann.

Der Reaktionsschritt (VIII) -> (IX) erfolgt vorzugsweise in Substanz ohne weiteres Lösungsmittel. Der Temperaturbereich für diese Reaktion liegt vorzugsweise zwischen 60°C und 120°C, insbesondere bei 80°C.

Der Reaktionsschritt (IX) + (X) -> (XI) erfolgt vorzugsweise in Tetrahydrofuran als Lösungsmittel. Der Temperaturbereich für diese Reaktion liegt vorzugsweise zwischen -78°C und Raumtemperatur, insbesondere bei -70°C. Als Base wird bevorzugt Lithiumdiisopropylamid eingesetzt.

Der Reaktionsschritt (XI) -> (II) erfolgt vorzugsweise in Dimethylformamid als Lösungsmittel. Der Temperaturbereich für diese Reaktion liegt vorzugsweise zwischen 0°C und 40°C, insbesondere bei Raumtemperatur. Als Base wird bevorzugt Triethylamin eingesetzt.

Die Verbindungen der Formel (V), (VII), (VIII) und (X) sind käuflich erhältlich, literaturbekannt oder nach üblichen Methoden herstellbar.

Die Verbindungen der Formel (I) zeigen ein überraschendes und wertvolles pharmakologisches Wirkungsspektrum und lassen sich daher als vielseitige Medikamente einsetzen.

Die pharmazeutische Wirksamkeit der Verbindungen der Formel (I) lässt sich durch ihre Wirkung als Adenosinkinase-Inhibitoren erklären.

Die Verbindungen der Formel (I) sind allein oder in Kombination mit einem oder mehreren anderen Wirkstoffen zur Prävention und/oder Behandlung inbesondere von ischämiebedingten peripheren und kardiovaskulären Erkrankungen geeignet. Geeignete Kombinationswirkstoffe sind insbesondere Arzneimitteln, die zur Standardtherapie der koronaren Herzkrankheit gehören, wie beispielsweise Calciumkanalblocker, Nitrovasodilatatoren, beta-Rezeptorenblocker, Thrombozytenaggregatioshemmer, Thrombolytika (Fibrinolytika), Antikoagulantien, ACE-Hemmer, Glykoprotein IIb/IIIa-Rezeptor-Antagonisten, Antiarrhythmika, beta-Adrenerge Agonisten oder Nukleosid-Transporter-Inhibitoren.

Im Sinne der vorliegenden Erfindung sind unter ischämiebedingten peripheren und kardiovaskulären Erkrankungen beispielsweise insbesondere die akute und chronische Behandlung von ischämischen Erkrankungen des Herz-Kreislauf-Systems, wie z.B. der koronaren Herzkrankheit, der stabilen und instabilen Angina pectoris, von peripheren und arteriellen Verschlusskrankheiten, von thrombotischen Gefäßverschlüssen, des Myocardinfarkts und von Reperfusionsschäden zu verstehen.

Darüber hinaus können die Verbindungen der Formel (I) beispielsweise insbesondere zur Prävention und/oder Behandlung von cerebraler Ischämie, Hirnschlag, Reperfusionsschäden, Hirntrauma, Ödemen, Krämpfen, Epilepsie, Atemstillstand, Herzstillstand, Reye-Syndrom, cerebraler Thrombose, Embolie, Tumoren, Blutungen, Encephalomyelitis, Hydroencephalitis, Rückenmarksverletzungen, post-operativen Himschäden, Verletzungen der Retina oder des optischen Nervs nach Glaukom, Ischämie, Hypoxie, Ödem oder Trauma sowie in der Behandlung von Schizophrenie, Schlafstörungen und akuten und/oder chronischen Schmerzen sowie neurodegenerativen Erkrankungen, insbesondere zur Prävention und/oder Behandlung von Krebs-induzierten Schmerzen und chronischen neuropathischen Schmerzen, wie zum Beispiel bei diabetischer Neuropathie, posttherapeutischer Neuralgie, peripheren Nervenbeschädigungen, zentralem Schmerz (beispielsweise als Folge von cerebraler Ischämie) und trigeminaler Neuralgie und anderen chronischen Schmerzen, wie zum Beispiel Lumbago, Rückenschmerz (lower back pain) oder rheumatischen Schmerzen, eingesetzt werden.

Des weiteren eignen sich die Verbindungen der Formel (I) beispielsweise insbesondere zur Prävention und/oder Behandlung von Bluthochdruck und Herzinsuffizienz, Myocarditis, Nephritis, Pancreatitis und diabetischer Nephropathie.

Die vorliegende Erfindung betrifft auch die Verwendung der Verbindungen der Formel (I) zur Herstellung von Arzneimitteln zur Prophylaxe und/oder Behandlung der zuvor genannten Krankheitsbilder.

Die vorliegende Erfindung betrifft weiterhin ein Verfahren zur Prophylaxe und/oder Behandlung der zuvor genannten Krankheitsbilder mit den Verbindungen der Formel (I).

Weiterer Gegenstand der vorliegenden Erfindung sind Arzneimittel, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Für die Applikation der Verbindungen der allgemeinen Formel (I) kommen alle üblichen Applikationsformen in Betracht, d.h. also oral, parenteral, inhalativ, nasal, sublingual, buccal, rektal, lokal wie beispielsweise bei Implantaten oder Stents, oder äußerlich wie z.B. transdermal, insbesondere bevorzugt oral oder parenteral. Bei der parenteralen Applikation sind insbesondere intravenöse, intramuskuläre, subkutane Applikation zu nennen, z.B. als subkutanes Depot. Bevorzugt ist die orale oder parenterale Applikation. Besonders bevorzugt ist die orale Applikation.

Hierbei können die Wirkstoffe allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen. Hierbei muss der Wirkstoff in einer solchen Menge vorliegen, dass eine therapeutische Wirkung erzielt wird. Im Allgemeinen kann der Wirkstoff in einer Konzentration von 0,1 bis 100 Gew.-%, insbesondere 0,5 bis 90 Gew.-%, vorzugsweise 5 bis 80 Gew.-%, vorliegen. Insbesondere sollte die Konzentration des Wirkstoffs 0,5 bis 90 Gew.-% betragen, d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin), Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten selbstverständlich auch Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserern oder Farbstoffen versetzt werden.

Bei oraler Applikation werden vorzugsweise Dosierungen von 0,001 bis 5 mg/kg, vorzugsweise 0,001 bis 3 mg/kg Körpergewicht je 24 Stunden appliziert.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben der nachfolgenden Beispiele beziehen sich, sofern nicht anders angegeben, jeweils auf das Gewicht; Teile sind Gewichtsteile.

### A. Bewertung der physiologischen Wirksamkeit

Die Wirksamkeit der erfindungsgemäßen Verbindungen lässt sich beispielsweise durch die im folgenden beschriebenen biologischen Tests prüfen:

### 1. Inbibition von humaner rekombinanter Adenosin-Kinase

Der Test wird auf 96er-Filterplatten (DE81 der Fa. Millipore, DEAE-Cellulose, 0,65 µm) durchgeführt. Der Testpuffer enthält 50 mM Tris/HCl pH 8,0, 10 mM DTT und 1 mM MgCl₂; unmittelbar vor Testbeginn wird ATP in einer Konzentration von 0,5 mM zugegeben. Als Substrat dient ¹⁴C-Adenosin, 1:30 verdünnt in kaltem Adenosin; die Endkonzentration des kalten Adenosin beträgt 4x10⁻⁶ M; die Menge an ¹⁴C-Adenosin wird so gewählt, dass bei einem Volumen von 10 µl pro Vertiefung der Filterplatte eine Aktivität von 0,5 kBq (= 30.000 Zerfälle pro Minute) vorliegt. Die zu testenden Substanzen werden in einer Konzentration von 10⁻² M in DMSO gelöst. Weitere Verdünnungen erfolgen in Wasser.

Bei der Testdurchführung werden zunächst 80 µl Puffer je Vertiefung vorgelegt. Nacheinander werden, jeweils in einem Volumen von 10 µl, die Testsubstanzen, das Substrat Adenosin und schließlich humane rekombinante Adenosinkinase (Mathews J. J. et al., Biochemistry (1998) 37, 15607-15620) in Testpuffer pH 7,4 zugegeben. Die Adenosinkinase-Konzentration wird so gewählt, dass maximal 30 % der Gesamtmenge des Adenosins umgesetzt werden. Die Platten werden nun 20 min. bei 37°C inkubiert. Anschließend wird die Reaktion durch Absaugen gestoppt. Die Filter werden je einmal mit eiskaltem Waschpuffer (15 mM Tris/HCl pH 8,5) und mit Ethanol gewaschen. Die Platte wird bei 37°C getrocknet (etwa 15 min.). Dann werden pro Vertiefung 50 µl Szintillations-Lösung pipettiert und die ¹⁴C-Aktivität im MicroBeta-Counter ermittelt.

Die Inhibition der Enzymaktivität durch die getesteten Substanzen wird nach Abzug des Leerwertes (keine Enzymzugabe) im Vergleich mit dem ungehemmten Enzym (100 %-Wert) ermittelt.

Die Ergebnisse des Tests sind als IC₅₀-Werte für die Inhibition der Adenosin-Kinase angegeben und in Tabelle 1 aufgeführt:

**Tabelle 1:**

| Adenosin-Kinase -Inhibition (in vitro): | |
|---|---|
| **Beispiel** | **IC**_{**50**} **[nM]** |
| 4 | 50 |
| 6 | 20 |
| 8 | 80 |
| 10 | 10 |
| 13 | 100 |
| 24 | 30 |
| 29 | 80 |
| 32 | 30 |
| 35 | 20 |
| 37 | 20 |
| 193 | 30 |
| 198 | 20 |
| 201 | 20 |
| 255 | 5 |
| 270 | 30 |
| 326 | 15 |

### 2. In vivo-Testmodell:

Erwachsene FBI (Foxhound-Beagle-Irish-Setter)-Hunde (20-30 kg Körpergewicht) werden initial mit einer Kombination von Trapanal 500 mg und Alloferin 55 mg narkotisiert. Die Narkose wird durch Infusion eines Gemisches von Fentanyl 0,072 mg/kg, Alloferin 0,02 mg/kg und Dihydrobenzpyridyl 0,25 mg/kg x min erhalten. Die Tiere werden intubiert und mit einem Gemisch aus O₂/N₂O (Verhältnis 1:5) mit einer Engström-Atempumpe mit 16 Atemzügen pro min und einem Volumen von 18-24 ml/kg beatmet. Die Körpertemperatur wird bei 38°C ± 0,1°C gehalten. Der arterielle Blutdruck wird über einen Katheder in der Femoralarterie gemessen. Es wird eine Thorakotomie auf der linken Seite am fünften Intercostalraum durchgeführt. Die Lunge wird zurückgelegt, fixiert und das Pericard eingeschnitten. Ein proximaler Abschnitt der LAD (left artery descendent) distal zur ersten diagonalen Verzweigung wird freipräpariert und ein kalibrierter elektromagnetischer Flussmesskopf (Fa. Gould Statham, Modell SP7515) um das Gefäß gelegt und mit einem Flussmessgerät (Fa. Statham, Modell SP-2202) verbunden. Ein mechanischer Okkluder wird distal zum Flussmesskopf so angebracht, dass keine Verzweigungen zwischen Flussmesskopf und Okkluder liegen.

Blutentnahmen und Substanzgaben werden durch einen Katheder in der Femoralvene durchgeführt. Ein peripheres EKG wird mit subcutan verankerten Nadeln abgeleitet. Ein Mikrotip-Druckmanometer (Fa. Millar, Modell PC-350) wird durch den linken Vorhof geschoben, um den linksventrikulären Druck zu messen. Die Messung der Herzfrequenz wird über die R-Zacke des EKGs getriggert. Die hämodynamischen Parameter und der Koronarfluss werden während des gesamten Versuchs über einen Vielfachschreiber aufgezeichnet.

Eine Okklusion von vier Minuten verursacht eine reaktive Hyperämie. Man misst die Differenz zwischen dem Koronarfluss unter Kontrollbedingungen und dem Maximalfluss während der reaktiven Hyperämie. Die Zeit, die benötigt wird, um die Hälfte dieses Maximalflusses im Abfall zu erreichen, ist ein geeigneter Parameter, um die reaktive Hyperämie zu beurteilen.

Nach einer Stabilisierungszeit von einer Stunde wird das Experiment mit einer vierminütigen Okklusion begonnen. Dreißig Minuten später wird die Substanz gegeben (i.v.) und zwei Minuten später erneut okkludiert. Die reaktive Hyperämie nach Verum und Placebo wird verglichen.

### B. Ausführungsbeispiele

Die vorliegende Erfindung wird an den folgenden bevorzugten Beispielen veranschaulicht, die die Erfindung jedoch keinesfalls beschränken.

| **Verwendete Abkürzungen:** | |
|---|---|
| DCI | direkte chemische Ionisation (bei MS) |
| DMAP | *4-N,N-*Dimethylaminopyridin |
| DMF | *N,N*-Dimethylformamid |
| DMSO | Dimethylsulfoxid |
| EDC | *N'*-(3-Dimethylaminopropyl)-*N*-ethylcarbodiimid x HCl |
| EI | Elektronenstoß-Ionisation (bei MS) |
| ESI | Elektronenspray-Ionisation (bei MS) |
| HOBt | 1-Hydroxy-1H-benzotriazol x H₂O |
| HPLC | Hochdruck-, Hochleistungsflüssigkeitschromatographie |
| MPLC | Mitteldrukflüssigkeitschromatographie |
| MS | Massenspektroskopie |
| NMR | Kernresonanzspektroskopie |
| RP | reverse phase (bei HPLC) |
| RT | Raumtemperatur |
| TFA | Trifluoressigsäure |
| THF | Tetrahydrofuran |

### Ausgangsverbindungen:

### Beispiel I

### Phenyl-[trimethylsilyl)oxy]-methylphosphonsäurediethylester

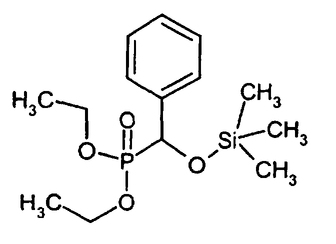

In eine Mischung von 100 g (601,84 mmol) Triethylphosphit und 71,92 g (662,03 mmol) Chlortrimethylsilan werden 70,26 g (662,03 mmol) Benzaldehyd getropft. Es wird 1 Stunde bei Raumtemperatur, dann 24 Stunden bei 80°C nachgerührt. Das Rohprodukt wird durch fraktionierte Destillation gereinigt. Man erhält 178,17 g (94 %) einer farblosen Flüssigkeit mit einem Siedepunkt von 110 bis 113°C (bei 0,59 mbar).
¹H-NMR (200 MHz, DMSO-d₆): δ = 0.0 (m, 9H), 1.1 (m, 6H), 3.7-4.02 (m, 4H), 5.05 (d, 1H), 7.15-7.45 (m, 5H).

### Beispiel IIa

### 2-Hydroxy-2-(3,5-dimethoxyphenyl)-1-phenylethanon

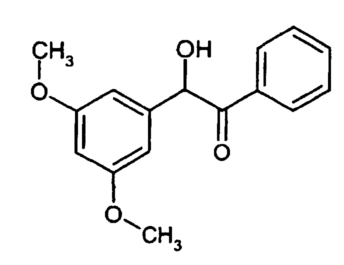

und

### Beispiel IIb

### 1-(3,5-Dimethoxyphenyl)-2-hydroxy-2-phenylethanon

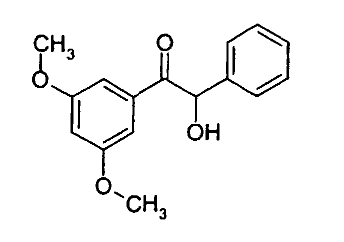

In eine Lösung von 12,81 g (126,58 mmol) Diisopropylamin in 50 ml THF werden bei -70°C 87,02 ml (139,24 mmol) einer 1.6 molaren Lösung von n-Butyllithium in Hexan unter Argon getropft. Es wird 30 Minuten nachgerührt, dann 40,05 g (126,58 mmol) der Verbindung aus Beispiel I gelöst in 50 ml THF bei -70°C zugetropft. Es wird erneut 30 min nachgerührt und in die so erhaltene Suspension eine Lösung von 18,93 g (113,92 mmol) 3,5-Dimethoxybenzaldehyd in 50 ml THF langsam zugetropft und nochmals 30 min nachgerührt. Der Ansatz wird auf Raumtemperatur erwärmt, tropfenweise mit 2 molarer Natronlauge hydrolysiert und 1h nachgerührt. Es wird Wasser zugegeben und 3 mal mit Diethylether extrahiert. Die vereinigten organischen Phasen werden mit 2 molarer Salzsäure und ges. Kochsalzlösung gewaschen, getrocknet und im Vakuum eingeengt. Man erhält ca. 43 g eines Öls, aus welchem man durch Kristallisation aus Ethanol sowie Flash- oder Säulenchromatographie an Kieselgel (Eluens: Cyclohexan/Essigsäureethylester) insgesamt 11,52 g (33 %) der Verbindung IIa als farblose Kristalle vom Schmelzpunkt 104 bis 105°C sowie 2,268 g (7 %) der Verbindung IIb als farblose Kristalle vom Schmelzpunkt 92 bis 93°C erhält.

### Beispiel III

### 2-Amino-5-(3,5-dimethoxyphenyl)-4-phenyl-furonitril

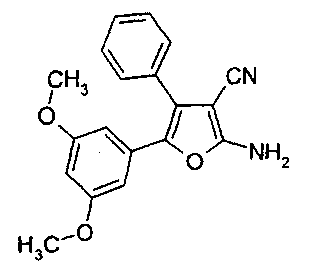

11,86 g (43,56 mmol) der Verbindung des Beispiels IIa werden in 21,23 ml DMF gelöst, mit 3,74 g (56,62 mmol) Malonsäuredinitril und 4,41 g (43,56 mmol) Triethylamin versetzt und über Nacht bei Raumtemperatur gerührt. Anschließend wird das Gemisch im Vakuum eingeengt, 2 mal in Toluol gelöst und erneut eingeengt, wobei Kristallisation eintritt. Es wird mit Diethylether und wenig Essigsäureethylester verrührt, abgesaugt und mit diesem Gemisch nachgewaschen. Die Mutterlauge wird eingeengt, wobei erneute Kristallisation eintritt. Die Kristalle werden abgesaugt und man erhält 10,2 g (73 %) hellbeigefarbene Kristalle vom Schmelzpunkt 179 bis 181°C, die ohne weitere Reinigung in der nächsten Stufe eingesetzt werden.

### Beispiel IV

### Ethyl 3-cyano-5-(3,5-dimethoxyphenyl)-4-phenyl-2-furanylimidoformiat

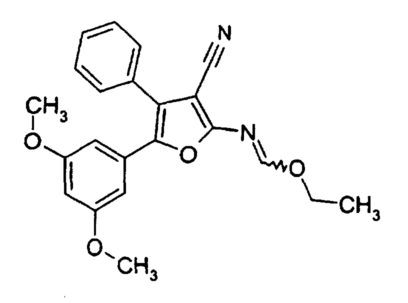

3,41 g (10,64 mmol) der Verbindung des Beispiels III werden in einer Mischung aus 26,56 ml Orthoameisensäureethylester und 0,5 ml Essigsäureanhydrid 5 Stunden unter Rückfluss erhitzt, wobei sich eine Lösung bildet. Anschließend wird die Reaktionsmischung im Vakuum eingeengt und mittels Säulenchromatographie gereinigt (Kieselgel, Eluent: Cyclohexan / Methylenchlorid). Man erhält 3,62 g (90 %) beigefarbene Kristalle vom Schmelzpunkt 103 bis 104°C.

### Beispiel V

### 6-(3,5-Dimethylphenyl)-5-phenylfuro[2,3-d]pyrimidin-4(3H)-on

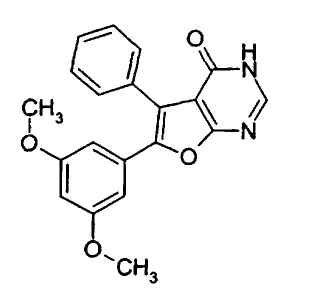

Zu 60 ml Acetanhydrid werden bei 0°C tropfenweise 30 ml Ameisensäure gegeben. Es wird 30 min bei 0°C nachgerührt, mit 10,2 g (31,84 mmol) der Verbindung des Beispiels III versetzt und unter Rückfluss gerührt. Nach 48 Stunden wird die Reaktionsmischung gekühlt, wobei das Produkt in kristalliner Form anfällt und anschließend abgesaugt und mit Diethylether gewaschen werden kann. Man erhält 5,845 g (53 %) eines farblosen Feststoffs vom Schmelzpunkt >250°C. Aus dem Filtrat werden durch erneute Umsetzung mit 12 ml Acetanhydrid und 6 ml Ameisensäure weitere 1,6 g (14 %) des Produktes isoliert.
¹H-NMR (400 MHz, DMSO-d₆): δ = 3.61 (s, 6H), 6.46 (m, 1H), 6.55 (d, 2H), 7.38-7.48 (m, 5H), 8.17 (s, 1H), 12.64 (br. s, 1H).

### Beispiel VI

### 4-Chlor-6-(3,5-dimethoxyphenyl)-5-phenylfuro[2,3-d]pyrimidin

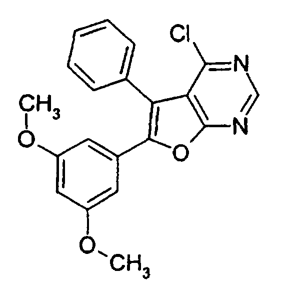

5,7 g (16,36 mmol) der Verbindung des Beispiels V werden mit 57 ml (611,53mmol) Phosphorylchlorid 3 Stunden unter Rückfluss erhitzt. Anschließend wird die Reaktionsmischung im Vakuum eingeengt, 30 min mit Eiswasser verrührt, mit Dichlormethan extrahiert, getrocknet und eingeengt. Man erhält 5,6 g (93 %) eines hellbeige gefärbten Feststoffes vom Schmelzpunkt 138 bis 140°C, der ohne weitere Reinigung in der nächsten Stufe eingesetzt wird.

### Beispiel VII

### 4-Chlor-6-(6-chlor-3-pyridinyl)-5-phenylfuro[2,3-d]pyrimidin

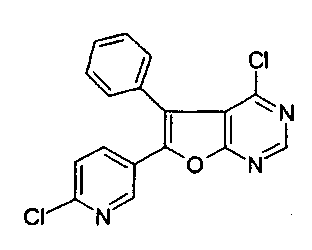

Die Synthese des eingesetzten Ketons 6-(6-Chlor-3-pyridinyl)-5-phenylfuro[2,3-d]-pyrimidin-4(3H)-on erfolgt analog der Herstellungsvorschrift des Beispiels V ausgehend von 2-(6-Chlor-3-pyridinyl)-2-hydroxy-1-phenylethanon, welches analog zur Herstellungsvorschrift der Beispiele IIa/IIb ausgehend von 6-Chlornicotinaldehyd synthetisiert wird.

Analog der Reaktionssequenz zur Synthese des Beispiels VI werden 2,6 g (8,03 mmol) 6-(6-Chlor-3-pyridinyl)-5-phenylfuro[2,3-d]pyrimidin-4(3H)-on in 26 ml Phosphorylchlorid umgesetzt. Man erhält 2,41 g (87,7 %) des Produktes als farblose Kristalle, die ohne weitere Reinigung direkt umgesetzt werden.

### Beispiel VIIIa

### 2-(4-Bromphenyl)-2-hydroxy-1-phenylethanon

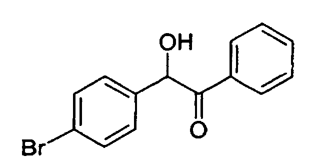

und

### Beispiel VIIIb

### 1-(4-Bromphenyl)-2-hydroxy-2-phenylethanon

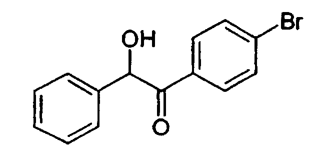

Analog zur Herstellung der Verbindungen der Beispiele IIa/IIb werden 10,0 g (31,60 mmol) der Verbindung des Beispiels I mit 5,26 g (28,44 mmol) 4-Brombenzaldehyd umgesetzt. Man erhält 4 g (43 %) eines nicht trennbaren Isomerengemischs von Beispiel VIIIa und Beispiel VIIIb im Verhältnis 65:35, das im weiteren Schritt direkt umgesetzt wird.
¹H-NMR des Isomerengemisches (200 MHz, CDCl₃): δ = 4.51 (dd, 1H), 5.85-5.95 (m, 1H), 7.16-7.6 (m, 7H), 7.73-7.94 (m, 2H).
MS (DCIpos): m/z = 308 (M+NH₄)⁺

### Beispiel IXa

### 2-Amino-5-(4-bromphenyl)-4-phenyl-3-furonitril

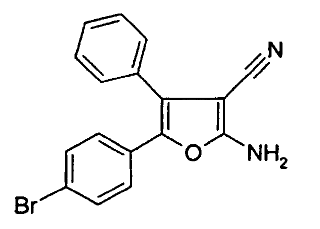

und

### Beispiel IXb

### 2-Amino-4-(4-bromphenyl)-5-phenyl-3-furonitril

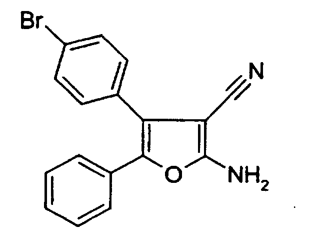

Analog zur Herstellung der Verbindung des Beispiels III werden 4,0 g (13,74 mmol) des Produktgemisches der Beispiele VIIIa/VIIIb umgesetzt. Man erhält 4,6 g (99 %) eines nicht trennbaren Isomerengemisches von Beispiel IXa und Beispiel IXb im Verhältnis 65:35, das im weiteren Schritt direkt umgesetzt wird.
¹H-NMR des Isomerengemisches (300 MHz, DMSO-d₆): δ = 7.1-7.17 (m, 2H), 7.34-7.53 (m, 7H), 7.79 (s, 2H).
MS (EIpos): m/z = 339.2 (M)⁺

### Beispiel Xa

### 5-(4-Bromphenyl)-3-cyano-5-phenyl-2-furylimidoethylformiat

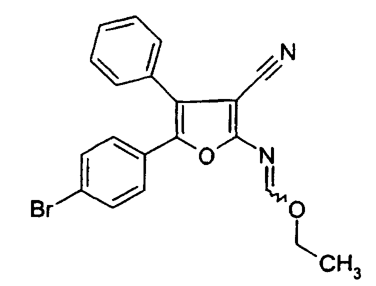

und

### Beispiel Xb

### 4-(4-Bromphenyl)-3-cyano-4-phenyl-2-furylimidoethylformiat

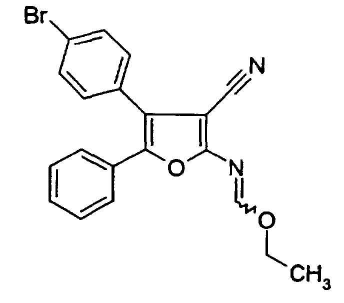

Analog zur Herstellung der Verbindung des Beispiels IV werden 4,6 g (13,56 mmol) des Produktgemisches der Beispiele IXa/IXb umgesetzt. Man erhält 3,9 g (69 %) eines nicht trennbaren Isomerengemisches von Beispiel Xa und Beispiel Xb im Verhältnis 65:35, das im weiteren Schritt direkt umgesetzt wird.
¹H-NMR des Isomerengemisches (300 MHz, CDCl₃): δ = 1.43 (t, 3H), 4.47 (q, 2H), 7.23-7.45 (m, 8H), 7.52-7.58 (m, 1H), 8.45 (s, 1H).
MS (DCIpos): m/z = 413 (M+NH₄)⁺

### Beispiel XIa

### 6-(4-Bromphenyl)-N-isobutyl-5-phenylfuro[2,3-d]pyrimidin-4-amin

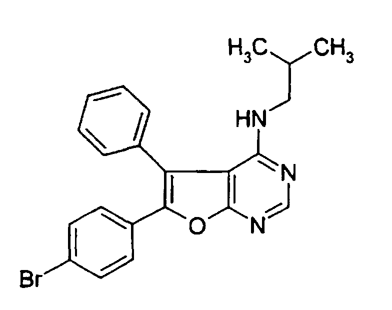

und

### Beispiel XIb

### 5-(4-Bromphenyl)-N-isobutyl-6-phenylfuro[2,3-d]pyrimidin-4-amin

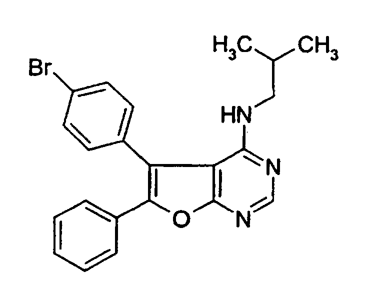

2,2 g (5,57 mmol) des Produktgemisches der Verbindungen aus Beispiel Xa und Beispiel Xb werden in 110 ml Ethanol gelöst, mit 2,44 g (33,4 mmol) Isobutylamin versetzt und eine Stunde bei 40°C gerührt. Anschließend versetzt man die Mischung mit 33 ml 1 molarer Natronlauge und rührt eine Stunde bei 90°C. Der entstandene Niederschlag wird abgesaugt und das Filtrat mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält 1,0 g (39 %) der Verbindung des Beispiels XIa und 450 mg (19 %) der Verbindung des Beispiels XIb in Form von amorphen Feststoffen.
¹H-NMR von Beispiel XIa (300 MHz, CDCl₃): δ = 0.75 (d, 6H), 1.68 (sept., 1H), 3.24 (t, 2H), 4.66 (br. t, 1H), 7.34-7.61 (m, 9H), 8.40 (s, 1H).
MS (EIpos): m/z = 424.3 (M+H)⁺
¹H-NMR von Beispiel XIb (300 MHz, CDCl₃): δ = 0.82 (d, 6H), 1.72 (sept., 1H), 3.28 (t, 2H), 4.61 (br. t, 1H), 7.25-7.721 (4m, 9H), 8.40 (s, 1H).
MS (EIpos): m/z = 424.4 (M+H)⁺

### Beispiel XII

### 6-(4-Bromphenyl)-4-chlor-5-phenylfuro[2,3-d]pyrimidin

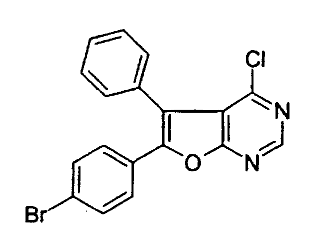

Die Synthese des eingesetzten Ketons 6-(4-Bromphenyl)-5-phenylfuro[2,3-d]pyrimidin-4(3H)-on erfolgt analog zur Herstellung der Verbindung des Beispiels V ausgehend vom Gemisch der Verbindungen aus Beispiel VIIIa/VIIIb (65:35).

Eine Suspension von 1630 mg (4,44 mmol) 6-(4-Bromphenyl)-5-phenylfuro[2,3-d]-pyrimidin-4(3H)-on in 7 ml (75,10 mmol) Phosphorylchlorid wird 2 Stunden zum Rückfluss erhitzt (Ölbadtemperatur 135°C). Nach dem Abkühlen wird die Lösung auf Eis gegeben und mit 25 %iger wässriger Ammoniak-Lösung leicht alkalisch gestellt. Der anfallende Niederschlag wird abgesaugt, mit DMSO gewaschen und im Vakuum getrocknet. Man erhält 680 mg (38 %) Produkt als hellbeigen Feststoff.
¹H-NMR (300 MHz, DMSO-d₆): δ = 7.38-7.68 (9H, m), 8.89 (1H, s).
MS (EIpos): m/z = 386 (M+H)⁺

### Beispiel XIII

### 6-(4-Bromphenyl)-4-chlor-5-(4-fluorphenyl)furo[2,3-d]pyrimidin (Isomerenge misch)

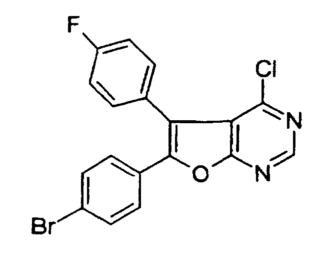

Die Synthese des eingesetzten Ketons 6-(4-Bromphenyl)-5-(4-fluorphenyl)-furo[2,3-d]pyrimidin-4(3H)-on erfolgt analog zur Herstellung der Verbindung des Beispiels V ausgehend vom entsprechenden Benzoin 2-(4-Bromphenyl)-1-(4-fluorphenyl)-2-hydroxyethanon, das im Gemisch mit 1-(4-Bromphenyl)-2-(4-fluorphenyl)-2-hydroxyethanon im Verhältnis 5:4 eingesetzt wird. Dieses Gemisch der beiden isomeren Benzoine wird analog zur Vorschrift der Synthese der Verbindungen der Beispiele VIIIa/VIIIb ausgehend von 4-Brombenzaldehyd und 4-Fluorphenyl-[trimethylsilyl)oxy]-methylphosphonsäurediethylester (hergestellt analog Verbindung des Beispiels I aus 4-Fluorbenzaldehyd) synthetisiert.

Analog zur Herstellung der Verbindung des Beispiels VI werden 19,0 g (49,3 mmol) 6-(4-Bromphenyl)-5-(4-fluorphenyl)furo[2,3-d]pyrimidin-4(3H)-on mit 67 ml (718,8 mmol) Phosphorylchlorid umgesetzt. Man erhält 19,7 g (94 %) des Produktes als Isomerengemisch [(6-(4-Bromphenyl)-4-chlor-5-(4-fluorphenyl)furo[2,3-d]pyrimidin / 5-(4-Bromphenyl)-4-chlor-6-(4-fluorphenyl)furo[2,3-d]pyrimidin = 5:4] in Form eines weiss-braunen Feststoffes.

¹H-NMR (300 MHz, DMSO-d₆) des Isomerengemischs: δ = 7.29-7.78 (8H, m), 8.88 (1H, s, Isomer), 8.89 (1H, s).
MS (EIpos): m/z = 404 (M+H)⁺

### Beispiel XIV

### 4-Chlor-5-(4-fluorphenyl)-6-phenylfuro[2,3-]pyrimidin

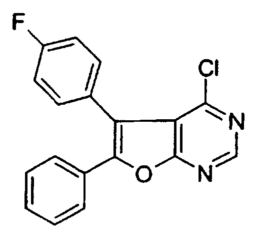

Die Synthese des eingesetzten Ketons 5-(4-Fluorphenyl)-6-phenylfuro[2,3-d]pyrimidin-4(3H)-on erfolgt analog zur Herstellung der Verbindung des Beispiels V ausgehend vom entsprechenden Benzoin 1-(4-Fluorphenyl)-2-hydroxy-2-phenylethanon. Das Benzoin wird analog zur Vorschrift der Synthese der Verbindung der Beispiele VIIIa/VIIIb ausgehend von Benzaldehyd und 4-Fluorphenyl-[trimethylsilyl)oxy]-methylphosphonsäurediethylester (hergestellt analog Verbindung des Beispiels I aus 4-Fluorbenzaldehyd) synthetisiert. Hierbei gelingt es, das gewünschte Isomer, welches zur Synthese eingesetzt wird, durch Kristallisation aus Ethanol zu erhalten (Schmelzpunkt 107 bis 109°C).

Analog zur Herstellung der Verbindung des Beispiels VI werden 7,9 g (25,79 mmol) 5-(4-Fluorphenyl)-6-phenylfuro[2,3-d)pyrimidin-4(3H)-on in 80 ml Phosphorylchlorid umgesetzt. Man erhält 7,65 g (91,3 %) des Produktes als farblose Kristalle vom Schmelzpunkt 124 bis 127°C, die direkt weiter umgesetzt werden.

### Beispiel XV

### 4-Chlor-5,6-bis(4-methoxyphenyl)furo[2,3-d]pyrimidin

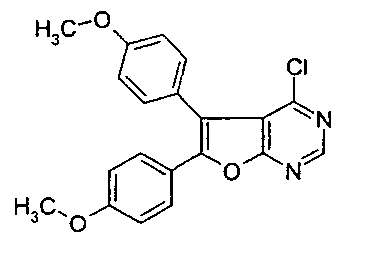

Die Synthese des eingesetzten Ketons 5,6-Bis(4-methoxyphenyl)furo[2,3-d]pyrimidin-4(3H)-on erfolgt analog zur Herstellung der Verbindung des Beispiels V ausgehend von 2-Hydroxy-1,2-bis(4-methoxyphenyl)ethanon.

Eine Suspension von 1,02 g (2,93 mmol) 5,6-Bis(4-methoxyphenyl)furo[2,3-d]pyrimidin-4(3H)-on in 4,0 ml (42,9 mmol) Phosphorylchlorid wird 45 min zum Rückfluss erhitzt. Nach üblicher Aufarbeitung erhält man 1,05 g (89 %) Produkt in Form eines weißen Feststoffes.
¹H-NMR (300 MHz, DMSO-d₆): δ = 3.78 (3H, s), 3.84 (3H, s), 6.99 (2H, d), 7.09 (2H, d), 7.43 (2H, d), 7.49 (2H, d), 8.80 (1H, s).
MS (Elpos): m/z = 367 (M+H)⁺

### Beispiel XVI

### N-(Diphenylmethylen)-N-{4-[5-(4-fluorphenyl)-4-(4-methyl-1-piperazinyl)-furo[2,3-d]pyrimidin-6-yl]phenyl}amin

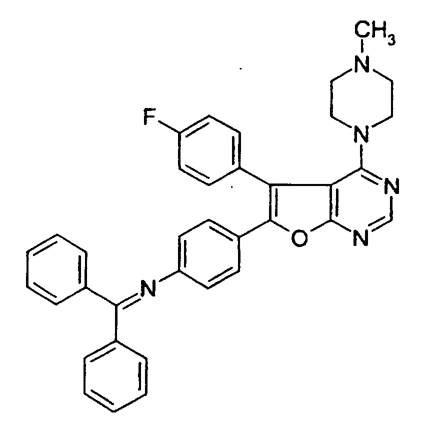

Zu einer Suspension von 0,98 mg (0,001 mmol) Tris(dibenzylidenaceton)-dipalladium(0) und 2,0 mg (0,003 mmol) rac-2,2'-Bis(diphenylphosphino)-1,1'-binaphthyl in 3 ml trockenem Toluol werden unter einer Argon-Atmosphäre 200 mg (0,428 mmol) der Verbindungen aus Beispiel 21, 93,1 mg (0,51 mmol) Benzophenonimin und 57,6 mg (0,60 mmol) Natrium-tert-butylat zugegeben. Das gelbe Reaktionsgemisch wird drei Stunden bei 80°C gerührt. Anschließend wird mit 10 ml Toluol und 10 ml gesättigter wässriger Natriumhydrogencarbonat-Lösung verdünnt. Nach Trennung der Phasen wird die organische Phase über Magnesiumsulfat getrocknet und im Vakuum eingeengt. Das Gemisch wird mittels präparativer MPLC (Säule: 50 g Kieselgel; Eluent: Dichlormethan/Ethanol 50:1 bis 10:1; Fluss 80 ml/min; UV-Detektion bei 210 nm) aufgetrennt. Nach Konzentration im Vakuum erhält man 190 mg (78 %) des Produktes als gelben Feststoff.

¹H-NMR (300 MHz, DMSO-d₆): δ = 1.97-2.09 (4H, m), 2.06 (3H, s), 3.08-3.21 (4H, m), 6.18 (2H, d), 7.10-7.22 (4H, m), 7.37-7.50 (10H, m), 7.61-7.69 (2H, m), 8.41 (1H, s).
MS (EIpos): m/z = 568 (M)⁺

### Beispiel XVII

### N-(5,6-Diphenylfuro[2,3-d]pyrimidin-4-yl)-N-isobutylamin

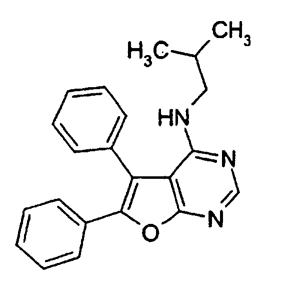

Analog zur Herstellungsvorschrift der Verbindungen der Beispiele XIa und XIb wurde das Produkt ausgehend von 2-Hydroxy-1,2-diphenylethanon hergestellt.

¹H-NMR (200 MHz, DMSO-d₆): δ = 0.72 (d, 6H), 1.66 (sept., 1H), 3.20 (d, 2H), 4.89 (br. s, 1H), 7.25-7.68 (m, 10H), 8.32 (s, 1H).
MS (Elpos): m/z = 344 (M+H)⁺

### Beispiel XVIII

### 5-(4-Fluorphenyl)-4-(4-methyl-1-piperazinyl)-6-phenylfuro[2,3-d]pyrimidin

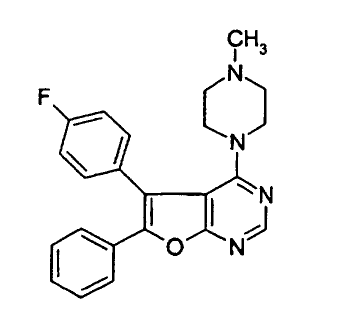

Zu einer Suspension von 500 mg (1.54 mmol) der Verbindung des Beispiels XIV werden 616,9 mg (6,16 mmol) *N*-Methylpiperazin gegeben und 4,5 h unter Siedehitze gerührt. Anschließend werden 8 ml Wasser zugegeben und es wird auf Raumtemperatur abgekühlt. Die ausgefallenen Kristalle werden abgesaugt und mit Ethanol/Wasser (1:1) gewaschen. Man erhält nach Trocknen 384 mg (64,2 %) des Produktes in Form eines hellgelben Pulvers vom Schmelzpunkt 143-144°C.

¹H-NMR (200 MHz, CDCl₃): δ = 2.05-2.25 (m, 7H), 3.22-3.25 (m, 4H), 7.10-7.50 (m, 9H), 8.49 (s, 1H).
MS (EIpos): m/z = 389 (M+H)⁺

### Beispiel XIX

### 4-Chlor-6-(6-chlor-3-pyridinyl)-5-(4-fluorphenyl)furo[2,3-d]pyrimidin

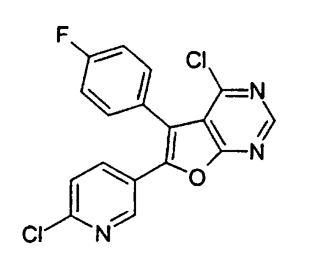

Die Synthese des eingesetzten Ketons 6-(6-Chlor-3-pyridinyl)-5-(4-fluorphenyl)-furo[2,3-d]pyrimidin-4(3H)-on erfolgt analog der Herstellungsvorschrift des Beispiels V ausgehend von 2-(6-Chlor-3-pyridinyl)-2-hydroxy-1-(4-fluorphenyl)-ethanon, welches analog zur Herstellungsvorschrift der Beispiele IIa/IIb ausgehend von 6-Chlornicotinaldehyd und 4-Fluorbenzaldehyd synthetisiert wird.

Analog der Reaktionssequenz zur Synthese des Beispiels VII werden 3.34 g (9.77 mmol) 6-(6-Chlor-3-pyridinyl)-5-(4-fluorphenyl)furo[2,3-d]pyrimidin-4(3H)-on in 35 ml Phosphorylchlorid umgesetzt. Man erhält 3.4 g (97 %) des Produktes als farblosen bis leicht gelben Feststoff.

¹H-NMR (300 MHz, DMSO-d₆): δ = 7.36-7.46 (m, 2H), 7.56-7.67 (m, 3H), 7.89 (dd, 1H), 8.50 (d, 1H), 8.91 (s, 1H)
MS (ESIpos): m/z = 360.2 (M+H)⁺

### Beispiel XX

### 4-Chlor-6-(6-chlor-3-pyridinyl)-5-phenylfuro[2,3-d]pyrimidin

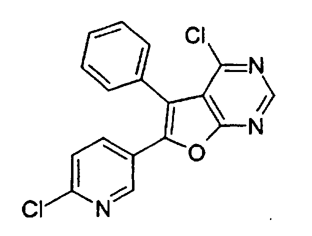

Die Synthese des eingesetzten Ketons 6-(6-Chlor-3-pyridinyl)-5-phenylfuro[2,3-d]pyrimidin-4(3H)-on erfolgt analog der Herstellungsvorschrift des Beispiels V ausgehend von 2-(6-Chlor-3-pyridinyl)-2-hydroxy-1-phenylethanon, welches analog zur Herstellungsvorschrift der Beispiele IIa/IIb ausgehend von 6-Chlornicotinaldehyd und Benzaldehyd synthetisiert wird.

Analog der Reaktionssequenz zur Synthese des Beispiels VII werden 2.07 g (6.39 mmol) 6-(6-Chlor-3-pyridinyl)-5-phenylfuro[2,3-d]pyrimidin-4(3H)-on in 22.6 ml Phosphorylchlorid umgesetzt. Man erhält 1.48 g (68 %) des Produktes als farblosen bis leicht gelben Feststoff.

MS (ESIpos): m/z = 342 (M+H)⁺.

### Herstellungsbeispiele:

### Beispiel 1

### N-Cycloheptyl-6-(3,5-dimethoxyphenyl)-5-phenylfuro[2,3-d]pyrimidin-4-amin

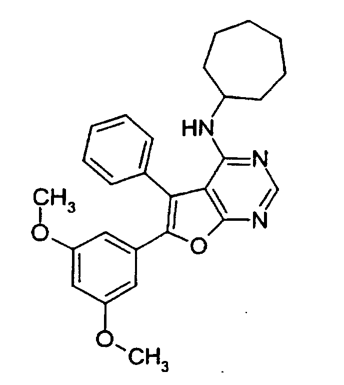

100 mg (0,27 mmol) der Verbindung des Beispiels VI wird in 2 ml Ethanol mit 180 mg (1,59 mmol) Cycloheptylamin 2 Stunden bei 40°C gerührt, wobei sich ein Niederschlag bildet. Anschließend wird mit 2 ml 1 N Natronlauge versetzt und 4 Stunden bei 90°C gerührt, wobei sich wieder eine Lösung bildet. Nach Rühren über Nacht wird das kristalline Produkt abgesaugt und mit Wasser/Ethanol gewaschen. Man erhält 91 mg (77 %) farblose Kristalle vom Schmelzpunkt 151 bis 152°C.

¹H-NMR (300 MHz, CDCl₃): δ = 1.22-1.6 (m, 10H), 1.75-1.88 (m, 2H), 3.61 (s, 6H), 4.2-4.34 (m, 1H), 4.65 (d, 1H), 6.35 (m, 1H), 6.7 (d, 2H), 7.47-7.61 (m, 5H), 8.39 (s,1H).
MS (ESIpos): m/z = 444 (M+H)⁺

### Beispiel 2

### 6-(3,5-Dimethoxyphenyl)-5-phenyl-4-(1-piperidinyl)furo[2,3-d]pyrimidin

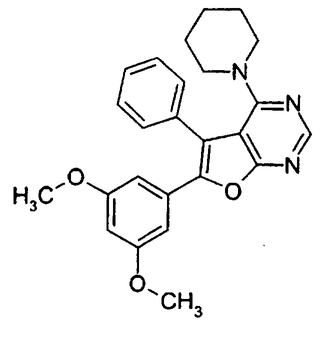

Zu einer Suspension von 50 mg (0.14 mmol) der Verbindung des Beispiels VI in 1 ml Ethanol wird 46,4 mg (0,55 mmol) Piperidin gegeben und 3 Stunden bei 80°C gerührt. Anschließend wird in der Hitze 1 ml Wasser hinzugegeben und die Mischung auf Raumtemperatur gekühlt. Hierbei fällt das Produkt in Form von Kristallen aus, die abgesaugt und mit Ethanol/Wasser 1:1 gewaschen werden. Man erhält 37 mg (65 %) farbloser Kristalle vom Schmelzpunkt 152 bis 153°C.

¹H-NMR (300 MHz, CDCl₃): δ = 1.12-1.29 (m, 4H), 1.34-1.5 (m, 2H), 3.2 (t, 4H), 3.6 (s, 6H), 6.35 (t, 1H), 6.62 (d, 2H), 7.35-7.55 (m, 5H), 8.45 (s, 1H):
MS (ESIpos): m/z = 416 (M+H)⁺

### Beispiel 3

### 5-(4-Fluorphenyl)-4-(4-methyl-1-piperazinyl)-6-(4-nitrophenyl)furo[2,3-d]pyrimidin

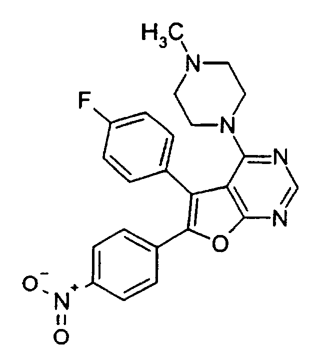

1g (3,08 mmol) der Verbindung des Beispiels XIV wird in Acetonitril in der Wärme gelöst und anschließend unter schnellem Rühren gekühlt (Eis/Methanol-Kühlung). Jetzt wird die Mischung mit 613,4 mg (4,62 mmol) Nitronium-tetrafluorborat versetzt, 1 Stunde bei 0°C gerührt, mit 1,85 g (18,48 mmol) 1-Methylpiperazin versetzt und 1,5 Stunden unter Siedehitze erwärmt, wobei ein gelber Feststoff ausfällt. Die Reaktionsmischung wird abgekühlt, der Feststoff abgesaugt und mit Acetonitril gewaschen. Man erhält 777,2 mg (58 %) eines gelb gefärbten Produktes vom Schmelzpunkt >250°C.

¹H-NMR (300 MHz, CDCl₃): δ = 2.15 (t, 4H), 2.19 (s, 3H), 3.3 (t, 4H), 7.17-7.32.(m, 2H), 7.33-7.46 (m, 2H), 7.55-7.65 (m, 2H), 8.09-8.18 (m, 2H), 8.51 (s, 1H).
MS (ESIpos): m/z = 434 (M+H)⁺

### Beispiel 4

### 4-[5-(4-Fluorphenyl)-4-(4-methyl-1-piperazinyl)furo[2,3-d]pyrimidin-6-yl]anilin

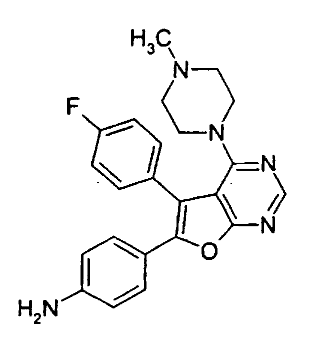

110 mg (0,25 mmol) der Verbindung aus Beispiel 3 werden in einer Mischung aus 3 ml Methanol und 3 ml THF gelöst, mit 11 mg Palladium auf Kohle (10 Gew.-%) versetzt und bei Normaldruck in einer Wasserstoffatmosphäre hydriert. Anschließend wird die Mischung über Celite filtriert, mit THF gewaschen und im Vakuum eingeengt. Man erhält 98 mg (96 %) farblose Kristalle vom Schmelzpunkt 214 bis 215°C, die ohne weitere Reinigung umgesetzt wurden.

MS (ESIpos): m/z = 404 (M+H)⁺

### Beispiel 5

### 6-(4-tert-Butytoxycarbonylamino-methylcarbonylamino-phenyl)-5-(4-fluorphenyl)-4-(4-methyl-1-piperazinyl)-furo(2,3-d]pyrimidin

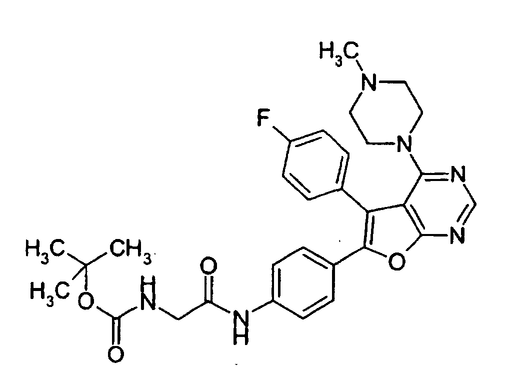

Eine Lösung aus 30 mg (0,07 mmol) der Verbindung aus Beispiel 4 in 3 ml THF und 2 ml DMF wird mit 19,5 mg (0,11 mmol) tert.-Butyloxycarbonylglycin und 28,5 mg (0,15 mmol) EDC versetzt und über Nacht bei Raumtemperatur gerührt. Anschließend wird die Mischung direkt mittels Säulenchromatographie (Kieselgel, Eluent: Dichlormethan/Methanol) gereinigt. Nach Umkristallisation aus Essigsäureethylester erhält man 15,4 mg (37 %) des Produktes als farblosen Feststoff.

¹H-NMR (200 MHz, CDCl₃): δ = 1.46 (s, 9H), 2.16 (t, 4H), 2.21 (s, 3H), 3.26 (t, 4H), 3.9 (d, 2H), 4.19 (br. s, 1H), 7.1-7.24 (m, 2H), 7.32-7.5 (m, 6H), 8.25 (br. s, 1H), 8.47 (s, 1H).
MS (ESIpos): m/z = 561 (M+H)⁺

### Beispiel 6

### N-1-{4-[5-(4-Fluorphenyl)-4-(4-methyl-1-piperazinyl)furo[2,3-d]pyrimidin-6-yl]-phenylglycinamid-dihydrochlorid

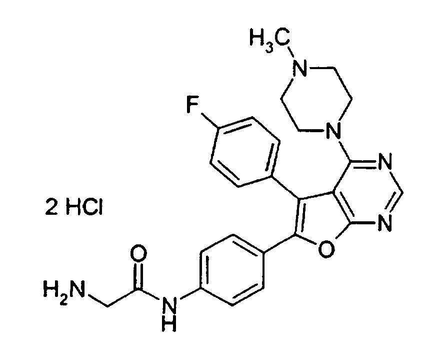

133 mg (0,24 mmol) der Verbindung aus Beispiel 5 werden in 20 ml 4 N HCI in Dioxan 2 Stunden gerührt, wobei das Produkt als Feststoff ausfällt. Die Mischung wird im Vakuum eingeengt, der Feststoff in wenig Wasser gelöst, filtriert und getrocknet. Man erhält 105 mg (83 %) eines farblosen Pulvers.

¹H-NMR (400 MHz, D₂O): δ = 2.56 (t, 2H), 2.84 (s, 3H), 3.08 (t, 2H), 3.31 (d, 2H), 3.71 (d, 2H), 4.02 (s, 2H), 7.29-7.46 (m, 8H), 8.34 (s, 1H).
MS (ESIpos): m/z = 461 (M - 2 HCI + H)⁺

### Beispiel 7

### 4-(4-Methyl-1-piperazinyl)-6-[N-(4-morpholinyl)-3-pyridinyl]-5-phenylfuro[2,3-d]-pyrimidin

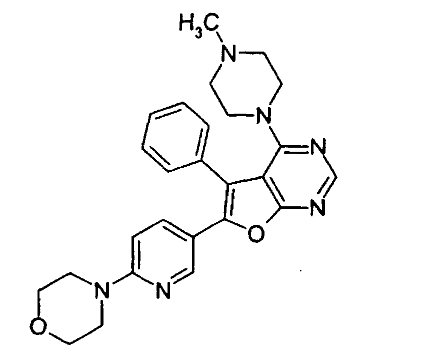

Eine Mischung aus 50 mg (0,12 mmol) der Verbindung aus Beispiel 94 und 2 ml Morpholin wird 1,5 Stunden bei 130°C gerührt. Nach Abkühlen fällt das Produkt als Feststoff aus. Die Mischung wird mit Essigsäureethylester verdünnt, der Feststoff abgesaugt und anschließend mit Ethanol und Wasser gewaschen. Man erhält 54 mg (96 %) hellbeige gefärbte Kristalle vom Schmelzpunkt 255 bis 257°C.

¹H-NMR (400 MHz, CDCl₃): δ = 2.13 (s, 4H), 2.19 (s, 3H), 3.26 (t, 4H), 3.53 (t, 4H), 3.79 (t, 4H), 6.52 (d, 1H), 7.36-7.5 (m, 5H), 7.57 (dd, 1H), 8.28 (m, 1H), 8.45 (s, 1H).
MS (DCI): m/z = 457 (M+H)⁺

### Beispiel 8

### 5-[4-(4-Methyl-1-piperazinyl)-5-phenylfuro[2,3-d]pyrimidin-6-yl]-2-pyridinamin

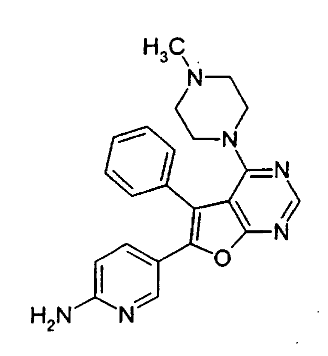

Eine Mischung aus 50 mg (0,12 mmol) der Verbindung aus Beispiel 94, 1,6 g Acetamid (27,09 mmol) und 220 mg Kaliumcarbonat wird 32 Stunden bei 210°C gerührt. Nach Abkühlen auf 70°C wird mit Wasser und Essigester versetzt, die wässrige Phase mit Essigsäureethylester extrahiert, die vereinigten organischen Phasen mit Wasser gewaschen, getrocknet und eingeengt. Das Rohprodukt wird anschließend in einem Gemisch aus Essigsäureethylester und Diethylether verrührt und abfiltriert. Man erhält 26,8 mg (56 %) beige gefärbte Kristalle vom Schmelzpunkt 229 bis 231 °C.

¹H-NMR (300 MHz, DMSO-d₆): δ = 1.97 (t, 4H), 2.04 (s, 3H), 3.14 (t, 4H), 6.33 (s, 2H), 6.38 (d, 1H), 7.32-7.57 (m, 6H), 7.91 (d, 1H), 8.4 (s, 1H).
MS (ESIpos): m/z = 387 (M+H)⁺

### Beispiel 9

### 1-[6-(6-Chlor-3-pyridinyl)-5-phenylfuro[2,3-d]pyrimidin-4-yl]-4-piperidinol

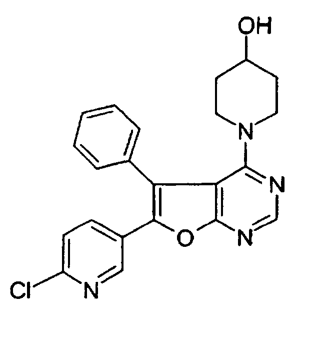

Die Verbindung wird analog der Versuchsvorschrift zur Synthese von Beispiel 2 aus der Verbindung des Beispiels VII und 4-Piperidinol in quantitativer Ausbeute hergestellt.

¹H-NMR (300 MHz, CDCl₃): δ = 1.14-1.32 (m, 3H), 1.50-1.65 (m, 2H), 2.89-3.02 (m, 2H), 3.59-3.79 (m, 3H), 7.21-7.28 (m, 1H), 7.36-7.54 (m, 5H), 7.70-7.74 (m, 1H) 8.38-8.41 (m, 1H), 8.48 (s, 1H).
MS (ESIpos): m/z = 407 (M+H)⁺

### Beispiel 10

### 1-{6-[6-(4-Methyl-1-piperazinyl)-3-pyridinyl]-5-phenylfuro[2,3-d]pyrimidin-4-yl}-4-piperidinol

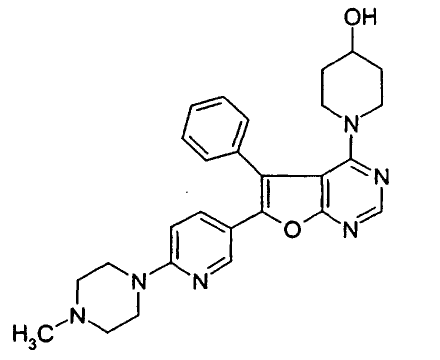

Die Verbindung wird analog der Versuchsvorschrift für Beispiel 7 aus Beispiel 9 und *N*-Methylpiperazin hergestellt (Ausbeute: 81 %).

¹H-NMR (300 MHz, CDCl₃): δ = 1.10-1.30 (m, 3H), 1.50-1.68 (m, 2H), 2.38 (s, 3H), 2.50-2.78 (m, 4H), 2.80-2.98 (m, 2H), 3.50-3.68 (m, 6H), 3.92-4.08 (m, 1H), 6.65 (d, 1H), 7.15-7.60 (m, 6H), 8.23-8.29 (m, 1H), 8.46 (s, 1H).
MS (ESIpos): m/z = 471 (M+H)⁺

### Beispiel 11

### 4-[4-(Isobutylamino)-5-phenylfuro[2,3-d]pyrimidin-6-yl]benzoesäuremethylester

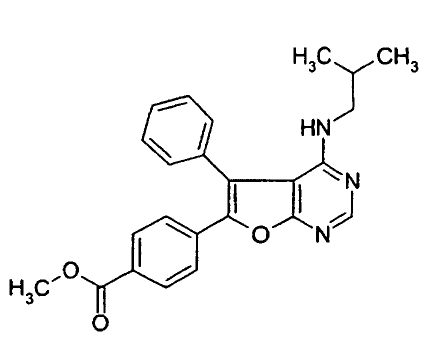

Eine Mischung aus 21,4 mg (0,05 mmol) Bis(diphenylphosphino)propan und 10,6 mg Palladium(II)acetat werden unter Schutzgas und Sauerstoffausschluss in 2,0 ml DMF vorgelegt. Hierzu werden 200,0 mg (0,47 mmol) der Verbindung aus Beispiel XIa (gelöst in einer Mischung aus 4,0 ml Methanol und 4,0 ml DMF) und 479,2 mg (4,74 mmol) Triethylamin gegeben und eine Stunde bei 120°C gerührt. Anschließend gießt man die Reaktionsmischung auf Wasser und extrahiert mit Methylenchlorid. Die vereinigten organischen Phasen werden noch einmal mit Wasser und einmal mit gesättigter Natriumchlorid-Lösung gewaschen, mit Natriumsulfat getrocknet und im Vakuum eingeengt. Der Rückstand wird mittels Chromatographie (Kieselgel, Eluent: Cyclohexan/Essigsäureethylester 1:1) gereinigt. Man erhält 137 mg (64,8 %) des Produktes als amorphen Feststoff.

¹H-NMR (300 MHz, CDCl₃): δ = 0.77 (d, 6H), 1.67 (sept., 1H), 3.26 (t, 2H), 3.89 (s, 3H), 4.66-4.70 (m, 1H), 7.48-7.60 (m, 7H), 7.93 (d, 2H), 8.41 (s, 1H).
MS (ESIpos): m/z = 402 (M+H)⁺

### Beispiel 12

### 4-[4-(Isobutylamino)-5-phenylfuro[2,3-d]pyrimidin-6-yl]benzoesäure

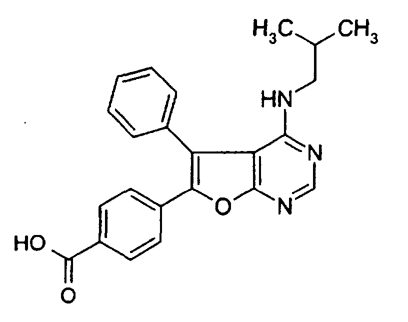

Zu einer Mischung aus 590 mg (1,37 mmol) der Verbindung aus Beispiel 11 in 14 ml Methanol und 5 ml Wasser gibt man 163,6 mg (6,83 mmol) Lithiumhydroxid und rührt vier Stunden bei 50°C. Anschließend wird das Methanol im Vakuum entfernt und die zurückgebliebene wässrige Phase unter Eisbadkühlung mit 1 N wässriger Salzsäure angesäuert. Nach Einengen der Mischung im Vakuum fällt das Produkt aus. Die gebildeten Kristalle werden mit Wasser gewaschen und im Vakuum getrocknet. Man erhält 485 mg (91,6 %) des Produktes als amorphen Feststoff, der direkt weiter umgesetzt wurde.

### Beispiel 13

### N-Isobutyl-6-{4-[(4-methyl-1-piperazinyl)carbonyl]phenyl}-5-phenylfuro[2,3-d]-pyrimidin-4-amin

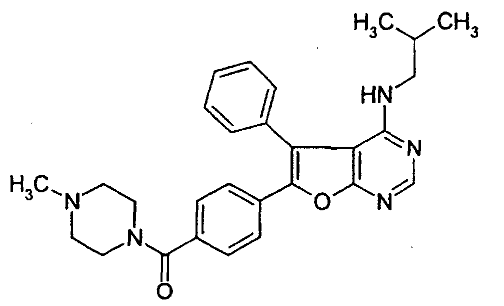

Zu einer Lösung aus 80,0 mg (0,21 mmol) der Verbindung aus Beispiel 12 in 5 ml DMF werden nacheinander 30,6 mg (0,23 mmol) HOBt, 45,5 mg (0,24 mmol) EDC, 62,0 mg (0,62 mmol) 1-Methylpiperazin, 62,6 mg (0,62 mmol) N-Methylmorpholin und eine katalytische Menge DMAP hinzugegeben und die Reaktionsmischung über Nacht bei RT gerührt. Das Gemisch wird mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält nach Konzentration im Vakuum 52 mg (53,6 %) des Produkts als farblosen Feststoff.

¹H-NMR (300 MHz, CDCl₃): δ = 0.76 (s, 3H), 0.78 (s, 3H), 1.5-1.75 (m, 1H), 2.3 (s, 3H), 2.3-2.55 (br.s, 4H), 3.26 (m, 2H), 3.34-3.88 (m, 4H), 4.65-4.70 (t, 1H), 7.25-7.33 (d, 2H), 7.47-7.59 (m, 7H), 8.41 (s, 1H).
MS (ESIpos): m/z = 470 (M+H)⁺

### Beispiel 14

### 2-(4-{4-[4-(Isobutylamino)-5-phenylfuro[2,3-d]pyrimidin-6-yl]phenyl}-1-piperazinyl)-ethanol

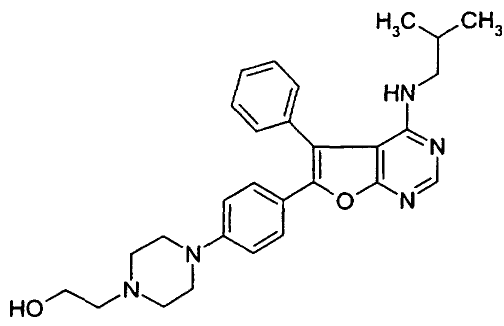

Unter Schutzgas und rigorosem Sauerstoffausschluss werden 75,0 mg der Verbindung des Beispiels XIa, 4,88 mg (0,005 mmol) Tris(dibenzylidenaceton)dipalladium, 11,0 mg (0,018 mmol) rac-2,2'-Bis(diphenylphosphino)-1,1'-binaphthyl, und 23,9 mg (0,21 mmol) Kalium-tert.-butylat in 5 ml Toluol vorgelegt und mit 69,3 mg (0,53 mmol) 2-(1-Piperazinyl)ethanol versetzt. Die Reaktionsmischung wird 2 Stunden bei 60°C gerührt, erneut mit der oben angegebenen katalytischen Menge Tris(dibenzylidenaceton)dipalladium und rac-BINAP versetzt und über Nacht bei 60°C gerührt. Nach Entfernen des Lösungsmittels im Vakuum wird das Gemisch mittels präparativer HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält nach Konzentration im Vakuum 76 mg (90 %) des Produkts als farblosen Feststoff.

¹H-NMR (300 MHz, DMSO-d₆): δ = 0.74 (d, 6H), 1.18 (sept., 1H), 2.40 (t, 2H), 3.13-3.34 (m, 10H), 3.47-3.59 (m, 2H), 4.38 (t, 1H), 4.78 (t, 1H), 6.85 (d, 2H), 7.27 (d, 2H), 7.51-7.61 (m, 5H), 8.27 (s, 1H).
MS (ESIpos): m/z = 472 (M+H)⁺

### Beispiel 15

### N-Isobutyl-6-(4-nitrophenyl)-5-phenylfuro[2,3-d]pyrimidin-4-amin

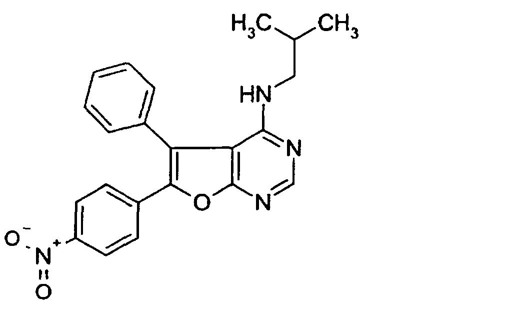

Zu einer Lösung aus 500 mg (1,46 mmol) der Verbindung des Beispiels XVII in 25,0 ml Dichlormethan gibt man bei 0°C portionsweise 352,7 mg (1,75 mmol) Nitroniumtetrafluorborat, rührt die Reaktionsmischung eine Stunde bei 0°C und anschließend über Nacht bei Raumtemperatur. Nach Zugabe von Wasser wird die Reaktionsmischung 2 mal mit Dichlormethan extrahiert, die organischen Phasen vereinigt, einmal mit gesättigter Natriumchlorid-Lösung gewaschen, über Natriumsulfat getrocknet und im Vakuum konzentriert. Nach Chromatographie des Rückstands (Kieselgel, Eluent: Cyclohexan / Essigsäureethylester) erhält man 414 mg (66,6 %) des Produktes als Feststoff.

¹H-NMR (200 MHz, DMSO-d₆): δ = 0.73 (d, 6H), 1.68 (sept., 1H), 3.23 (t, 2H), 5.03 (br. t, 1H), 7.55-7.72 (m, 7H), 8.15-8.27 (m, 2H), 8.41 (s, 1H).
MS (DCIpos): m/z = 389 (M+H)⁺

### Beispiel 16

### 6-(4-Aminophenyl)-N-isobutyl-5-phenylfuro[2,3-d]pyrimidin-4-amin

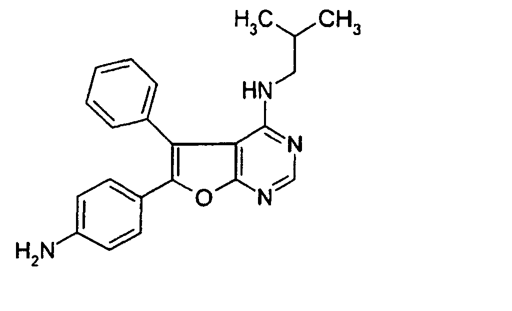

Eine Lösung aus 5,0 g (12,87 mmol) der Verbindung aus Beispiel 15 in 102 ml THF und 102 ml Methanol versetzt man mit 0,25 g (0,13 mmol) Palladium auf Kohle (10 Gew.-%) und hydriert über Nacht bei Raumtemperatur in einer Wasserstoffatmosphäre bei 1 bar. Anschließend wird die Mischung über Celite filtriert und im Vakuum eingeengt. Nach Chromatographie des Rückstands (Kieselgel, Eluent: Cyclohexan / Essigsäureethylester) erhält man 1,84 g (39 %) des Produktes in Form hellbeige gefärbter Kristalle.

¹H-NMR (200 MHz, CDCl₃): δ = 0.77 (d, 6H), 1.67 (sept., 1H), 3.24 (t, 2H), 3.80 (br. s, 2H), 4.58-4.63 (m, 1H), 6.55 (d, 2H), 7.33 (d, 2H), 7.47-7.54 (m, 5H), 8.36 (s, 1H).
MS (DCIpos): m/z = 359 (M+H)⁺

### Beispiel 17

### N-{4-[4-(Isobutylamino)-5-phenylfuro[2,3-d]pyrimidin-6-yl]phenyl}-N',N"dimethylglycinamid

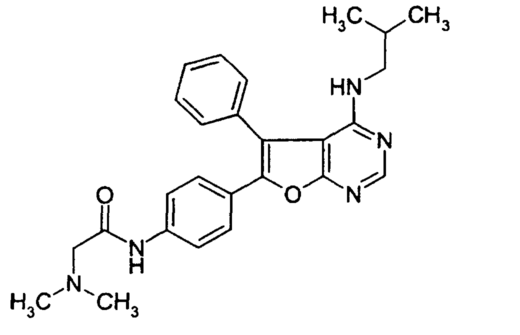

Zu einer Lösung aus 43,1 mg (0,42 mmol) *N,N*-Dimethylglycin in 5,0 ml DMF werden 41,4 mg (0,31 mmol) HOBt und 61,5 mg (0,32 mmol) EDC gegeben und 30 min bei Raumtemperatur gerührt. Dann gibt man 100,0 mg (0,28 mmol) der Verbindung aus Beispiel 16, 112,8 mg (1,12 mmol) N-Methylmorpholin und 3,41 mg (0,028 mmol) DMAP zu und rührt über Nacht. Das Rohgemisch wird anschließend direkt mittels präparativer HPLC (Säule: YMC Gel ODS-AQ S-11 µm, 250 x 30 mm; Eluent: Acetonitril/Wasser; Fluss: 50 mL/min; UV-Detektion bei 210 nm) getrennt. Nach weiterer Trennung mittels Säulenchromatographie (Kieselgel, Eluent: Methylenchlorid/Methanol) erhält man nach Konzentration im Vakuum 49 mg (37 %) des Produktes als farblosen Feststoff.

¹H-NMR (200 MHz, DMSO-d₆): δ = 0.72 (d, 6H), 1.61 (sept., 1H), 2.25 (s, 6H), 3.06 (s, 2H), 3.21 (t, 2H), 4.89 (t, 1H), 7.35 (d, 2H), 7.53-7.67 (m, 7H), 8.32 (s, 1H), 9.87 (s, 1H).
MS (DCIpos): m/z = 444 (M+H)⁺

### Beispiel 18

### N-{4-[4-(Isobutylamino)-5-phenylfuro[2,3-d]pyrimidin-6-yl]phenyl}methansulfonamid

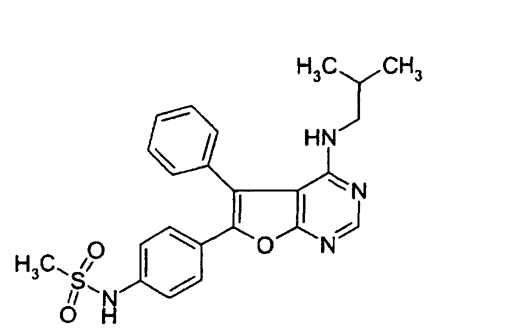

Zu einer Lösung aus 60,0 mg (0,17 mmol) der Verbindung aus Beispiel 16 in 2,0 ml Pyridin werden 23,0 mg (0,20 mmol) Methansulfonsäurechlorid gegeben. Das Reaktionsgemisch wird über Nacht bei Raumtemperatur gerührt und anschließend direkt mittels präparativer HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält 67 mg (90 %) des Produktes als farblosen Feststoff.

¹H-NMR (300 MHz, CDCl₃): δ = 0.78 (d, 6H), 1.67 (sept., 1H), 3.04 (s, 3H), 3.25 (t, 2H), 4.65 (t, 1H), 7.12 (d, 2H), 7.45-7.6 (m, 7H), 8.40 (s, 1H).
MS (ESIpos): m/z = 437 (M)⁺

### Beispiel 19

### tert-Butyl 4-[4-(isobutylamino)-5-phenylfuro[2,3-d]pyrimidin-6-yl]phenylcarbamat

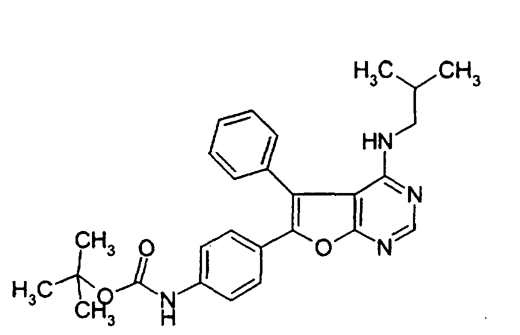

Zu einer Lösung aus 100,0 mg (0,28 mmol) der Verbindung aus Beispiel 16 in 2,0 ml Wasser und 1,0 ml THF werden 66,9 mg (0,31 mmol) Pyrokohlensäure-di-tert.butylester und 73,9 mg (0,7 mmol) Natriumcarbonat gegeben und über Nacht gerührt. Anschließend wird das organische Lösungsmittel im Vakuum entfernt und mit Dichlormethan extrahiert. Da die Umsetzung nicht vollständig war, wird das Gemisch erneut im Vakuum konzentriert, in 3 ml Dioxan aufgenommen, 66,98 mg (0,31 mmol) Pyrokohlensäure-di-tert.butylester hinzugefügt und erneut über Nacht gerührt. Nach Konzentration im Vakuum wird die Mischung direkt mittels präparativer HPLC (Säule: YMC Gel ODS-AQ S-11 µm, 250 x 30 mm; Eluent: Acetonitril/Wasser; Fluss: 50 ml/min; UV-Detektion bei 210 nm) getrennt. Man erhält 49 mg (38 %) des Produktes als farblosen Feststoff.

¹H-NMR (300 MHz, CDCl₃): δ = 0.73 (d, 6H), 1.47 (s, 9H), 1.65 (sept., 1H), 3.20 (t, 2H), 4.85 (t, 1H), 7.31 (d, 2H), 7.40 (d, 2H), 7.5-7.64 (m, 5H), 8.30 (s, 1H).
MS (ESIpos): m/z = 459 (M)⁺

### Beispiel 20

### N-{4-[4-(Isobutylamino)-5-phenylfuro[2,3-d]pyrimidin-6-yl]phenyl}-N'-(4-methoxyphenyl)harnstoff

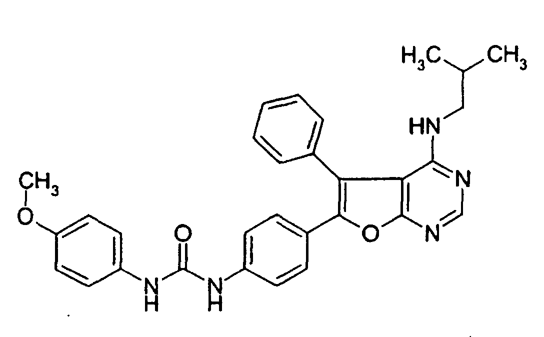

Zu einer Lösung aus 60,0 mg (0,17 mmol) der Verbindung aus Beispiel 16 in 3,0 ml Dichlormethan werden bei 0°C 50,8 mg (0,5 mmol) Triethylamin und eine katalytische Menge DMAP gegeben. Anschließend tropft man 37,4 mg (0,25 mmol) 1-Isocyanato-4-methoxybenzol zu und rührt 24 h bei Raumtemperatur. Die Reaktionsmischung wird auf Wasser gegeben und mit Dichlormethan extrahiert. Die organische Phase wird anschließend mit gesättigter Natriumchlorid-Lösung gewaschen, über Natriumsulfat getrocknet und im Vakuum konzentriert. Das Produkt wird anschließend direkt mittels präparativer HPLC (Säule: YMC Gel ODS-AQ S-11 µm, 250 x 30 mm; Eluent: Acetonitril/Wasser, Fluss: 50 ml/min; UV-Detektion bei 210 nm) getrennt. Man erhält 37 mg (44 %) des Produktes als farblosen Feststoff.

¹H-NMR (300 MHz, CDCl₃): δ = 0.74 (d, 6H), 1.66 (sept., 1H), 3.21 (t, 2H), 3.71 (s, 3H), 4.85 (t, 1H), 6.85 (d, 2H), 7.28-7.38 (m, 4H), 7.39-7.45 (m, 2H), 7.53-7.65 (m, 5H), 8.3 (s, 1H), 8.5 (s, 1H), 8.72 (s, 1H).
MS (ESIpos): m/z = 508 (M)⁺

### Beispiel 21

### 6-(4-Bromphenyl)-5-(4-fluorphenyl)-4-(4-methyl-1-piperazinyl)furo[2,3-d]pyrimidin

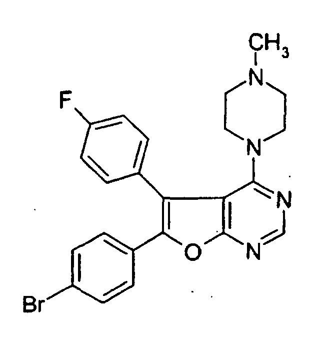

3,00 g (7,4 mmol) der Verbindung des Beispiels XIII (Isomerengemisch 5:4) werden in 60 ml Dioxan suspendiert und nach Zugabe von 4,47 g (44,6 mmol) 1-Methylpiperazin 12 Stunden zum Rückfluss erhitzt (Ölbadtemperatur 82°C). Nach üblicher Aufarbeitung wird der erhaltene Rückstand mittels präparativer HPLC (Säule: 250 x 20 mm Kromasil 100 C-18, 5 µm; Eluent: Wasser/Acetonitril/1 %ige Trifluoressigsäure 44:45:11; Fluss: 25 ml/min; UV-Detektion bei 210 nm) aufgetrennt. Es werden 957 mg (28 %) des Produktes in Form eines leicht gelben Feststoffes neben 344 mg (10 %) des entsprechenden Isomers erhalten.

¹H-NMR (300 MHz, DMSO-d₆): δ = 2.13 (4H, t), 2.20 (3H, s), 3.29 (4H, t), 7.12-7.47 (8H, m), 8.48 (1H, s).
MS (ESIpos): m/z = 468 (M)⁺

### Beispiel 22

### 6-(4-Bromphenyl)-4-(4-methyl-1-piperazinyl)-5-phenylfuro[2,3-d]pyrimidin

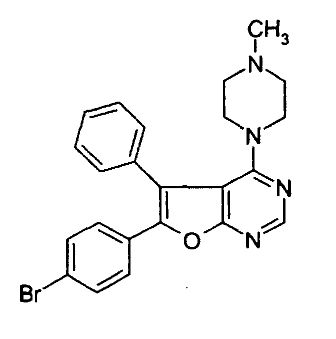

Zu einer Suspension von 630 mg (1,63 mmol) der Verbindung des Beispiels XII in 50 ml Tetrahydrofuran werden 1,35 g (9,80 mmol) Kaliumcarbonat gegeben. Nach Zugabe von 654 mg (6,53 mmol) 1-Methylpiperazin wird die Suspension 90 min zum Rückfluss erhitzt (Ölbadtemperatur 88°C). Nach Abkühlen wird die Reaktionslösung mit 20 ml Wasser und 20 ml Essigsäureethylester versetzt. Die wässrige Phase wird dreimal mit Essigsäureethylester extrahiert. Die vereinigten organischen Phasen werden zweimal mit gesättigter wässriger Natriumchlorid-Lösung gewaschen, über Magnesiumsulfat getrocknet und im Vakuum eingeengt. Man erhält 710 mg (88 %) des Produktes in Form eines weißen Feststoffes.

¹H-NMR (300 MHz, DMSO-d₆): δ = 1.91-2.08 (4H, m), 2.03 (3H, s), 3.09-3.21 (4H, m), 7.24-7.36 (2H, m), 7.38-7.61 (7H, m), 8.46 (1H, s).
MS (ESIpos): m/z = 450 (M+H)⁺

### Beispiel 23

### 1-[6-(4-Bromphenyl)-5-(4-fluorphenyl)furo[2,3-d]pyrimidin-4-yl]-4-piperidinol

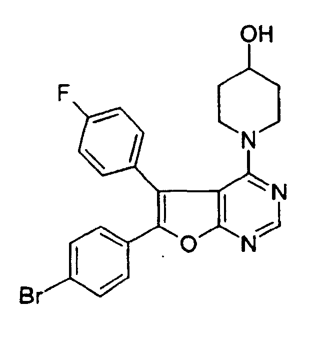

Analog der Reaktion zu Beispiel 21 werden 2,00 g (4,95 mmol) der Verbindung des Beispiels XIII (Isomerengemisch) mit 2,00 g (19,82 mmol) 4-Hydroxypiperidin umgesetzt. Man erhält 1,50 g (64 %) des Produktes als weißen Feststoff.

¹H-NMR (300 MHz, DMSO-d₆): δ = 0.98-1.12 (2H, m), 1.38-1.50 (2H, m), 2.82-2.97 (2H, m), 3.45-3.59 (3H, m), 4.48 (1H, d), 7.29 (2H, d), 7.32-7.53 (4H, m), 7.57 (2H, d), 8.43 (1H, s).
MS (ESIpos): m/z = 469 (M+H)⁺

### Beispiel 24

### 1-{4-[5-(4-Fluorphenyl)4-(4-methyl-1-piperazinyl)furo[2,3-d]pyrimidin-6-yl]phenyl}-2-azetidinon

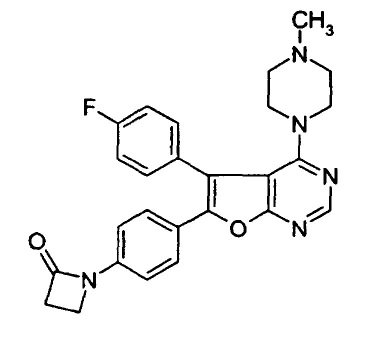

In einem ausgeheizten Schlenk-Rohr werden unter Argon 2,0 mg (0,002 mmol) Tris-(dibenzylidenaceton)dipalladium, 3,7 mg (0,006 mmol) 9,9-Dimethyl-4,5-bis(diphenylphosphino)xanthen, 18,3 mg (0,251 mmol) 2-Azetidinon, 97,6 mg (97,61 mmol) Cäsiumcarbonat und 100,0 mg (0,21 mmol) der Verbindung aus Beispiel 21 vorgelegt. Nach weiterem Spülen mit Argon werden 0,30 ml trockenes Dioxan zugegeben. Die erst rötliche, später gelbe Suspension wird 48h auf 100°C erhitzt und dabei heftig gerührt. Nach Abkühlen wird die Reaktionsmischung mit Dichlormethan (ca. 5 ml) versetzt, filtriert und im Vakuum eingeengt. Das Gemisch wird mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält nach Konzentration im Vakuum 39,0 mg (39,8 %) des Produkts als farblosen Feststoff.

¹H-NMR (300 MHz, CDCl₃): δ = 2.11-2.17 (4H, m), 2.19 (3H, s), 3.19 (2H, t), 3.22-3.31 (4H, m), 3.64 (2H, t), 7.14-7.21 (2H, m), 7.23-7.30 (2H, m), 7.33-7.45 (m, 4H), 8.48 (1H, s).
MS (EIpos): m/z = 458.4 (M+H)⁺

### Beispiel 25

### 4-[5-(4-Fluorphenyl)-4-(4-methyl-1-piperazinyl)furo[2,3-d]pyrimidin-6-yl]-phenyl-amin

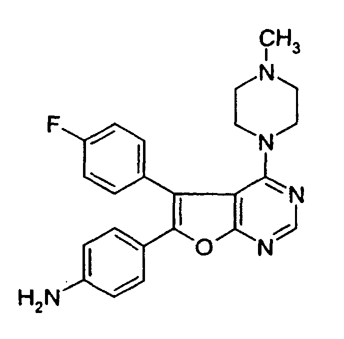

Zu einer Lösung von 150 mg (0,26 mmol) der Verbindung des Beispiels XVI in 3 ml Methanol werden bei 60°C 249,9 mg (3,96 mmol) Ammoniumformiat und 14,1 mg (0,013 mmol) Palladium auf Kohle (10 Gew.-%) zugegeben. Das Reaktionsgemisch wird 4 Stunden bei der angegebenen Temperatur gerührt und anschließend über einen Seitz-Filter filtriert. Das Filtrat wird im Vakuum eingeengt und nach Lösen in DMSO mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210nm) aufgetrennt. Man erhält nach Konzentration im Vakuum 89 mg (65 %) des Produktes als TFA-Salz in Form eines farblosen Feststoffs.

¹H-NMR (300 MHz, DMSO-d₆): δ = 2.73 (3H, s), 2.81-3.01 (2H, m), 3.12-3.29 (2H, m), 3.60-3.85 (4H, m), 6.50 (2H, d), 7.09 (2H, d), 7.29-7.54 (4H, m), 8.48 (1H, s), 9.55 (2H, br s).
MS (Elpos): m/z = 404 (M+H)⁺

### Beispiel 26

### N-(4-{[5,6-bis(4-methoxyphenyl)furo[2,3-d]pyrimidin-4-yl]amino}transcyclohexyl)-5-oxotetrahydro-2-furancarbonsäureamid

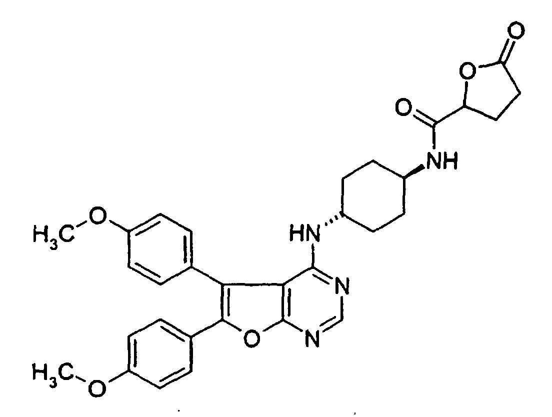

Zu einer Lösung von 35;1 mg (0;270 mmol) 5-Oxotetrahydro-2-furancarbonsäure in 2 ml Dichlormethan werden 120 mg (0;27 mmol) der Verbindung aus Beispiel 168, 56,9 mg (0,297 mmol) EDC, 40,1 mg (0,297 mmol) HOBt und 109 mg (1,08 mmol) Triethylamin zugegeben. Es wird 24 Stunden bei Raumtemperatur gerührt und nach Zugabe von 2 ml gesättigter wässriger Natriumhydrogencarbonat-Lösung wird über Extrelut filtriert und das Filtrat im Vakuum konzentriert. Das Gemisch wird mittels MPLC (Säule: 50 g Kieselgel; Eluent: Dichlormethan/Ethanol 20:1; Fluss 80 ml/min; UV-Detektion bei 210 nm) getrennt. Man erhält 50 mg (33 %) des Produkts als farblosen Feststoff.

¹H-NMR (300 MHz, DMSO-d₆): δ = 0.94-1.12 (2H, m), 1.21-1.42 (2H, m), 1.65-1.78 (2H, m), 1.88-2.11 (3H, m), 2.25-2.59 (3H, m), 3.46-3.61 (1H, m), 3.75 (3H, s), 3.80-3.94 (m, 1H), 3.86 (3H, s), 4.19 (1H, d), 4.80 (1H, dd), 6.94 (2H, d), 7.17 (2H, d), 7.40 (2H, d), 7.44 (2H, d), 8.08 (1H, d), 8.31 (1H, s).
MS (EIpos): m/z = 557 (M+H)⁺

### Beispiel 27

### N-(4-{[5,6-Bis(4-methoxyphenyl)furo[2,3-d]pyrimidin-4-yl]amino}transcyclohexyl)methansulfonsäureamid

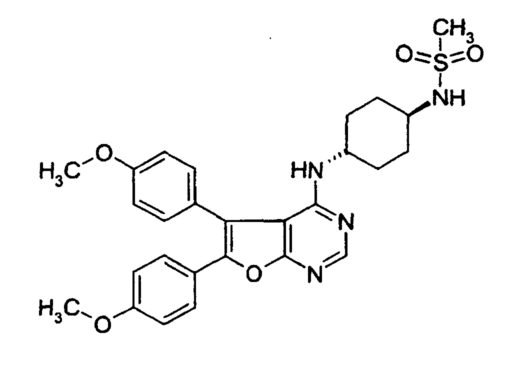

Zu einer Lösung von 12,3 mg (0,11 mmol) Methansulfonsäurechlorid in 2 ml Dichlormethan wird eine Lösung von 47,6 mg (0,11 mmol) der Verbindung aus Beispiel 168 in 0,5 ml Dichlormethan und 43,3 mg (0,43 mmol) Triethylamin zugetropft. Nach Zugabe einer Spatelspitze 4-Dimethylaminopyridin wird 24 h bei RT gerührt. Es werden 2 ml gesättigter wässriger Natriumhydrogencarbonat-Lösung zugegeben und über Extrelut filtriert. Das Filtrat wird im Vakuum eingeengt und das Gemisch direkt mittels präparativer HPLC (Säule: YMC Gel ODS-AQ S-11 µm, 250 x 30 mm; Eluent: Acetonitril/Wasser; Fluss: 50 ml/min; UV-Detektion bei 210 nm) getrennt. Man erhält 20,1 mg (35,2 %) des Produkts als farblosen Feststoff.

¹H-NMR (300 MHz, DMSO-d₆): δ = 0.94-1.10 (2H, m), 1.21-1.40 (2H, m), 1.76-1.97 (4H, m), 2.88 (3H, s), 3.02-3.17 (1H, m), 3.75 (3H, s), 3.78-3.89 (1H, m), 3.86 (3H, s), 4.15 (1H, d), 6.90-6.99 (3H, m), 7.16 (2H, d), 7.90 (2H, d), 7.94 (2H, d), 8.30 (1H, s).
MS (EIpos): m/z = 523 (M+H)⁺

### Beispiel 28

### N-(4-{[5,5-Bis(4-methoxyphenyl)furo[2,3-d]pyrimidin-4-yl]amino}transcyclohexyl)-N'-ethylharnstoff

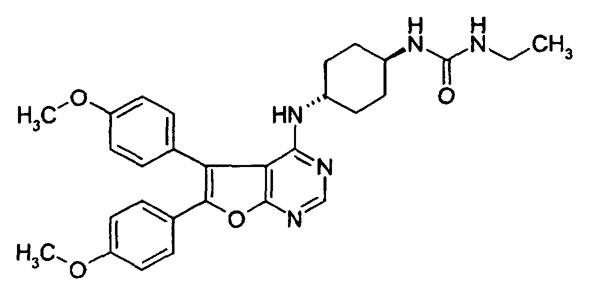

Zu einer Lösung von 9,5 mg (0,13 mmol) 1-Isocyanatoethan in 3 ml THF werden 59,6 mg (0,13 mmol) der Verbindung aus Beispiel 168 gegeben und die Reaktionsmischung 24 Stunden bei RT gerührt. Anschließend wird die Mischung mit 5 ml Ethanol versetzt und das als Niederschlag anfallende Produkt abgesaugt und mit Ethanol gewaschen. Man erhält 40 mg (57,9 %) Produkt als farblosen Feststoff.

¹H-NMR (300 MHz, DMSO-d₆): δ = 0.96 (3H, t), 1.01-1.28 (4H, m), 1.64-1.77 (2H, m), 1.82-1.94 (2H, m), 2.98 (2H, dq), 3.76 (3H, s), 3.87 (3H, s), 4.68 (1H, d), 5.58-5.69 (2H, m), 6.93 (2H, d), 7.16 (2H, d), 7.41 (2H, d), 7.46 (2H, d), 8.31 (1H, s).
MS (EIpos): m/z = 516 (M+H)⁺

### Beispiel 29

### N-Cycloheptyl-5,6-bis(4-methoxyphenyl)-N-methylfuro[2,3-d]pyrimidin-4-amin

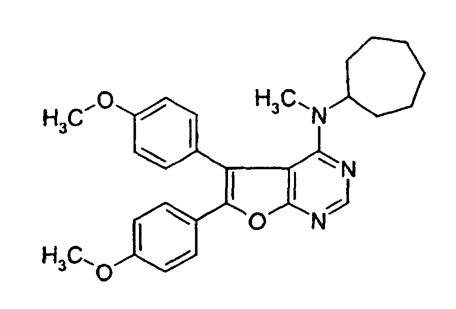

Das zur Synthese eingesetzte Cycloheptylmethylamin wurde analog folgender Literaturvorschrift hergestellt: K.A. Neidigh, M.A. Avery, J.S. Williamson, S. Bhattacharyya, *J. Chem. Soc., Perkin Trans. 1* **1998**, 2527-2531.
Zu einer Lösung von 176 mg (0,48 mmol) der Verbindung des Beispiels XV in 10 ml THF werden 398 mg (2,88 mmol) Kaliumcarbonat und 366 mg (2,88 mmol) Cycloheptylmethylamin gegeben, und die Reaktionsmischung 12 Stunden unter Rückfluss gerührt. Anschließend wird der Ansatz mit Essigsäureethylester versetzt und mit gesättigter wässriger Natriumhydrogencarbonat-Lösung gewaschen. Die abgetrennte organische Phase wird über Magnesiumsulfat getrocknet und im Vakuum eingeengt. Das Gemisch wird mittels präparativer MPLC (Säule: 100 g Kieselgel; Eluent: Cyclohexan/Essigsäureethylester 10:1; Fluss 80 ml/min; UV-Detektion bei 210 nm) aufgetrennt. Nach Konzentration im Vakuum erhält man 180 mg (82 %) des Produkts als farblosen Feststoff.

¹H-NMR (300 MHz, DMSO-d₆): δ = 1.06-1.70 (12H, m), 2.47 (3H, s), 3.74 (3H, s), 3.83 (3H, s), 4.08-4.23 (1H, m), 6.91 (2H, d), 7.07 (2H, d), 7.27-7.36 (4H, m), 8.33 (1H, s).
MS (EIpos): m/z = 458.3 (M+H)⁺

### Beispiel 30

### rac-4-[cis-3,5-Dimethyl-1-piperazinyl]-5,6-bis(4-methoxyphenyl)furo[2,3-d]-pyrimidin

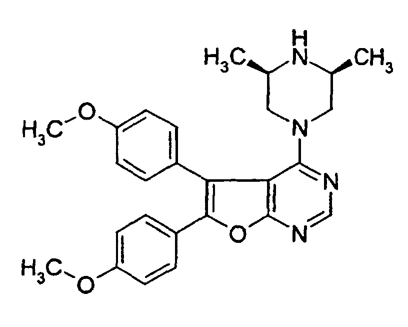

Analog zur Herstellungsvorschrift für Beispiel 29 werden zu einer Lösung von 80 mg (0,22 mmol) der Verbindung des Beispiels XV in 3 ml THF 181 mg (1,31 mmol) Kaliumcarbonat und 100 mg (0,87 mmol) cis-2,6-Dimethylpiperazin gegeben, und die Reaktionsmischung 12 h unter Rückfluss gerührt. Nach Aufarbeitung wird das Gemisch mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) getrennt. Man erhält 35 mg (36 %) des Produkts als farblosen Feststoff.

¹H-NMR (300 MHz, DMSO-d₆): δ = 0.70 (6H, d), 2.09 (2H, dd), 2.30-2.59 (3H, m), 3.66 (2H, dd), 3.77 (3H, s), 3.82 (3H, s), 6.92 (2H, d), 7.08 (2H, d), 7.28-7.47 (4H, m), 8.48 (1H, s).
MS (EIpos): m/z = 445.4 (M+H)⁺

### Beispiel 31

### rac-1-[6-{4-[cis-3,5-Dimethyl-1-piperazinyl]phenyl}-5-(4-fluorphenyl)furo[2,3-d]-pyrimidin-4-yl]-4-piperidinol

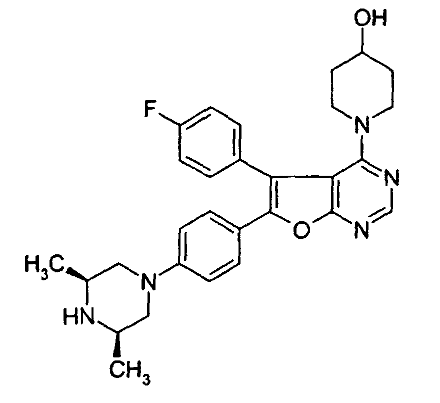

Analog zur Herstellungsvorschrift für Beispiel 14 werden 70 mg (0,22 mmol) der Verbindung des Beispiels 23, 4,1mg (0,004 mmol) Tris(dibenzylidenaceton)dipalladium, 9,3 mg (0,015 mmol) rac-2,2'-Bis(diphenylphosphino)-1,1'-binaphthyl und 20,1 mg (0,18 mmol) Kalium-tert-butylat in 6 ml Toluol vorgelegt. Nach Zugabe von 51 mg (0,45 mmol) cis-2,6-Dimethylpiperazin wird 36 h bei 60°C gerührt. Die Reaktionslösung wird mit Essigsäureethylester verdünnt und mit wässriger gesättigter Natriumhydrogencarbonat-Lösung gewaschen. Die wässrige Phase wird mit Dichlormethan extrahiert und die organischen Phasen vereinigt. Nach Entfernen des Lösungsmittels im Vakuum erhält man 42 mg (56 %) des Produkts als farblosen Feststoff.

¹H-NMR (300 MHz, DMSO-d₆): δ = 0.92-1.49 (2H, m), 1.01 (6H, d), 1.37-1.49 (2H, m), 2.14 (2H, dd), 2.54-2.60 (1H, m), 2.70-2.92 (4H, m), 3.43-3.58 (3H m), 3.62 (2H, dd), 4.58 (1H, d), 6.87 (2H, d), 7.20 (2H, d), 7.29-7.50 (4H, m), 8.38 (1H, s).
MS (EIpos): m/z = 502.3 (M+H)⁺

### Beispiel 32

### N-{4-[4-(4-methyl-1-piperazinyl)-5-phenylfuro[2,3-d]pyrimidin-6-yl]phenyl}-nicotinamid

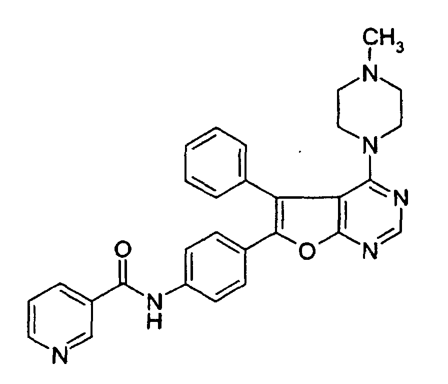

Analog zur Herstellung von Beispiel 24 werden 2,0 mg (0,002 mmol) Tris(dibenzylidenaceton)dipalladium, 5,2 mg (0,009 mmol) 9,9-Dimethyl-4,5-bis(diphenylphosphino)xanthen, 32,6 mg (0,27 mmol) Nicotinsäureamid und 102 mg (0,31 mmol) Cäsiumcarbonat in 0,25 ml trockenem Dioxan vorgelegt. Nach Zugabe von 100,0 mg (0,22 mmol) der Verbindung aus Beispiel 22 wird 30 h bei 100°C gerührt. Nach üblicher Aufarbeitung und Reinigung mittels präparativer HPLC (Säule: YMC Gel ODS-AQ S-11 µm, 250 x 30 mm; Eluent: Acetonitril/Wasser; Fluss: 50 ml/min; UV-Detektion bei 210 nm) erhält man 19 mg (17 %) des Produkts in Form eines farblosen Feststoffs.

¹H-NMR (300 MHz, DMSO-d₆): δ = 1.94-2.08 (4H, m), 2.05 (3H, s), 3.12-3.23 (4H, m), 7.34-7.62 (8H, m), 7.75 (2H, d), 8.34-8.40 (1H, m), 8.44 (1H, s), 8.73-8.79 (1H, m), 9.08 (1H, s), 10.54 (1H, s).
MS (EIpos): m/z = 491.4 (M+H)⁺

### Beispiel 33

### N-{4-[5-(4-Fluorphenyl)-4-(4-methyl-1-piperazinyl)furo[2,3-d]pyrimidin-6-yl]-phenyl}-N-methylamin

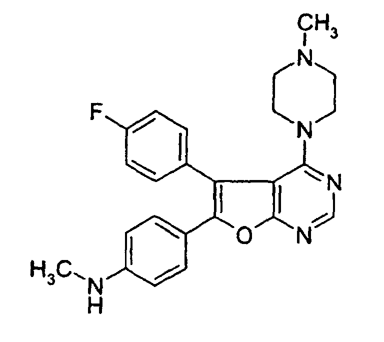

Analog der Reaktion zu Beispiel 24 werden 100,0 mg (0,21 mmol) der Verbindung aus Beispiel 21 mit 15,2 mg (0,26 mmol) *N*-Methylformamid umgesetzt. Es wird jedoch 40 h bei 100°C gerührt. Nach üblicher Aufarbeitung erhält man 39 mg (43 %) des decarbonylierten Amidierungsprodukts in Form eines farblosen Feststoffs.

¹H-NMR (300 MHz, DMSO-d₆): δ = 1.93-2.08 (4H, m), 2.06 (3H, s), 2.67 (3H, d), 3.05-3.19 (4H, m), 6.10-6.18 (1H, m), 6.48 (2H, d), 7.13 (2H, d), 7.28-7.41 (2H, m), 7.41-7.51 (2H, m), 8.39 (1H, s)
MS (ESIpos): m/z = 418.4 (M+H)⁺.

Die in der folgenden Tabelle aufgeführten Beispiele 34 bis 184 werden in Analogie zu den oben aufgeführten Vorschriften hergestellt:

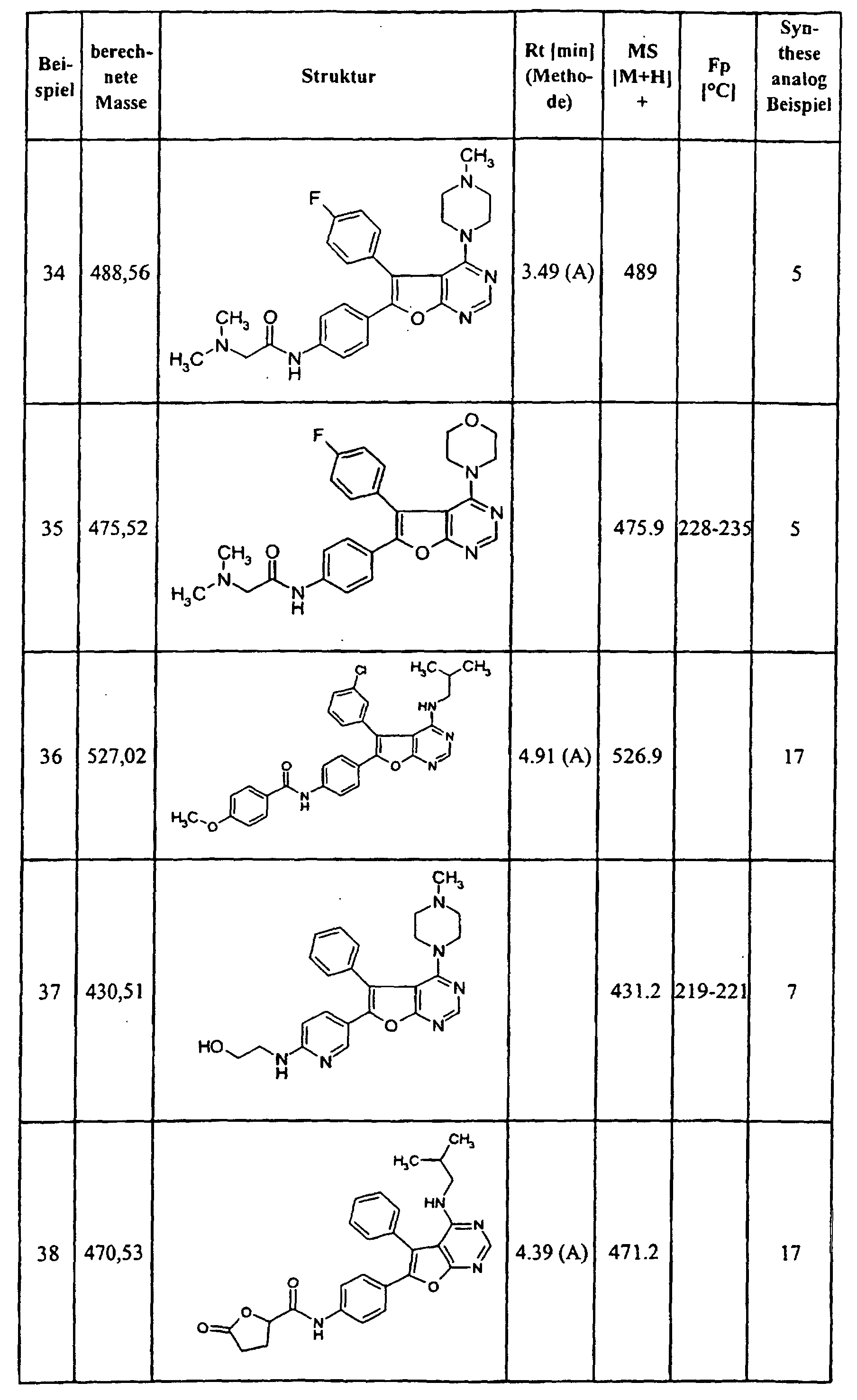

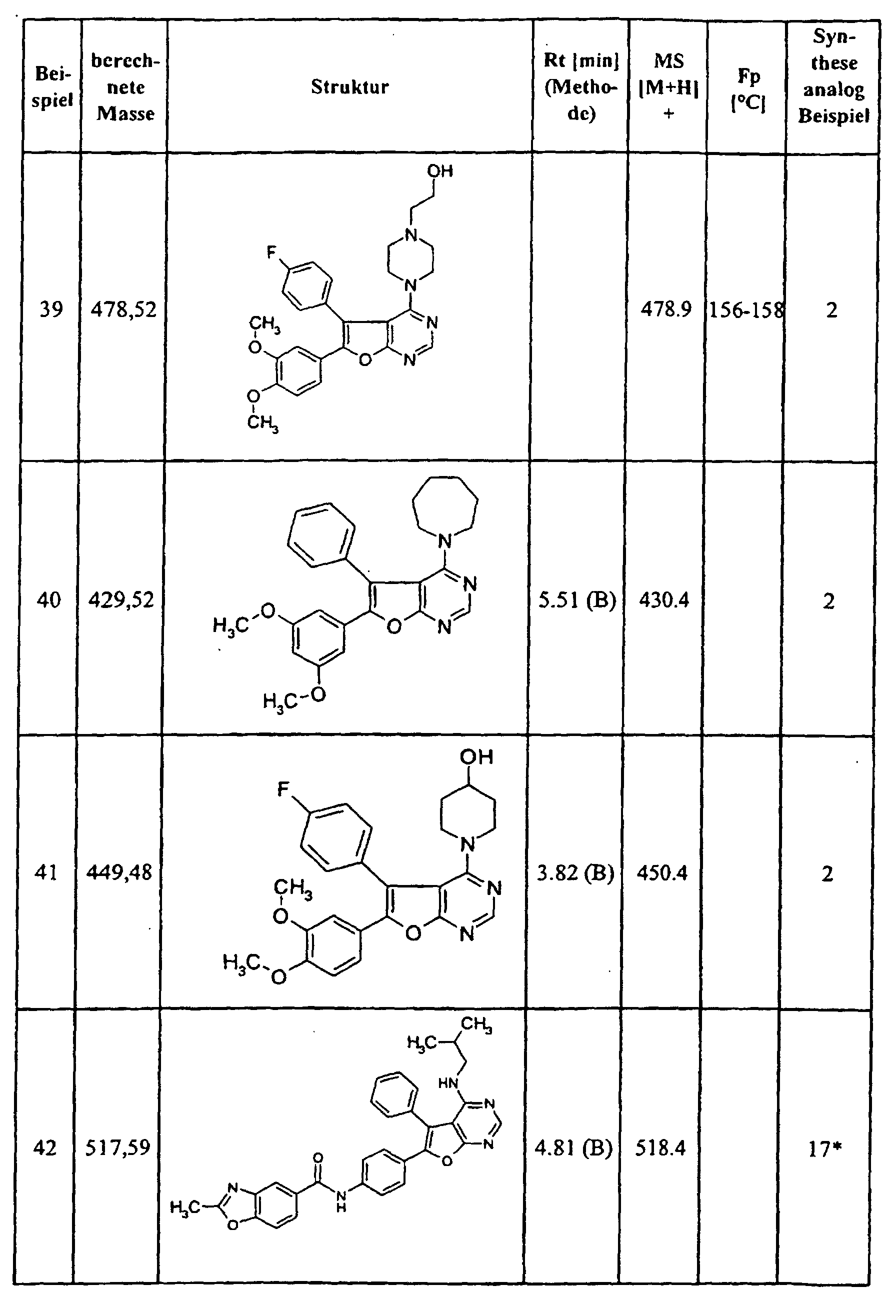

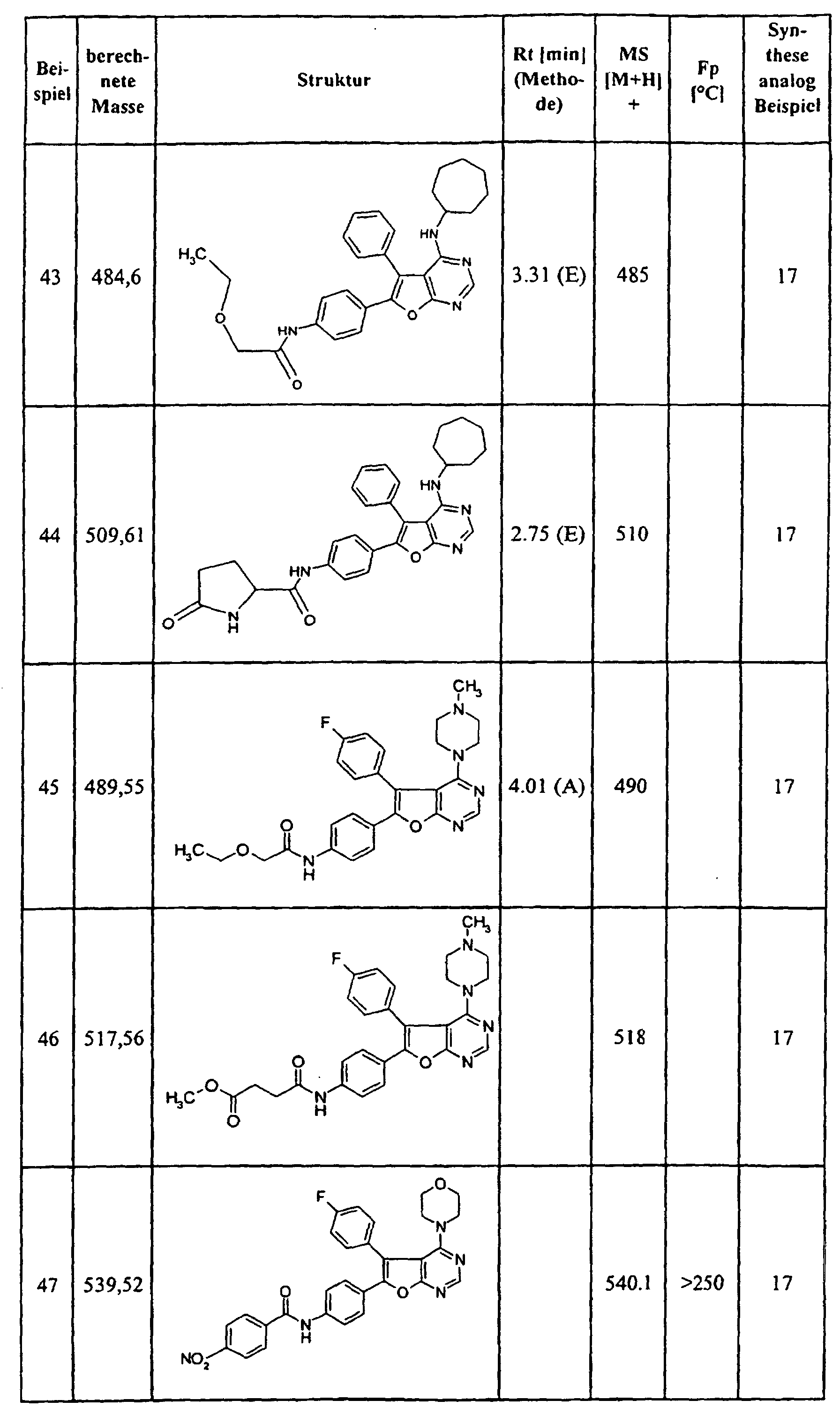

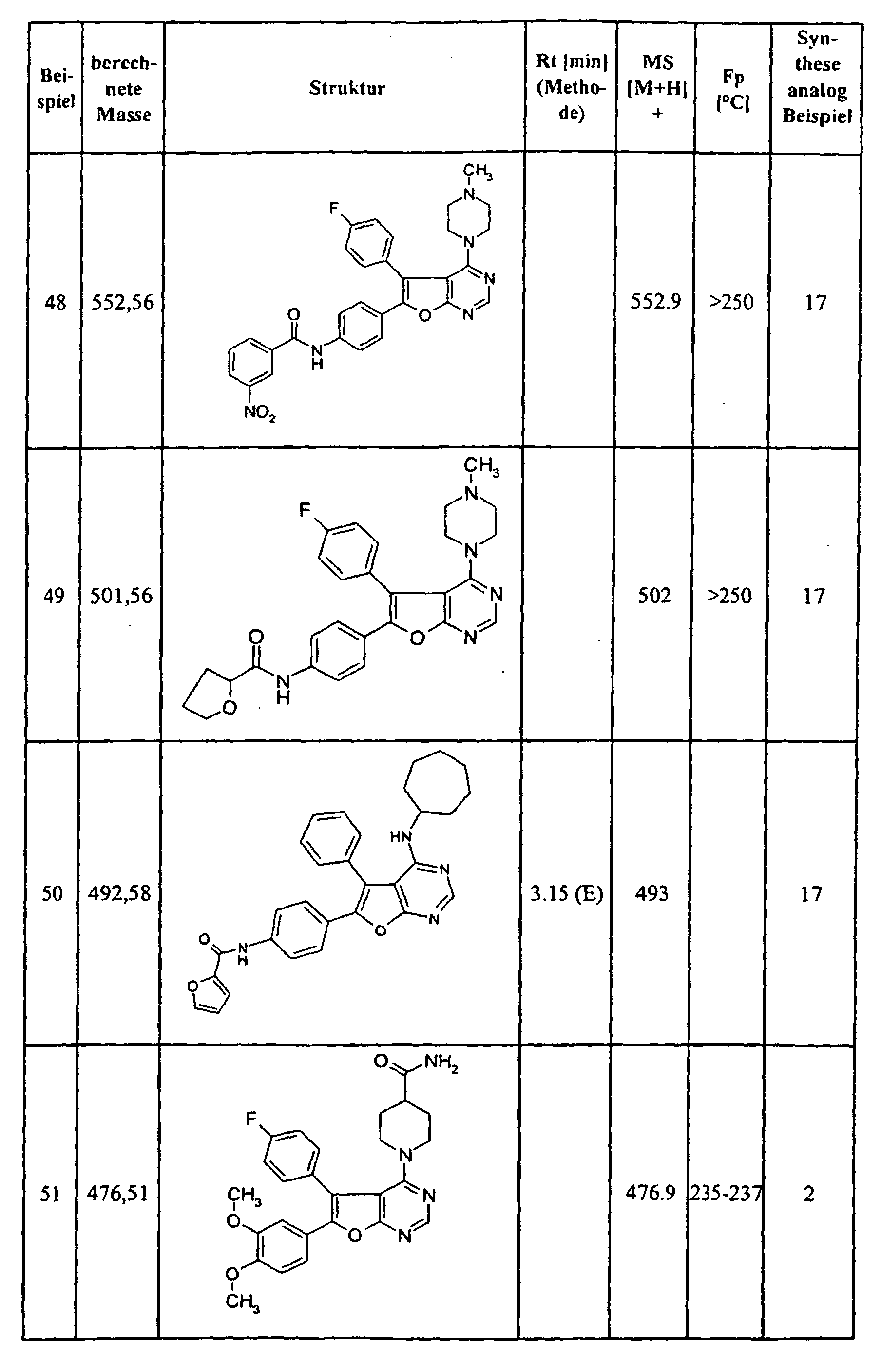

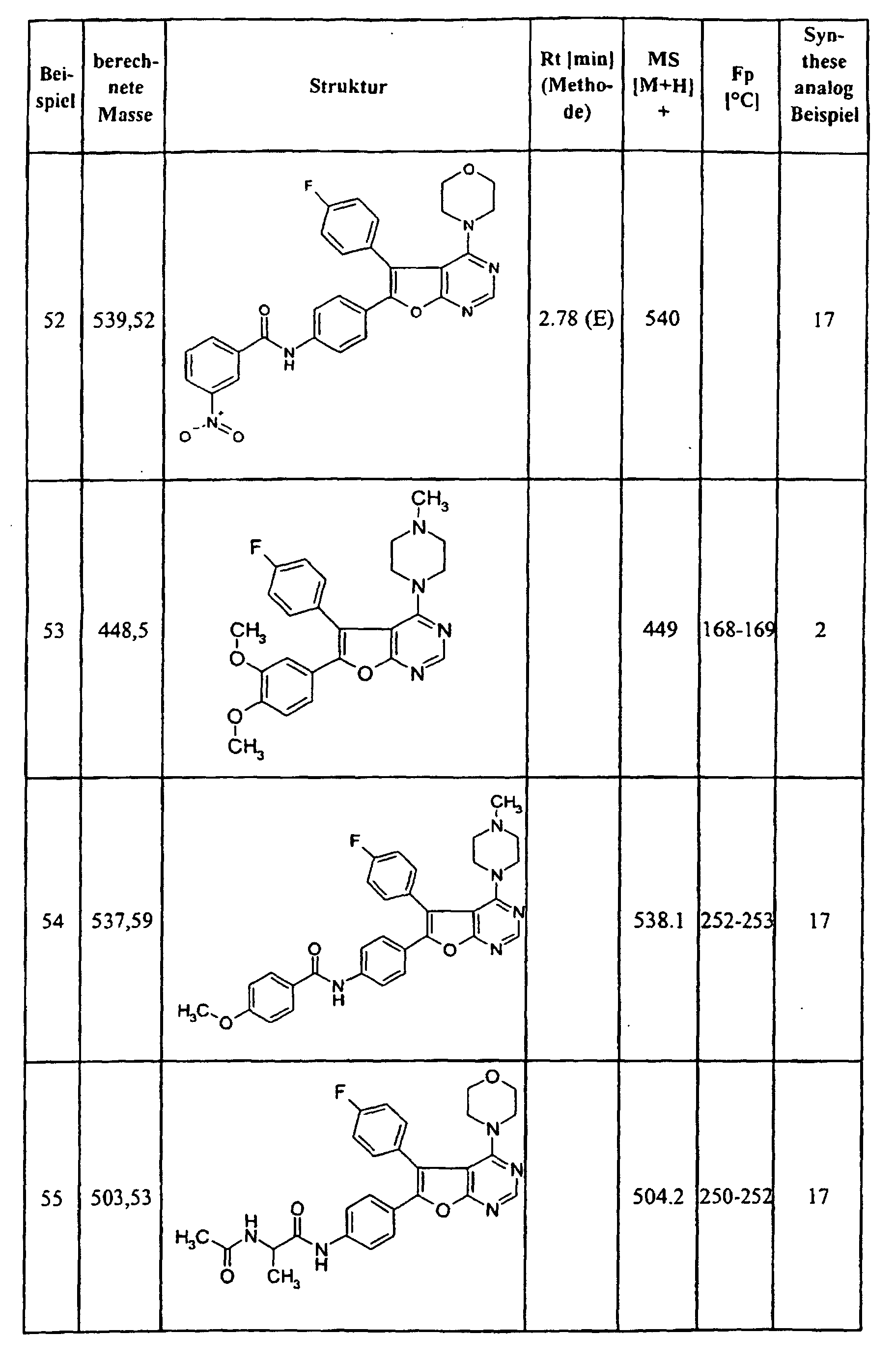

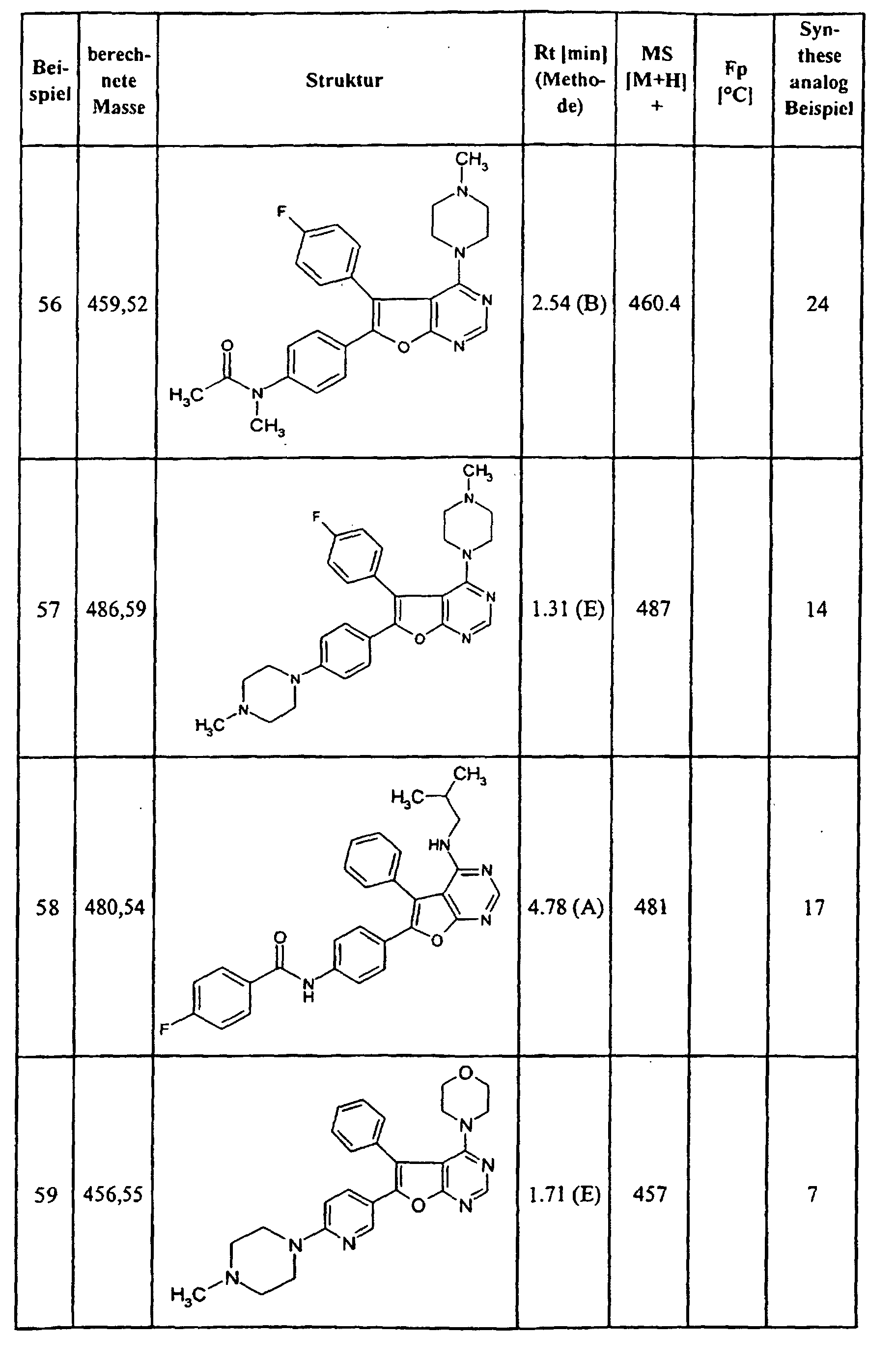

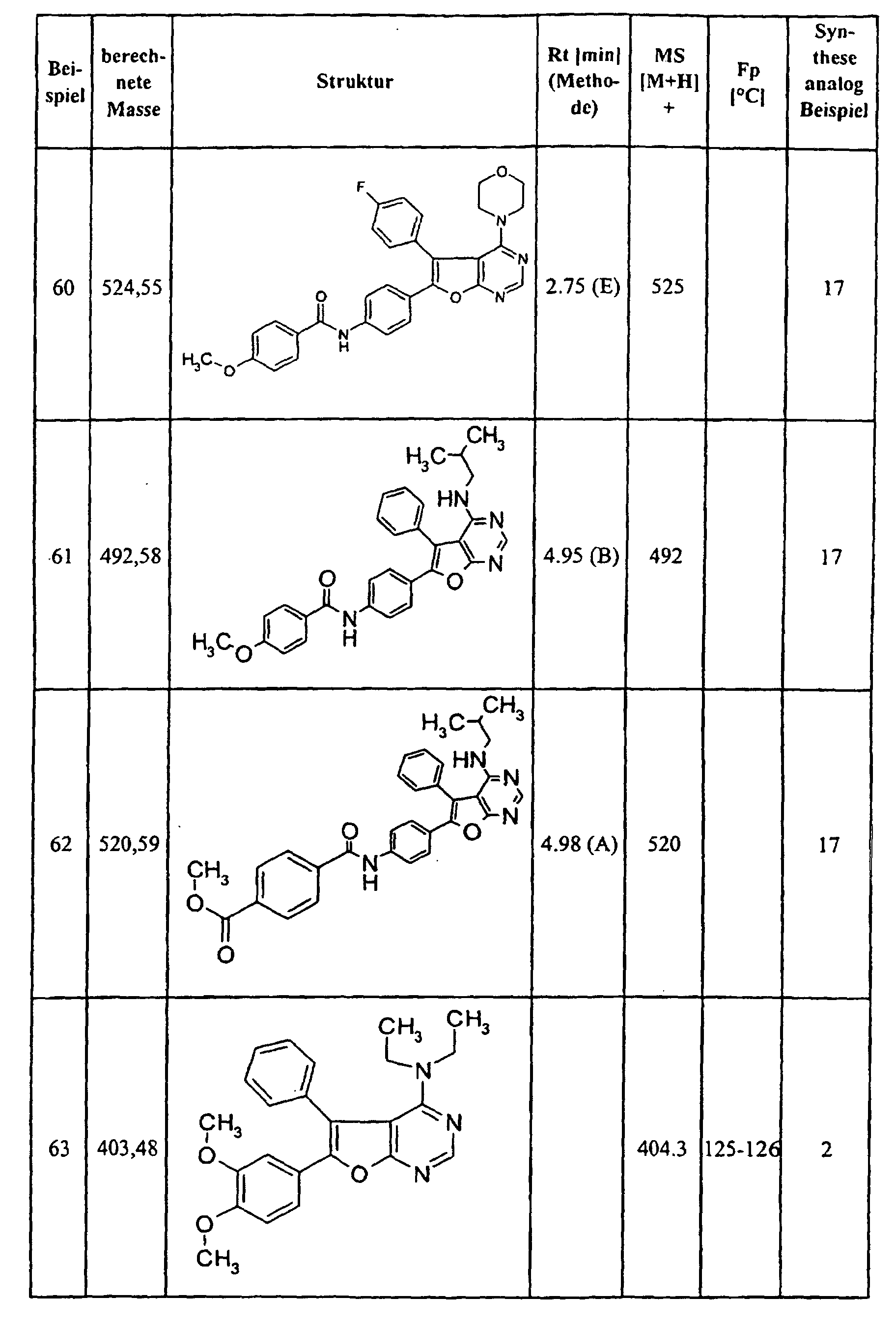

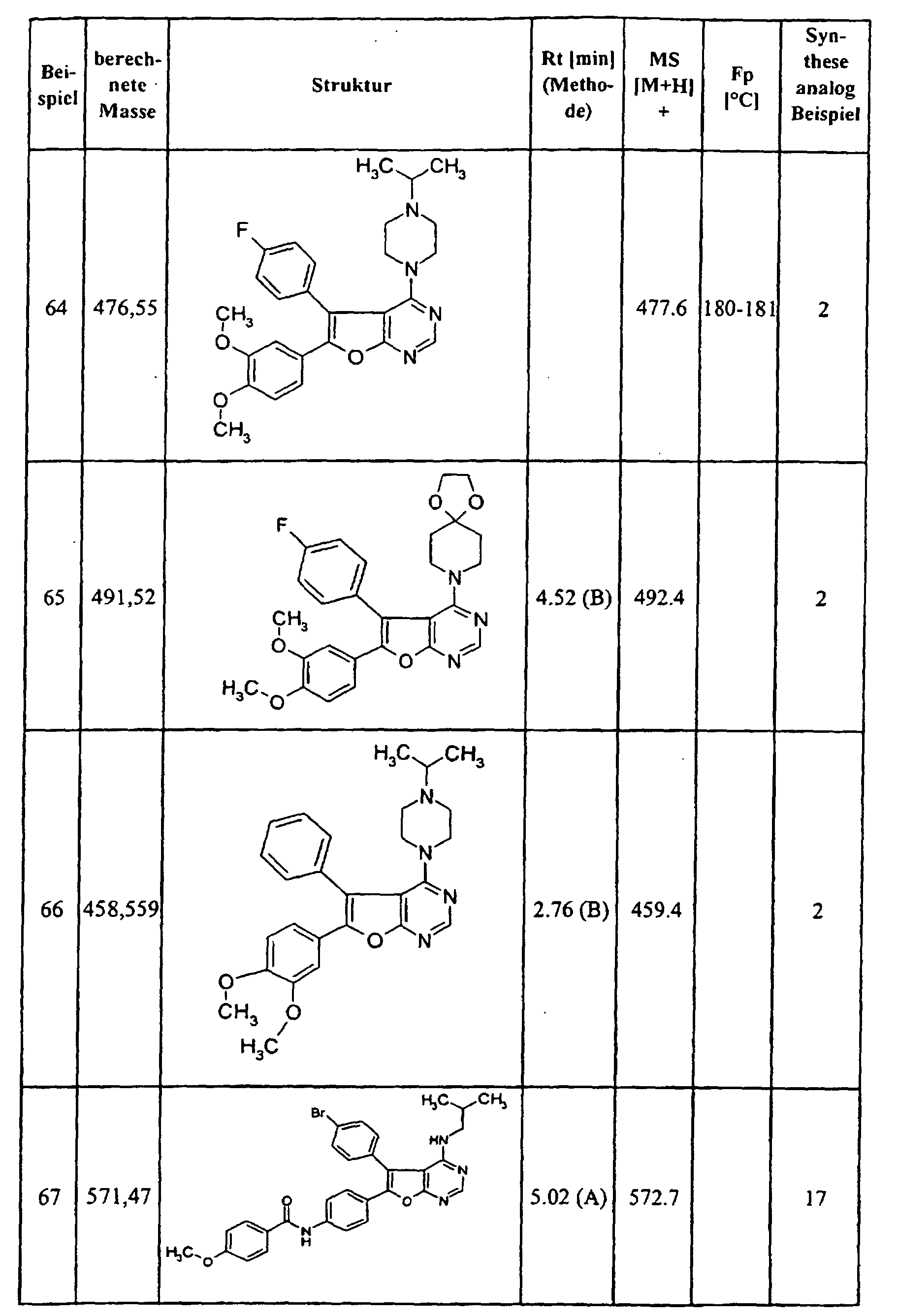

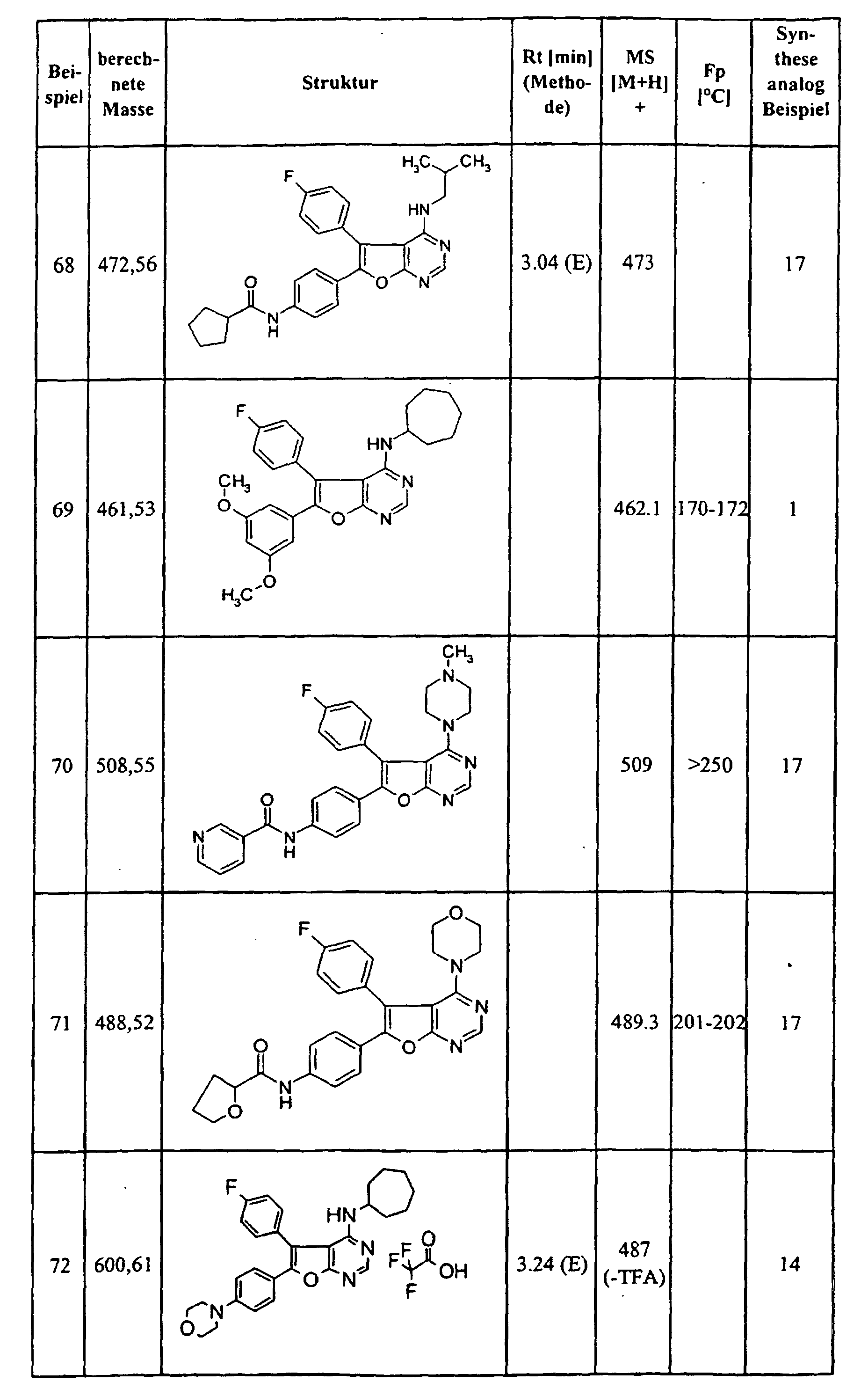

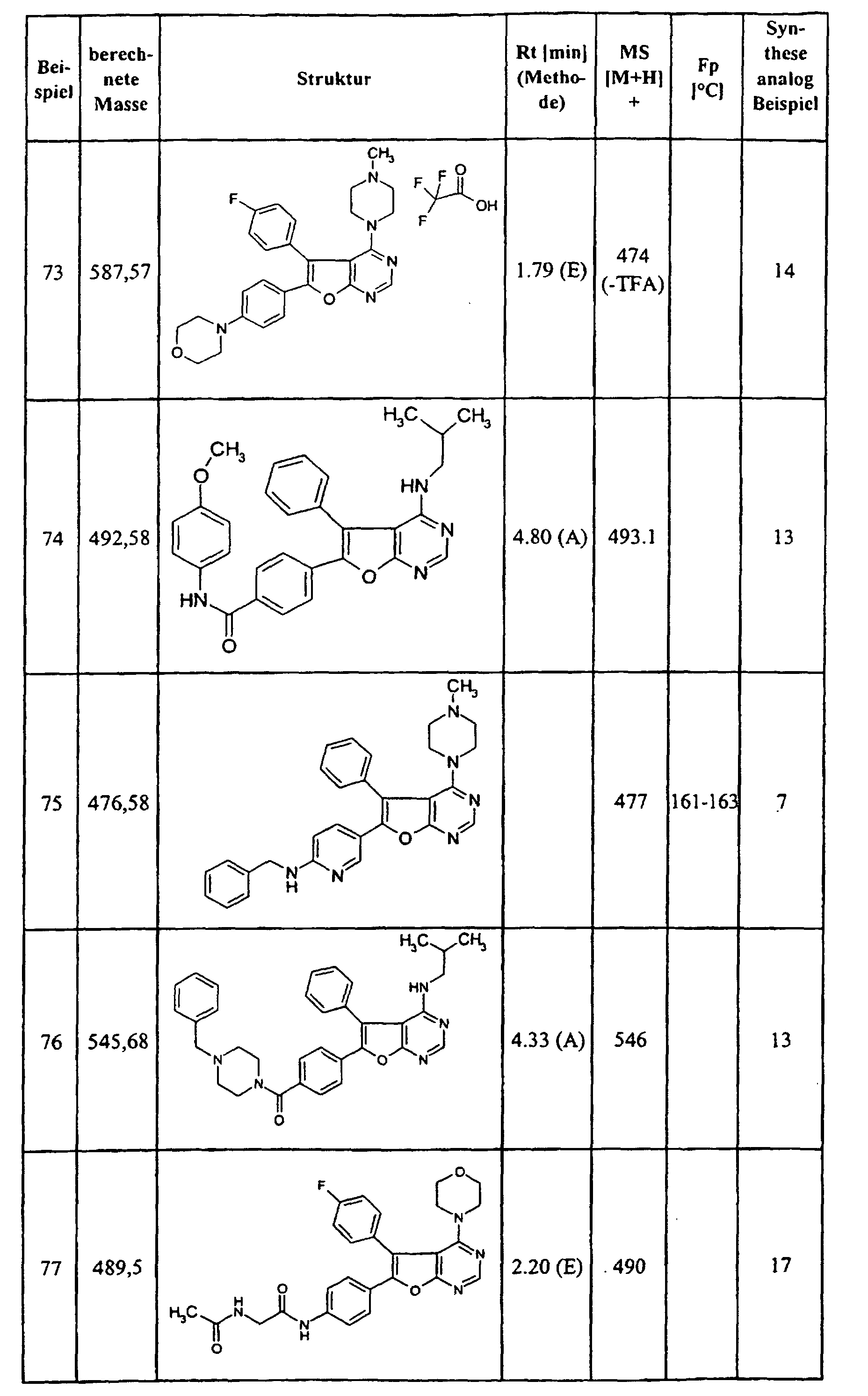

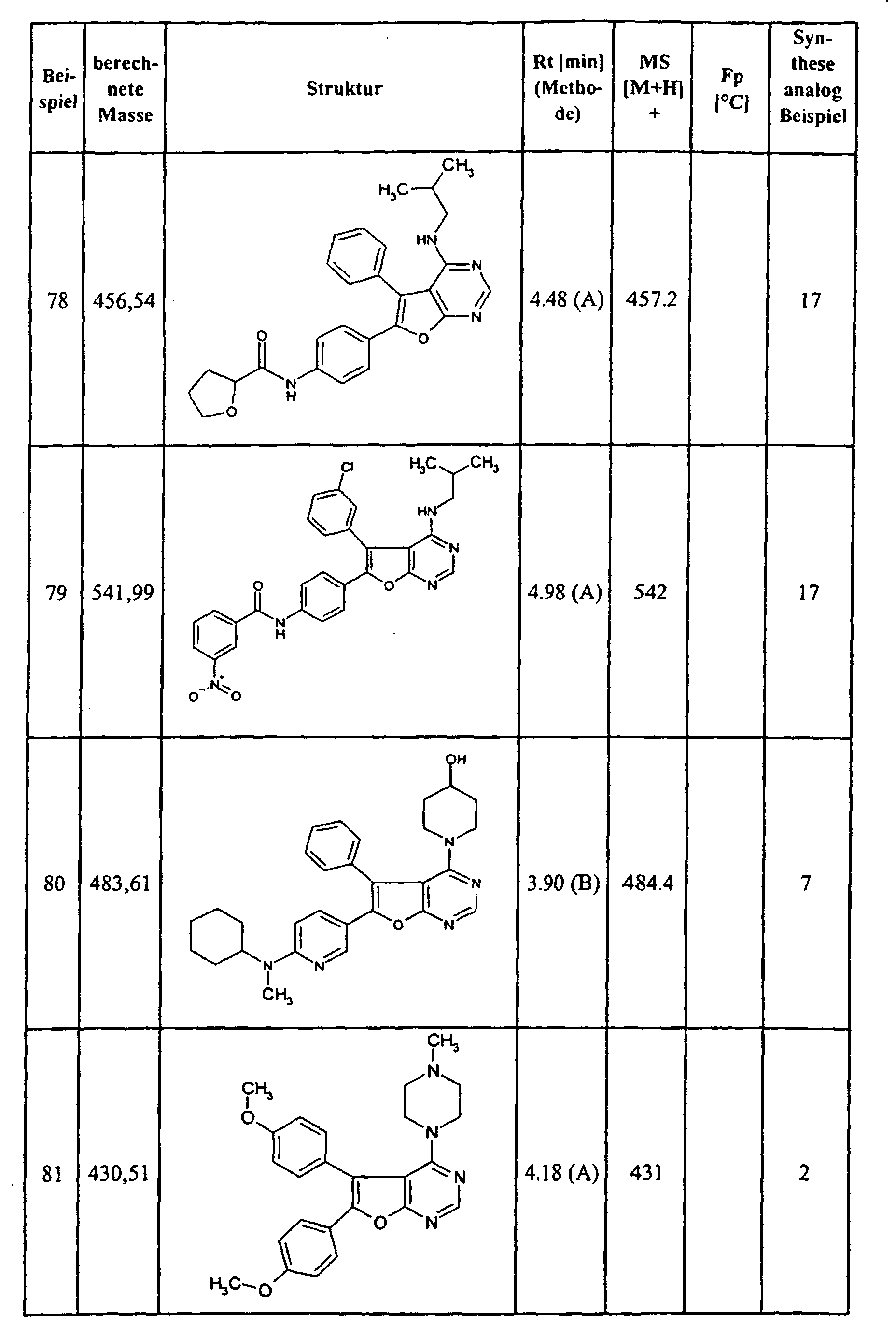

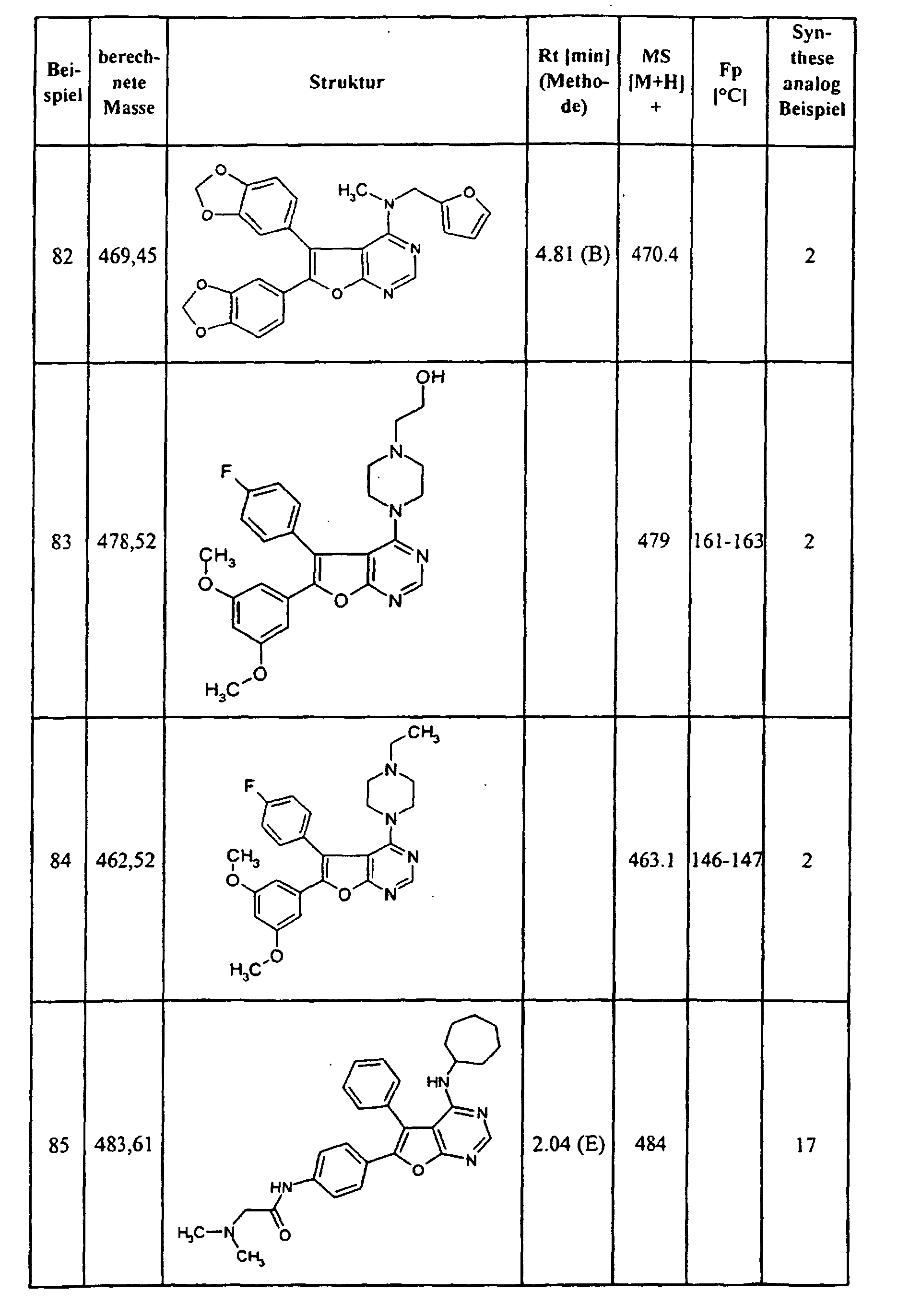

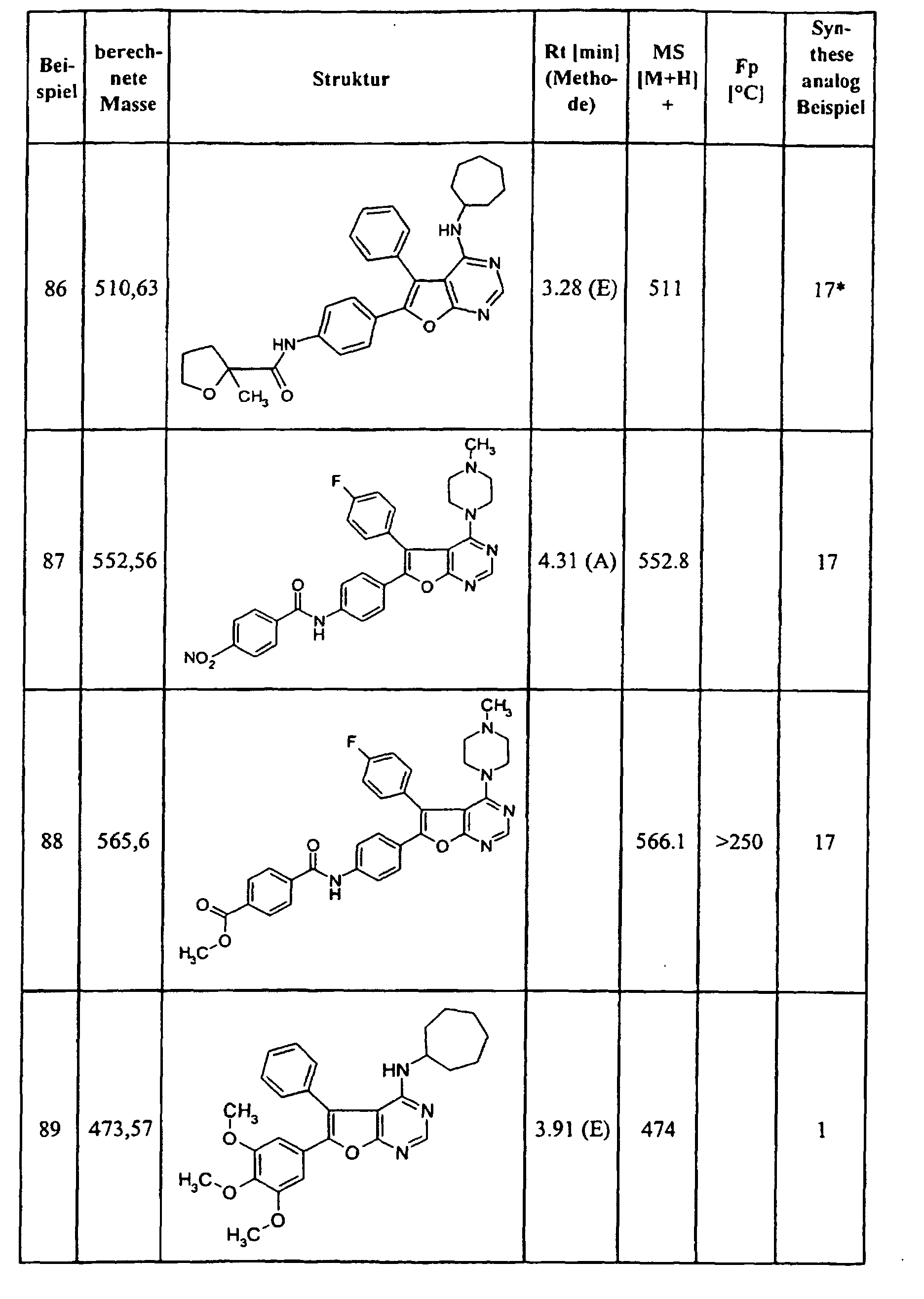

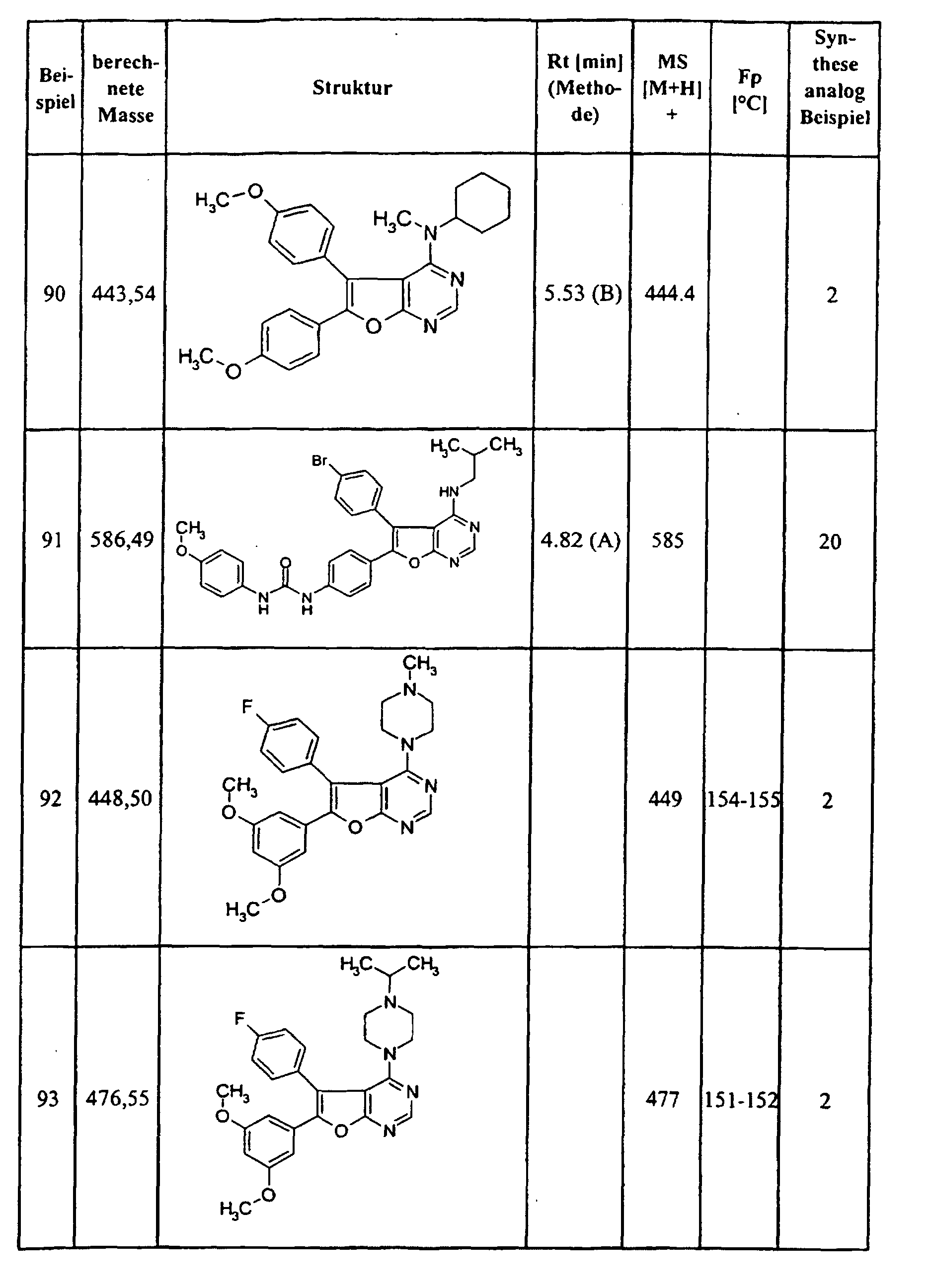

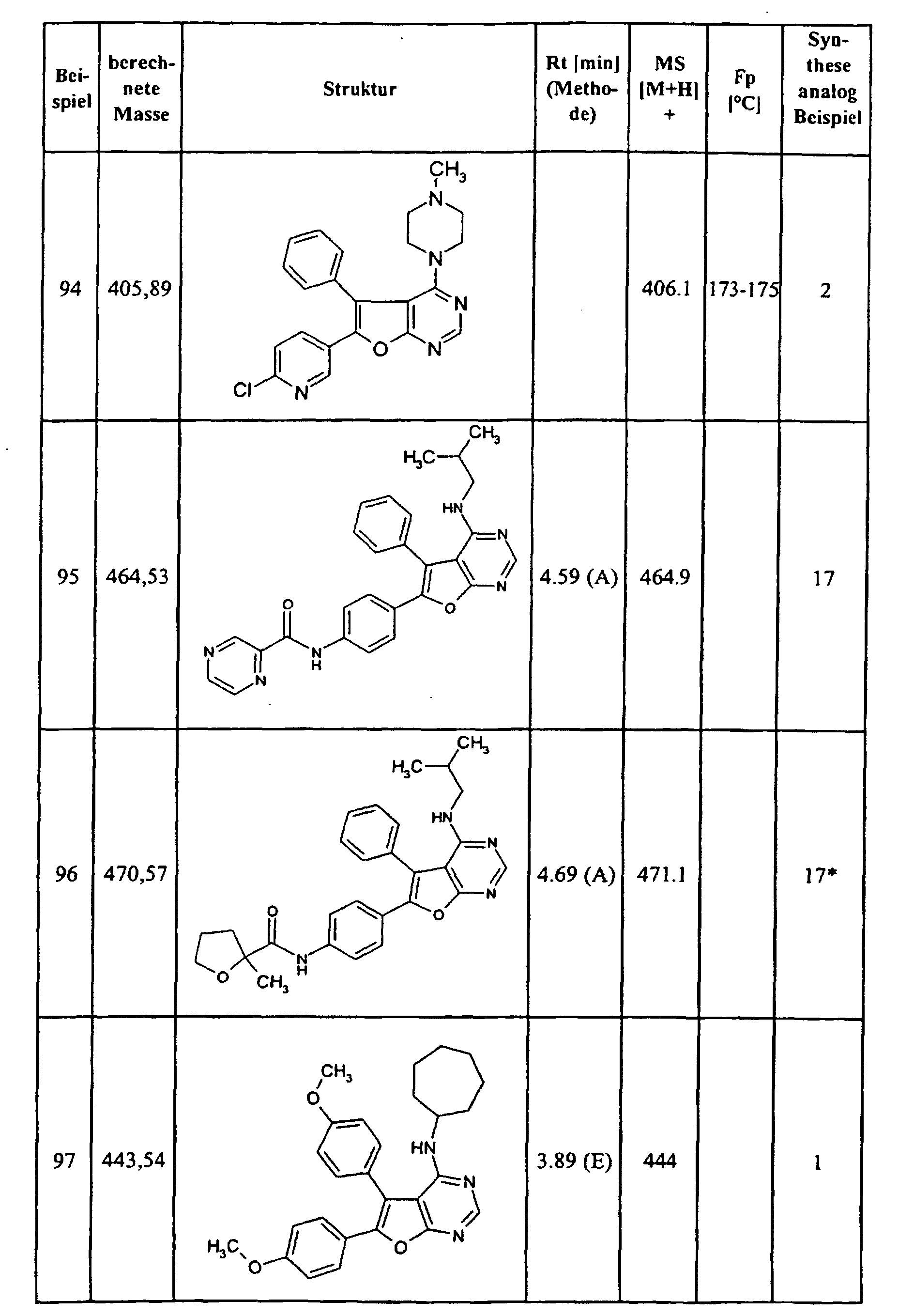

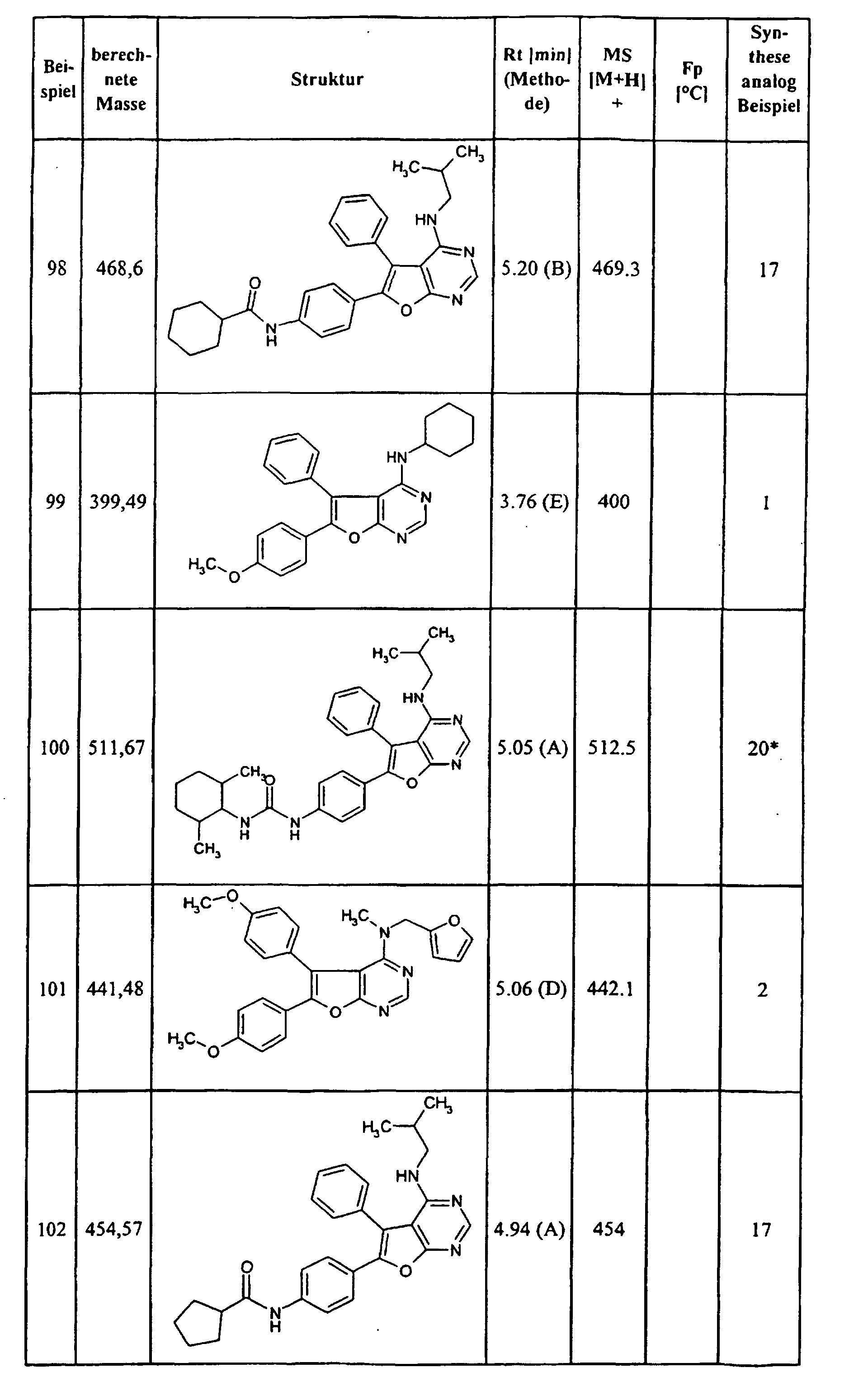

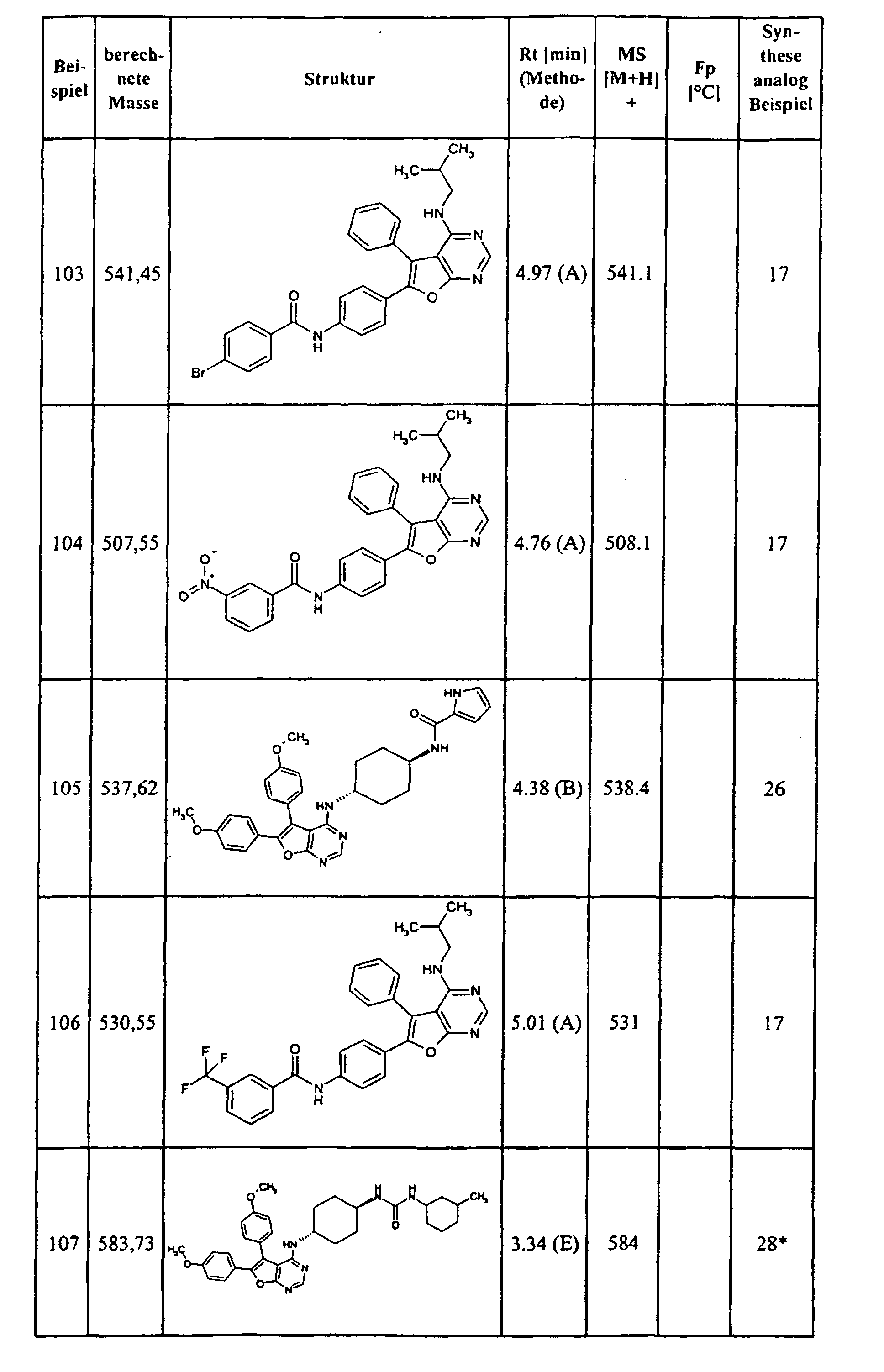

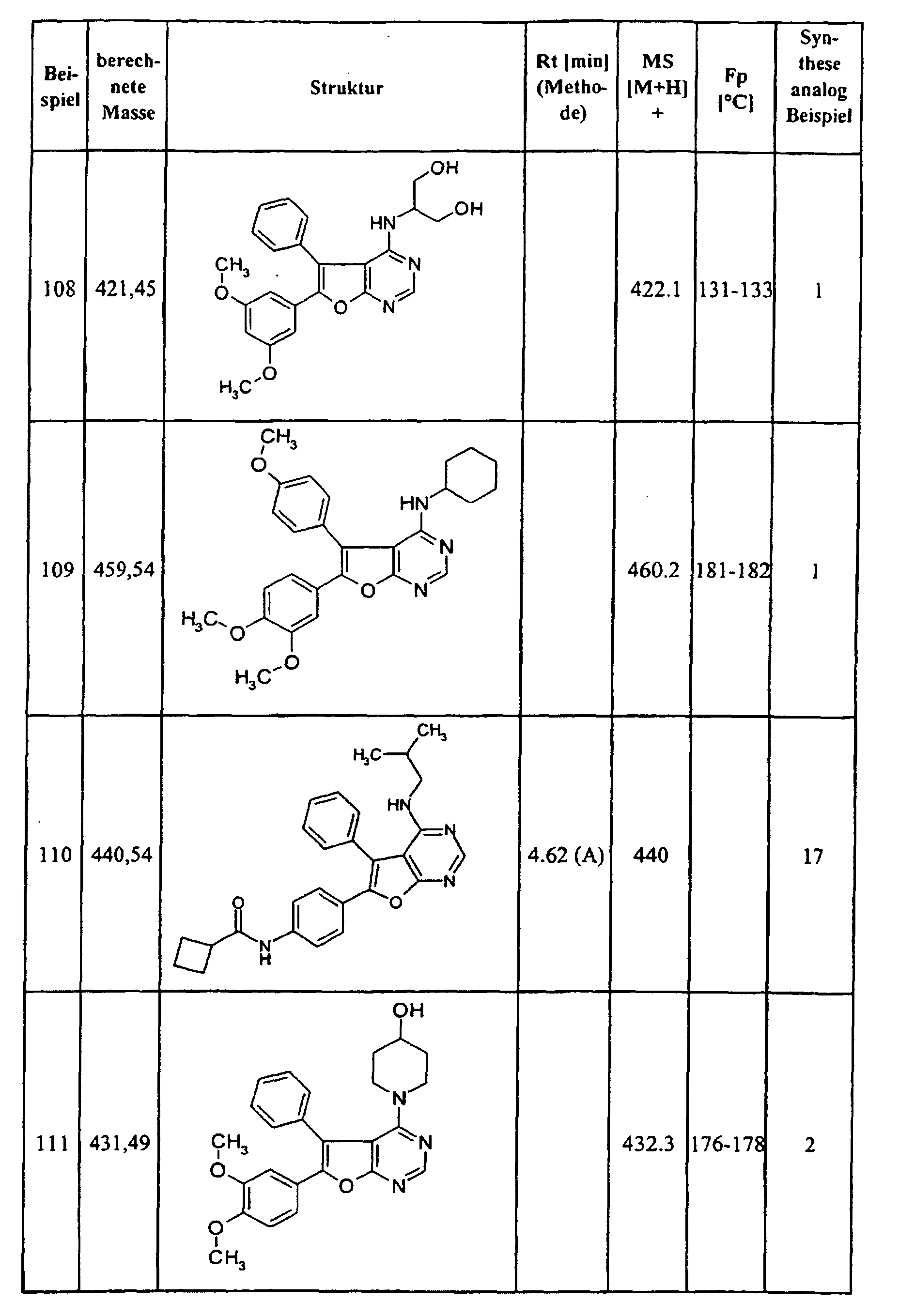

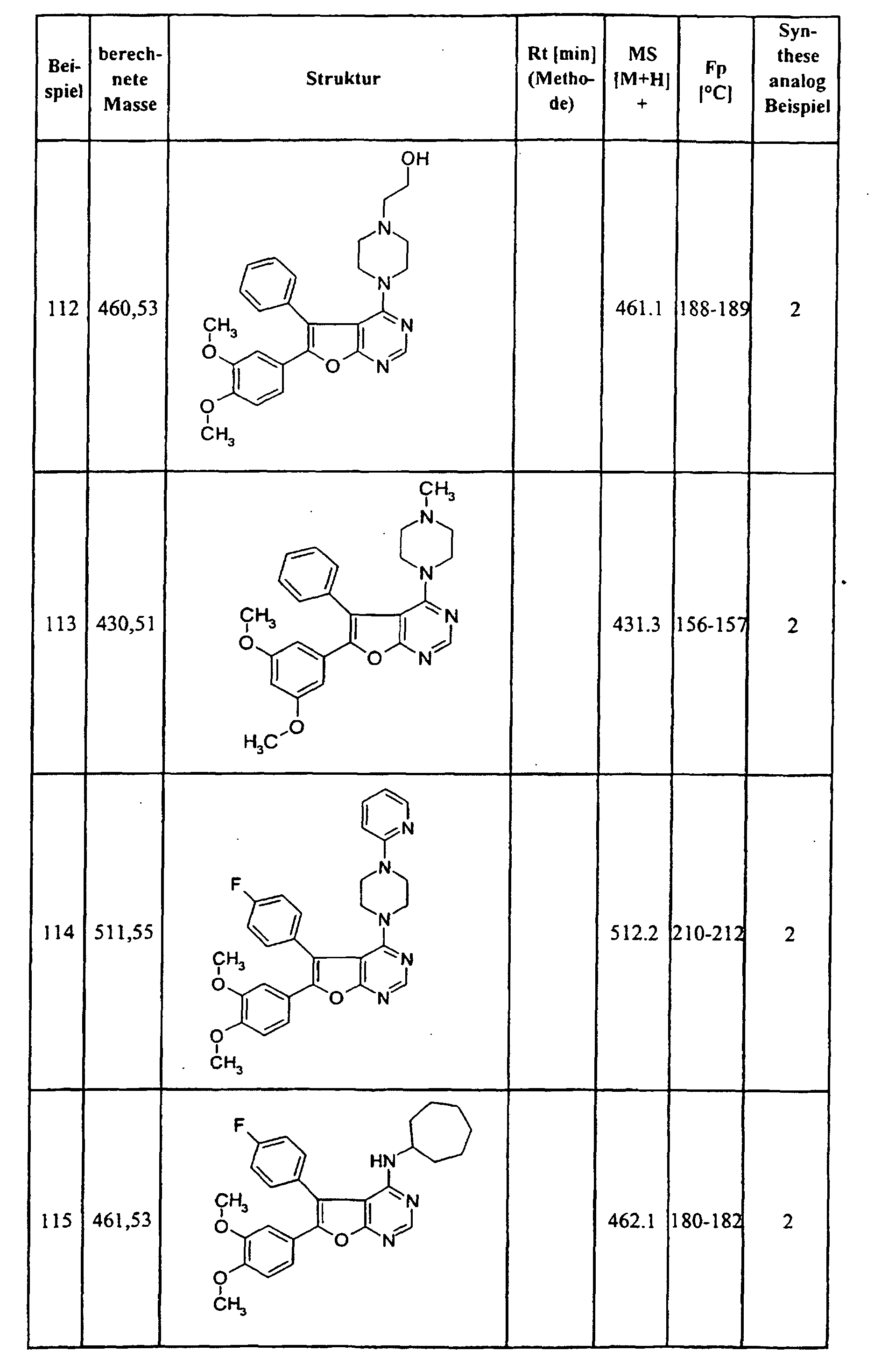

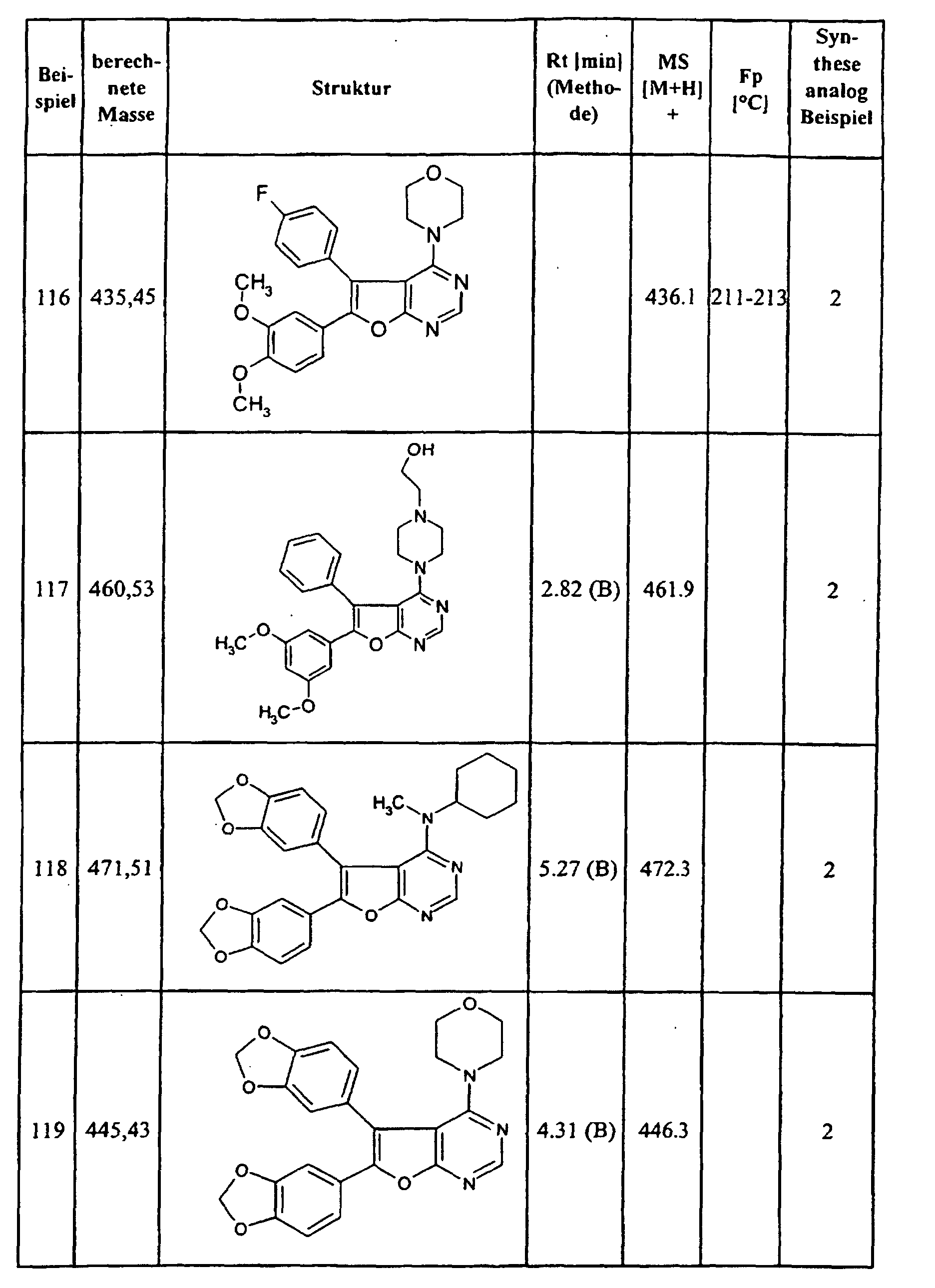

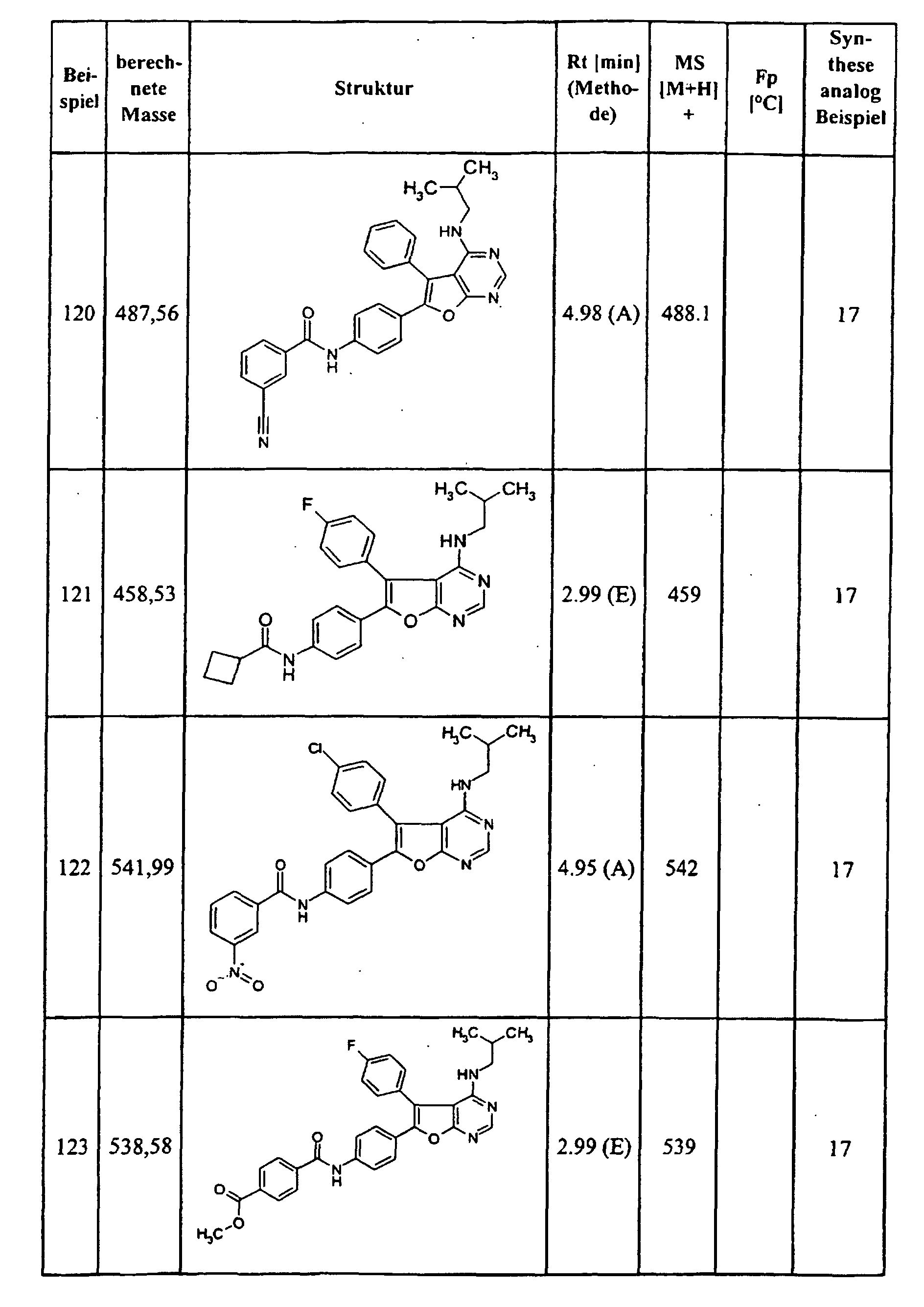

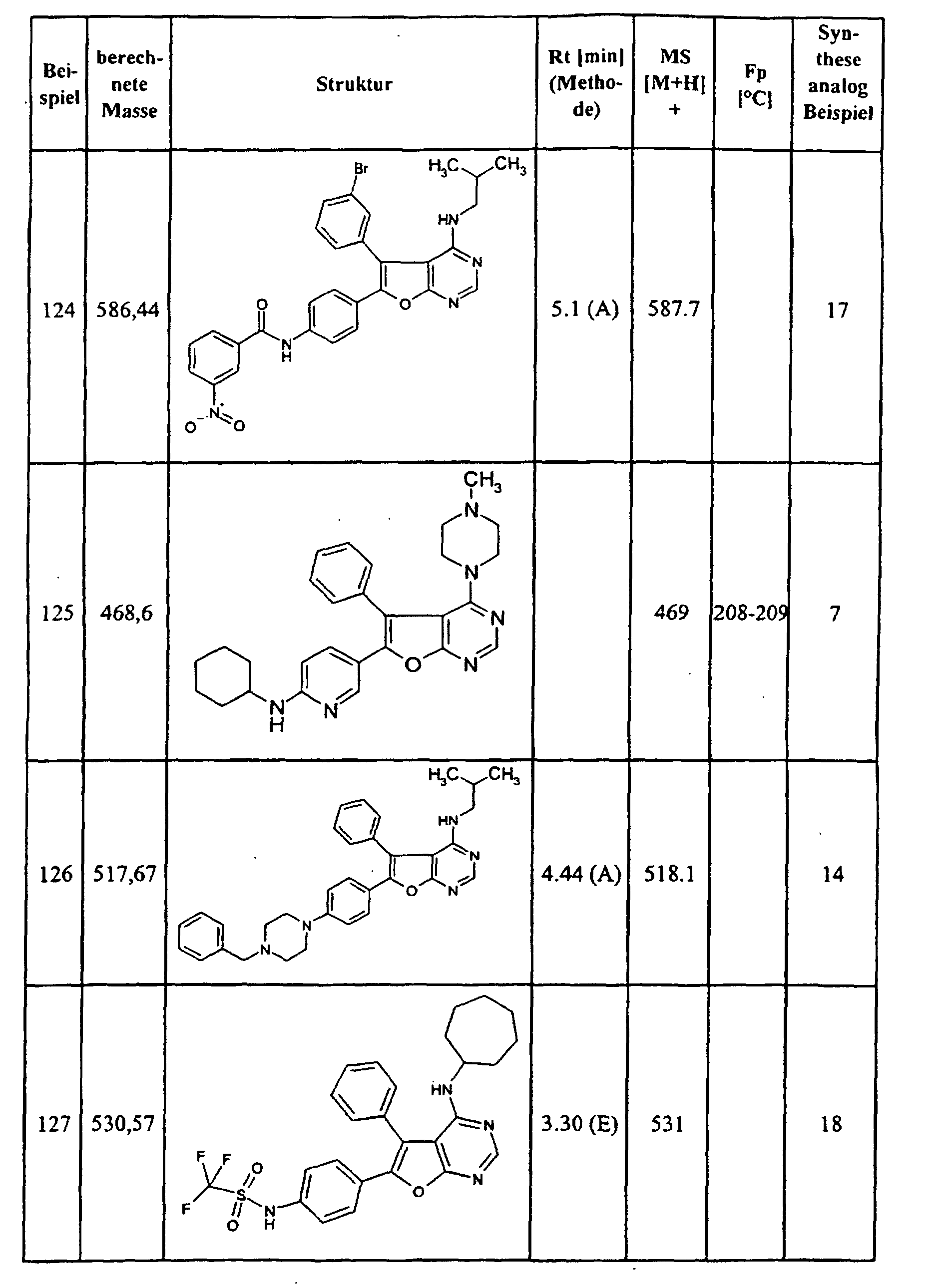

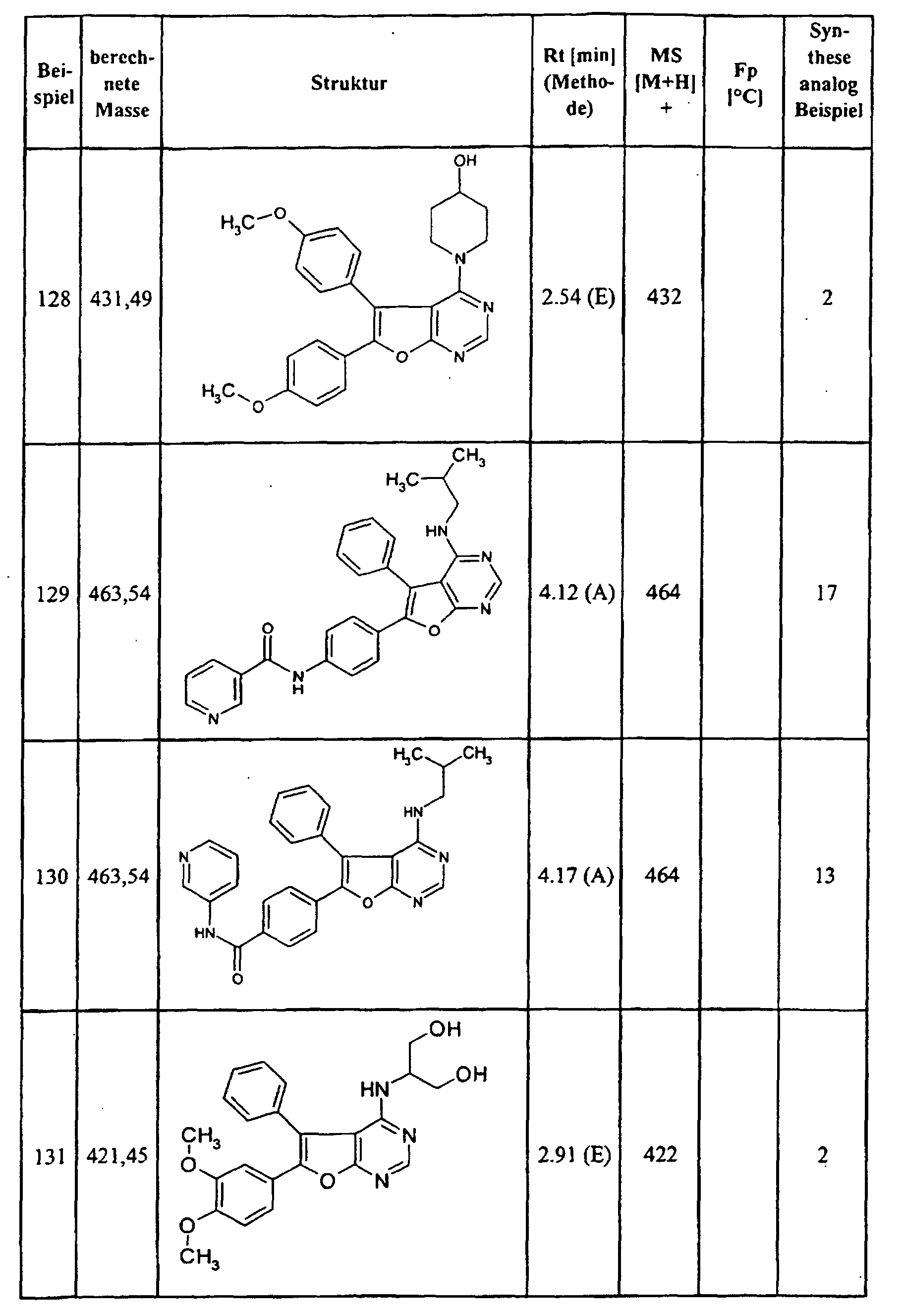

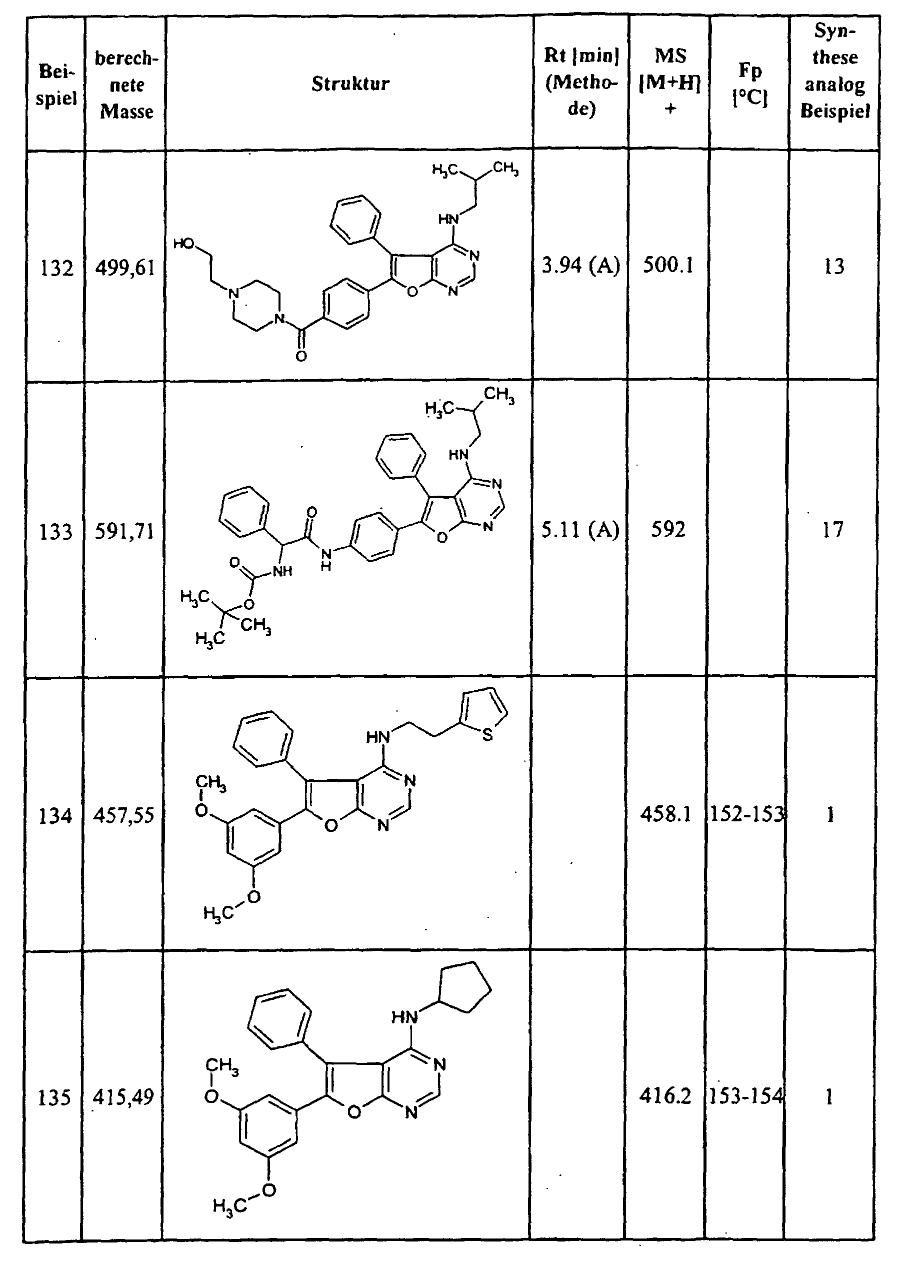

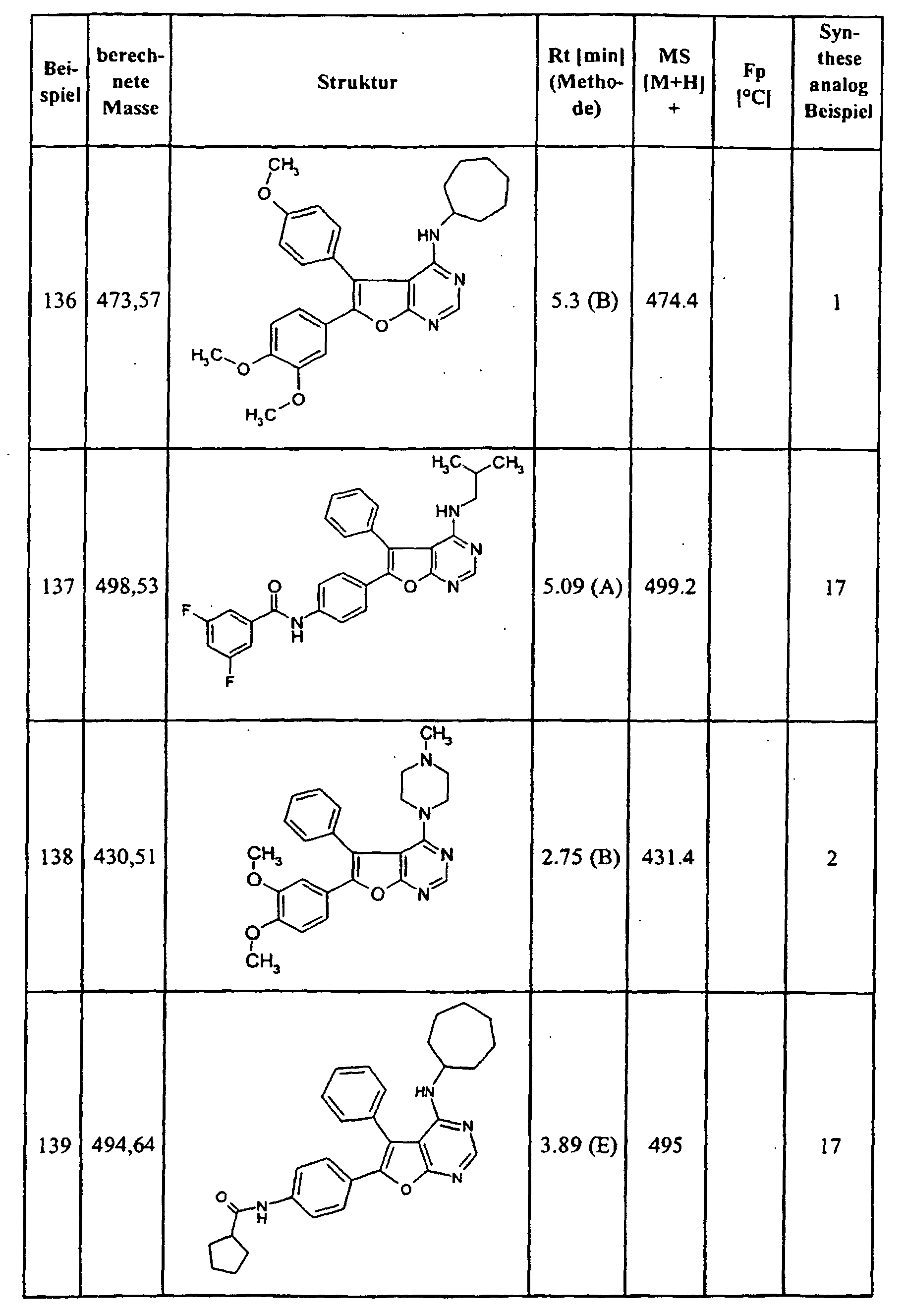

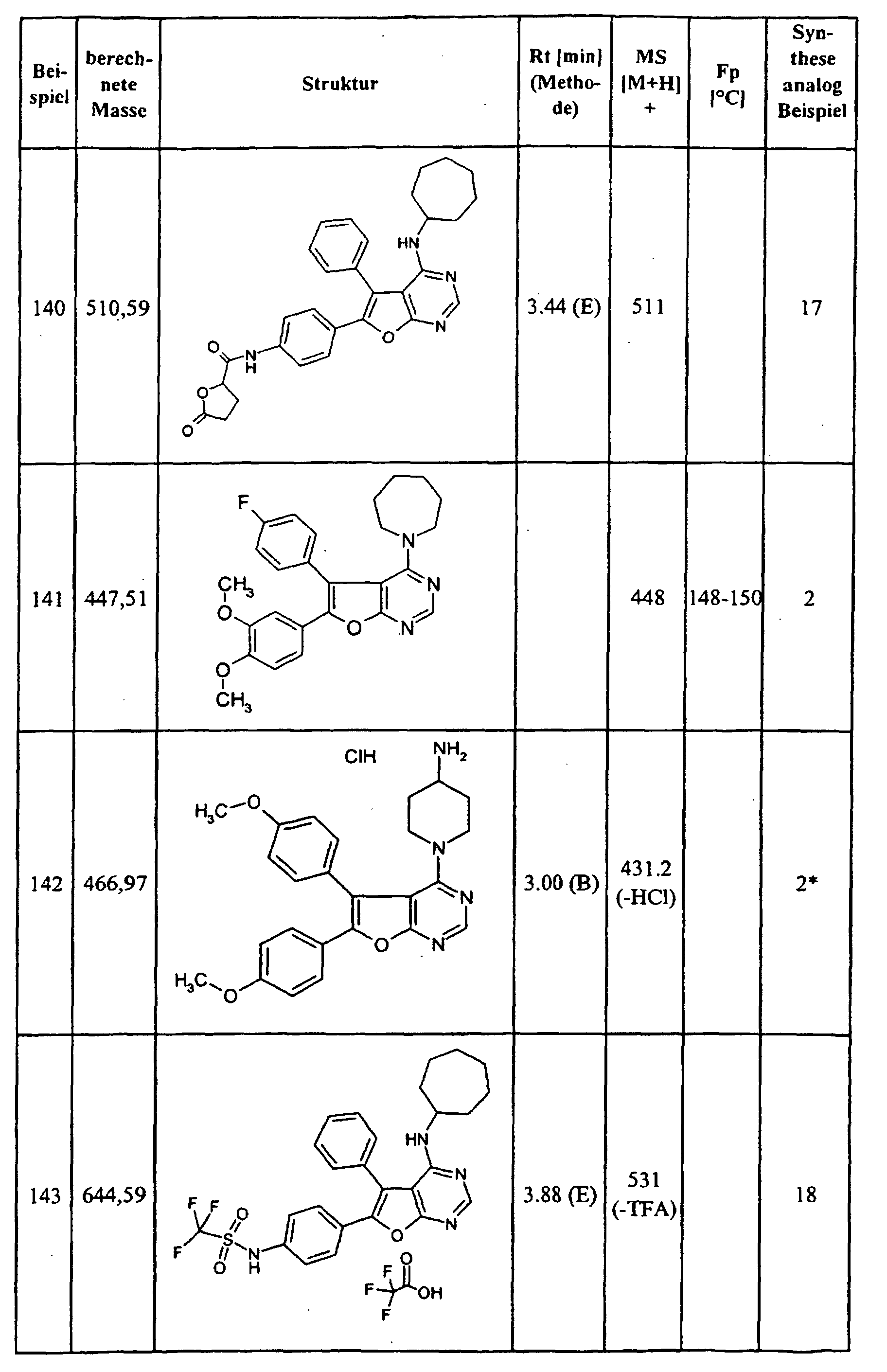

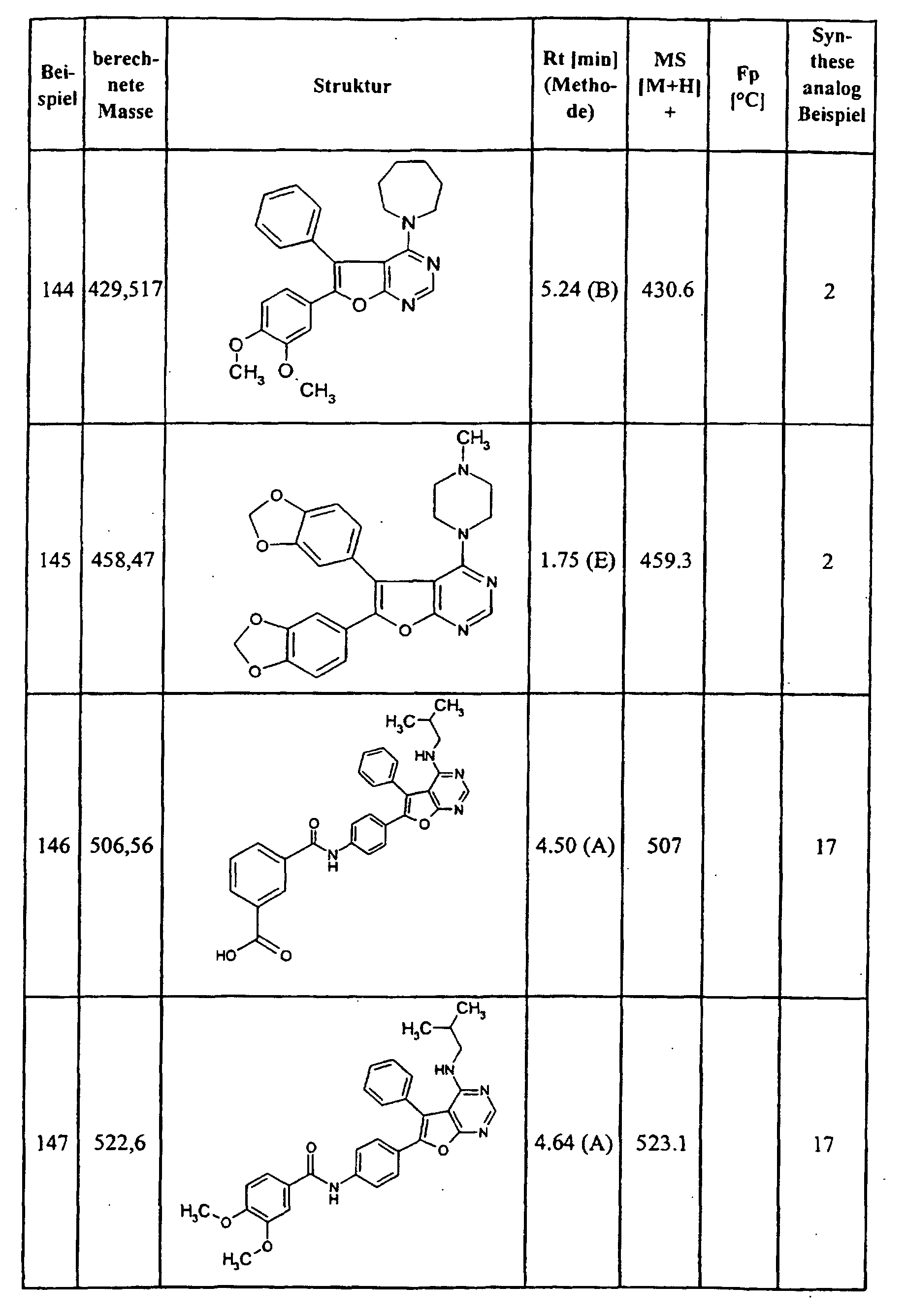

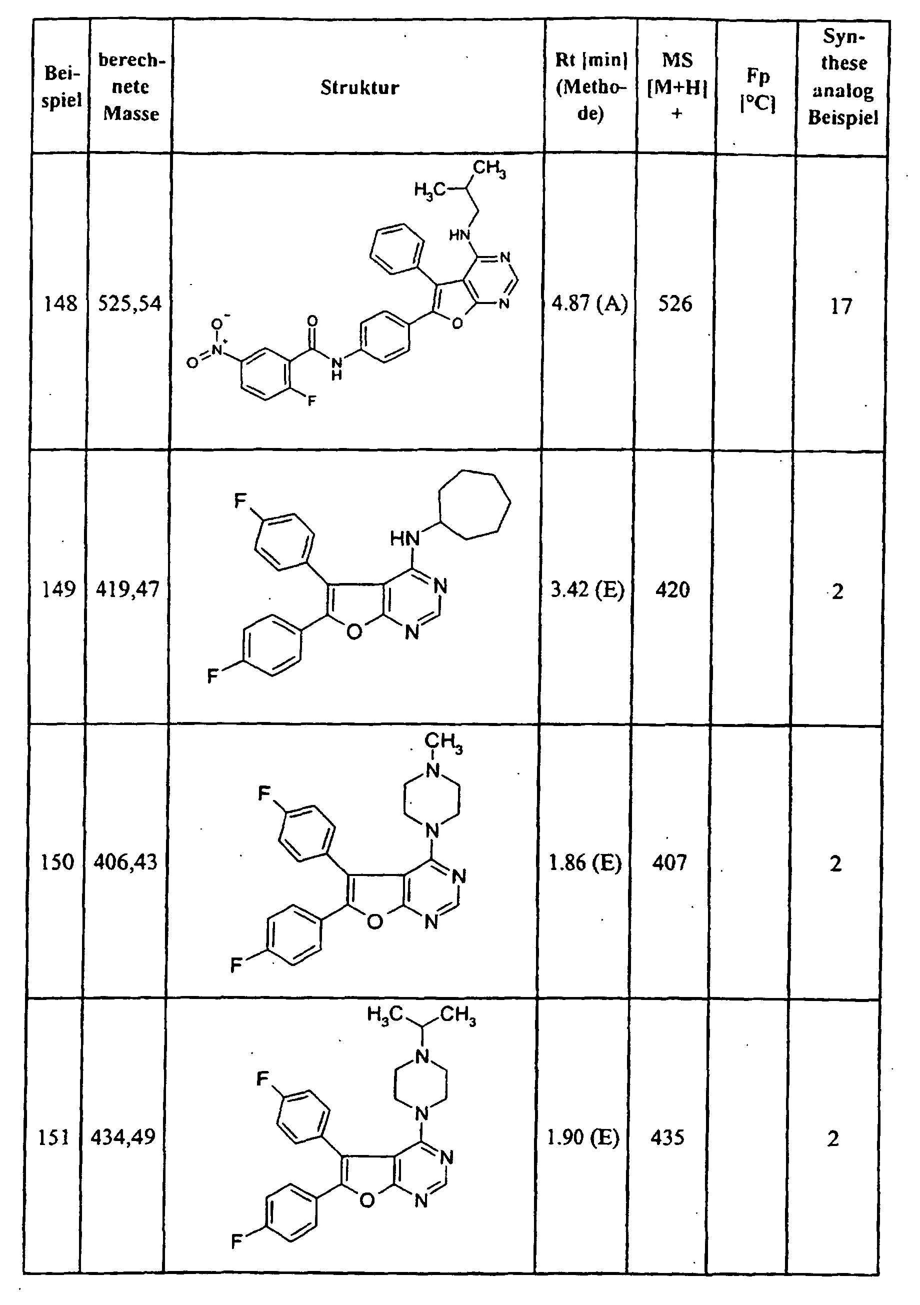

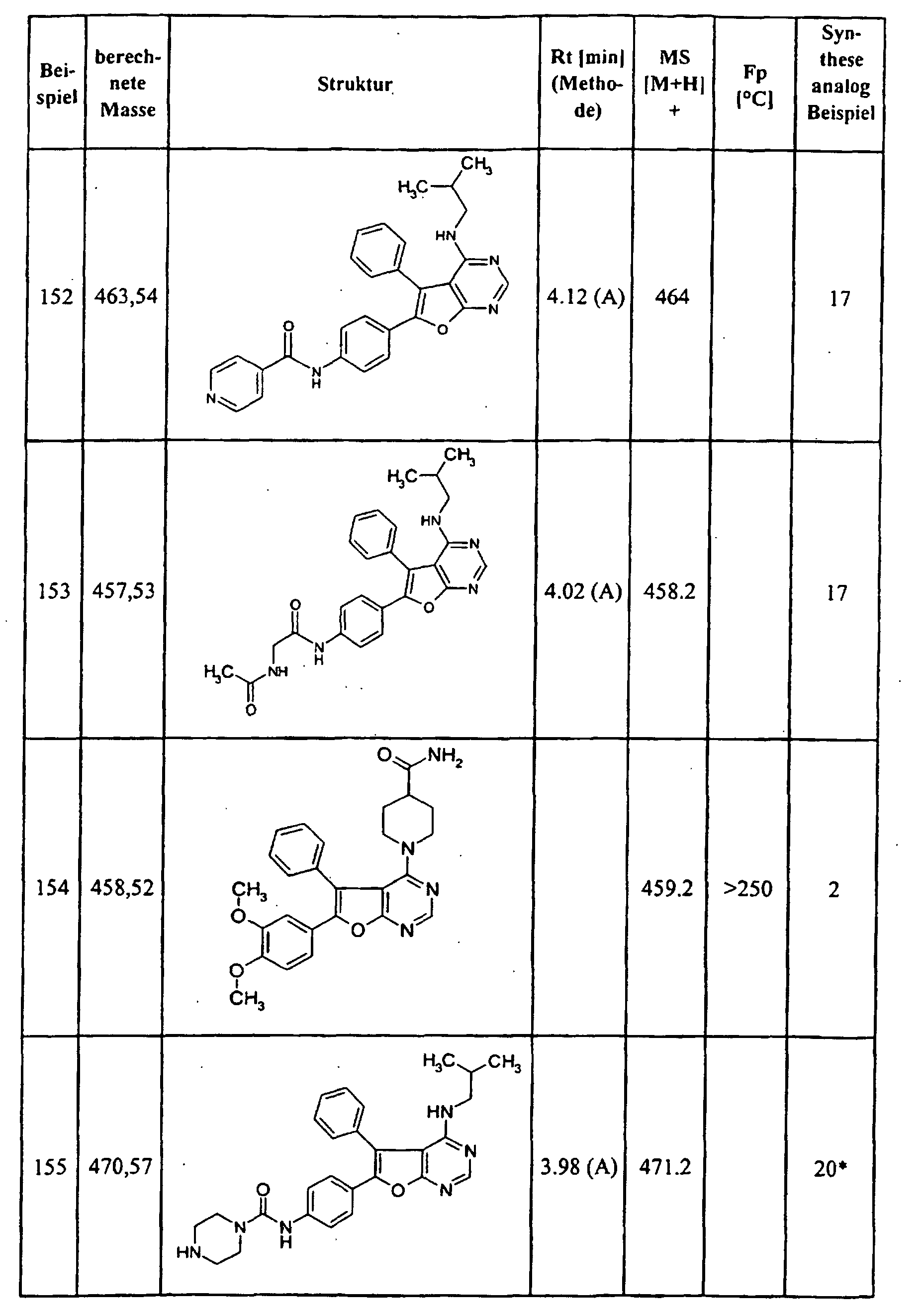

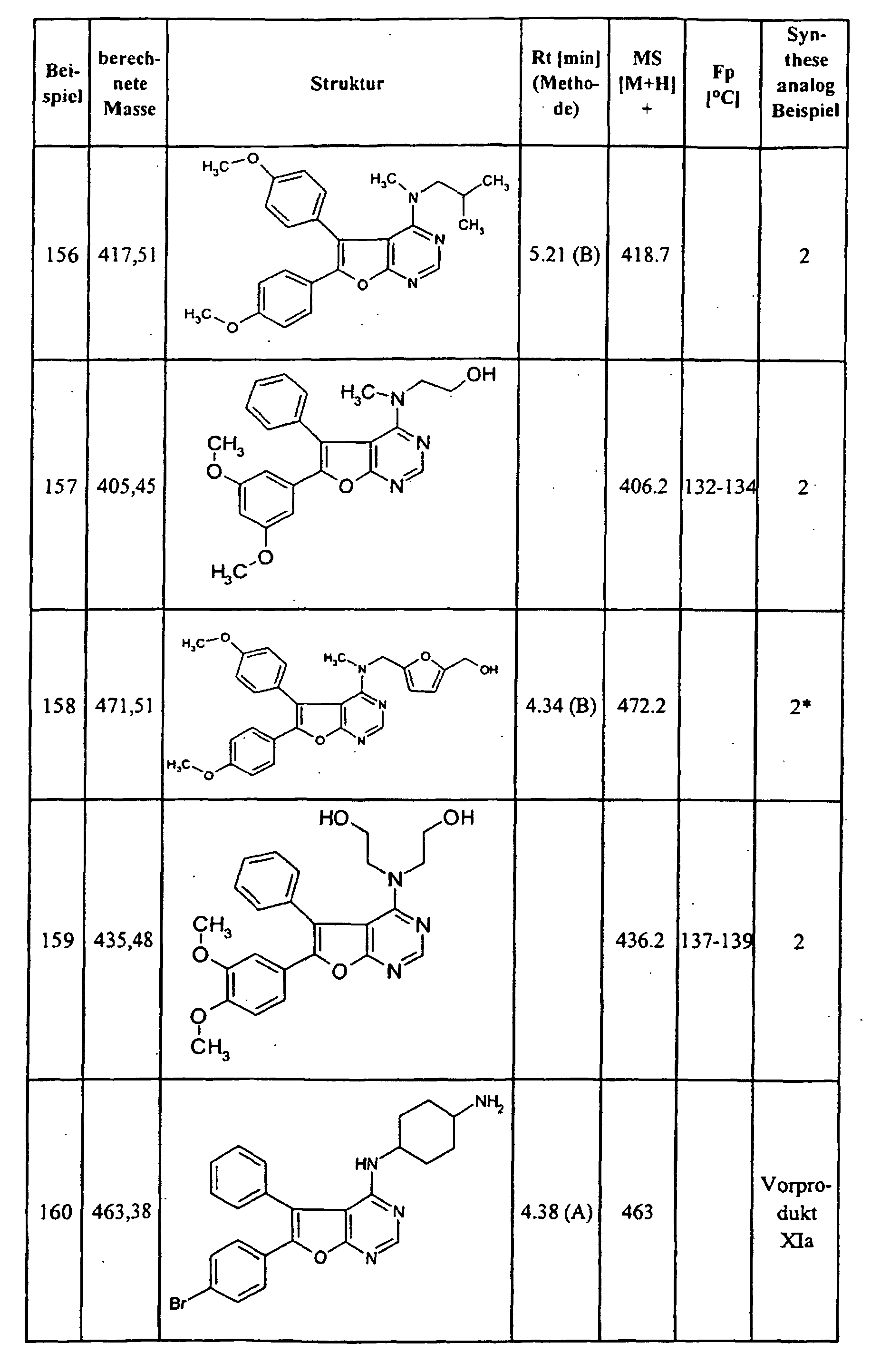

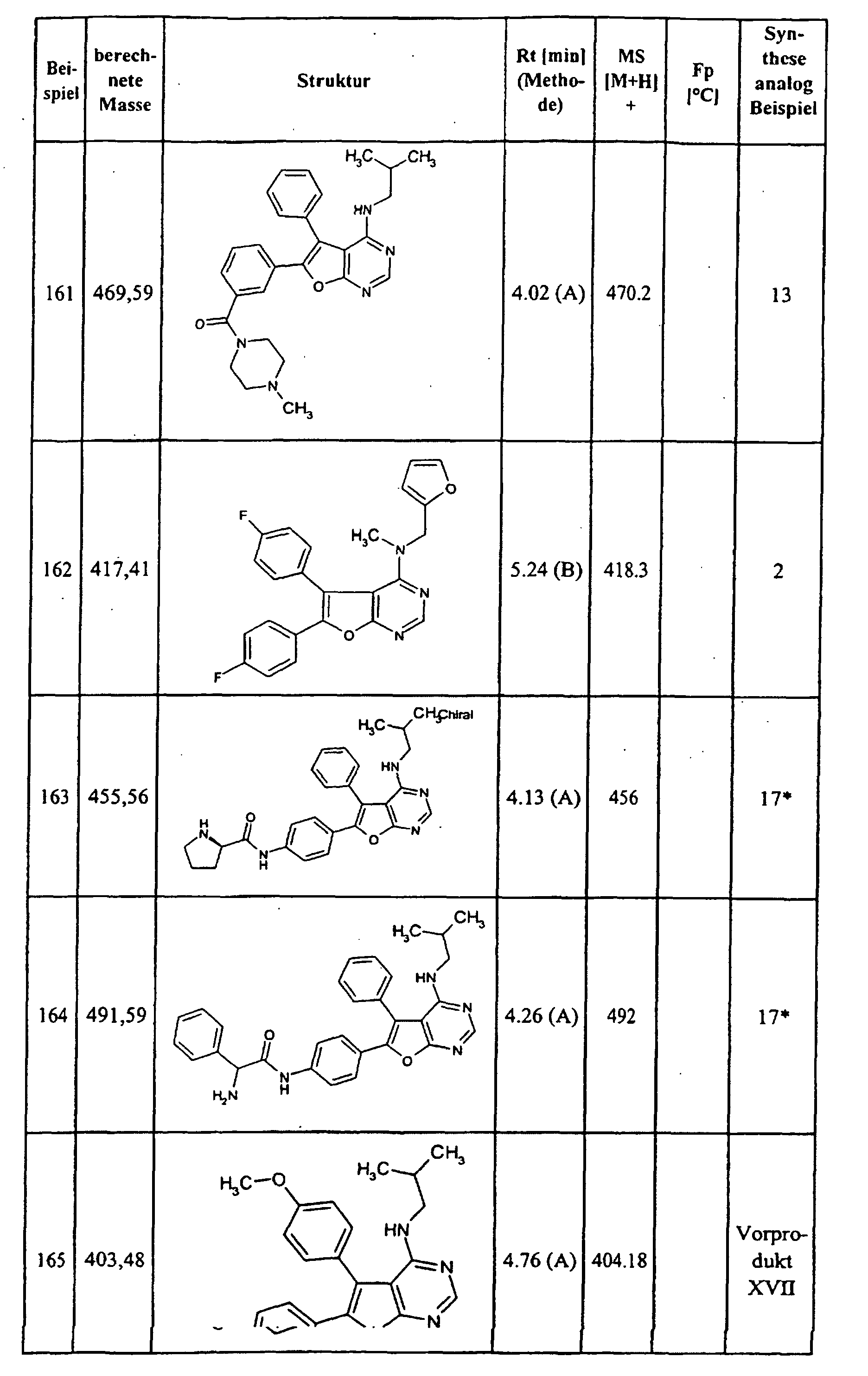

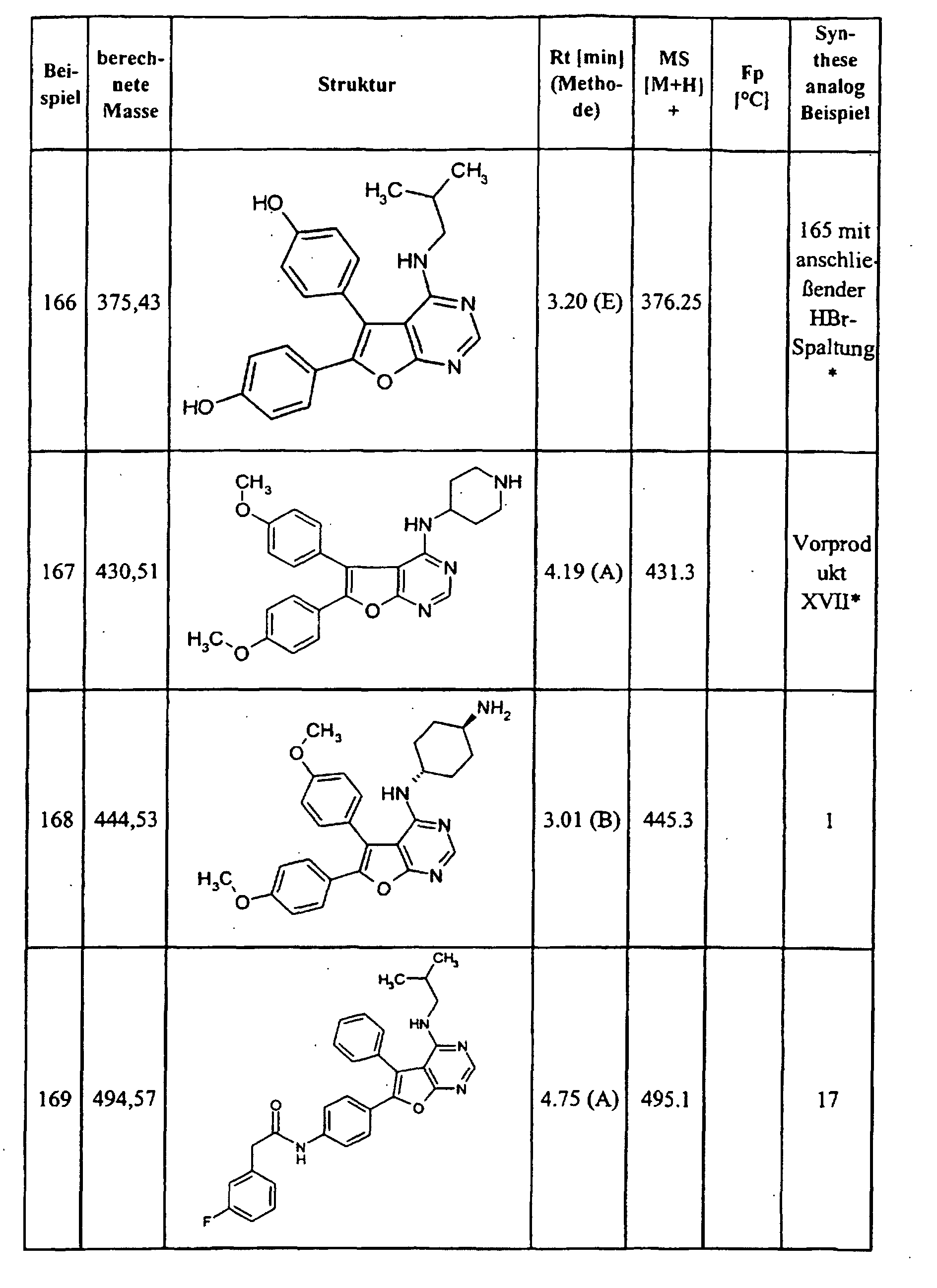

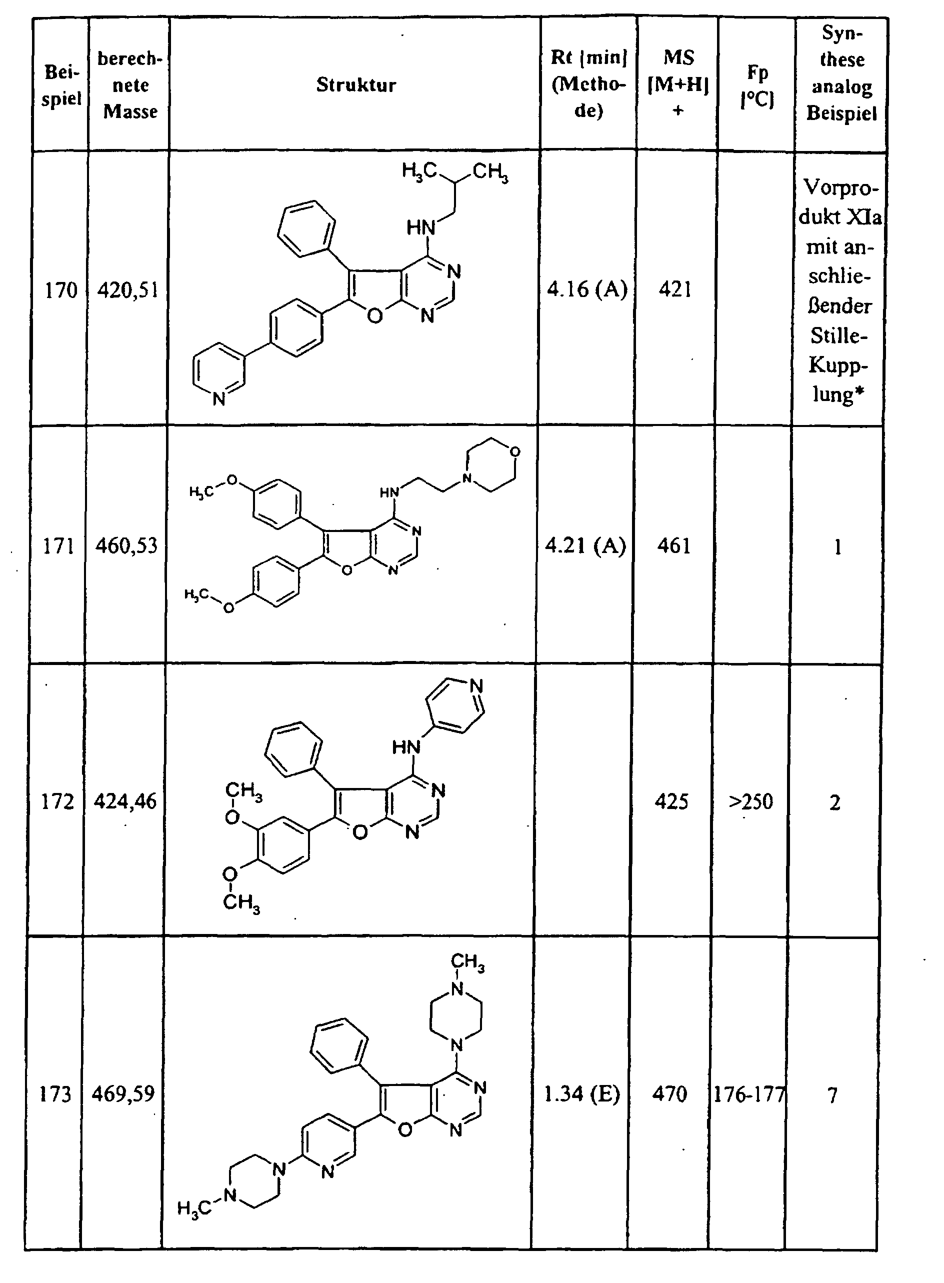

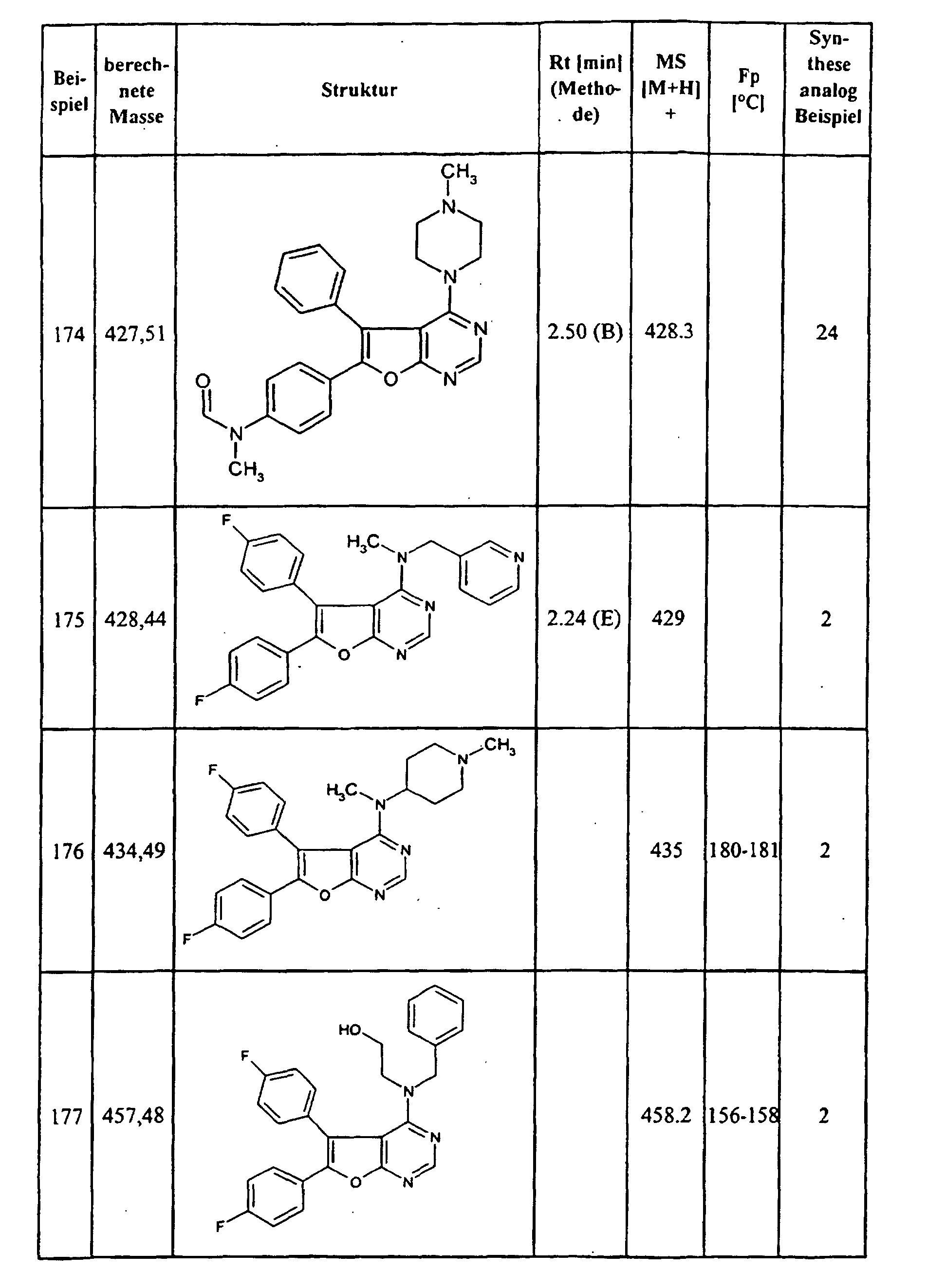

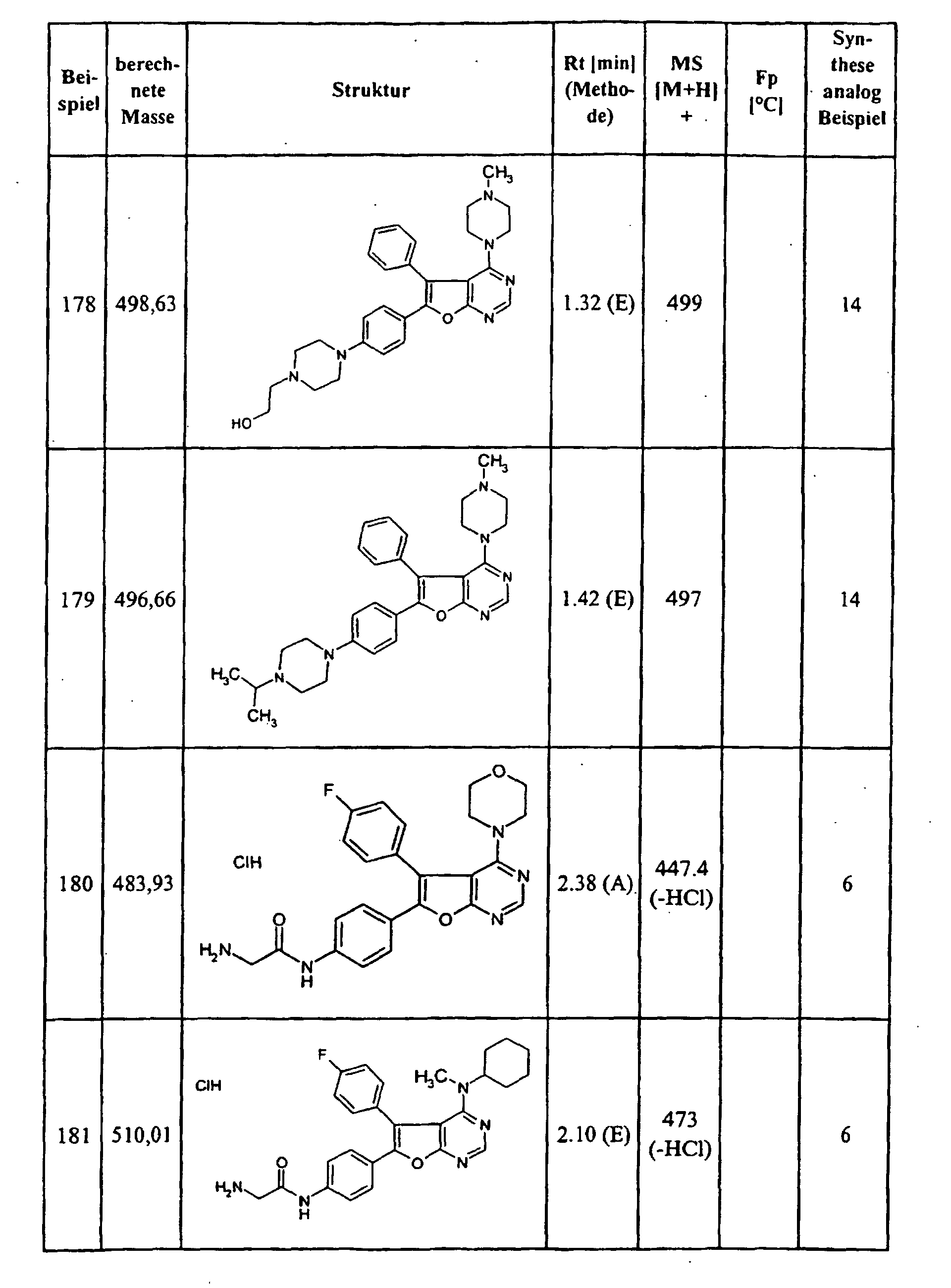

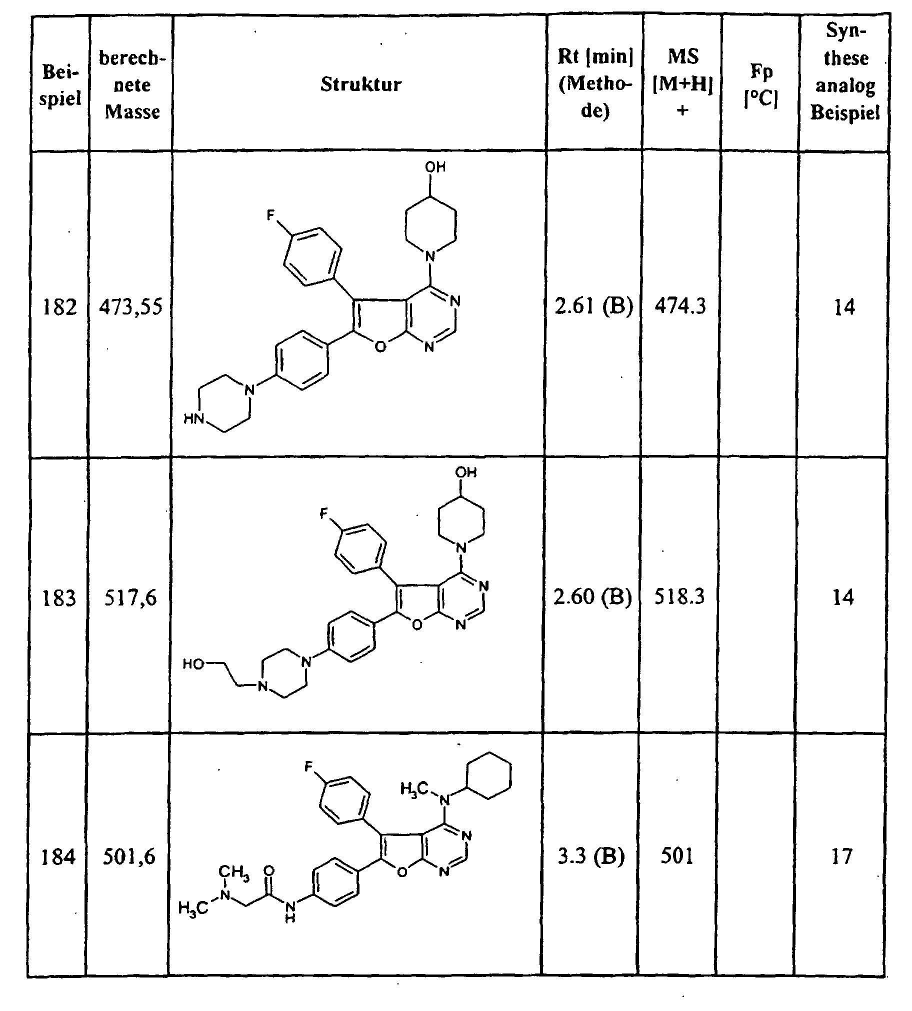

### Beispiel 185

### 1-[6-(4-Bromphenyl)-5-phenylfuro[2,3-d]pyrimidin-4-yl]-4-piperidinol

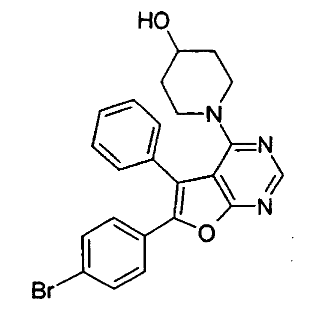

Analog der Reaktion zu Beispiel 21 werden 3.00 g (7.78 mmol) der Verbindung des Beispiels XII mit 3.15 g (31.12 mmol) 4-Hydroxypiperidin umgesetzt. Man erhält 3.12 g (89 %) des Produktes als weissen Feststoff.

¹H-NMR (300 MHz, DMSO-d₆): δ = 0.96-1.10 (m, 2H), 1.33-1.45 (m, 2H), 2.81-2.93 (m, 2H), 3.43-3.57 (m, 3H), 4.56 (d, 1H), 7.26-7.33 (m, 2H), 7.38-7.47 (m, 2H), 7.49-7.59 (m, 5H), 8.42 (s, 1H)
MS (ESIpos): m/z = 450 (M+H)⁺.

### Beispiel 186

### 1-(6-{4-[(3-Chlorphenyl)amino]phenyl}-5-phenylfuro[2,3-d]pyrimidin-4-yl)-4-piperidinol

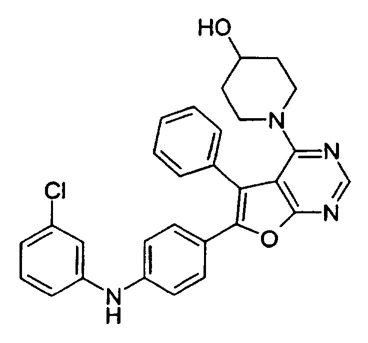

Zu einer rötlichen Suspension von 6.1 mg (0.01 mmol) Tris(dibenzylidenaceton)-dipalladium(0), 11.1 mg (0.02 mmol) rac-2,2'-Bis(diphenylphosphino)-1,1'-binaphthyl, 100.0 mg (0.22 mmol) der Verbindung des Beispiels 185 und 29.9 mg (0.27 mmol) Kalium-tert.-butylat in 2 ml Toluol werden 85.0 mg (0.67 mmol) 3-Chloranilin gegeben. Das Reaktionsgemisch wird 24 h auf 60°C erhitzt und dabei heftig gerührt. Nach Abkühlen wird die Reaktionsmischung mit Dichlormethan (ca. 5 ml) versetzt, filtriert und im Vakuum eingeengt. Das Gemisch wird mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält nach Konzentration im Vakuum 36 mg (33 %) des Produkts als farblosen Feststoff.

¹H-NMR (300 MHz, DMSO-d₆): δ = 0.95-1.09 (m, 2H), 1.32-1.46 (m, 2H), 2.79-2.91 (m, 2H), 3.41-3.56 (m, 3H), 4.54 (d, 1H), 6.86-6.92 (m, 1H), 6.98-7.09 (m, 4H), 7.21-7.33 (m, 2H), 7.40-7.58 (m, 5H), 8.39 (s, 1H), 8.63 (s, 1H)
MS (ESIpos): m/z = 497.4 (M+H)⁺.

### Beispiel 187

### tert.-Butyl-1-[6-(4-bromphenyl)-5-(4-fluorphenyl)furo[2,3-d]pyrimidin-4-yl]-4-piperidinylcarbamat

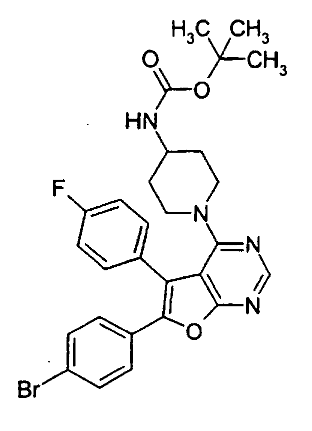

Analog der Reaktion zu Beispiel 21 werden 1 g (2.59 mmol) der Verbindung des Beispiels XII mit 2.08 g (10.37 mmol) tert.-Butyl 4-piperidinylcarbamat umgesetzt. Man erhält 1.4 g (98 %) des Produktes als weissen Feststoff.

¹H-NMR (300 MHz, CDCl₃): δ = 0.93-1.08 (m, 2H), 1.41 (s, 9H), 1.58-1.69 (m, 2H), 2.68-2.81 (m, 2H), 3.37-3.52 (m, 1H), 4.28 (br s, 1H), 7.27-7.50 (m, 8H), 8.47 (s, 1H)
MS (ESIpos): m/z = 550 (M+H)⁺.

### Beispiel 188

### tert.-Butyl 1-(5-(4-fluorphenyl)-6-{4-[formyl(methyl)amino]phenyl}furo[2,3-d]-pyrimidin-4-yl)-4-piperidinylcarbamat

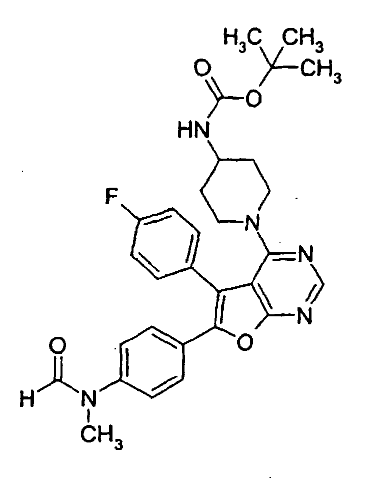

Analog zur Herstellung von Beispiel 24 werden 3.33 mg (0.003 mmol) Tris(dibenzylidenaceton)dipalladium, 8.42 mg (0.01 mmol) 9,9-Dimethyl-4,5-bis(diphenylphosphino)xanthen, 12.9 mg (0.22 mmol) N-Methylformamid und 71.16 mg (0.22 mmol) Cäsiumcarbonat in 1 ml trockenem Dioxan vorgelegt. Nach Zugabe von 100.0 mg (0.18 mmol) der Verbindung aus Beispiel 187 wird 18 h bei 100°C gerührt. Nach üblicher Aufarbeitung und Reinigung mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) erhält man 41 mg (39 %) des Produkts in Form eines gelben Feststoffs.

MS (ESIpos): m/z = 528 (M+H)⁺.

### Beispiel 189

### 4-[4-(4-Amino-1-piperidinyl)-5-(4-fluorphenyl)furo[2,3-d]pyrimidin-6-yl]-phenyl(methyl)formamid-Hydrochlorid

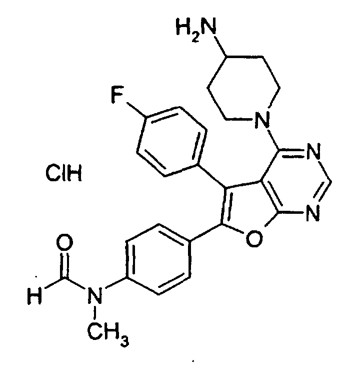

Zu einer Lösung von 30 mg (0.06 mmol) der Verbindung aus Beispiel 188 in 0.5 ml Dioxan werden 0.43 ml (1.71 mmol) einer 4 M Lösung von HCl in Dioxan gegeben, und die klare Lösung wird 4 h bei RT gerührt. Dabei bildet sich ein weisser Feststoff. Das Lösungsmittel wird im Vakuum entfernt und man erhält 25 mg (88 %) Produkt in Form eines farblosen Feststoffs.

¹H-NMR (300 MHz, DMSO-d₆): δ = 1.09-1.48 (m, 4H), 1.55-1.67 (m, 2H), 2.63-2.77 (m, 2H), 2.99-3.14 (m, 1H), 3.18 (s, 3H), 3.72-3.83 (m, 2H), 7.3-7.61 (m, 7H), 7.87-7.99 (m, 2H), 8.47 (s, 1H), 8.62 (s, 1H)
MS (ESIpos): m/z = 428 (M+H)⁺.

### Beispiel 190

### 1-[6-(2'-Nitro-1,1'-biphenyl-4-yl)-5-phenylfuro[2,3-d]pyrimidin-4-yl]-4-piperidinol

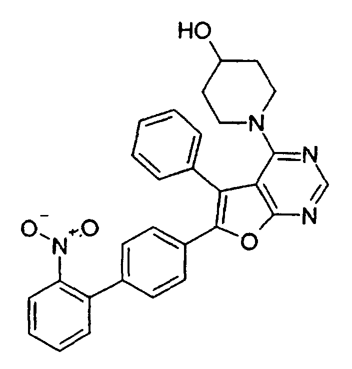

Eine Suspension aus 23.38 mg (0.03 mmol) Bis(triphenylphosphin)palladium(II)-chlorid, 150 mg (0.33 mmol) der Verbindung aus Beispiel 185 und 72.28 mg (0.43 mmol) (2-Nitrophenyl)boronsäure in 4 ml N,N-Dimethylformamid wird 1 h bei 70°C gerührt. Zu der gelben Lösung werden anschließend 0.5 ml einer 2 M wässrigen Natriumcarbonat-Lösung gegeben, und das Reaktionsgemisch wird über Nacht bei RT gerührt. Das rohe Reaktionsgemisch wird mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält 40 mg (24 %) Produkt in Form eines farblosen Feststoffs.

¹H-NMR (300 MHz, DMSO-d₆): δ = 0.95-1.10 (m, 2H), 1.33-1.46 (m, 2H), 2.82-2.96 (m, 2H), 3.44-3.58 (m, 3H), 7.31 (d, 1H), 7.42-7.80 (m, 10H), 7.98 (d, 1H), 8.44 (s, 1H)
MS (ESIpos): m/z = 493.3 (M+H)⁺.

### Beispiel 191

### 5-(4-Fluorphenyl)-N-methyl-6-[6-(methylamino)-3-pyridinyl]furo[2,3-d]-pyrimidin-4-amin

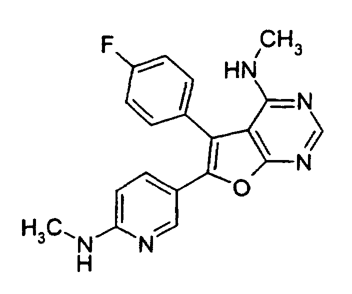

Zu einer Lösung von 200 mg (0.56 mmol) der Verbindung aus Beispiel XIX in 1.67 ml Methanol werden 1.67 ml einer 33%-igen Lösung von Methylamin in Ethanol gegeben. Nach Versetzen der Reaktionsmischung mit 0.08 ml (0.59 mmol) Triethylamin wird 16 h bei 70°C in einem verschlossenen Gefäß gerührt. Das Rohgemisch wird direkt mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält 38 mg (14 %) Produkt in Form eines farblosen Feststoffs.

¹H-NMR (300 MHz, CDCl₃): δ = 2.93 (d, 3H), 2.98 (d, 3H), 4.51 (br d, 1H), 4.82 (br d, 1H), 6.35 (d, 1H), 7.19-7.29 (m, 2H), 7.41-7.49 (m, 2H), 7.50 (dd, 1H), 8.17 (d, 1H), 8.42 (s, 1H)
MS (DCI): m/z = 350 (M+H)⁺.

### Beispiel 192

### 1-[6-(6-Chlor-3-pyridinyl)-5-(4-fluorphenyl)furo[2,3-d]pyrimidin-4-yl]-4-piperidinol

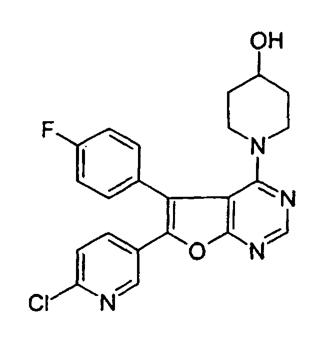

Die Verbindung wird analog der Versuchsvorschrift zur Synthese von Beispiel 9 aus der Verbindung des Beispiels XIX und 4-Piperidinol in 70%-iger Ausbeute hergestellt.

¹H-NMR (300 MHz, DMSO-d₆): δ = 0.99-1.13 (m, 2H), 1.39-1.51 (m, 2H), 2.88-2.98 (m, 2H), 3.48-3:59 (m, 3H), 4.60 (d, 1H), 7.36-7.45 (m, 2H), 7.48-7.59 (m, 3H), 7.77 (dd, 1H), 8.34 (d, 1H), 8.47 (s, 1H)
MS (ESIpos): m/z = 425 (M+H)⁺.

### Beispiel 193

### 1-(5-(4-Fluorphenyl)-6-{6-[(2-hydroxyethyl)amino]-3-pyridinyl}furo[2,3-d]-pyrimidin-4-yl)-4-piperidinol

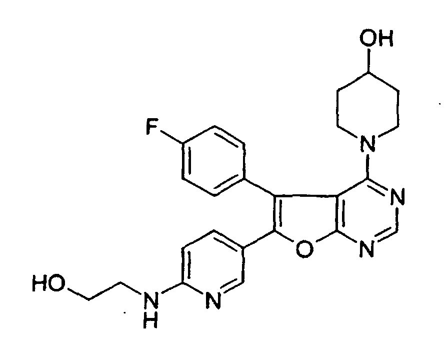

Eine Mischung aus 1000 mg (2.35 mmol) der Verbindung aus Beispiel 192 und 2-Aminoethanol wird 12 h bei 135°C gerührt. Nach Abkühlen wird mit Essigsäureethylester verdünnt, und nach üblicher Aufarbeitung wird das Gemisch mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält 919 mg (87 %) Produkt in Form eines farblosen Feststoffs.

¹H-NMR (300 MHz, DMSO-d₆): δ = 0.97-1.11 (m, 2H), 1.38-1.49 (m, 2H), 2.80-2.93 (m, 2H), 3.42-3.57 (m, 5H), 4.58 (br s, 2H), 6.49 (d, 1H), 6.98 (br s, 1H), 7.29-7.41 (m, 3H), 7.42-7.51 (m, 2H), 7.97 (d, 1H), 8.39 (s, 1H)
MS (ESIpos): m/z = 450.4 (M+H)⁺.

### Beispiel 194

### 1-{5-(4-Fluorphenyl)-6-[6-(2-hydroxyethoxy)-3-pyridinyl]furo[2,3-d]pyrimidin-4-yl}-4-piperidinol

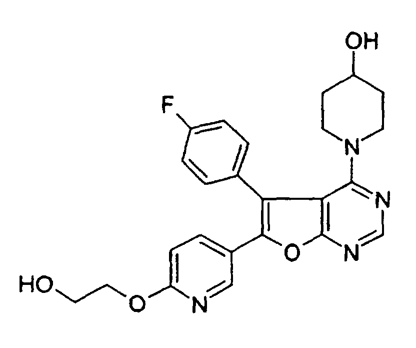

Zu einer Lösung aus 100 mg (0.24 mmol) der Verbindung aus Beispiel 192 in 1 ml 1,2-Ethandiol werden 14.5 mg (0.26 mmol) pulverisiertes Kaliumhydroxid gegeben, und das Reaktionsgemisch wird 2.5 h bei 100°C gerührt. Nach Abkühlen wird mit Wasser (ca. 15 ml) versetzt, der entstandene Niederschlag abgesaugt und im Vakuum getrocknet. Das Rohprodukt wird mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält 7 mg (6 %) Produkt in Form eines farblosen Feststoffs.

¹H-NMR (300 MHz, DMSO-d₆): δ = 0.96-1.11 (m, 2H), 1.34-1.50 (m, 2H), 2.8-2.96 (m, 2H), 3.42-3.59 (m, 3H), 3.41-3.73 (m, 2H), 4.19-4.31 (m, 2H), 4.49 (d, 1H), 4.79 (t, 1H), 6.83 (d, 1H), 7.29-7.54 (m, 4H), 7.56-7.67 (m, 1H), 8.13 (d, 1H), 8.41 (s, 1H)
MS (ESIpos): m/z = 451.3 (M+H)⁺.

### Beispiel 195

### 1-{5-(4-Fluorphenyl)-6-[6-(4-morpholinyl)-3-pyridinyl]furo[2,3-d]pyrimidin-4-yl}-4-piperidinol

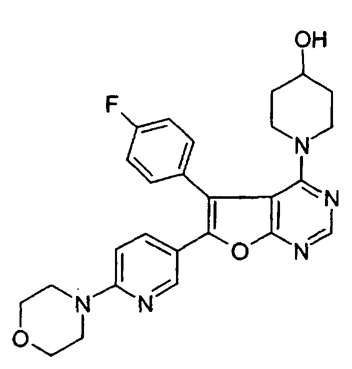

Die Verbindung wird analog der Versuchsvorschrift für Beispiel 7 aus Beispiel 192 und Morpholin hergestellt (Ausbeute: 85 %).

¹H-NMR (300 MHz, CDCl₃): δ = 0.97-1.11 (m, 2H), 1.38-1.49 (m, 2H), 2.81-2.93 (m, 2H), 3.44-3.57 (m, 7H), 3.62-3.71 (m, 4H), 4.58 (d, 1H), 6.82 (d, 1H), 7.31-7.41 (m, 2H), 7.43-7.54 (m, 3H), 8.12 (d, 1H), 8.39 (s, 1H)
MS (ESIpos): m/z = 476.5 (M+H)⁺.

### Beispiel 196

### 1-{5-(4-Fluorphenyl)-6-[6-(4-morpholinyl)-3-pyridinyl]furo[2,3-d]pyrimidin-4-yl}-4-piperidinol-Hydrochlorid

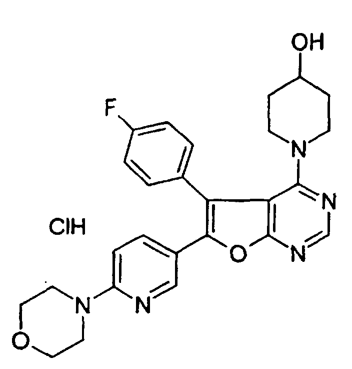

Zu 40 mg (0.08 mmol) der Verbindung aus Beispiel 195 werden 2 ml einer 4 M Lösung von HCI in Dioxan gegeben, und die Reaktionsmischung wird 3 h bei RT gerührt. Nach Entfernen des Lösungsmittels erhält man 43 mg (95 %) Produkt in Form eines farblosen Feststoffes.

MS (ESIpos): m/z = 476.4 (M+H)⁺.

### Beispiel 197

### 4-(4-Isopropyl-1-piperazinyl)-5-phenyl-6-[4-(1-piperazinyl)phenyl]furo[2,3-d]-pyrimidin-Hydrochlorid

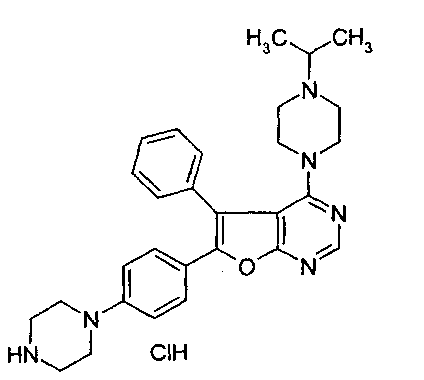

Die Synthese des eingesetzten Bromids 6-(4-Bromphenyl)-4-(4-isopropyl-1-piperazinyl)-5-phenylfuro[2,3-d]pyrimidin erfolgt analog der Herstellungsvorschrift des Beispiels 22 ausgehend von der Verbindung des Beispiels XII und 4-Isopropylpiperazin.

Unter Schutzgas und rigorosem Sauerstoffausschluss werden 100 mg (0.21 mmol) 6-(4-Bromphenyl)-4-(4-isopropyl-1-piperazinyl)-5-phenylfuro[2,3-d]pyrimidin, 5.75 mg (0.01 mmol) Tris(dibenzylidenaceton)dipalladium, 13.0 mg (0.02 mmol) rac-2,2'-Bis(diphenylphosphino)-1,1'-binaphthyl und 28.2 mg (0.29 mmol) Natriumtert.-butylat in 4 ml Toluol vorgelegt und mit 54.1 mg (0.63 mmol) Piperazin versetzt. Die Reaktionsmischung wird 20 h bei 60°C gerührt. Nach Entfernen des Lösungsmittels wird mit Essigsäureethylester versetzt und nach der üblichen Art aufgearbeitet. Das Gemisch wird mittels präparativer RP-HPLC (Säule: YMC Gel ODS-AQ S-11 µm, 250 x 30 mm; Eluent: Acetonitril/Wasser + 0.3% HCl; Fluss: 50 ml/min; UV-Detektion bei 210 nm) aufgetrennt. Man erhält 16 mg (18 %) Produkt in Form eines gelben Feststoffs.

¹H-NMR (300 MHz, DMSO-d₆): δ = 0.17 (d, 6H), 2.48-2.60 (m, 2H), 3.05-3.23 (m, 8H), 3.29-3.40 (m, 1H), 3.41-3.48 (m, 4H), 3.73 (d, 1H), 6.95 (d, 2H), 7.33 (d, 2H), 7.43-7.61 (m, 5H), 8.52 (s, 1H), 9.05 (br s, 1H), 10.51 (br s, 1H)
MS (ESIpos): m/z = 477 (M+H)⁺.

### Beispiel 198

### 1-{5-[5-(4-Fluorphenyl)-4-(4-hydroxy-1-piperidinyl)furo[2,3-d]pyrimidin-6-yl]-2-pyridinyl}-4-piperidinol

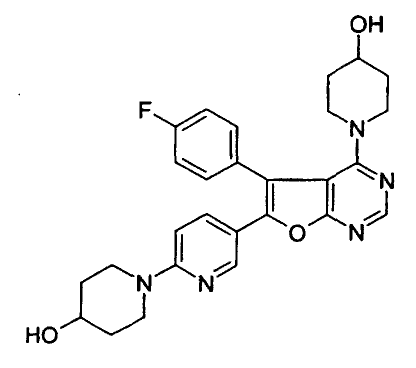

Zu 593.4 mg (1.65 mmol) der Verbindung aus Beispiel XIX werden 8.33 g (82.38 mmol) 4-Hydroxypiperidin gegeben. Die Reaktionsmischung wird 14 h bei 135°C gerührt. Nach Abkühlen bildet sich ein Feststoff, der in Wasser und Ethanol gelöst wird. Nach üblicher Aufarbeitung wird das Gemisch mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) aufgetrennt. Man erhält 380 mg (47 %) Produkt in Form eines farblosen Feststoffs.

¹H-NMR (200 MHz, DMSO-d₆): δ = 1.18-1.57 (m, 4H), 1.66-1.86 (m, 2H), 2.77-2.99 (m, 2H), 3.03-3.26 (m, 2H), 3.42-3.59 (m, 3H), 3.61-3.80 (m, 1H), 3.91-4.10 (m, 2H), 4.62 (d, 1H), 4.72 (d, 1H), 6.82 (d, 1H), 7.30-7.57 (m, 5H), 8.09 (d, 1H), 8.40 (s, 1H)
MS (ESIpos): m/z = 490.4 (M+H)⁺.

### Beispiel 199

### 2-({5-[4-(1,4-Dioxa-8-azaspiro[4.5]dec-8-yl)-5-(4-fluorphenyl)furo[2,3-d]-pyrimidin-6-yl]-2-pyridinyl}amino)ethanol

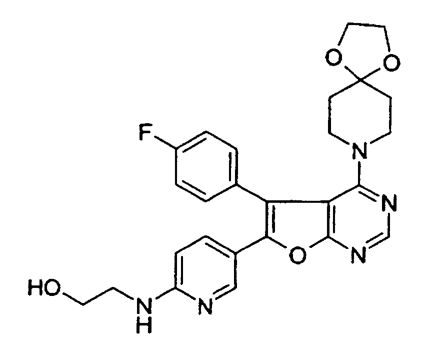

Die Synthese des eingesetzten Chlorids 8-[6-(6-Chlor-3-pyridinyl)-5-(4-fluorphenyl)-furo[2,3-d]pyrimidin-4-yl]-1,4-dioxa-8-azaspiro[4.5]decan erfolgt analog der Herstellungsvorschrift des Beispiels 22 ausgehend von der Verbindung des Beispiels XII und 1,4-Dioxa-8-azaspiro[4.5]decan.

Analog der Herstellungsvorschrift zu Beispiel 7 werden 135 mg (0.29 mmol) 8-[6-(6-Chlor-3-pyridinyl)-5-(4-fluorphenyl)furo[2,3-d]pyrimidin-4-yl]-1,4-dioxa-8-azaspiro[4.5]decan und 3.04 g (49.70 mmol) 2-Aminoethanol vermischt und 14 h bei 135°C gerührt. Nach üblicher Aufarbeitung und Trennung mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) erhält man 79 mg (56 %) Produkt in Form eines farblosen Feststoffs.

¹H-NMR (200 MHz, DMSO-d₆): δ = 1.23-1.38 (m, 4H), 3.15-3.27 (m, 4H), 3.27-3.33 (m, 2H), 3.41-3.55 (m, 2H), 3.80 (s, 4H), 4.70 (t, 1H), 6.48 (d, 1H), 7.02 (t, 1H), 7.28-7.52 (m, 5H), 7.98 (d, 1H), 8.41 (s, 1H)
MS (ESIpos): m/z = 492.3 (M+H)⁺.

### Beispiel 200

### 1-{5-(4-Fluorphenyl)-6-[6-(4-isopropyl-1-piperazinyl)-3-pyridinyl]furo[2,3-d]-pyrimidin-4-yl}-4-piperidinol

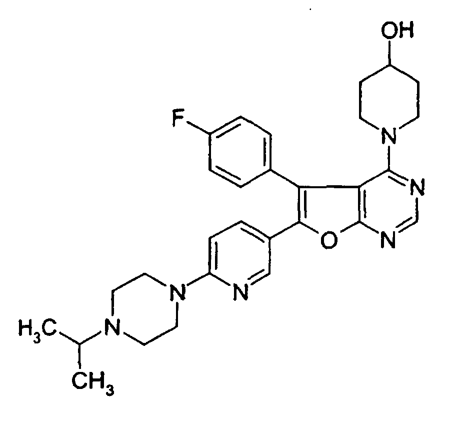

Analog der Herstellungsvorschrift zu Beispiel 7 werden 60 mg (0.14 mmol) der Verbindung aus Beispiel 192 und 905.36 mg (7.06 mmol) 4-Isopropylpiperazin vermischt und 16 h bei 135°C gerührt. Nach üblicher Aufarbeitung wird durch Zugabe von DMSO ein Niederschlag ausgefällt, der abgesaugt, mit DMSO gewaschen und im Vakuum getrocknet wird. Man erhält 12 mg (16 %) Produkt in Form eines farblosen Feststoffs.

¹H-NMR (300 MHz, DMSO-d₆): δ = 0.98 (d, 6H), 1.01-1.12 (m, 2H), 1.38-1.50 (m, 2H), 2.23-2.30 (m, 1H), 2.60-2.75 (m, 3H), 2.81-2.92 (m, 2H), 3.44-3.56 (m, 8H), 4.58 (d, 1H), 6.80 (d, 1H), 7.31-7.52 (m, 5H), 8.10 (d, 1H), 8.39 (s, 1H)
MS (ESIpos): m/z = 517.4 (M+H)⁺.

### Beispiel 201

### N-{4-[5-(4-Fluorphenyl)-4-(4-hydroxy-1-piperidinyl)furo[2,3-d]pyrimidin-6-yl]-phenyl}-L-prolinamid-Hydrochlorid

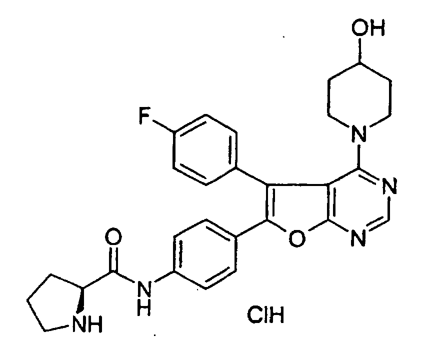

Analog der Herstellungsvorschrift zu Beispiel 17 werden 60 mg (0.15 mmol) der Verbindung aus Beispiel 23 und 38.32 mg (0.18 mmol) 1-(tert.-Butoxycarbonyl)-Lprolin umgesetzt. Nach anschließender Entschützung mit einer Lösung von HCI in Dioxan erhält man 23 mg (13 %) Produkt in Form eines farblosen Feststoffs.

¹H-NMR (300 MHz, DMSO-d₆): δ = 0.96-1.12 (m, 2H), 1.38-1.50 (m, 2H), 1.87-2.04 (m, 3H), 2.39-2.46 (m, 1H), 2.80-2.93 (m, 2H), 3.18-3.32 (m, 2H), 3.50-3.60 (m, 4H), 4.29-4.41 (m, 1H), 7.31-7.41 (m, 4H), 7.41-7.51 (m, 2H), 7.60 (d, 2H), 8.40 (s, 1H), 8.59-8.72 (m, 1H), 9.50-9.67 (m, 1H), 10.85 (s, 1H)
MS (ESIpos): m/z = 502.2 (M+H)⁺.

### Beispiel 202

### N-(1-{5-[4-(4-Amino-1-piperidinyl)-5-(4-fluorphenyl)furo[2,3-d]pyrimidin-6-yl]-2-pyridinyl}-4-piperidinyl)acetamid-Hydrochlorid

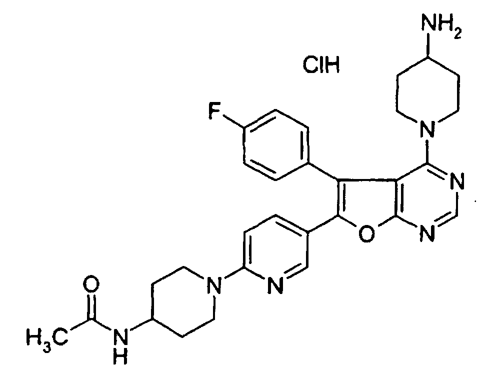

Die Synthese des eingesetzten Chlorids tert.-Butyl 1-[6-(6-chlor-3-pyridinyl)-5-(4-fluorphenyl)furo[2,3-d]pyrimidin-4-yl]-4-piperidinylcarbamat erfolgt analog der Herstellungsvorschrift des Beispiels 22 ausgehend von der Verbindung des Beispiels XII und tert.-Butyl 4-piperidinylcarbamat.

Analog der Herstellungsvorschrift zu Beispiel 7 werden 70 mg (0.11 mmol) tert.-Butyl 1-[6-(6-chlor-3-pyridinyl)-5-(4-fluorphenyl)furo[2,3-d]pyrimidin-4-yl]-4-piperidinylcarbamat und 434.21 mg (3.05 mmol) N-Acetylpiperidin vermischt und 14 h bei 135°C gerührt. Nach üblicher Aufarbeitung und Trennung mittels präparativer RP-HPLC (Säule: YMC Gel ODS-AQ S-11 µm, 250 x 30 mm; Eluent: Acetonitril/Wasser; Fluss: 50 ml/min; UV-Detektion bei 210 nm) erhält man 90 mg (75 %) tert.-Butyl-1-[6-{6-[4-(acetylamino)-1-piperidinyl]-3-pyridinyl}-5-(4-fluorphenyl)furo[2,3-d]pyrimidin-4-yl]-4-piperidinylcarbamat in Form eines farblosen Feststoffs. Anschließendes Versetzen von 70 mg (0.11 mmol) dieser Substanz mit einer Lösung von HCI in Dioxan in der üblichen Weise liefert 62.5 mg (96 %) des entsprechenden Hydrochlorids in Form eines farblosen Feststoffs.

¹H-NMR (400 MHz, D₂O): δ = 0.95-1.08 (m, 2H), 1.28-1.41 (m, 2H), 1.57 (d, 2H), 1.77 (s, 3H), 1.78-1.88 (m, 2H), 2.52-2.64 (m, 2H), 2.96-3.18 (m, 4H), 3.51-3.61 (m, 1H), 3.69-3.87 (m, 4H), 6.82 (d, 1H), 7.12 (dd, 2H), 7.21-7.29 (m, 2H), 7.40-7.46 (m, 1H), 7.70 (d, 1H), 8.12 (s, 1H)
MS (ESIpos): m/z = 530.3 (M+H)⁺.

### Beispiel 203

### N-{5-[4-(4-Hydroxy-1-piperidinyl)-5-phenylfuro[2,3-d]pyrimidin-6-yl]-2-pyridinyl}acetamid

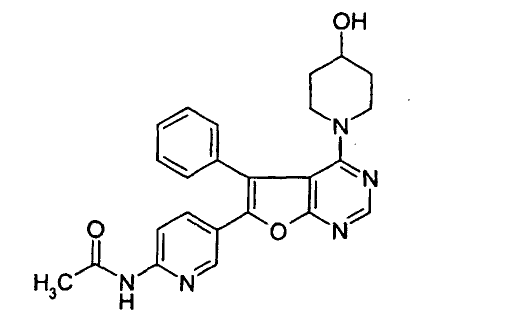

Die Synthese des eingesetzten Chlorids 1-[6-(6-Chloro-3-pyridinyl)-5-phenylfuro[2,3-d]pyrimidin-4-yl]-4-piperidinol erfolgt analog der Herstellungsvorschrift des Beispiels 192 ausgehend von der Verbindung des Beispiels XX und 4-Hydroxypiperidin.

200 mg (0.49 mmol) 1-[6-(6-Chloro-3-pyridinyl)-5-phenylfuro[2,3-d]pyrimidin-4-yl]-4-piperidinol, 5.8 g (98.19 mmol) Acetamid und 1.69 g (12.23 mmol) Kaliumcarbonat werden vermischt und 32 h bei 210°C gerührt. Es wird auf 70°C abgekühlt und Wasser zugegeben. Nach Verdünnen mit Essigsäureethylester werden die Phasen getrennt und die wässrige Phase mit Essigsäureethylester extrahiert. Die vereinigten organischen Phasen werden mit Wasser gewaschen, über Magnesiumsulfat getrocknet und im Vakuum eingeengt. Nach Trennung mittels präparativer RP-HPLC (Säule: YMC Gel ODS-AQ S-11 µm, 250 x 30 mm; Eluent: Acetonitril/ Wasser; Fluss: 50 ml/min; UV-Detektion bei 210 nm) erhält man 12 mg (6 %) Produkt in Form eines farblosen Feststoffs.

¹H-NMR (400 MHz, D₂O): δ = 0.97-1.11 (m, 2H), 1.33-1.46 (m, 2H), 2.08 (s, 3H), 2.83-2.95 (m, 2H), 3.45-3.59 (m, 3H), 4.56 (d, 1H), 7.41-7.59 (m, 5H), 7.72 (dd, 1H), 8.02 (d, 1H), 8.20 (d, 1H), 8.42 (s, 1H)
MS (ESIpos): m/z = 430.3 (M+H)⁺.

### Beispiel 204

### 1-[6-{6-[(2-Aminoethyl)amino]-3-pyridinyl}-5-(4-fluorphenyl)furo[2,3-d]-pyrimidin-4-yl]-4-piperidinol-Hydrochlorid

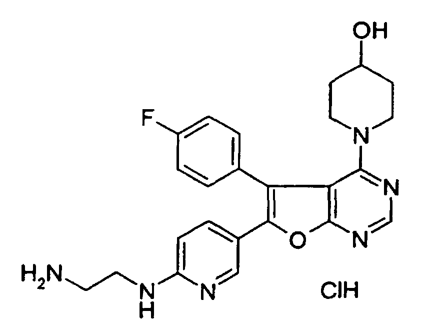

150 mg (0.35 mmol) der Verbindung aus Beispiel 192 werden in 1.06 g (17.65 mmol) Ethylendiamin suspendiert und 12 h bei 120°C gerührt. Nach Abkühlen wird das Reaktionsgemisch in N,N-Dimethylformamid aufgenommen und direkt mittels präparativer RP-HPLC (Säule: YMC Gel ODS-AQ S-11 µm, 250 x 30 mm; Eluent: Acetonitril/Wasser + 0.3% HCl; Fluss: 50 ml/min; UV-Detektion bei 210 nm) aufgetrennt. Man erhält 67 mg (39 %) Produkt in Form eines farblosen Feststoffs.

¹H-NMR (400 MHz, D₂O): δ = 0.97-1.12 (m, 2H), 1.38-1.50 (m, 2H), 2.82-3.07 (m, 4H), 3.45-3.62 (m, 5H), 6.75 (d, 1H), 7.31-7.57 (m, 5H), 7.91-8.12 (br m, 2H), 7.96 (d, 1H), 8.41 (s, 1H)
MS (ESIpos): m/z = 449.3 (M+H)⁺.

Die in der folgenden Tabelle aufgeführten Beispiele 205 bis 331 werden in Analogie zu den oben aufgeführten Vorschriften hergestellt:

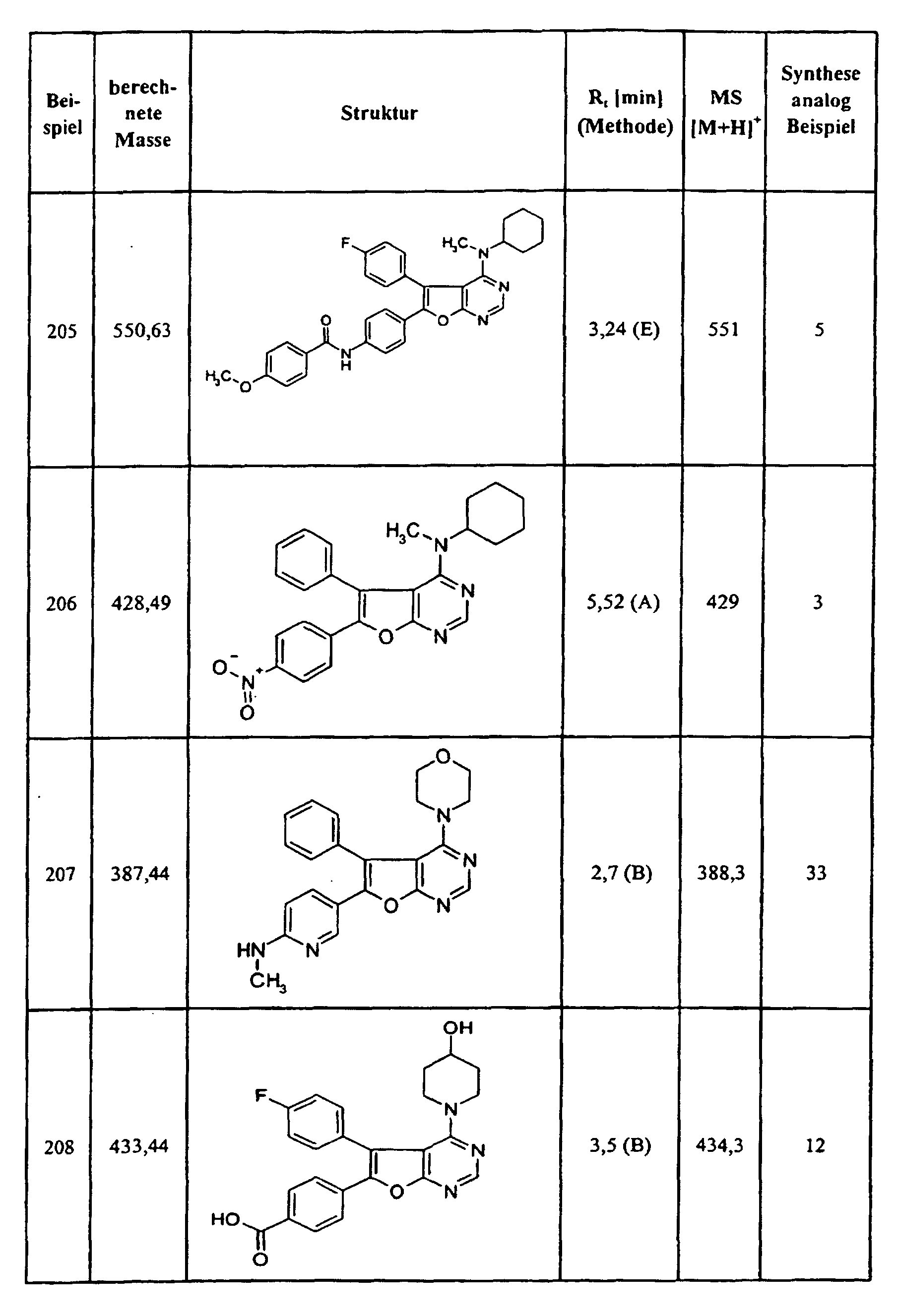

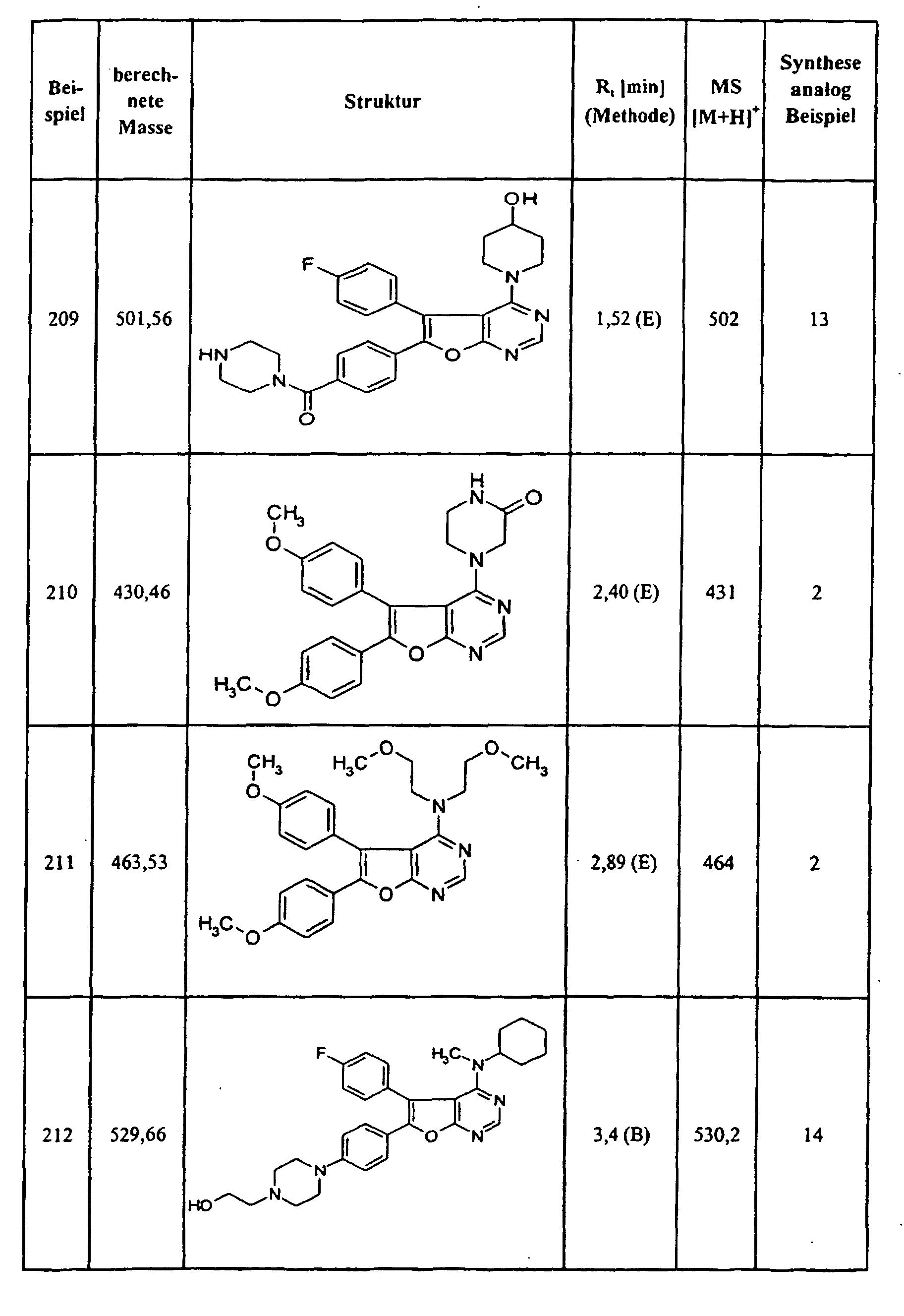

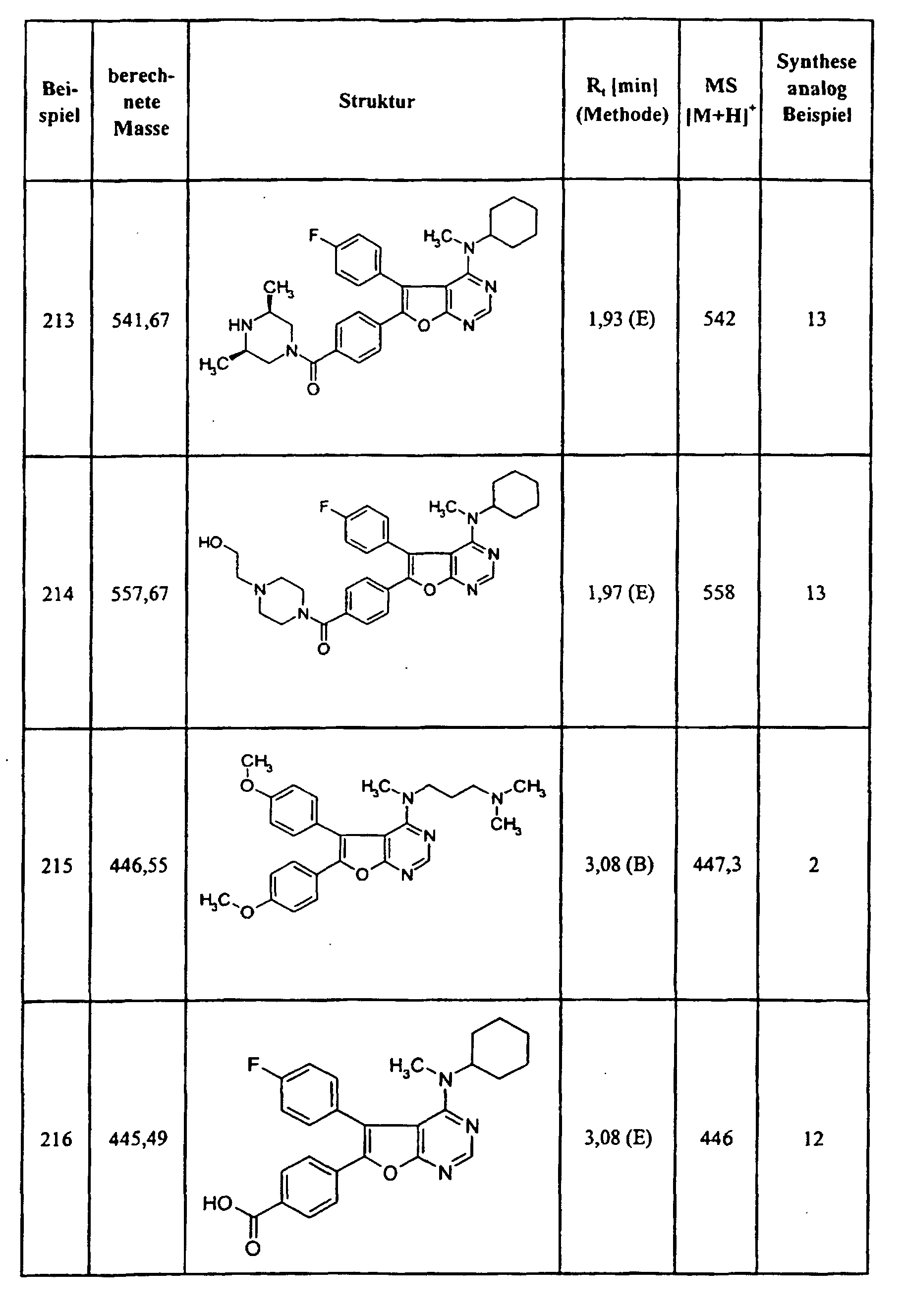

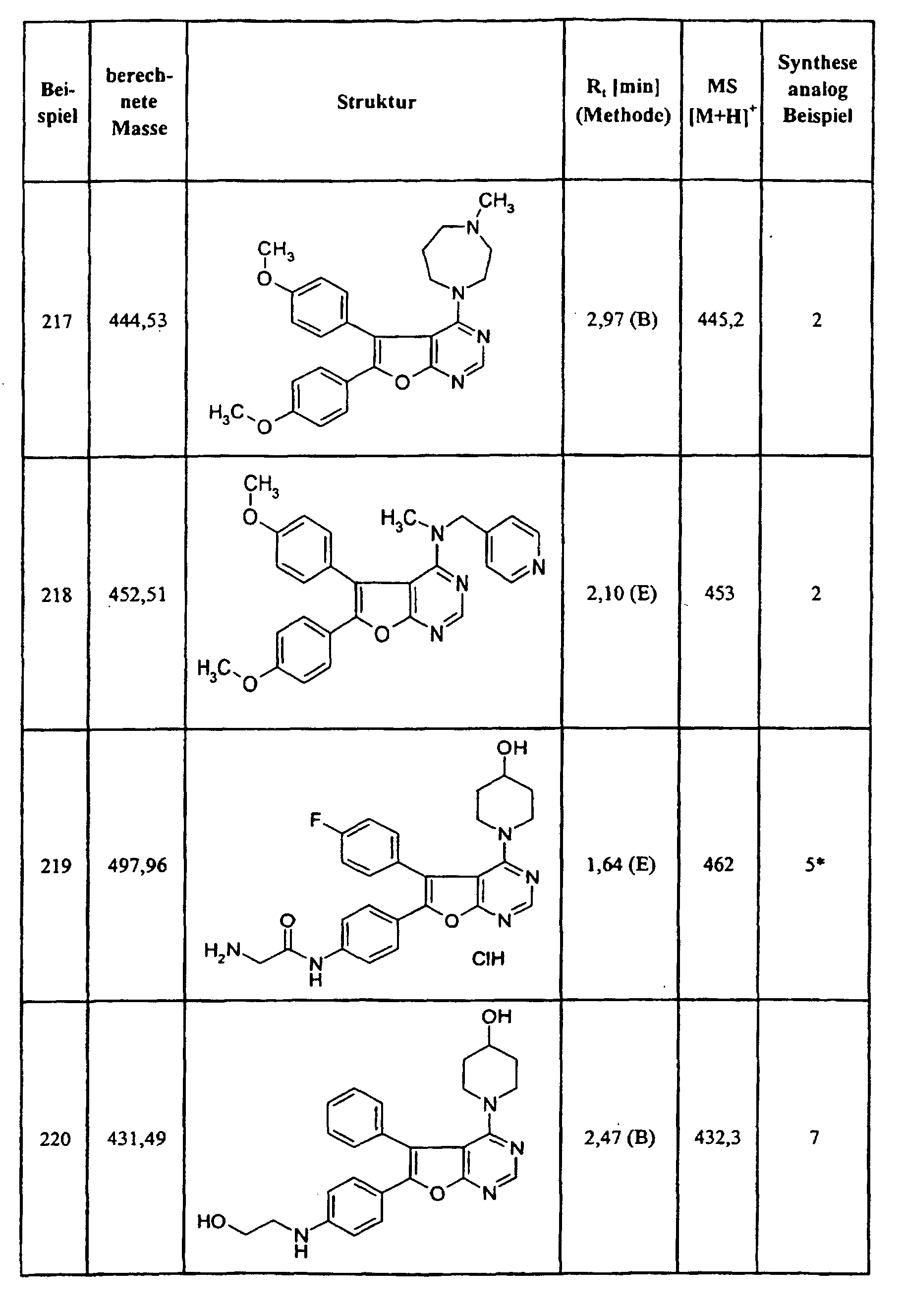

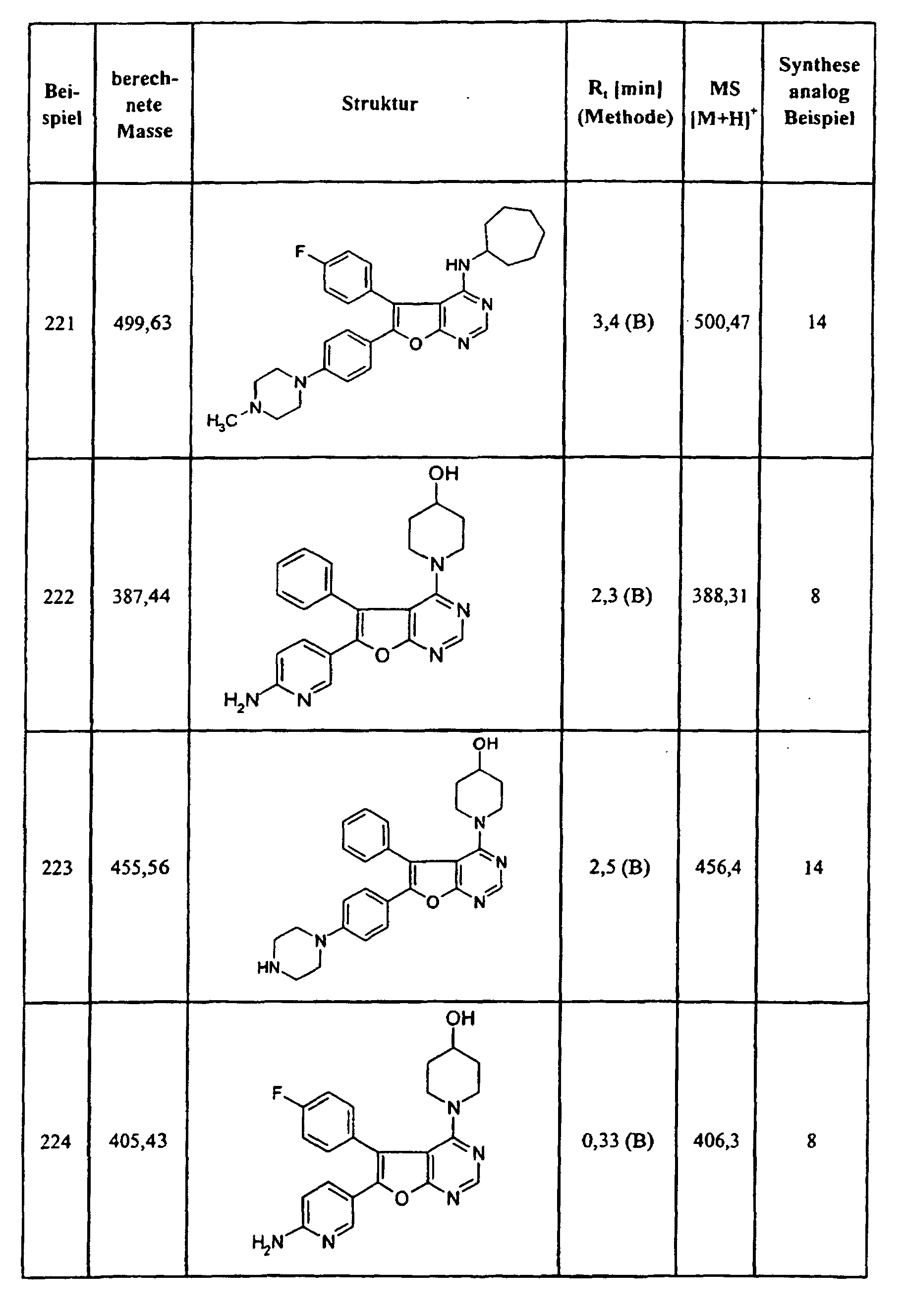

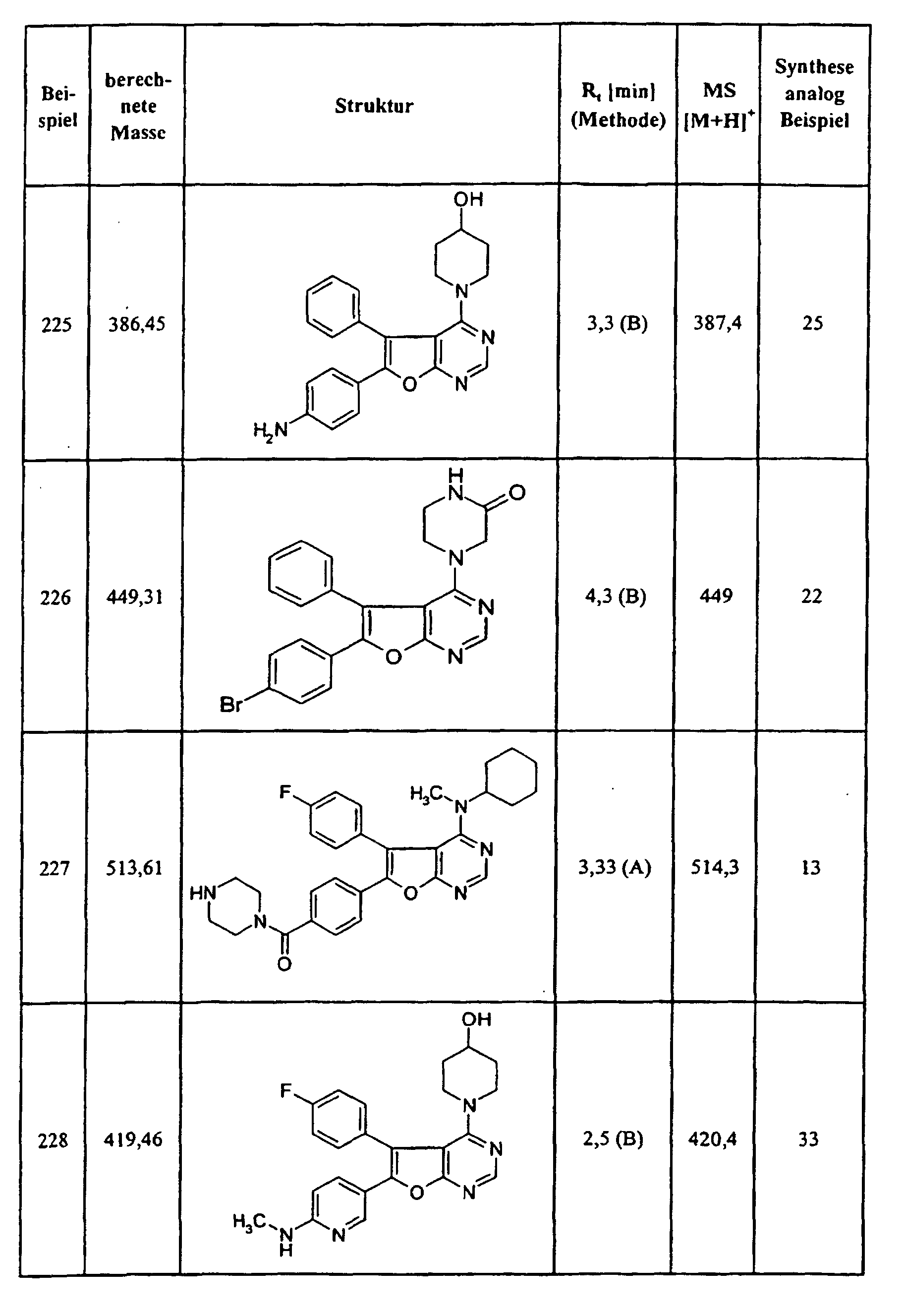

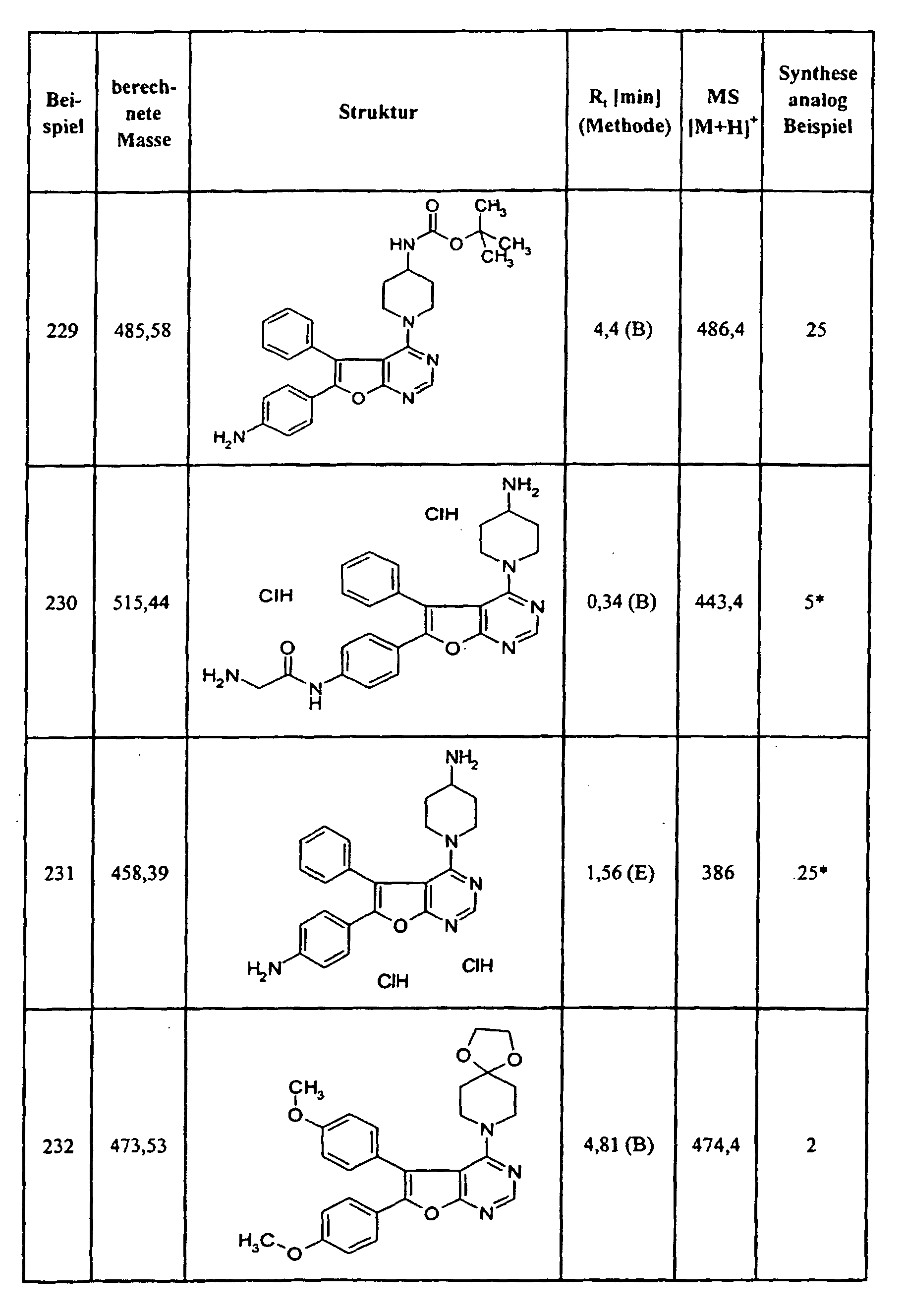

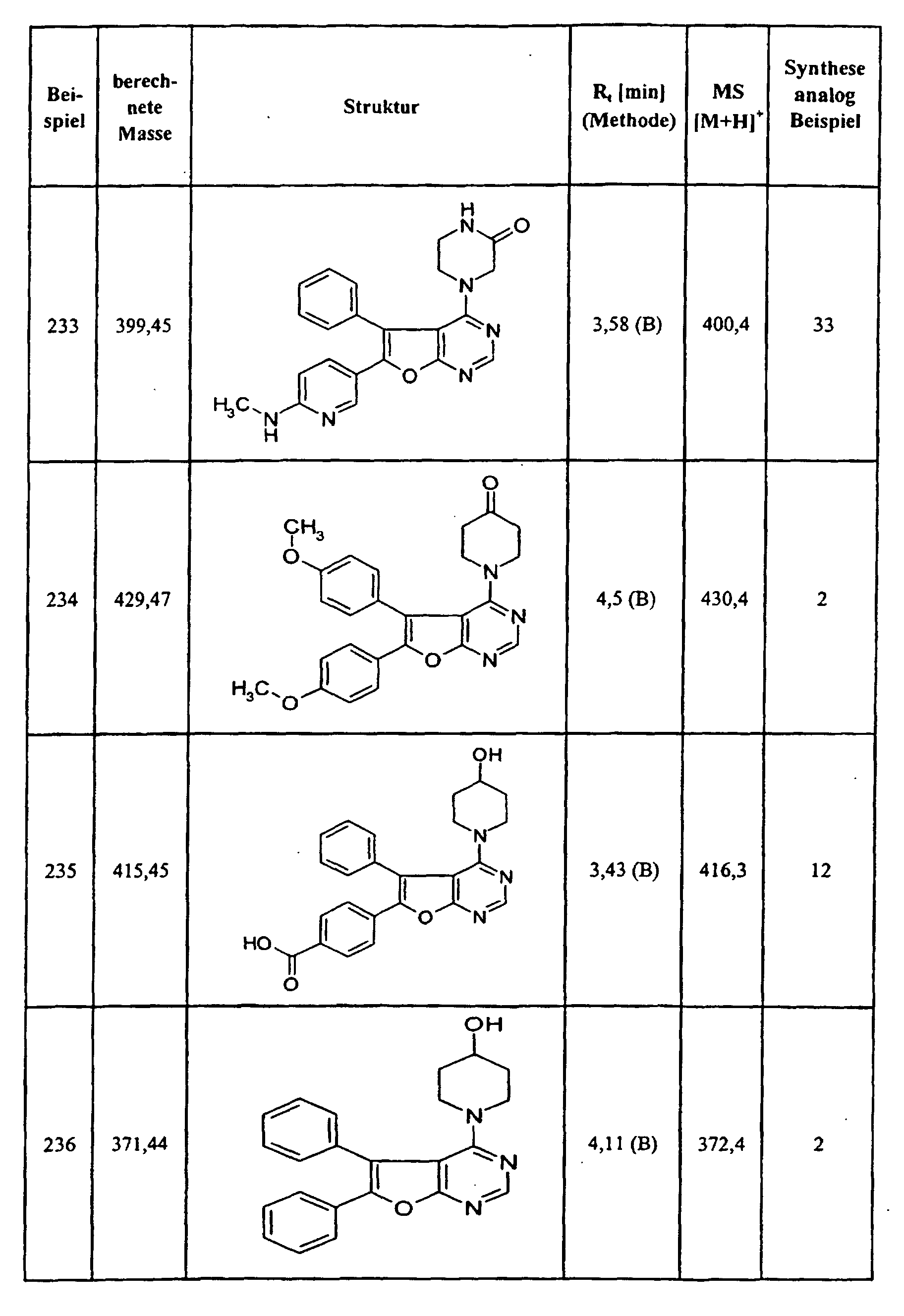

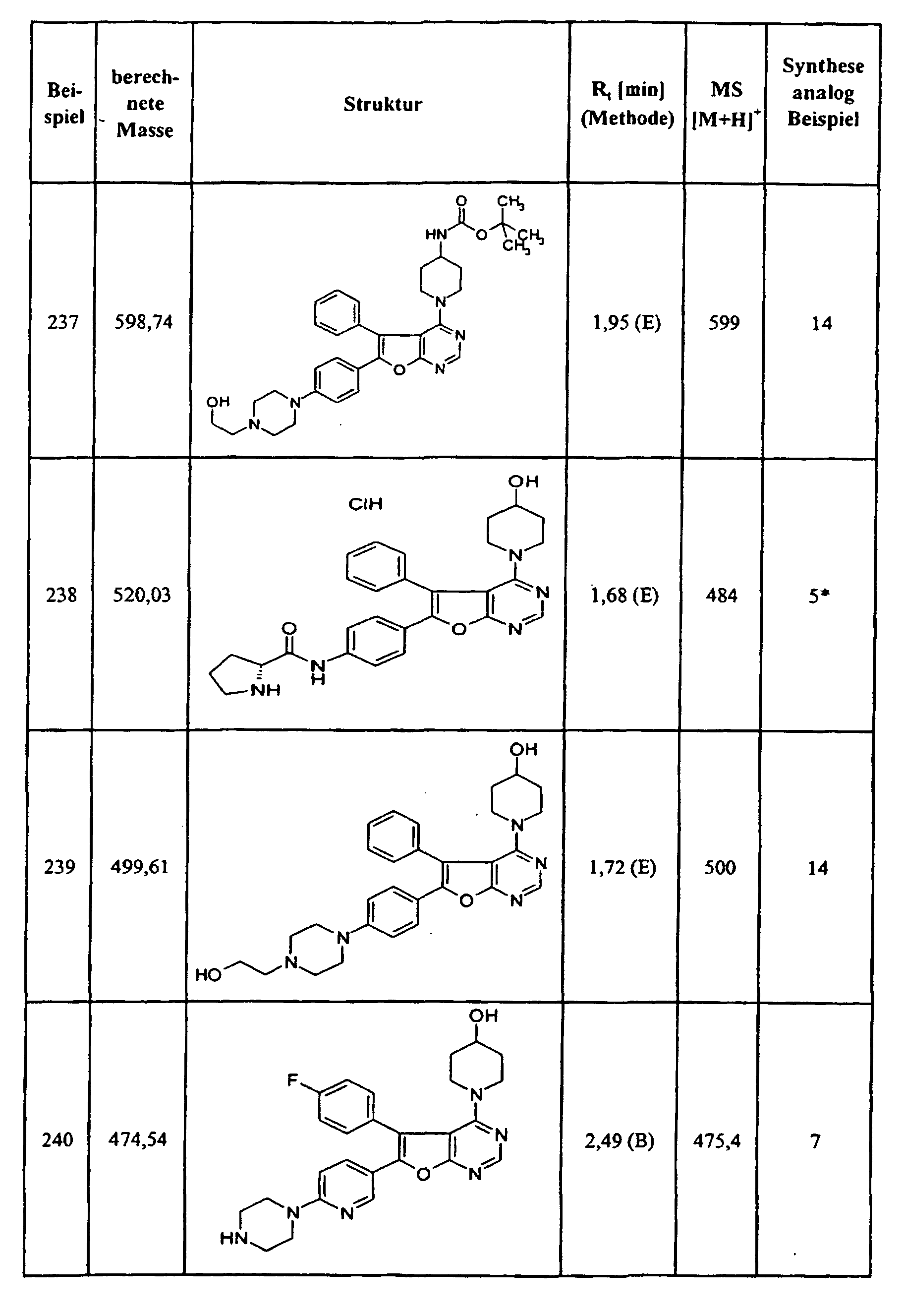

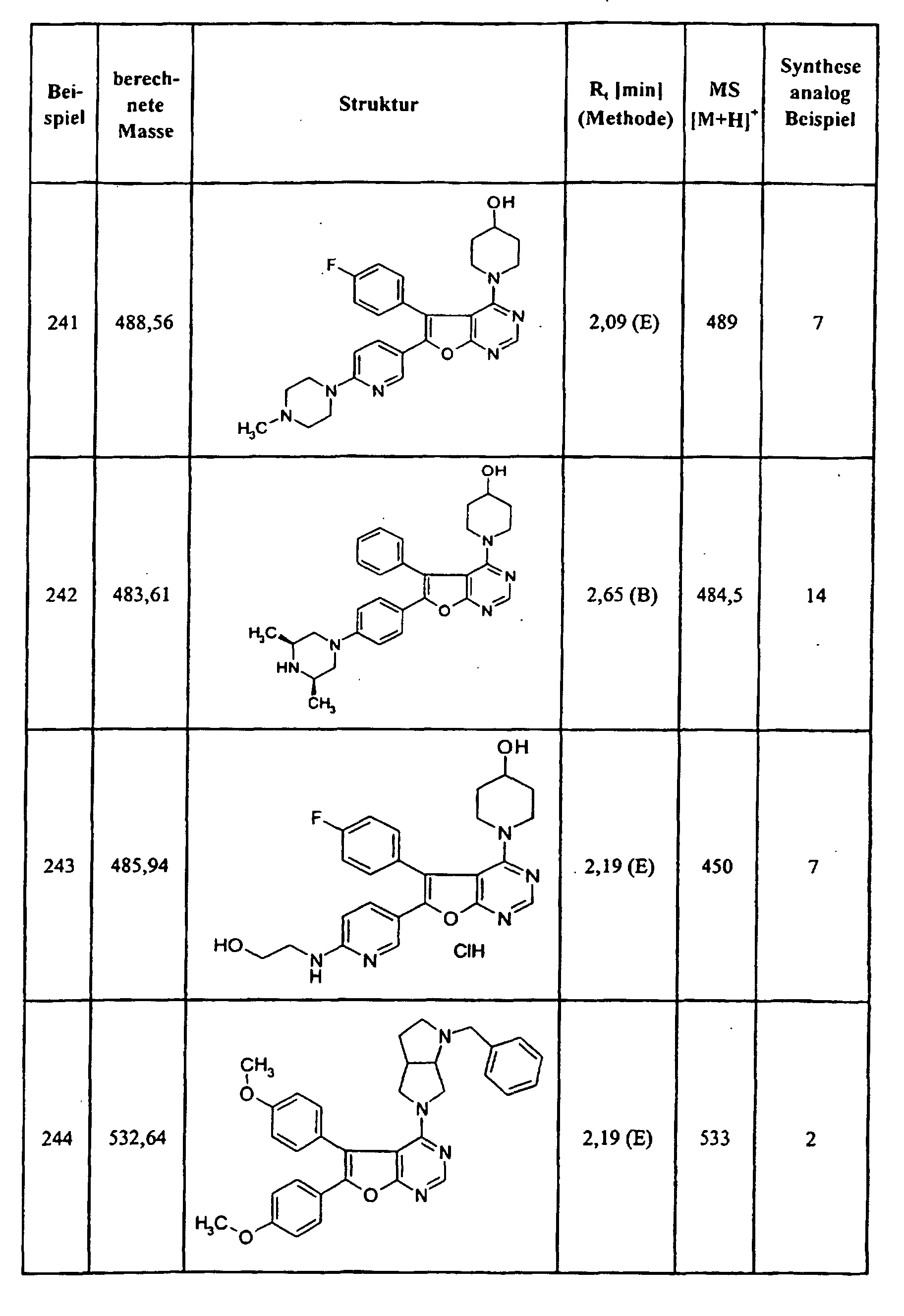

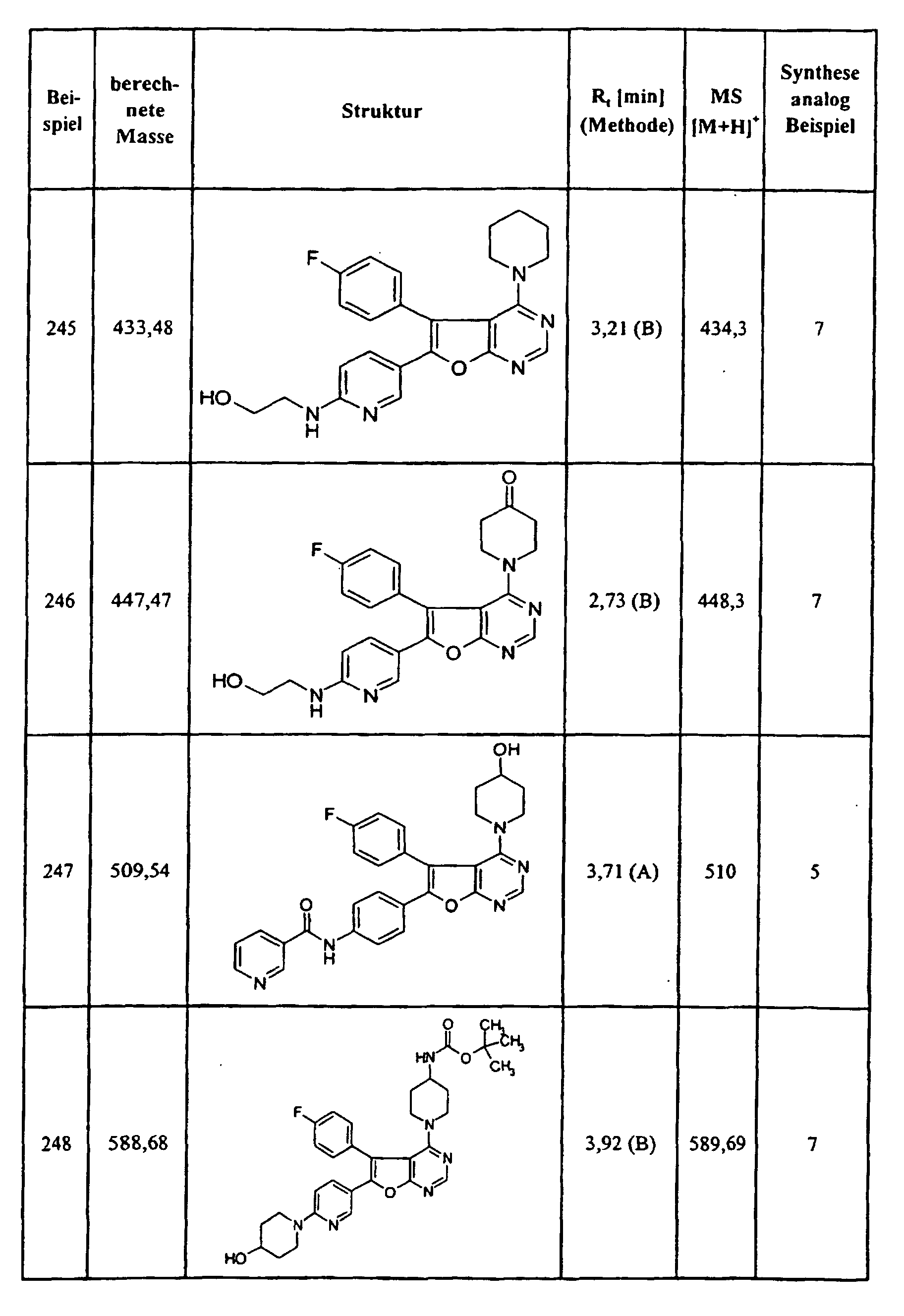

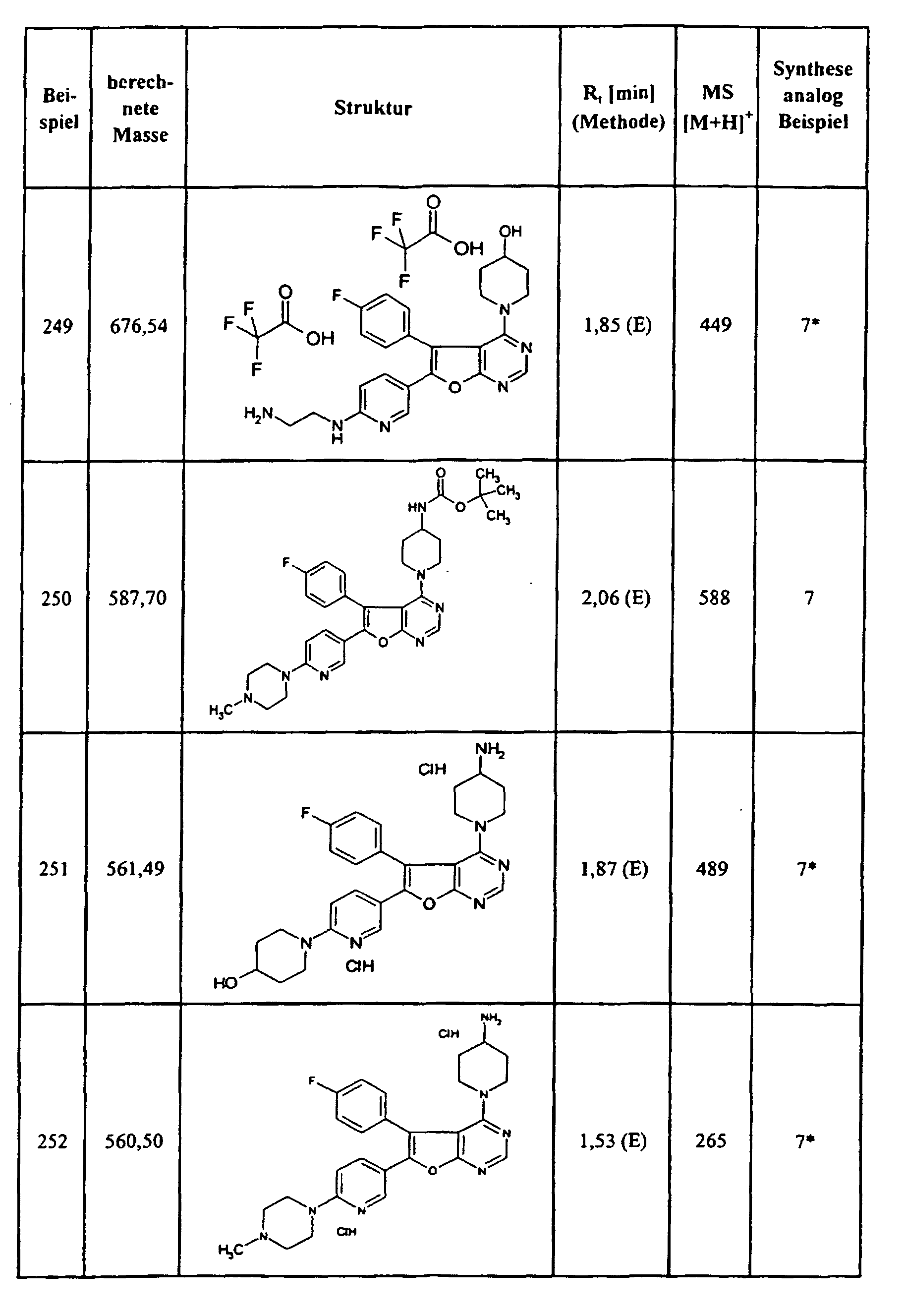

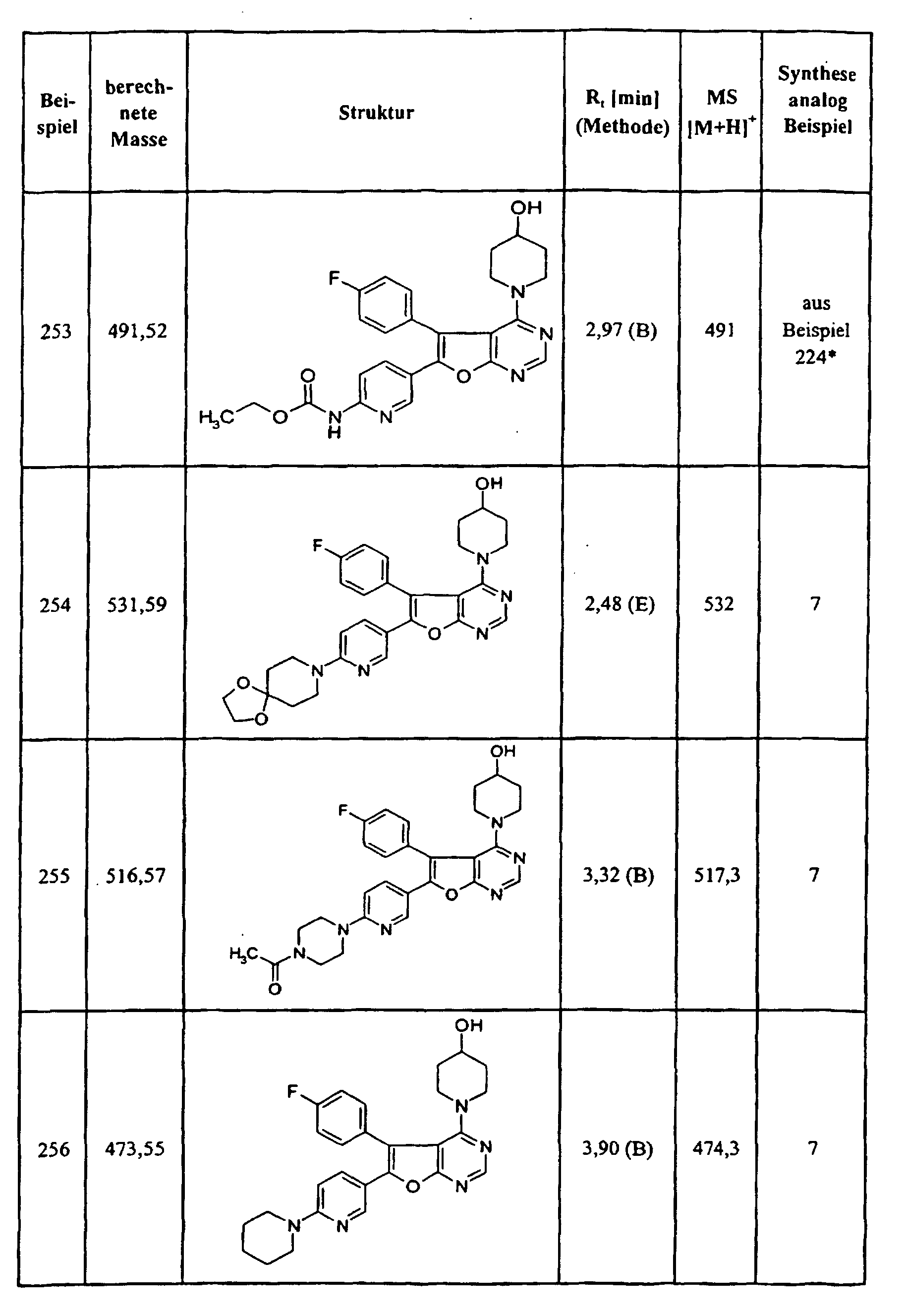

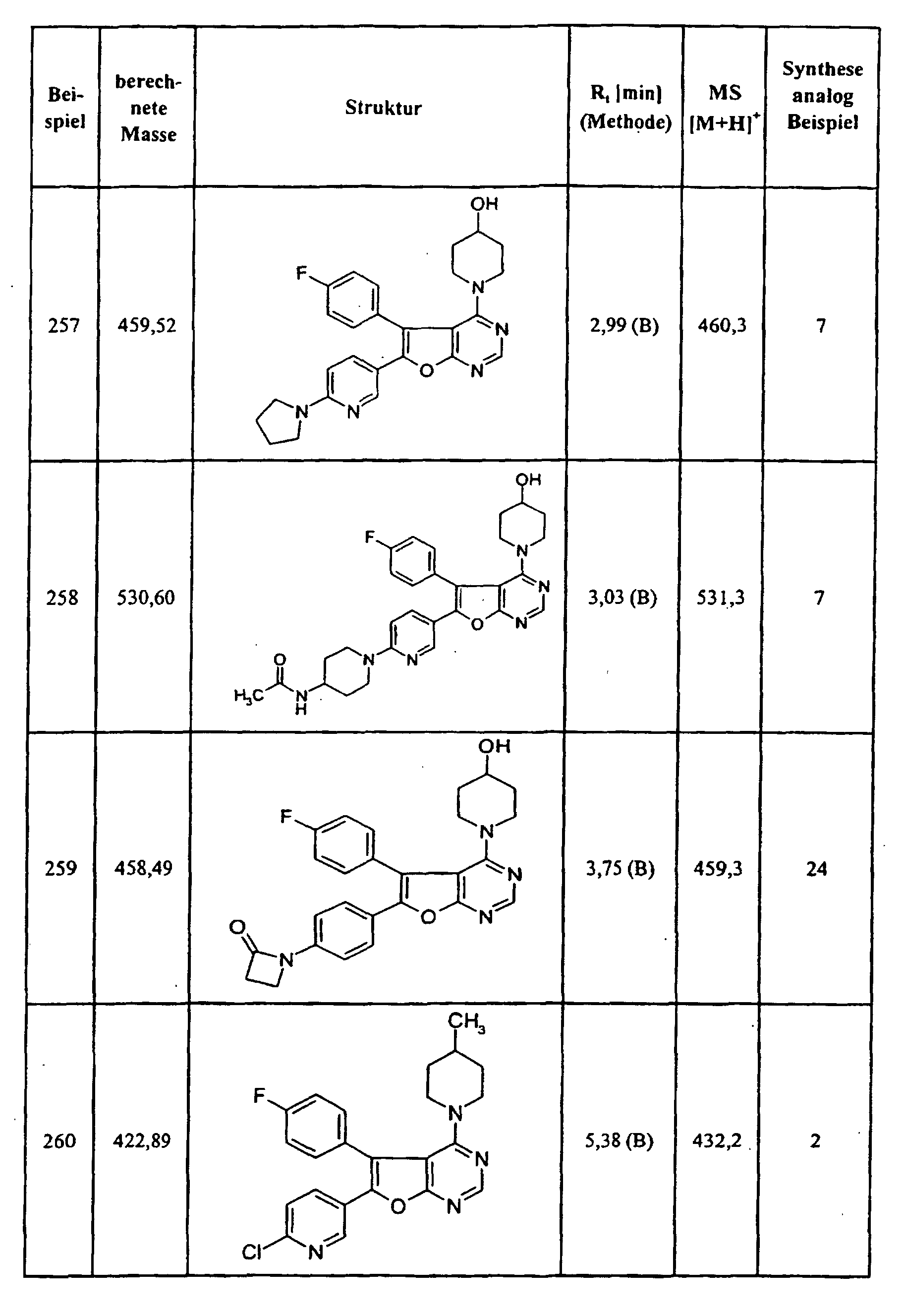

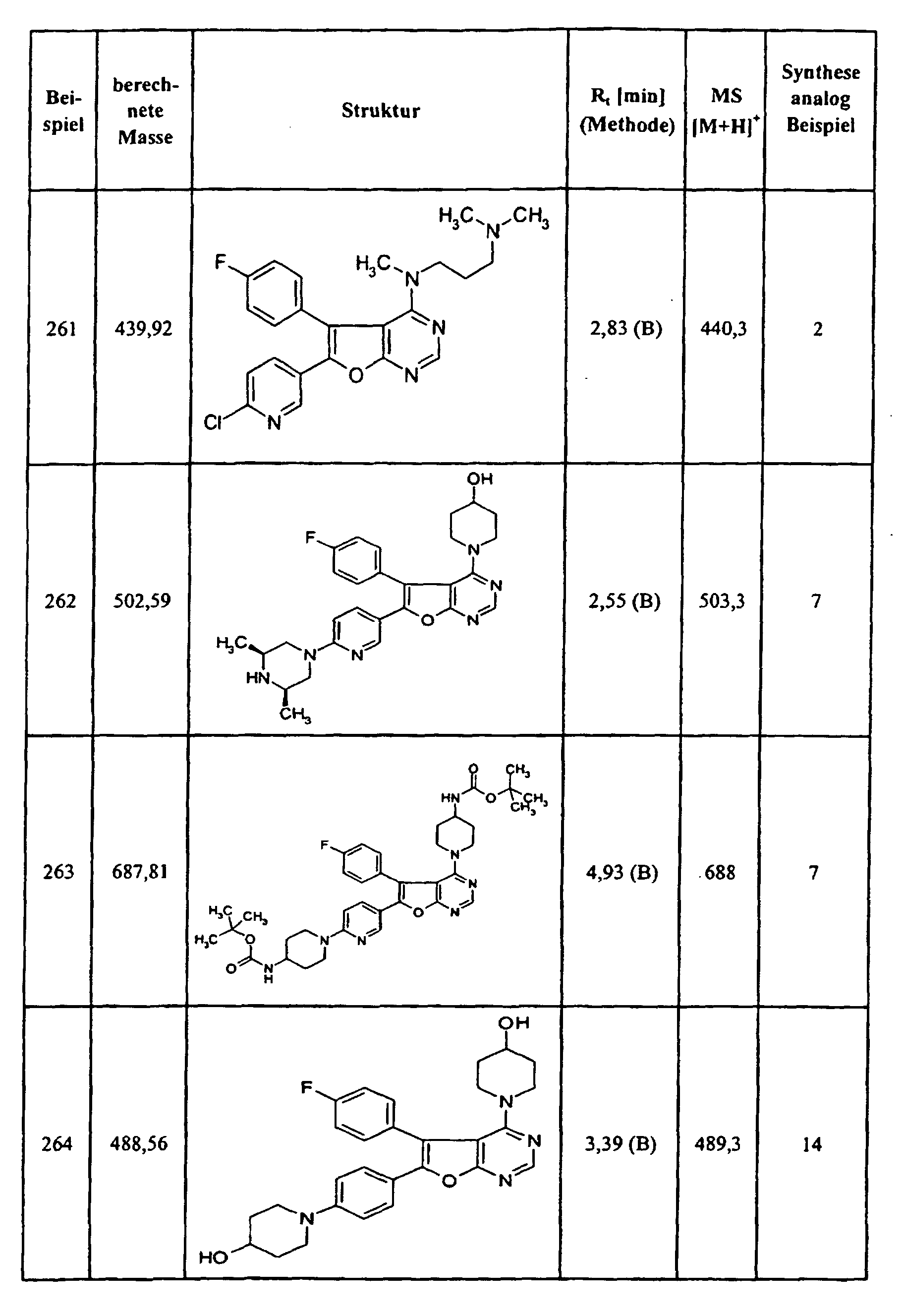

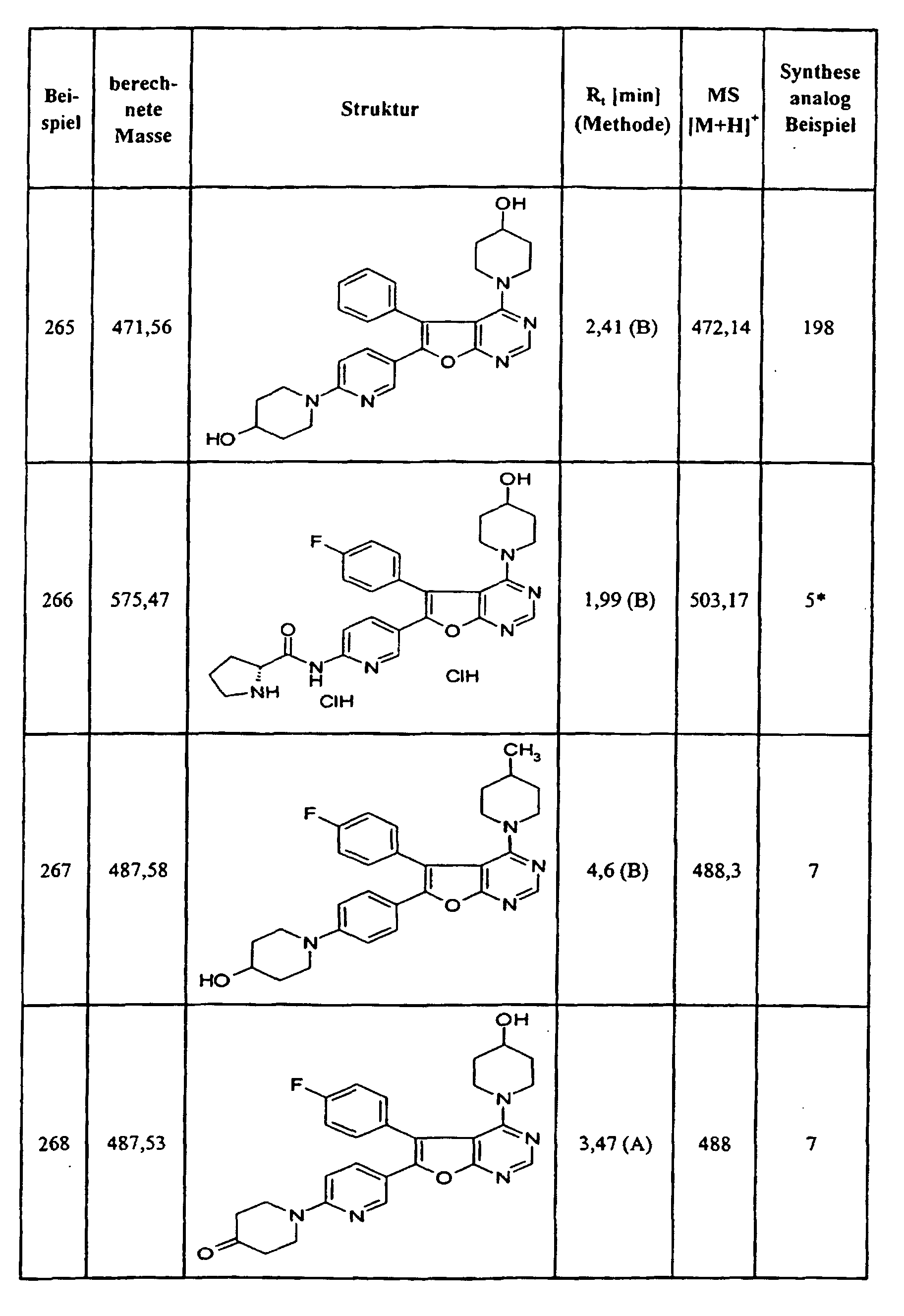

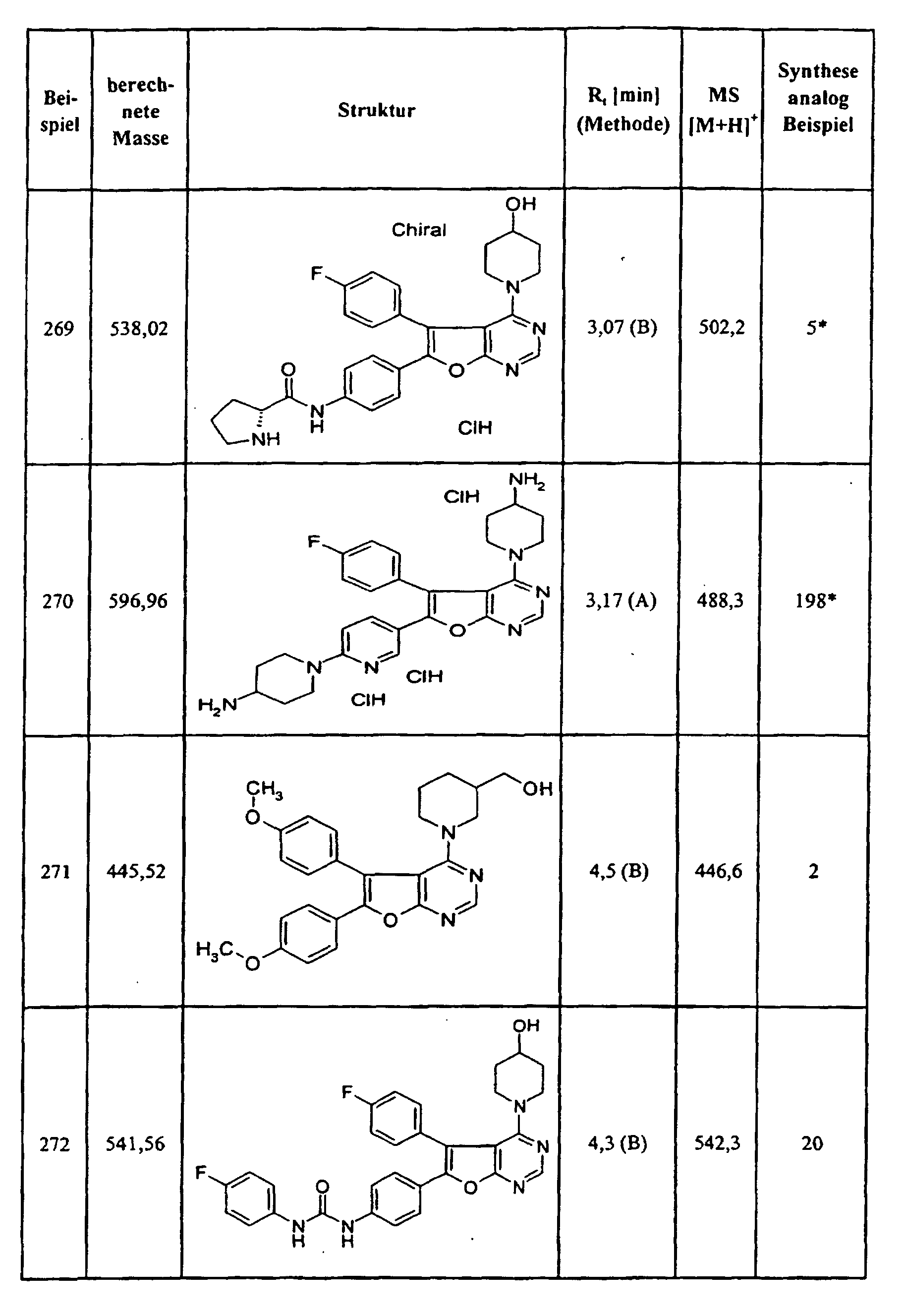

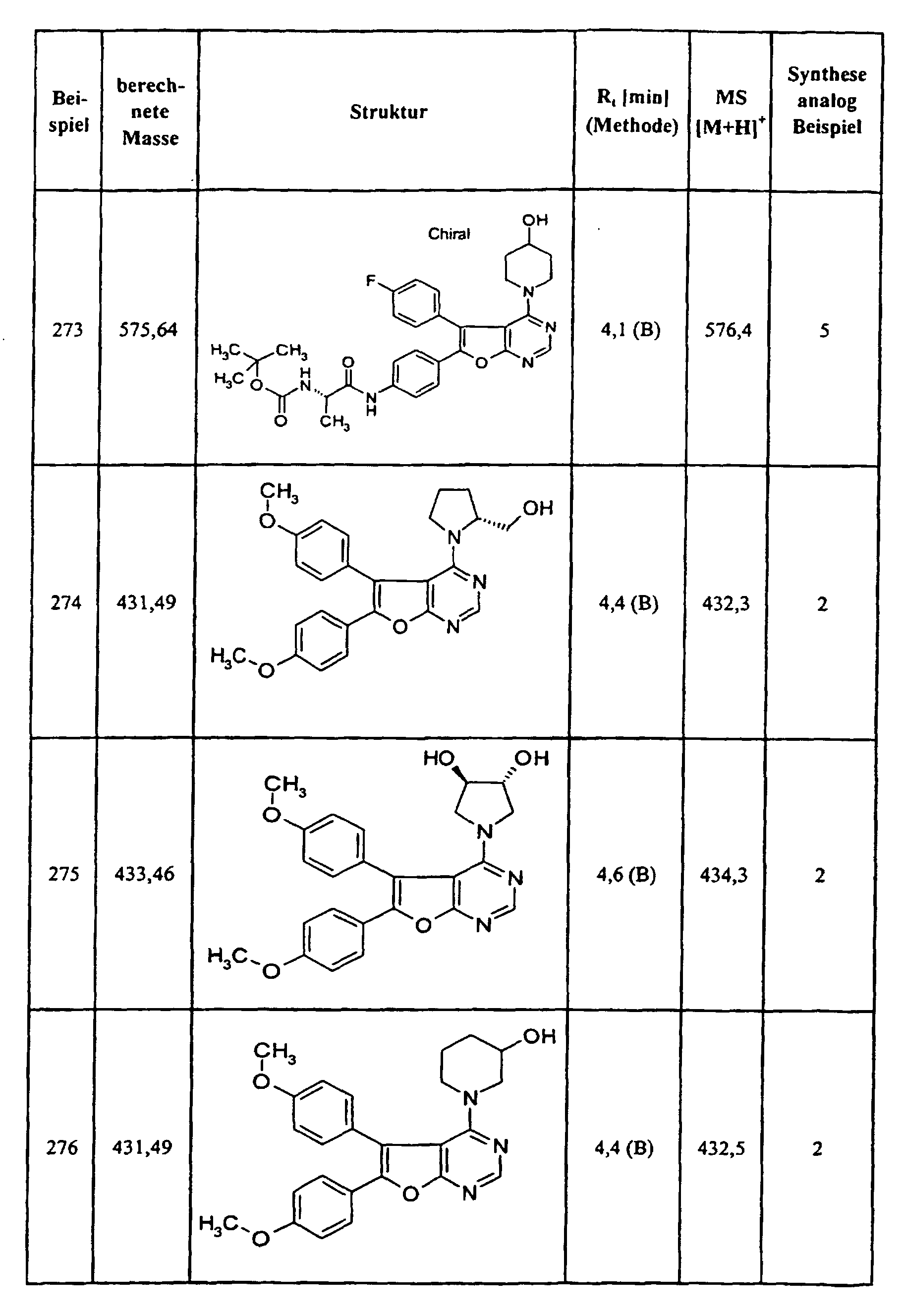

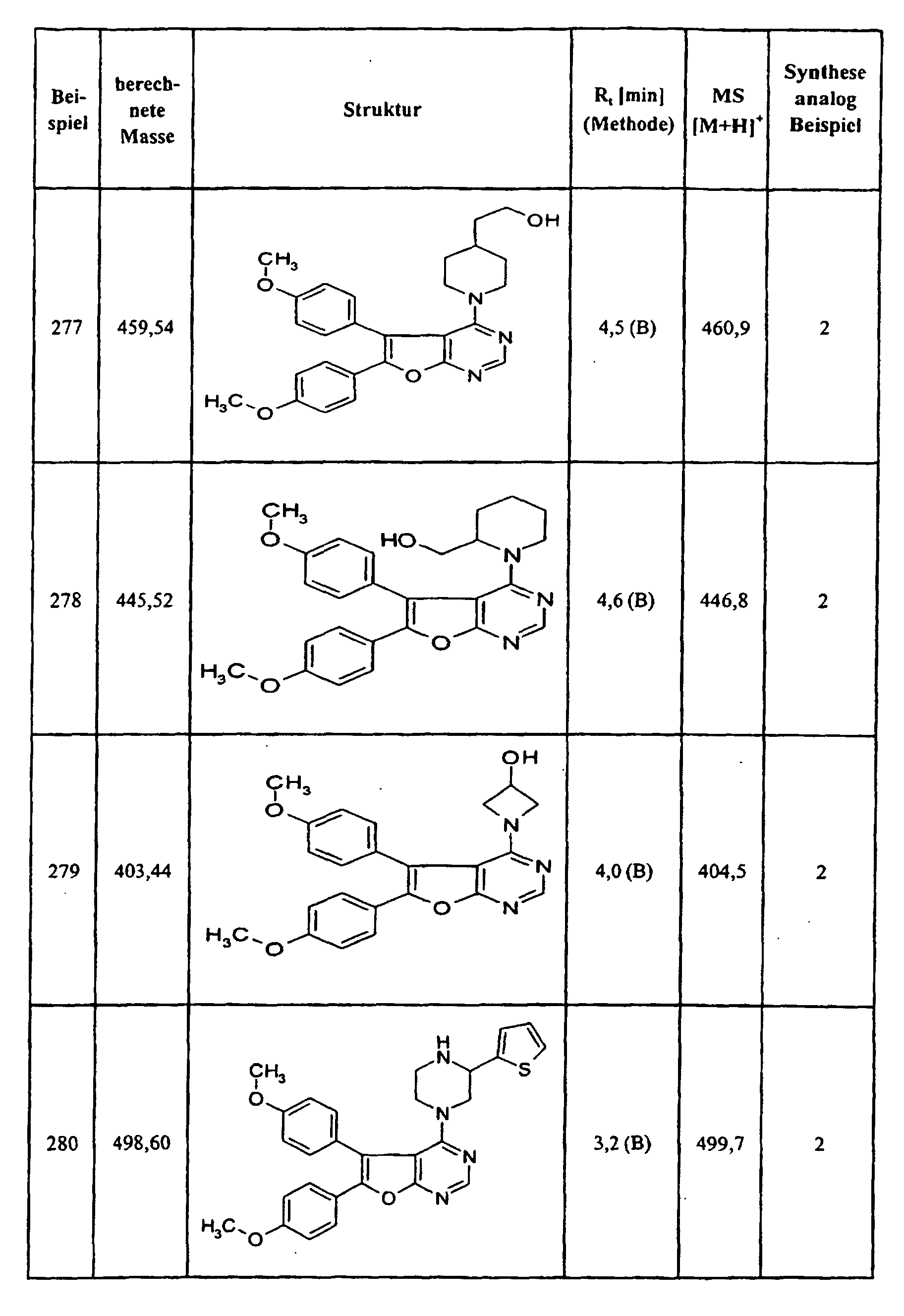

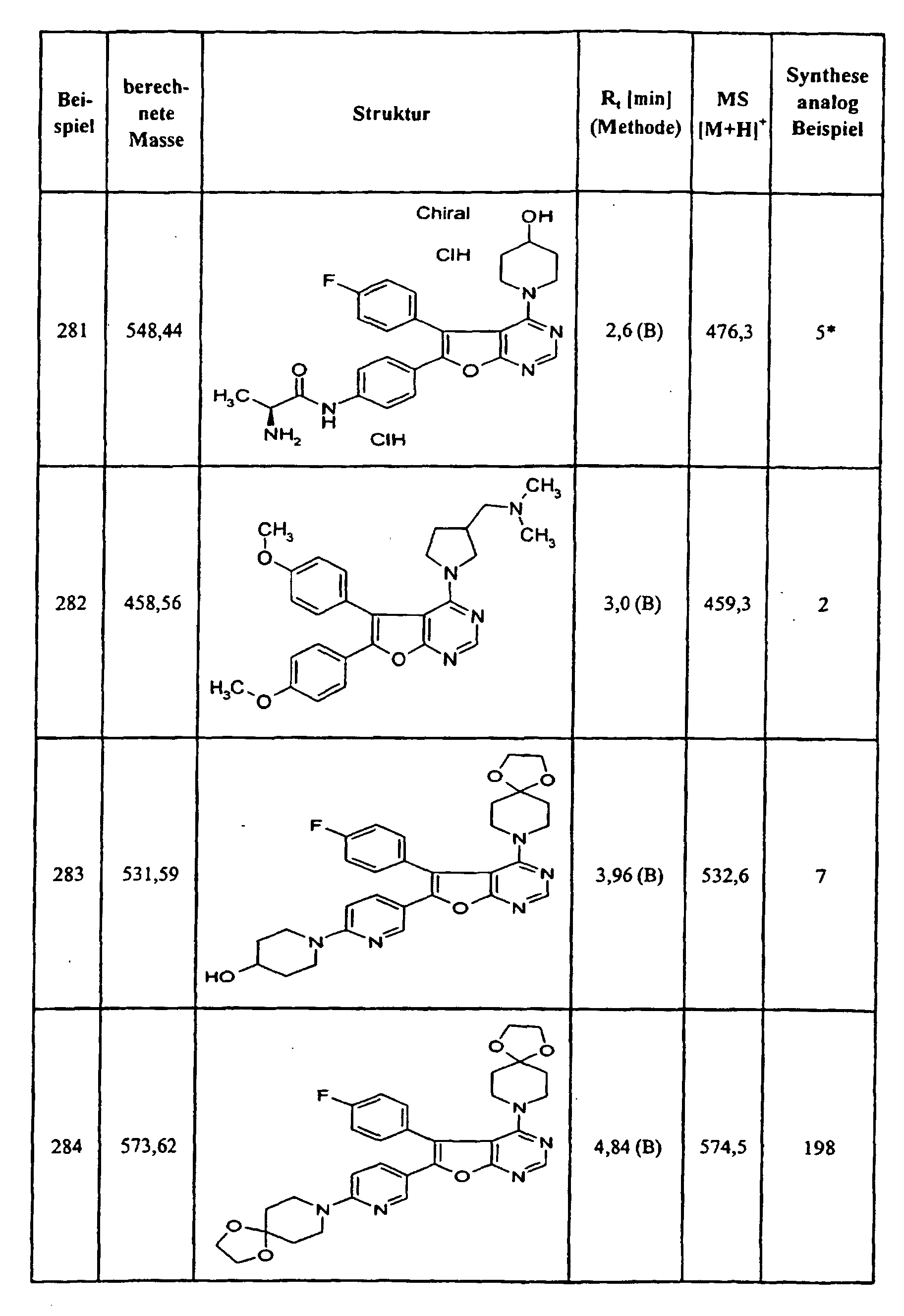

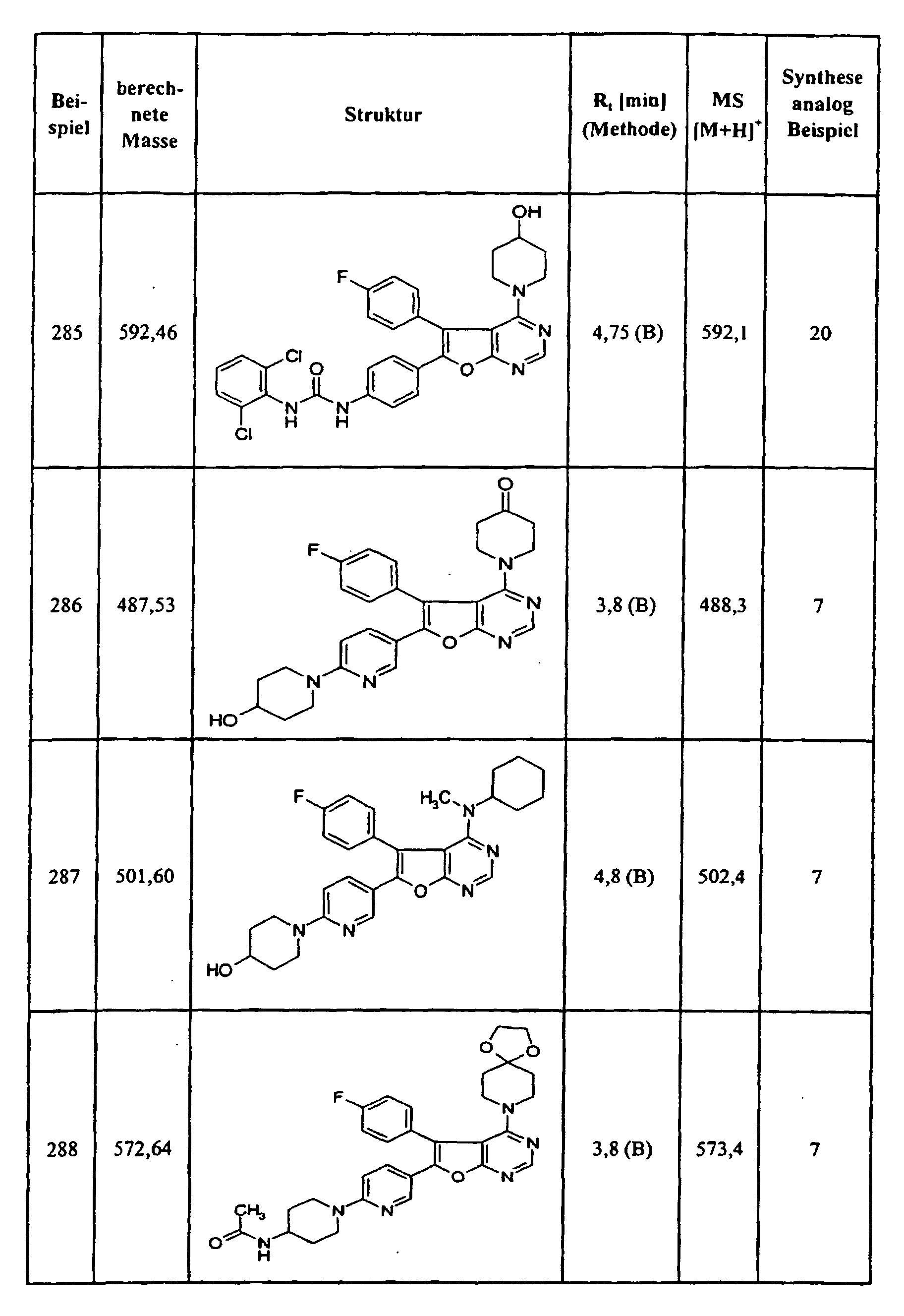

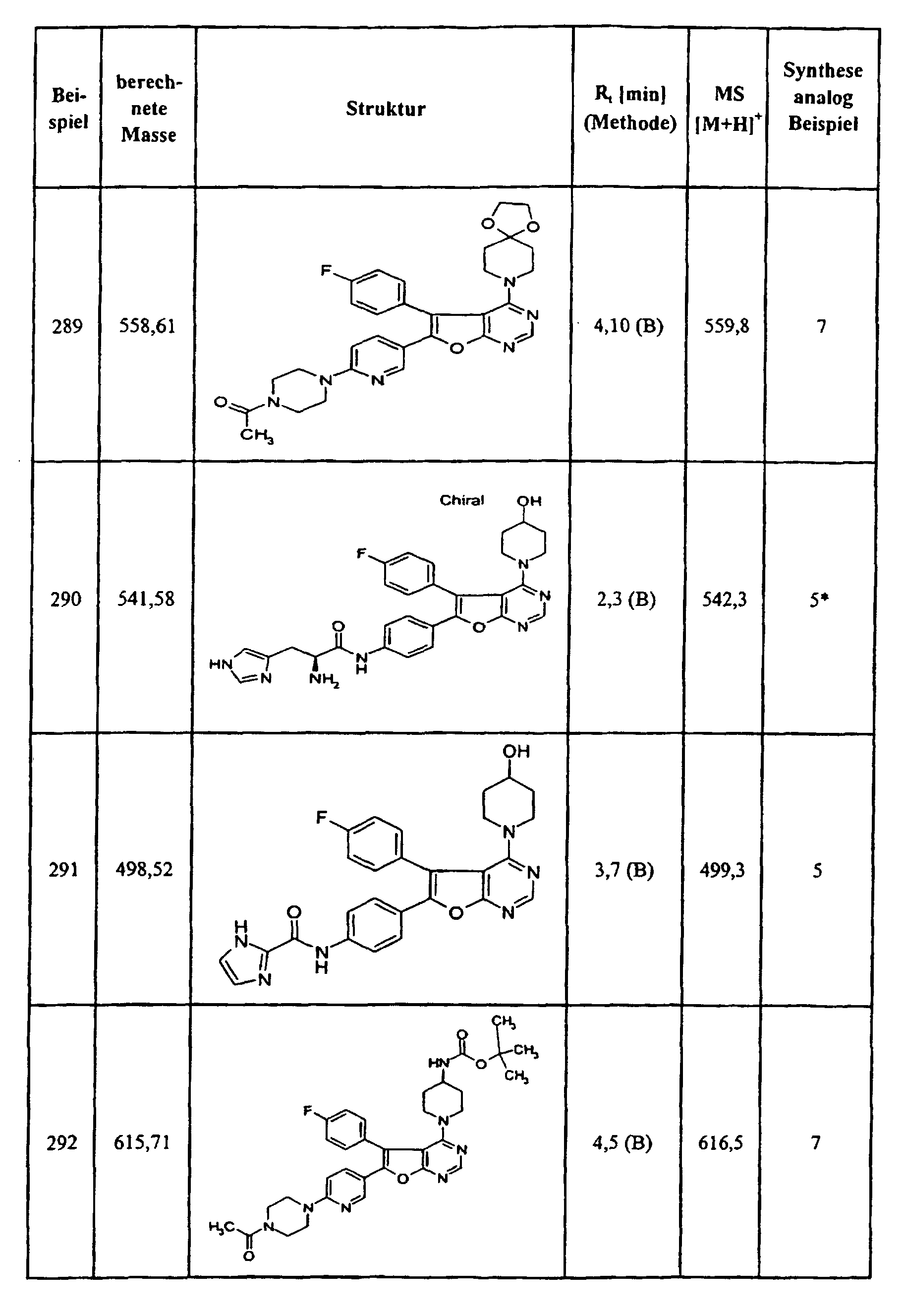

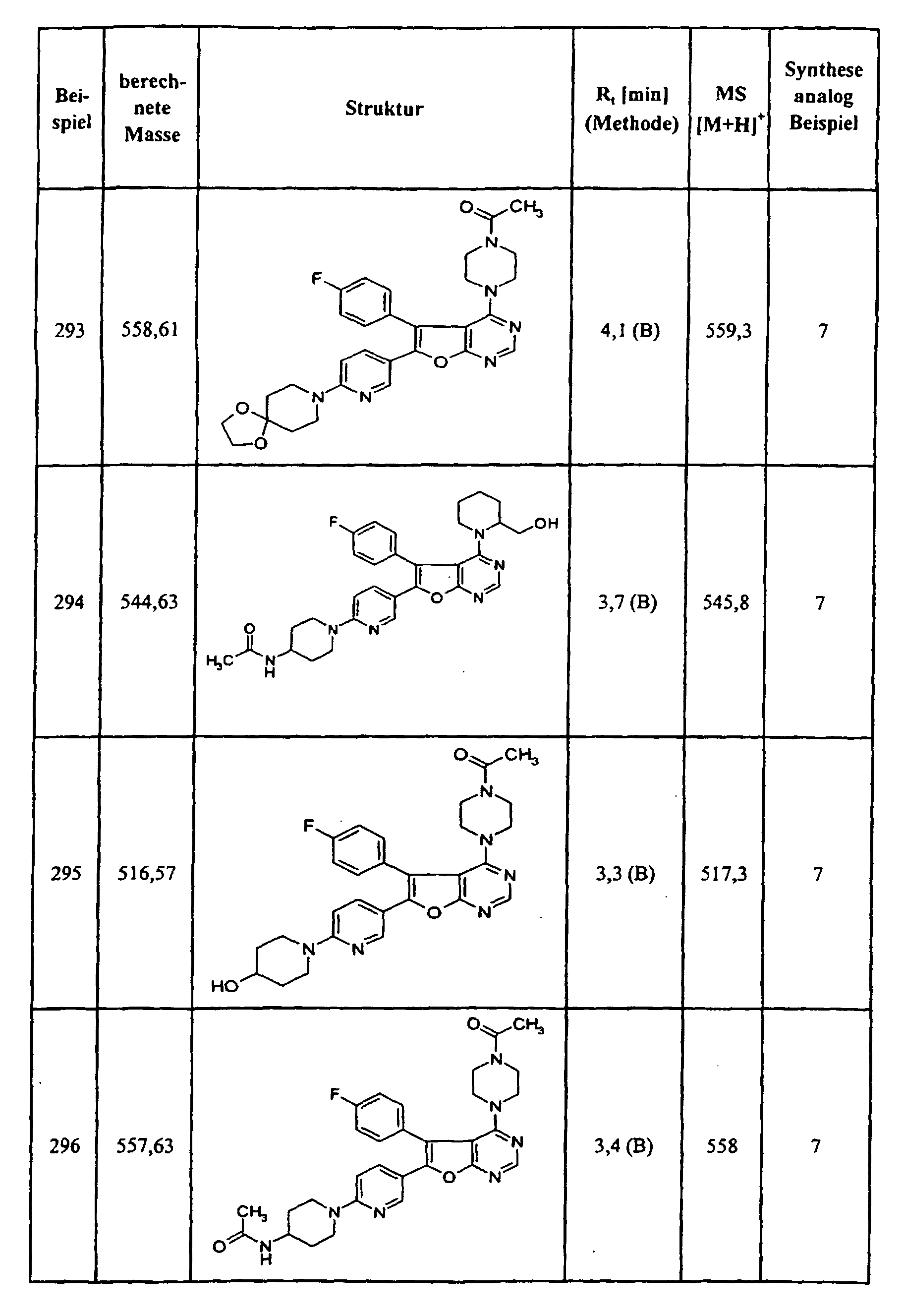

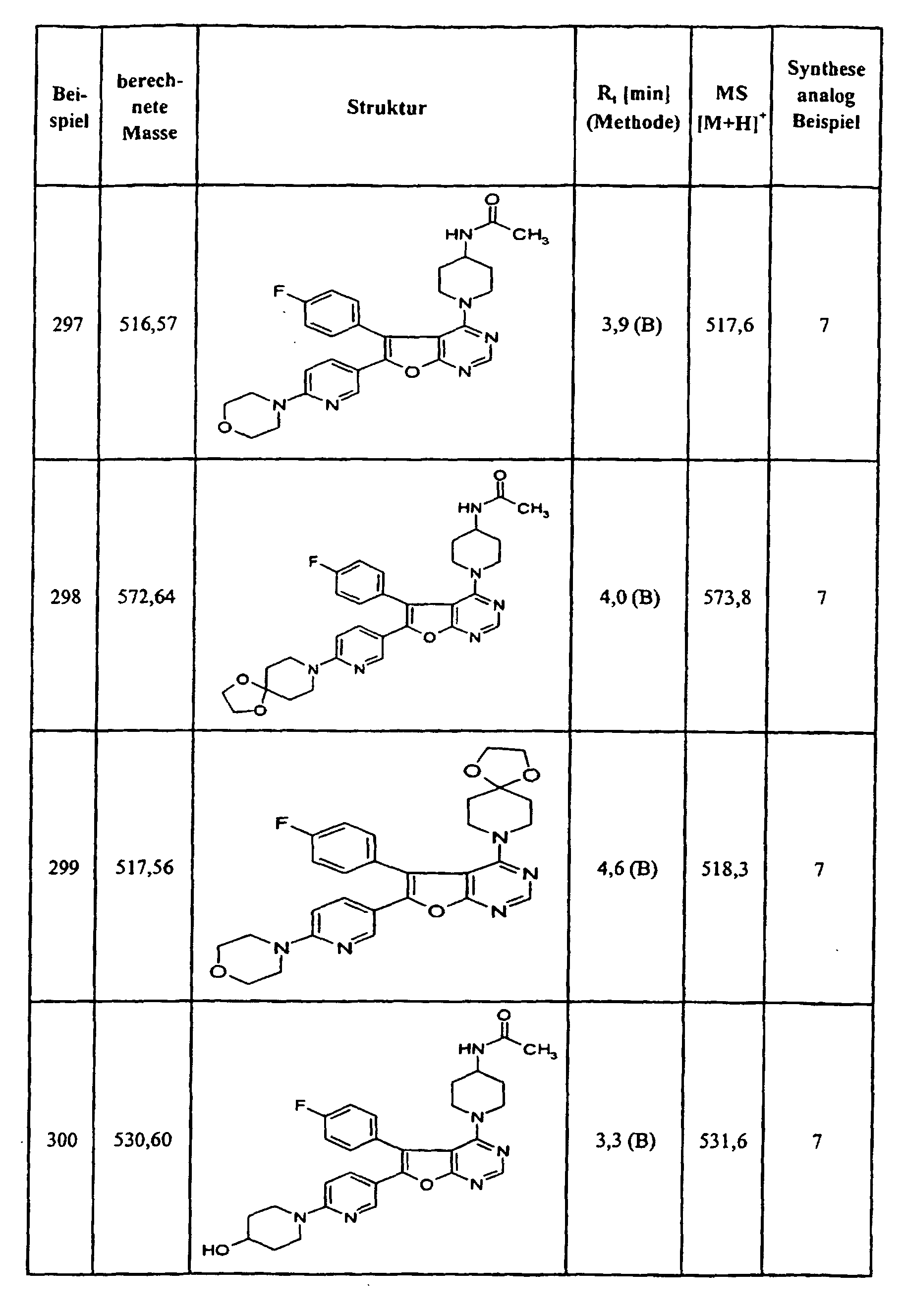

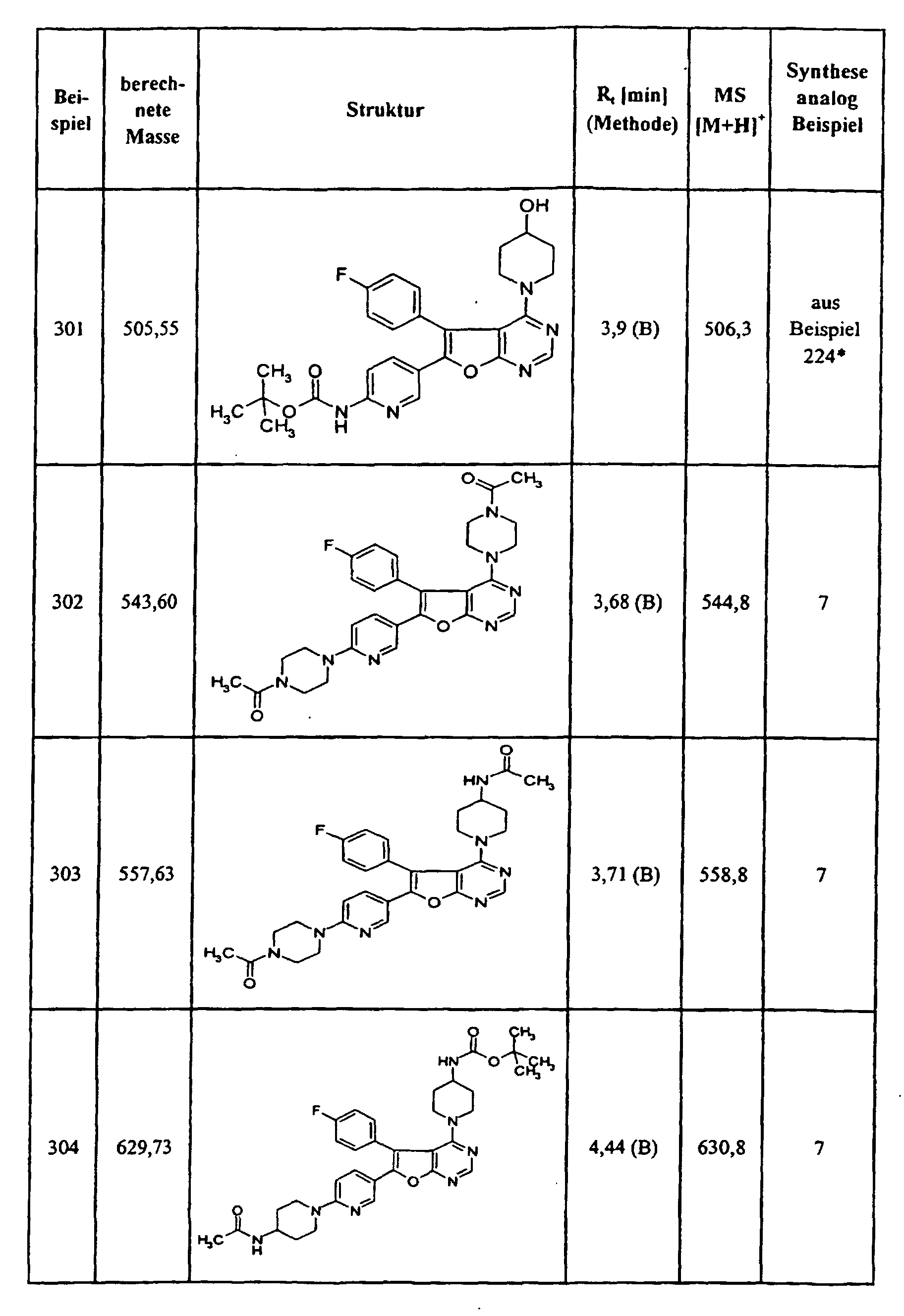

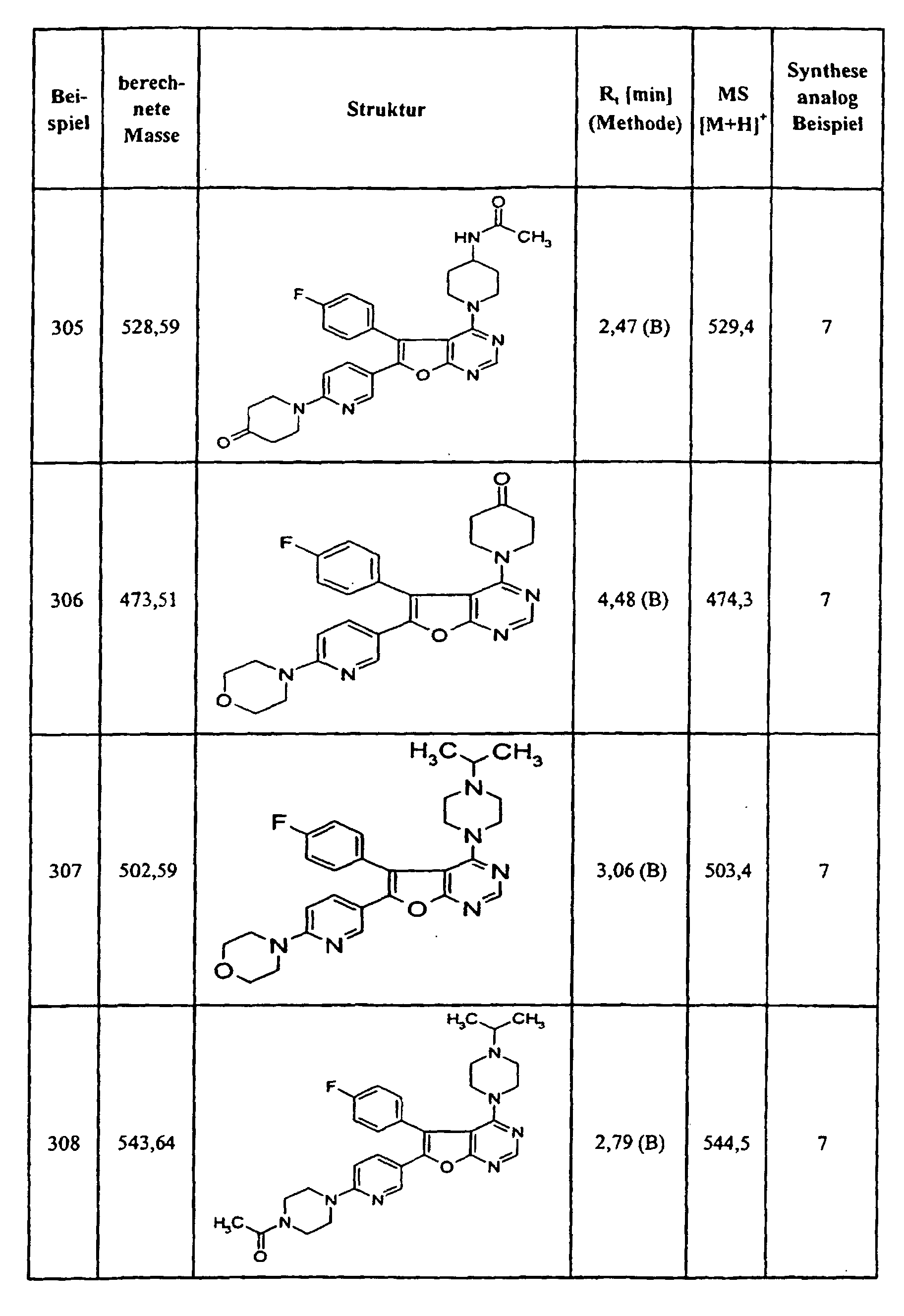

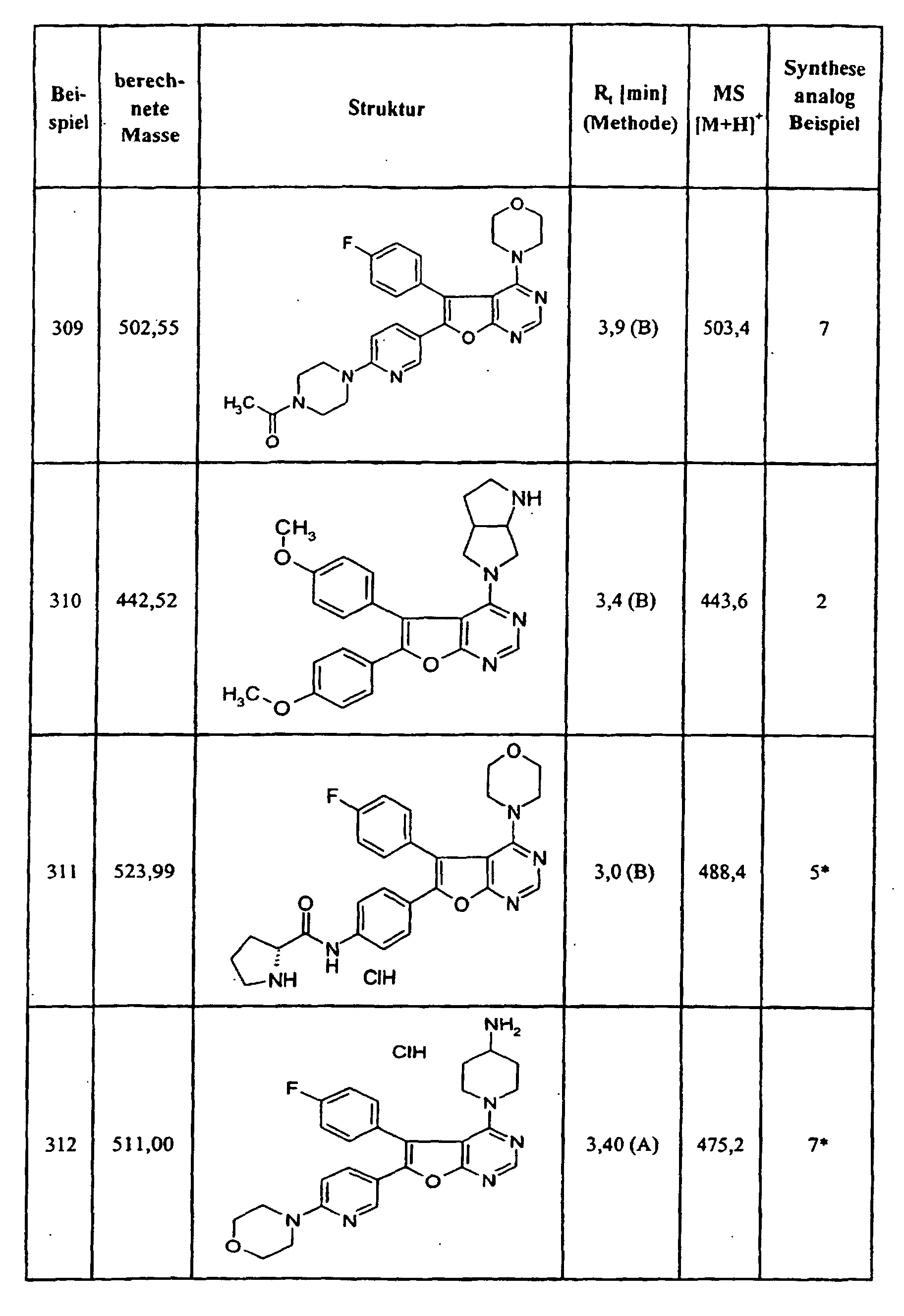

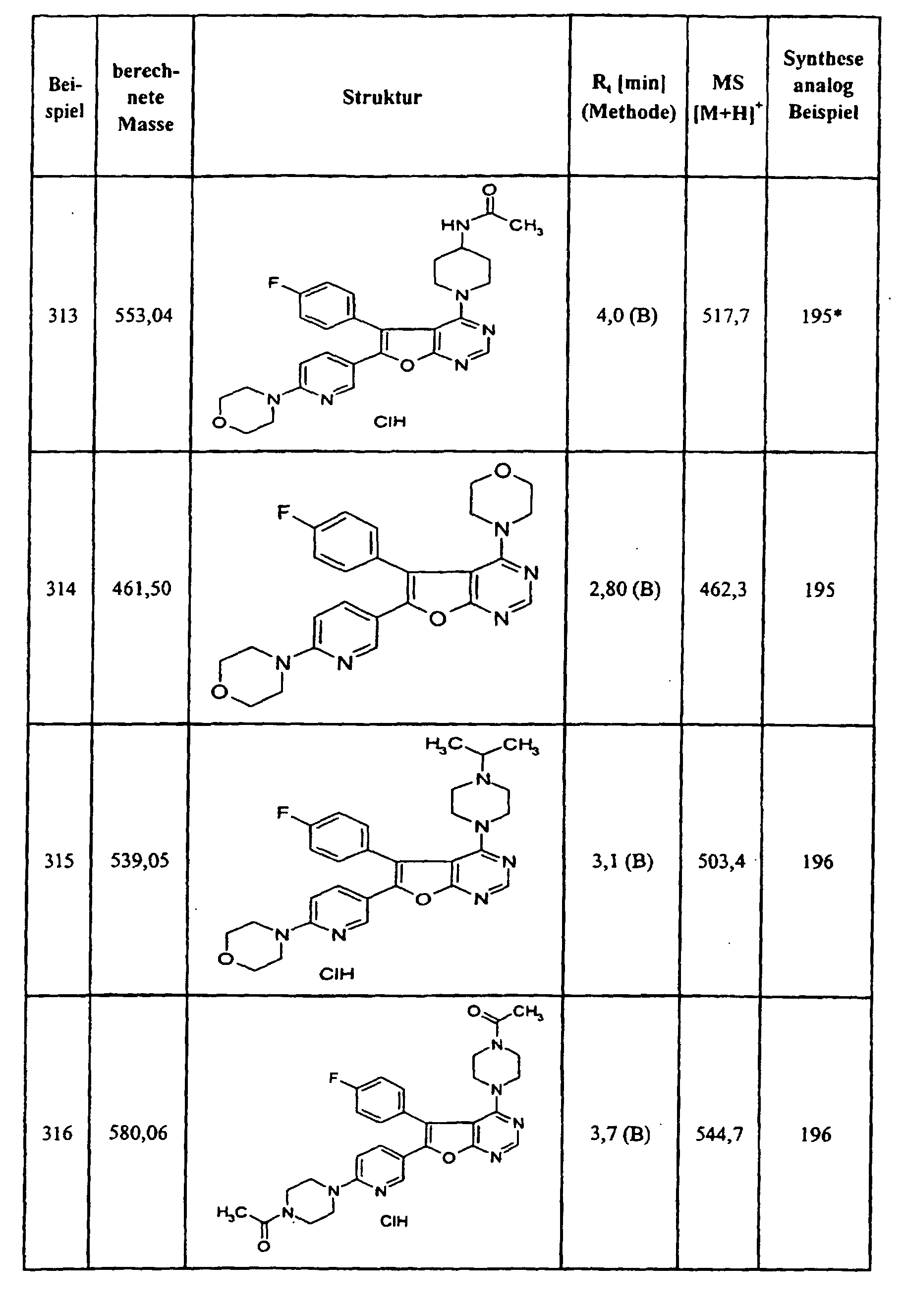

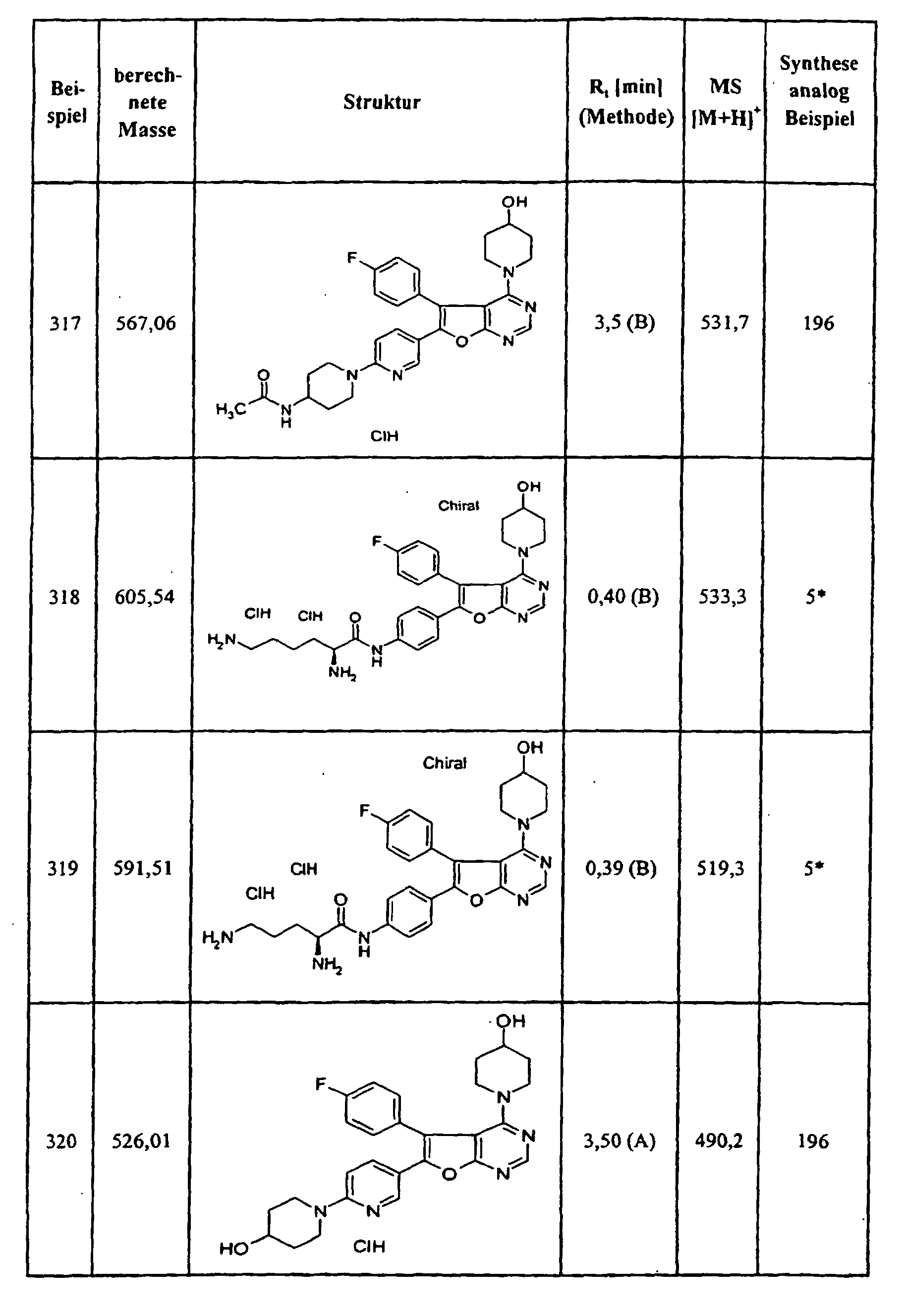

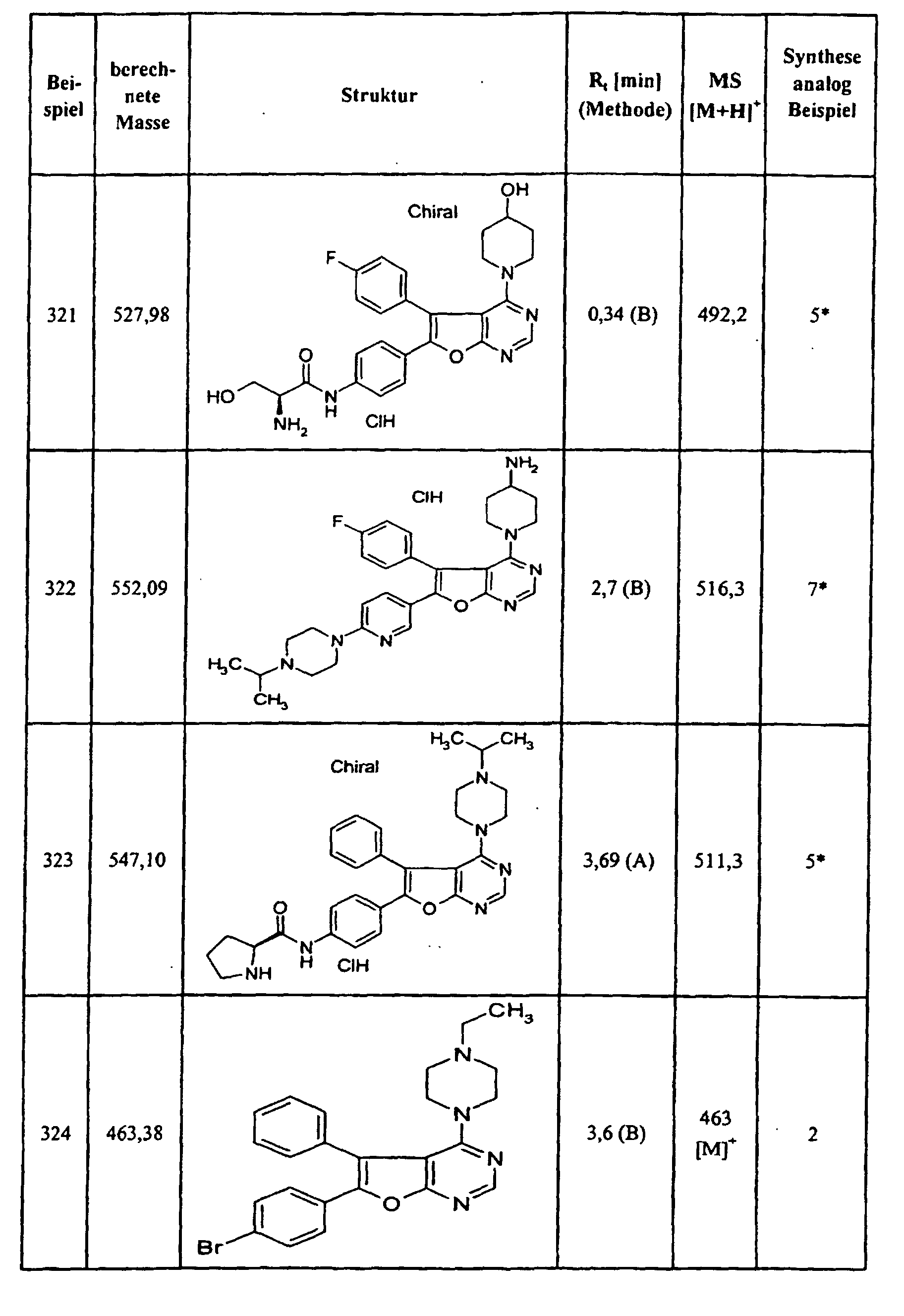

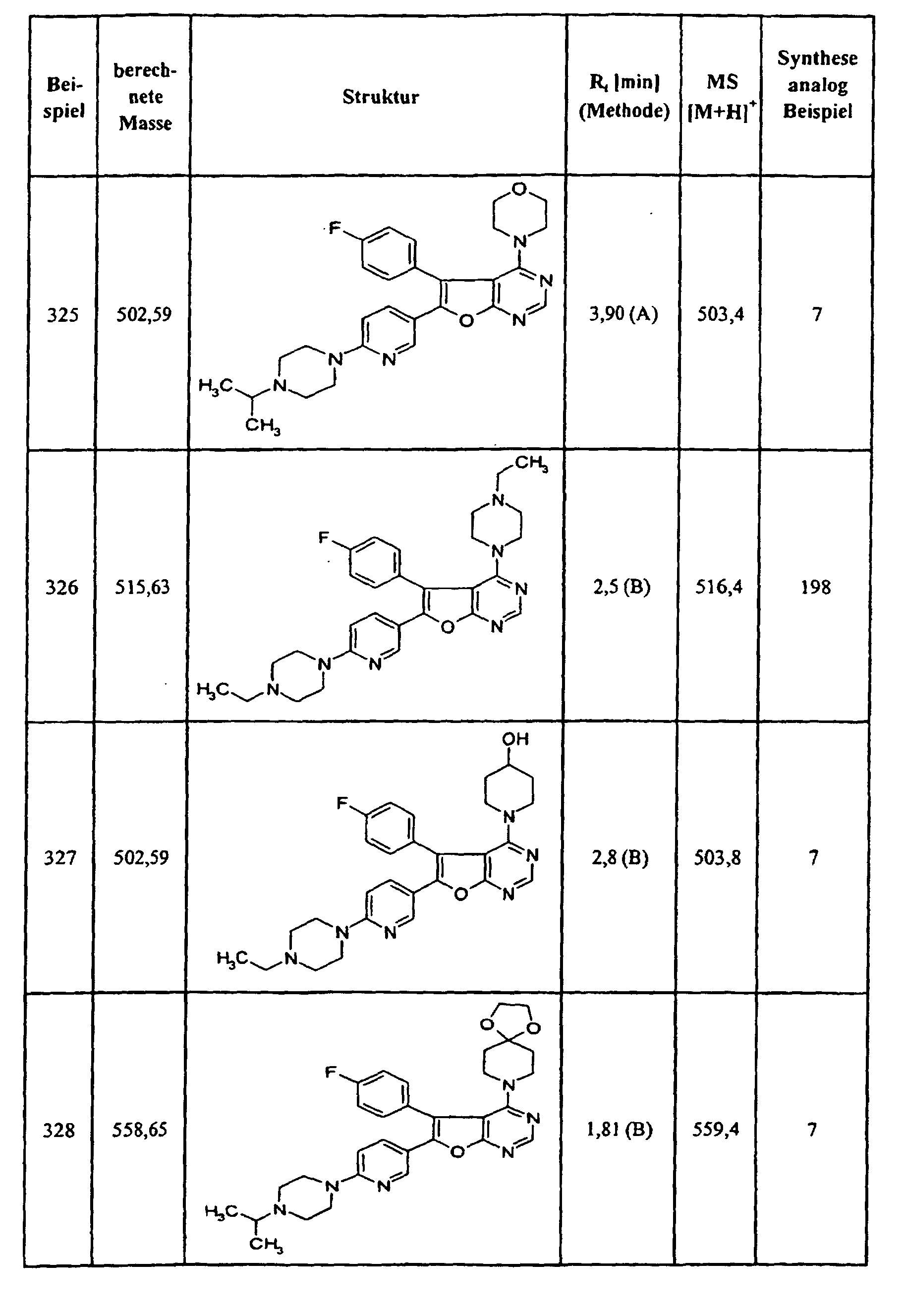

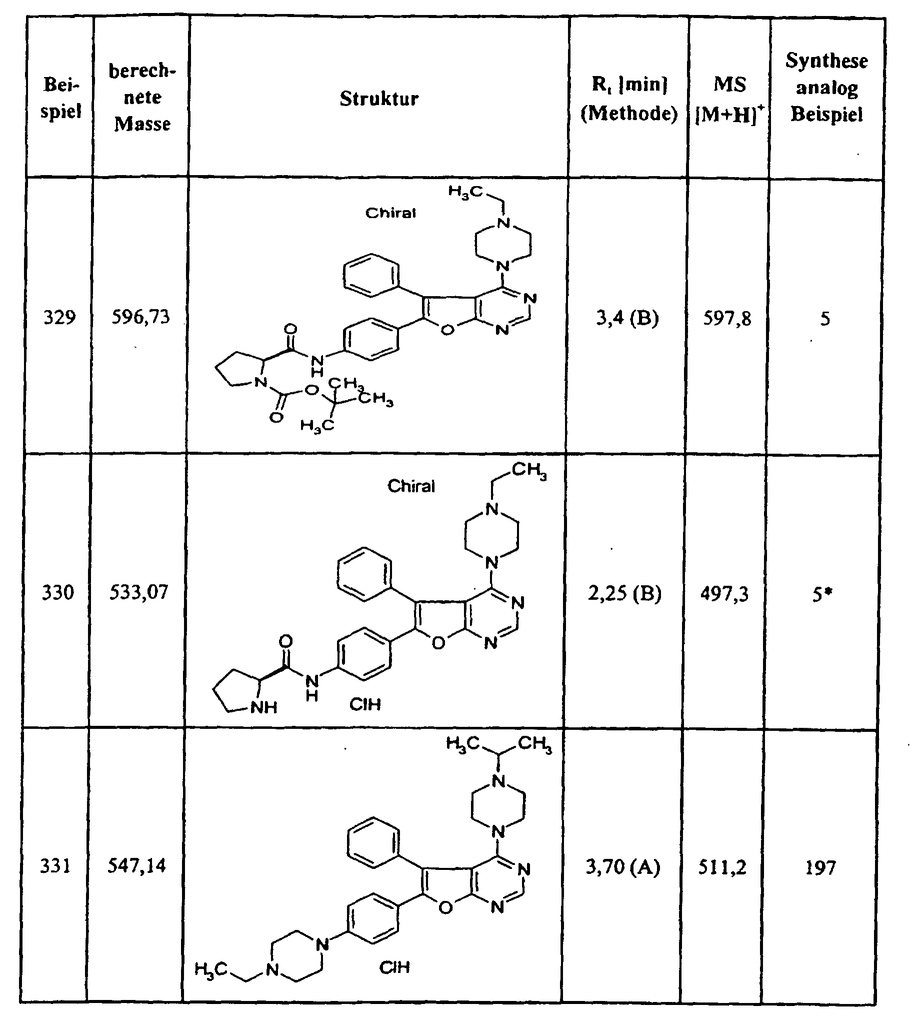

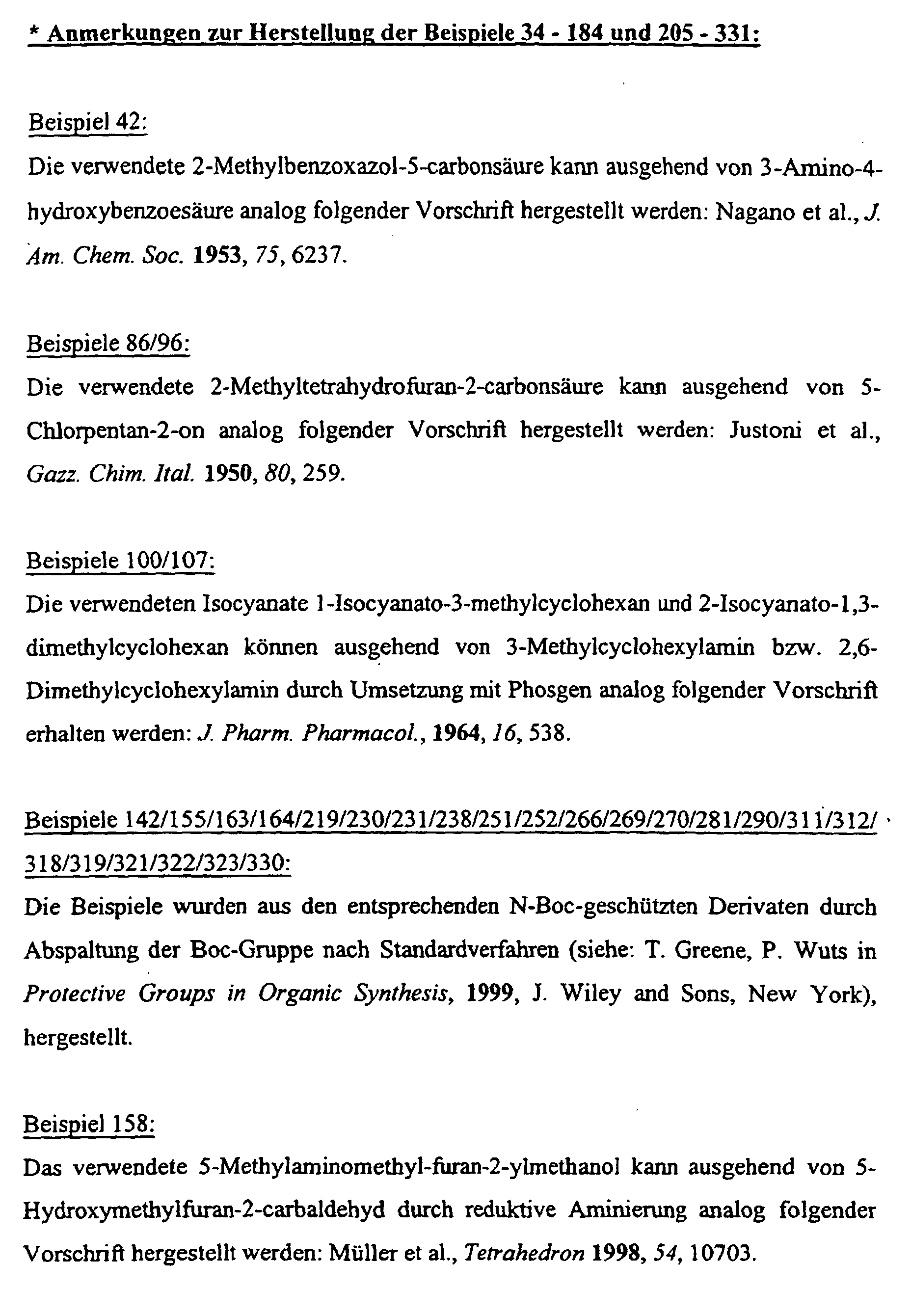

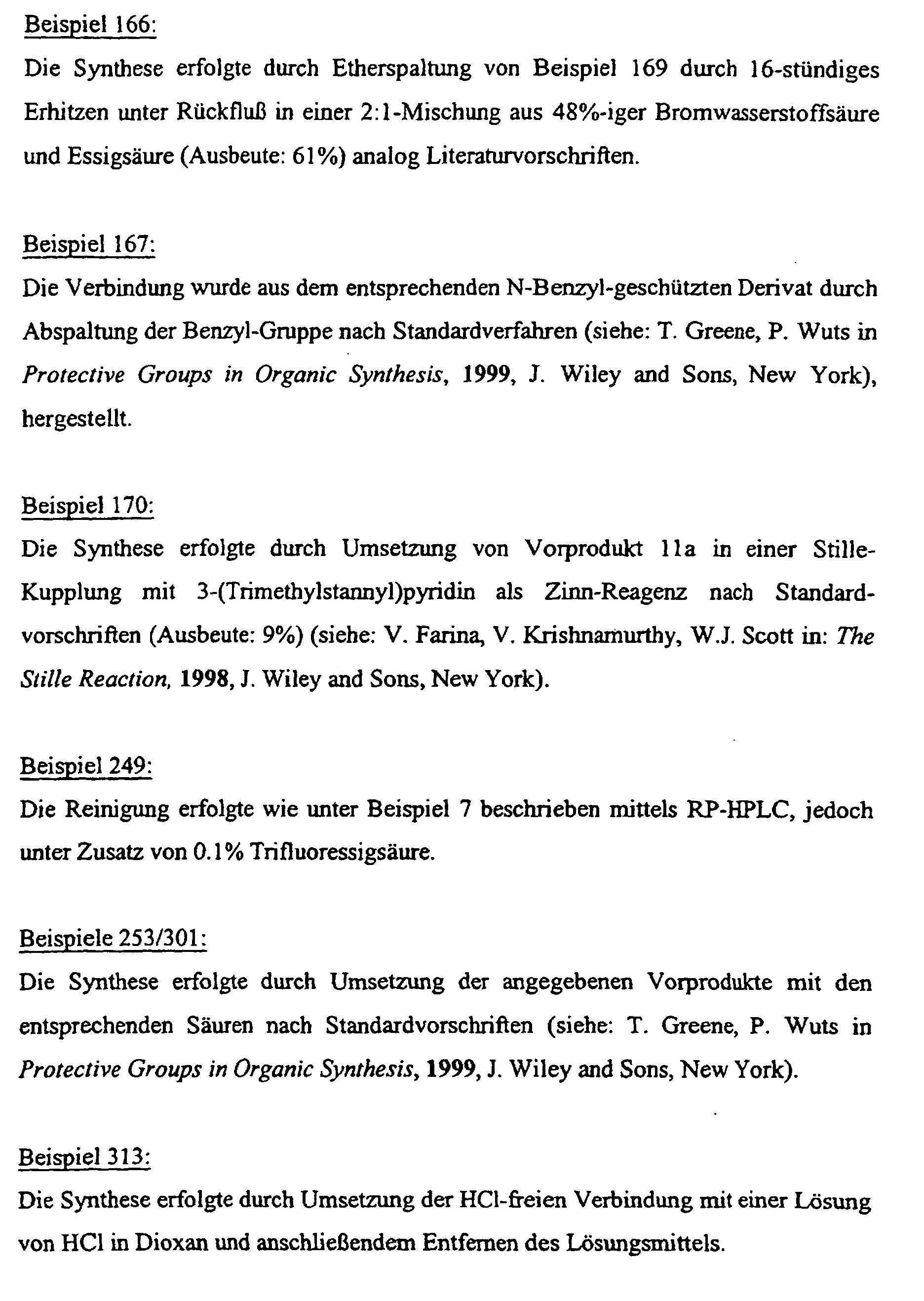

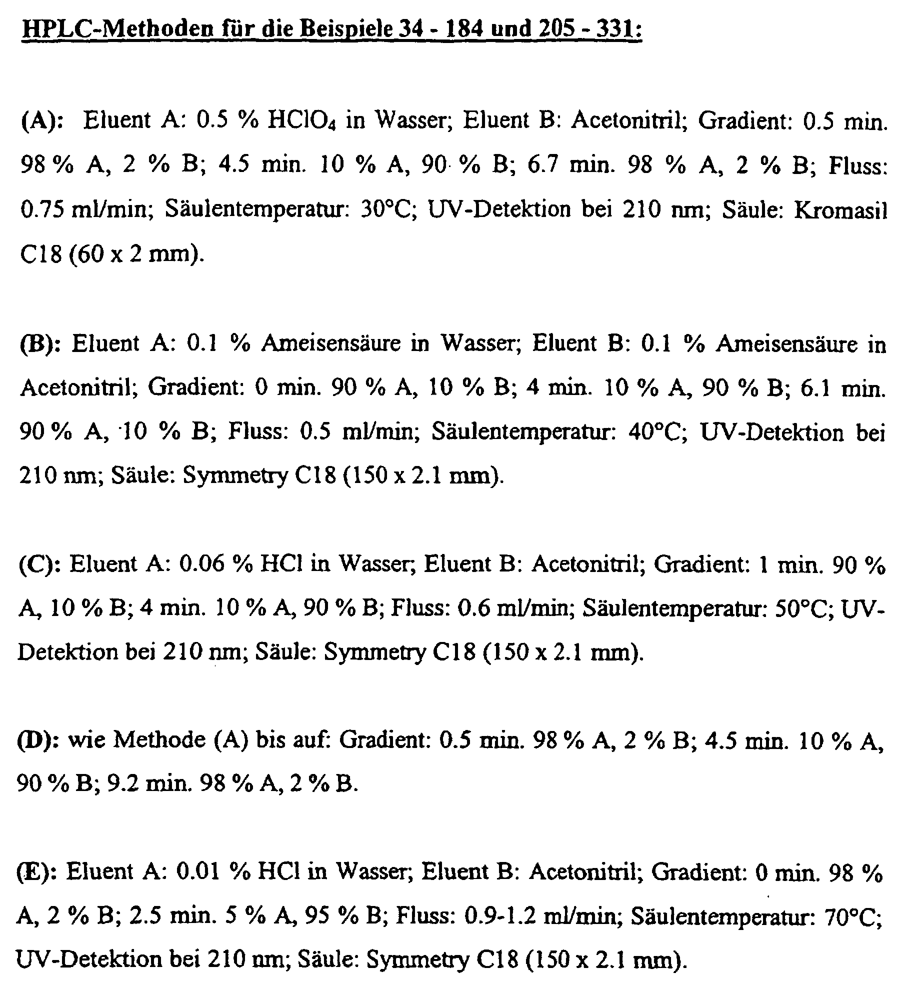

## Patentansprüche

1. Verbindungen der Formel (I) 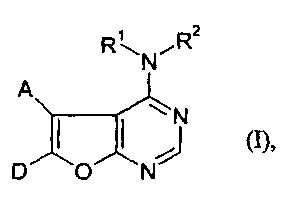 in welcher
A für Phenyl oder 5- oder 6-gliedriges Heteroaryl mit bis zu drei Heteroatomen aus der Reihe N, O und/oder S, die jeweils bis zu dreifach, unabhängig voneinander, durch Substituenten aus der Gruppe Halogen, Hydroxy, (C₁-C₆)-Alkoxy, Trifluormethyl, Trifluormethoxy, Amino, Carboxyl und (C₁-C₆)-Alkoxycarbonyl substituiert sein können, oder für eine Gruppe der Formel 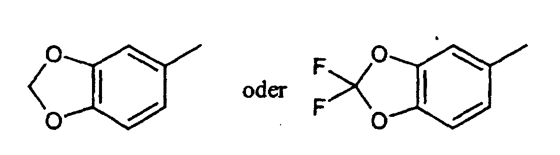 steht,
D für eine Gruppe der Formel
R³-E-G-
steht, worin
G Phenylen oder 5- oder 6-gliedriges Heteroarylen mit bis zu drei Heteroatomen aus der Reihe N, O und/oder S bedeutet, die jeweils bis zu zweifach, unabhängig voneinander, durch Substituenten aus der Gruppe Halogen, Trifluormethyl, Trifluormethoxy, (C₁-C₆)-Alkoxy, Amino, Nitro und Carboxyl substituiert sein können,
E für eine Bindung, eine Carbonylgruppe, eine Sulfonylgruppe oder für eine Gruppe der Formel *-C(O)-NR⁴- oder *-SO₂-NR⁴- steht,
worin * die Anknüpfungsstelle zur Gruppe R³ und R⁴ Wasserstoff oder (C₁-C₆)-Alkyl bedeutet,
und
R³ für Halogen, Trifluormethyl, Hydroxy, gegebenenfalls durch Hydroxy oder Amino substituiertes (C₁-C₆)-Alkoxy, Trifluormethoxy, Nitro, Carboxyl oder eine Gruppe der Formel H-C(O)-NR⁴-,
worin R⁴ die oben angegebene Bedeutung hat,
für (C₁-C₆)-Alkyl, das ein- bis zweifach, unabhängig voneinander, durch Substituenten ausgewählt aus der Gruppe Halogen, Trifluor-methyl, Hydroxy, (C₁-C₆)-Alkoxy, Amino, Mono- oder Di-(C₁-C₆)-alkylamino, (C₁-C₆)-Acylamino, (C₁-C₆)-Alkoxycarbonylamino, Amidino, Guanidino, Carboxyl, (C₁-C₆)-Alkoxycarbonyl, (C₆-C₁₀)-Aryl, (C₆-C₁₀)-Aryloxy und 5- bis 10-gliedriges Heteroaryl mit bis zu drei Heteroatomen aus der Reihe N, O und/oder S substituiert sein kann, wobei Aryl, Aryloxy und Heteroaryl ihrerseits jeweils ein- bis zweifach, unabhängig voneinander, durch Halogen, Hydroxy, Amino, (C₁-C₄)-Alkyl, (C₁-C₆)-Alkoxy, Cyano oder Nitro substituiert sein können,
für (C₃-C₇)-Cycloalkyl, das durch Phenyl oder bis zu vierfach durch (C₁-C₄)-Alkyl substituiert sein kann,
für (C₆-C₁₀)-Aryl, das ein- bis zweifach, unabhängig voneinander, durch Substituenten ausgewählt aus der Gruppe von Halogen, Trifluormethyl, (C₁-C₆)-Alkyl, Hydroxy, (C₁-C₆)-Alkoxy, (C₁-C₆)-Alkanoyl, Cyano, Nitro, Amino, Mono- und Di-(C₁-C₆)-alkylamino, (C₁-C₆)-Acylamino, Carboxyl, (C₁-C₆)-Alkoxycarbonyl und 5- bis 6-gliedriges Heteroaryl mit bis zu zwei Heteroatomen aus der Reihe N, O und/oder S substituiert sein kann,
für 4- bis 7-gliedriges, gesättigtes oder partiell ungesättigtes, über ein Ringkohlenstoffatom oder über ein Ringstickstoffatom gebundenes Heterocyclyl mit bis zu drei Heteroatomen aus der Reihe N, O und/oder S, das bis zu dreifach, unabhängig voneinander, durch (C₁-C₆)-Alkyl, welches seinerseits durch Hydroxy, (C₁-C₄)-Alkoxy oder Phenyl substituiert sein kann, (C₁-C₆)-Alkoxycarbonyl, (C₁-C₆)-Alkoxycarbonylamino, (C₁-C₆)-Alkanoyl, (C₁-C₆)-Alkylcarbonylamino, 1,2-Dioxyethylen, Carboxyl, Amino, Hydroxy, (C₁-C₆)-Alkoxy oder eine Oxo-Gruppe substituiert sein kann,
für 5- bis 10-gliedriges, über ein Ringkohlenstoffatom oder über ein Ringstickstoffatom gebundenes Heteroaryl mit bis zu drei Heteroatomen aus der Reihe N, O und/oder S, das seinerseits gegebenenfalls ein- bis zweifach, unabhängig voneinander, durch Substituenten ausgewählt aus der Gruppe von Halogen, Nitro, Amino, Hydroxy, (C₁-C₆)-Alkyl, (C₃-C₆)-Cycloalkyl, Phenyl, Benzyl und 5-gliedriges Heteroaryl mit bis zu zwei Heteroatomen aus der Reihe N, O und/oder S substituiert ist,
oder
für eine Gruppe der Formel -NR⁵R⁶ steht,
worin
R⁵ und R⁶ unabhängig voneinander für Wasserstoff, (C₁-C₆)-Alkyl, welches durch Hydroxy, Amino, (C₁-C₄)-Alkoxy, Mono- oder Di-(C₁-C₄)-alkylamino oder Phenyl substituiert sein kann, für (C₃-C₇)-Cycloalkyl, welches ein- bis zweifach, unabhängig voneinander, durch Hydroxy, Amino, (C₁-C₄)-Alkoxy, Mono- oder Di-(C₁-C₄)-alkylamino oder (C₁-C₄)-Alkyl substituiert sein kann, für (C₆-C₁₀)-Aryl, welches ein- bis zweifach, unabhängig voneinander, durch Hydroxy, Halogen, Amino, (C₁-C₄)-Alkoxy oder Nitro substituiert sein kann, oder für 5- bis 6-gliedriges Heterocyclyl oder 5-bis 6-gliedriges Heteroaryl mit jeweils bis zu zwei Heteroatomen aus der Reihe N, O und/oder S stehen,
oder
D für eine Gruppe der Formel 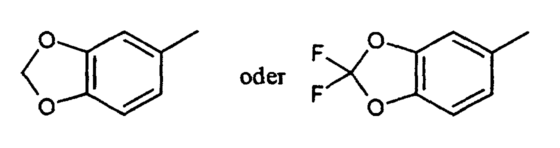 steht,
R¹ für Wasserstoff, (C₃-C₆)-Cycloalkyl oder für (C₁-C₆)-Alkyl steht, das ein- bis zweifach, unabhängig voneinander, durch Hydroxy, (C₁-C₆)-Alkoxy, Amino, Mono- oder Di-(C₁-C₆)-alkylamino substituiert sein kann,
und
R² für (C₁-C₆)-Alkyl, das ein- bis zweifach, unabhängig voneinander, durch Substituenten ausgewählt aus der Gruppe von Trifluormethyl, Hydroxy, (C₁-C₆)-Alkoxy, Amino, Mono- oder Di-(C₁-C₆)-alkylamino, Phenylamino, Carboxyl, (C₁-C₆)-Alkoxycarbonyl, (C₃-C₇)-Cycloalkyl, Phenyl, welches seinerseits gegebenenfalls ein- bis zwei-fach durch (C₁-C₄)-Alkoxy substituiert ist, 5- bis 6-gliedriges Heterocyclyl mit bis zu zwei Heteroatomen aus der Reihe N, O und/oder S und 5- bis 6-gliedriges Heteroaryl mit bis zu zwei Heteroatomen aus der Reihe N, O und/oder S, welches seinerseits gegebenenfalls durch (C₁-C₄)-Alkyl oder Hydroxy-(C₁-C₄)-alkyl substituiert ist, substituiert sein kann,
für (C₆-C₁₀)-Aryl, das durch Hydroxy, (C₁-C₆)-Alkoxy, Amino, Mono- oder Di-(C₁-C₆)-alkylamino substituiert sein kann,
für 5- bis 6-gliedriges Heteroaryl mit bis zu drei Heteroatomen aus der Reihe N, O und/oder S, das durch (C₁-C₆)-Alkyl, Trifluormethyl, Halogen, Hydroxy, (C₁-C₆)-Alkoxy, Trifluormethoxy, Amino, Mono- oder Di-(C₁-C₆)-alkyl-amino substituiert sein kann,
für 5- bis 6-gliedriges Heterocyclyl mit bis zu zwei Heteroatomen aus der Reihe N, O und/oder S, das durch Benzyl oder bis zu vierfach durch (C₁-C₄)-Alky) substituiert sein kann,
oder
für (C₄-C₈)-Cycloalkyl steht, das ein- bis zweifach, unabhängig voneinander, durch Hydroxy, Amino, (C₁-C₆)-Alkyl, (C₁-C₆)-Alkoxy, Mono- oder Di-(C₁-C₆)-alkyl-amino oder eine Gruppe der Formel R⁷-C(O)-NH- oder R⁷-SO₂-NH- substituiert sein kann,
worin
R⁷ für (C₁-C₆-Alkoxy, Phenyl, Benzyl, Phenylamino, Mono- oder Di-(C₁-C₆)-alkylamino, welche ihrerseits gegebenenfalls in der Alkyl-gruppe durch (C₁-C₄)-Alkoxycarbonyl oder Carboxyl substituiert sind, oder für (C₄-C₇)-Cycloalkylamino, welches seinerseits gegebenenfalls in der Cycloalkylgruppe durch (C₁-C₄)-Alkyl substituiert ist,
für (C₁-C₆)-Alkyl, das durch Hydroxy, (C₁-C₆)-Alkoxy, Amino, Mono- oder Di-(C₁-C₆)-alkylamino, (C₁-C₆)-Acylamino oder durch 5- oder 6-gliedriges Heterocyclyl mit bis zu zwei Heteroatomen aus der Reihe N, O und/oder S substituiert sein kann,
für 5- oder 6-gliedriges, über ein Ringkohlenstoffatom oder über ein Ringstickstoffatom gebundenes Heterocyclyl mit bis zu zwei Heteroatomen aus der Reihe N, O und/oder S, das gegebenenfalls durch eine Oxo-Gruppe substituiert ist,
oder
für 5- oder 6-gliedriges Heteroaryl mit bis zu drei Heteroatomen aus der Reihe N, O und/oder S, das gegebenenfalls einbis zweifach durch (C₁-C₄)-Alkyl substituiert ist, steht,
oder
R¹ und R² zusammen mit dem Stickstoffatom, an das sie gebunden sind, einen 4- bis 11-gliedrigen, mono-, bi- oder spiro-cyclischen Heterocyclus bilden, der bis zu zwei weitere Heteroatome aus der Reihe N, O und/oder S enthalten und ein- bis vierfach, unabhängig voneinander, durch Substituenten ausgewählt aus der Gruppe Amino, Mono- oder Di-(C₁-C₆)-alkylamino, Hydroxy, (C₁-C₆)-Alkoxy, Oxo, Carboxyl, Carbamoyl, (C₁-C₆)-Alkoxycarbonyl, (C₁-C₆)-Alkoxycarbonylamino, (C₁-C₆)-Alkanoyl, (C₁-C₆)-Alkylcarbonylamino, (C₁-C₆)-Alkyl, welches seinerseits gegebenenfalls durch Hydroxy, (C₁-C₆)-Alkoxy, Amino, Mono- oder Di-(C₁-C₆)-alkylamino, Phenyl oder durch 5-oder 6-gliedriges Heterocyclyl mit bis zu zwei Heteroatomen aus der Reihe N, O und/oder S substituiert ist, Phenyl, welches seinerseits gegebenenfalls durch Halogen substituiert ist, (C₃-C₇)-Cycloalkyl, Pyridyl, Thienyl und 5- oder 6-gliedriges Heterocyclyl mit bis zu zwei Heteroatomen aus der Reihe N, O und/oder S substituiert sein kann,
sowie deren pharmazeutisch verträgliche Salze, Solvate, Hydrate und Hydrate der Salze.

2. Verbindungen nach Anspruch 1 der Formel (Ia) 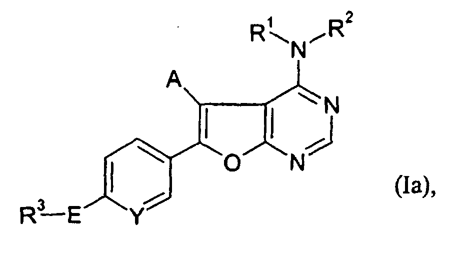 in welcher
A für Phenyl steht, das durch Fluor, Chlor, Brom oder Methoxy substituiert sein kann,
Y für CH oder N steht,
E für eine Bindung, für eine Carbonylgruppe oder für eine Gruppe der Formel *-C(O)-NH- steht,
worin * die Anknüpfungsstelle zur Gruppe R³ bedeutet,
R³ für Hydroxy, Methoxy, Amino oder Mono-(C₁-C₄)-alkylamino, das in der Alkylgruppe durch Hydroxy oder Amino substituiert sein kann,
für (C₁-C₄)-Alkyl, das gegebenenfalls durch Hydroxy, Methoxy, Ethoxy, Amino, Mono- oder Dimethylamino, (C₁-C₄)-Alkoxycarbonylamino, Acetamido, Carboxyl oder (C₁-C₄)-Alkoxycarbonyl substituiert ist,
oder
für einen 4- bis 6-gliedrigen gesättigten, über ein Ringkohlenstoffatom oder über ein Ringstickstoffatom gebundenen Heterocyclus mit bis zu zwei Heteroatomen aus der Reihe N und/oder O steht, der bis zu zweifach, unabhängig voneinander, durch (C₁-C₄)-Alkyl, welches seinerseits durch Hydroxy, Methoxy oder Ethoxy substituiert sein kann, durch Carboxyl, Amino, Hydroxy, Methoxy, Ethoxy oder eine Oxo-Gruppe substituiert sein kann,
R¹ für Wasserstoff, Methyl oder für Ethyl, welches durch Hydroxy oder Amino substituiert sein kann, steht,
und
R² für (C₁-C₄)-Alkyl, das ein- oder zweifach, unabhängig voneinander, durch Hydroxy, Amino, (C₁-C₄)-Alkoxy, Mono- oder Di-(C₁-C₄)-alkylamino substituiert sein kann, oder für (C₅-C₇)-Cycloalkyl steht,
oder
R¹ und R² zusammen mit dem Stickstoffatom, an das sie gebunden sind, einen Pyrrolidin-, Piperidin-, Piperazin- oder Morpholinring bilden, der ein- oder zweifach, unabhängig voneinander, durch (C₁-C₄)-Alkyl, welches seinerseits gegebenenfalls durch Hydroxy oder Amino substituiert ist, durch Amino, Mono- oder Di-(C₁-C₄)-alkylamino, Hydroxy, (C₁-C₄)-Alkoxy, Oxo, Carboxyl, Carbamoyl oder durch (C₁-C₄)-Alkoxycarbonyl substituiert sein kann.
sowie deren pharmazeutisch verträgliche Salze, Solvate, Hydrate und Hydrate der Salze.

3. Verbindungen nach Anspruch 1 der Formel (Ia),
in welcher
A für Phenyl steht, das durch Fluor substituiert sein kann,
Y für CH oder N steht,
E für eine Bindung oder für eine Gruppe der Formel *-C(O)-NH- steht,
worin * die Anknüpfungsstelle zur Gruppe R³ bedeutet,
R³ für Amino oder Mono-(C₁-C₄)-alkylamino, das in der Alkylgruppe durch Hydroxy oder Amino substituiert sein kann,
für (C₁-C₄)-Alkyl, das gegebenenfalls durch Hydroxy, Amino, Mono- oder Dimethylamino substituiert ist,
oder
für Pyrrolidin, Piperazin oder Piperidin steht, die bis zu zweifach, unabhängig voneinander, durch Methyl, Ethyl, n-Propyl, iso-Propyl, n-Butyl, sec-Butyl, iso-Butyl oder tert-Butyl, welche ihrerseits durch Hydroxy substituiert sein können, durch Amino oder Hydroxy substituiert sein können,
R¹ und R² zusammen mit dem Stickstoffatom, an das sie gebunden sind, einen Piperidin-, Piperazin- oder Morpholinring bilden, der ein- oder zweifach, unabhängig voneinander, durch Methyl, Ethyl, n-Propyl oder iso-Propyl, welche ihrerseits gegebenenfalls durch Hydroxy oder Amino substituiert sind, durch Amino, Hydroxy oder Oxo substituiert sein können,
sowie deren pharmazeutisch verträgliche Salze, Solvate, Hydrate und Hydrate der Salze.

4. Verfahren zur Herstellung der Verbindungen der Formel (I), wie in Anspruch 1 definiert, **dadurch gekennzeichnet, dass** man
Verbindungen der Formel (II) 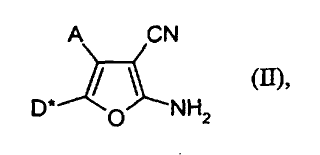 worin D* für D oder für einen unsubstituierten Phenylring steht und A und D die in Anspruch 1 angegebene Bedeutung haben,
entweder
[A] zunächst mit Ameisensäure in Acetanhydrid zu Verbindungen der Formel (III) 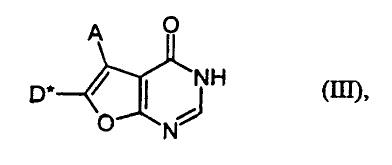 worin D* für D oder für einen unsubstituierten Phenylring steht und A und D die in Anspruch 1 angegebene Bedeutung haben,
umsetzt, dann mit Phosphorylchlorid in Verbindungen der Formel (IV) 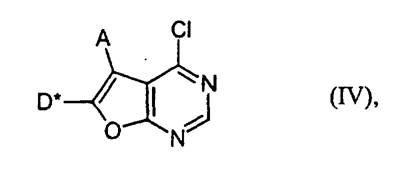 worin D* für D oder für einen unsubstituierten Phenylring steht und A und D die in Anspruch 1 angegebene Bedeutung haben,
überführt, anschließend für den Fall, dass D* für einen unsubstituierten Phenylring steht, diesen Phenylring nitriert, und schließlich mit Verbindungen der Formel (V) 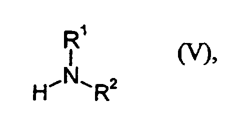 worin R¹ und R² die in Anspruch 1 angegebene Bedeutung haben,
zu Verbindungen der Formel (I) umsetzt
oder
[B] zunächst mit Orthoameisensäuretriethylester in Verbindungen der Formel (VI) 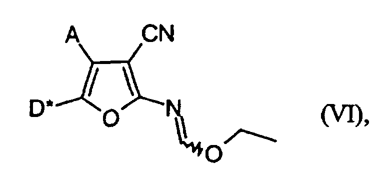 worin D* für D oder für einen unsubstituierten Phenylring steht und A und D die in Anspruch 1 angegebene Bedeutung haben, überführt und dann mit Verbindungen der Formel (VII) 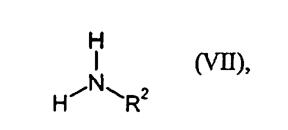 worin R² die in Anspruch 1 angegebene Bedeutung hat,
und anschließend mit einer Base direkt oder für den Fall, dass D* für einen unsubstituierten Phenylring steht, durch anschließende Nitrierung dieses Phenylringes, zu Verbindungen der Formel (I) umsetzt,
wobei sich bei den so erhaltenen Verbindungen der Formel (I) gegebenenfalls weitere Derivatisierungen, die nach üblichen Methoden durchgeführt werden können, anschließen können.

5. Verbindungen der Formel (I), wie in Anspruch 1 definiert, zur Prophylaxe und/oder Behandlung von Erkrankungen.

6. Arzneimittel, enthaltend mindestens eine Verbindung der Formel (I), wie in Anspruch 1 definiert, und mindestens einen weiteren Hilfsstoff.

7. Arzneimittel, enthaltend mindestens eine Verbindung der Formel (I), wie in Anspruch 1 definiert, und mindestens einen weiteren Wirkstoff.

8. Verwendung von Verbindungen der Formel (I), wie in Anspruch 1 definiert, zur Herstellung von Arzneimitteln zur Prophylaxe und/oder Behandlung von ischämiebedingten periphären und kardiovaskulären Erkrankungen.

9. Verwendung von Verbindungen der Formel (I), wie in Anspruch 1 definiert, zur Herstellung von Arzneimitteln zur akuten und chronischen Behandlung von ischämischen Erkrankungen des Herz-Kreislauf-Systems, wie z.B. der koronaren Herzkrankheit, der stabilen und instabilen Angina pectoris, von peripheren und arteriellen Verschlusskrankheiten, von thrombotischen Gefäßverschlüssen, des Myocardinfarkts und von Reperfusionsschäden.

10. Verwendung von Verbindungen der Formel (I), wie in Anspruch 1 definiert, zur Herstellung von Arzneimitteln zur Behandlung von akuten und/oder chronischen Schmerzen sowie neurodegenerativen Erkrankungen.

## Claims

1. Compounds of the formula (I) 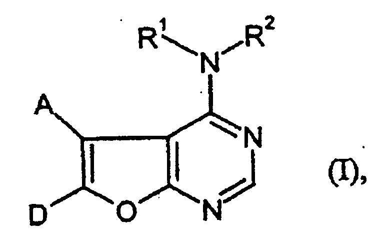 in which
A represents phenyl or 5- or 6-membered heteroaryl having up to three heteroatoms from the group consisting of N, O and/or S, each of which radicals may be substituted up to three times, independently of one another, by substituents from the group consisting of halogen, hydroxyl, (C₁-C₆)-alkoxy, trifluoromethyl, trifluoromethoxy, amino, carboxyl and (C₁-C₆)-alkoxycarbonyl, or represents a group of the formula 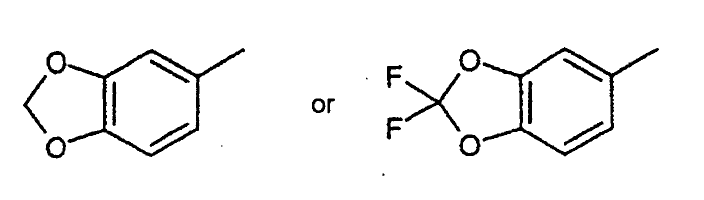
D represents a group of the formula
R³-E-G
in which
G represents phenylene or 5- or 6-membered heteroarylene having up to three heteroatoms from the group consisting of N, O and/or S, each of which radicals may be substituted up to two times, independently of one another, by substituents from the group consisting of halogen, trifluoromethyl, trifluoromethoxy, (C₁-C₆)-alkoxy, amino, nitro and carboxyl,
E represents a bond, a carbonyl group, a sulfonyl group or represents a group of the formula *-C(O)-NR⁴- or *-SO₂-NR⁴-,
in which * denotes the point of attachment to the group R³ and R⁴ represents hydrogen or (C₁-C₆)-alkyl,
and
R³ represents halogen, trifluoromethyl, hydroxyl, optionally hydroxyl- or amino-substituted (C₁-C₆)-alkoxy, trifluoromethoxy, nitro, carboxyl or a group of the formula H-C(O)-NR⁴-,
in which R⁴ is as defined above,
represents (C₁-C₆)-alkyl which may be mono- to disubstituted, independently of one another, by substituents selected from the group consisting of halogen, trifluoromethyl, hydroxyl (C₁-C₆)-alkoxy, amino, mono- or di-(C₁-C₆)-alkylamino, (C₁-C₆)-acylamino, (C₁-C₆)-alkoxycarbonylamino, amidino, guanidino, carboxyl, (C₁-C₆)-alkoxycarbonyl, (C₆-C₁₀)-aryl, (C₆-C₁₀)-aryloxy and 5- to 10-membered heteroaryl having up to three heteroatoms from the group consisting of N, O and/or S, where aryl, aryloxy and heteroaryl for their part may in each case be mono- to disubstituted, independently of one another, by halogen, hydroxyl, amino, (C₁-C₄)-alkyl, (C₁-C₆)-alkoxy, cyano or nitro,
represents (C₃-C₇)-cycloalkyl which may be substituted by phenyl or up to four times by (C₁-C₄)-alkyl,
represents (C₆-C₁₀)-aryl, which may be mono- to disubstituted, independently of one another, by substituents selected from the group consisting of halogen, trifluoromethyl, (C₁-C₆)-alkyl, hydroxyl, (C₁-C₆)-alkoxy, (C₁-C₆)-alkanoyl, cyano, nitro, amino, mono- and di-(C₁-C₆)-alkylamino, (C₁-C₆)-acylamino, carboxyl, (C₁-C₆)-alkoxycarbonyl and 5- to 6-membered heteroaryl having up to two heteroatoms from the group consisting of N, O and/or S,
represents 4- to 7-membered, saturated or partially unsaturated heterocyclyl which is attached via a ring carbon atom or via a ring nitrogen atom and has up to three heteroatoms from the group consisting of N, O and/or S, which may be substituted up to three times, independently of one another, by (C₁-C₆)-alkyl, which for its part may be substituted by hydroxyl, (C₁-C₄)-alkoxy or phenyl, (C₁-C₆)-alkoxycarbonyl, (C₁-C₆)-alkoxycarbonylamino, (C₁-C₆)-alkanoyl, (C₁-C₆)-alkylcarbonylamino, 1,2-dioxyethylene, carboxyl, amino, hydroxyl, (C₁-C₆)-alkoxy or an oxo group,
represents 5- to 10-membered heteroaryl which is attached via a ring carbon atom or via a ring nitrogen atom and has up to three heteroatoms from the group consisting of N, O and/or S, which for its part may optionally be mono- to disubstituted, independently of one another, by substituents selected from the group consisting of halogen, nitro, amino, hydroxyl (C₁-C₆)-alkyl, (C₃-C₆)-cycloalkyl, phenyl, benzyl and 5-membered heteroaryl having up to two heteroatoms from the group consisting of N, O and/or S,
or
represents a group of the formula -NR⁵R⁶,
in which
R⁵ and R⁶ independently of one another represent hydrogen, (C₁-C₆)-alkyl, which may be substituted by hydroxyl, amino, (C₁-C₄)-alkoxy, mono- or di-(C₁-C₄)-alkylamino or phenyl, represent (C₃-C₇)-cycloalkyl, which may be mono- to disubstituted, independently of one another, by hydroxyl, amino, (C₁-C₄)-alkoxy, mono- or di-(C₁-C₄)-alkylamino or (C₁-C₄)-alkyl, represent (C₆-C₁₀)-aryl, which may be mono- to disubstituted, independently of one another, by hydroxyl, halogen, amino, (C₁-C₄)-alkoxy or nitro, or represent 5- to 6-membered heterocyclyl or 5- to 6-membered heteroaryl having in each case up to two heteroatoms from the group consisting ofN, O and/or S,
or
D represents a group of the formula 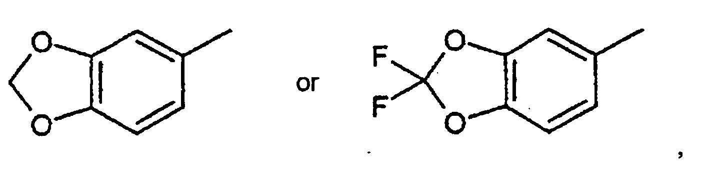
R¹ represents hydrogen, (C₃-C₆)-cycloalkyl or represents (C₁-C₆)-alkyl which may be mono- to disubstituted, independently of one another, by hydroxyl (C₁-C₆)-alkoxy, amino, mono- or di-(C₁-C₆)-alkylamino,
and
R² represents (C₁-C₆)-alkyl, which may be mono- to disubstituted, independently of one another, by substituents selected from the group consisting of trifluoromethyl, hydroxyl, (C₁-C₆)-alkoxy, amino, mono- or di-(C₁-C₆)-alkylamino, phenylamino, carboxyl, (C₁-C₆)-alkoxycarbonyl, (C₃-C₇)-cycloalkyl, phenyl, which for its part is optionally mono- to disubstituted by (C₁-C₄)-alkoxy, 5- to 6-membered heterocyclyl having up to two heteroatoms from the group consisting of N, O and/or S and 5- to 6-membered heteroaryl having up to two heteroatoms from the group consisting of N, O and/or S, which for its part is optionally substituted by (C₁-C₄)-alkyl or hydroxy-(C₁-C₄)-alkyl,
represents (C₆-C₁₀)-aryl which may be substituted by hydroxyl (C₁-C₆)-alkoxy, amino, mono or di-(C₁-C₆)-alkylamino,
represents 5- to 6-membered heteroaryl having up to three heteroatoms from the group consisting ofN, O and/or S which may be substituted by (C₁-C₆)-alkyl, trifluoromethyl, halogen, hydroxyl, (C₁-C₆)-alkoxy, trifluoromethoxy, amino, mono- or di-(C₁-C₆)-alkylamino,
represents 5- to 6-membered heterocyclyl having up to two heteroatoms from the group consisting of N, O and/or S which may be substituted by benzyl or up to four times by (C₁-C₄)-alkyl,
or
represents (C₄-C₈)-cycloalkyl which may be mono- to disubstituted, independently of one another, by hydroxyl amino, (C₁-C₆)-alkyl, (C₁-C₆)-alkoxy, mono- or di-(C₁-C₆)-alkylamino or a group of the formula R⁷-C(O)-NH- or R⁷-SO₂-NH-,
in which
R⁷ represents (C₁-C₆)-alkoxy, phenyl, benzyl, phenylamino, mono- or di-(C₁-C₆)-alkylamino, which for their part are optionally substituted in the alkyl group by (C₁-C₄)-alkoxycarbonyl or carboxyl, or represents (C₄-C₇)-cycloalkylamino which for its part is optionally substituted in the cycloalkyl group by (C₁-C₄)-alkyl,
represents (C₁-C₆)-alkyl which may be substituted by hydroxyl, (C₁-C₆)-alkoxy, amino, mono or di-(C₁-C₆)-alkylamino, (C₁-C₆)-acylamino or by 5- or 6-membered heterocyclyl having up to two heteroatoms from the group consisting of N, O and/or S,
represents 5- or 6-membered heterocyclyl which is attached via a ring carbon atom or via a ring nitrogen atom and has up to two heteroatoms from the group consisting of N, O and/or S, which is optionally substituted by an oxo group,
or
represents 5- or 6-membered heteroaryl having up to three heteroatoms from the group consisting of N, O and/or S, which is optionally mono- to disubstituted by (C₁-C₄)-alkyl,
or
R¹ and R² together with the nitrogen atom to which they are attached form a 4- to 11-membered mono-, bi- or spire-cyclic heterocycle which may contain up to two further heteroatoms from the group consisting of N, O and/or S and which may be mono- to tetrasubstituted, independently of one another, by substituents selected from the group consisting of amino, mono- or di-(C₁-C₆)-alkylamino, hydroxyl (C₁-C₆)-alkoxy, oxo, carboxyl, carbamoyl, (C₁-C₆)-alkoxycarbonyl, (C₁-C₆)-allcoxycarbonylamino, (C₁-C₆)-alkanoyl, (C₁-C₆)-alkylcarbonylamino, (C₁-C₆)-alkyl, which for its part is optionally substituted by hydroxyl, (C₁-C₆)-alkoxy, amino, mono or di-(C₁-C₆)-alkylamino, phenyl or by 5- or 6-membered heterocyclyl having up to two heteroatoms from the group consisting of N, O and/or S, phenyl which for its part is optionally substituted by halogen, (C₃-C₇)-cycloalkyl, pyridyl, thienyl and 5- or 6-membered heterocyclyl having up to two heteroatoms from the group consisting ofN, O and/or S,
and its pharmaceutically acceptable salts, solvates, hydrates and hydrates of the salts.

2. Compounds according to Claim 1 of the formula (Ia) 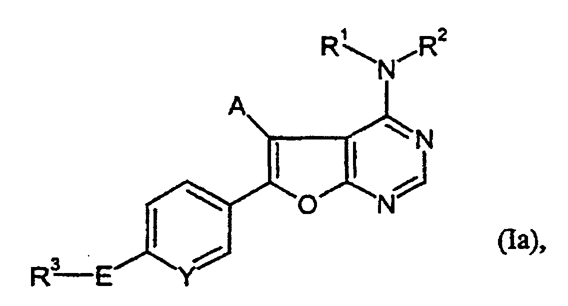 in which
A represents phenyl which may be substituted by fluorine, chlorine, bromine or methoxy,
Y represents CH or N,
E represents a bond, represents a carbonyl group or represents a group of the formula *-C(O)-NH-,
in which * denotes the point of attachment to the group R³,
R³ represents hydroxyl, methoxy, amino or mono-(C₁-C₄)-alkylamino, which may be substituted in the alkyl group by hydroxyl or amino,
represents (C₁-C₄)-alkyl which is optionally substituted by hydroxyl methoxy, ethoxy, amino, mono or dimethylamino, (C₁-C₄)-alkoxycarbonylamino, acetamido, carboxyl or (C₁-C₄)-alkoxycarbonyl,
or
represents a 4- to 6-membered, saturated heterocycle which is attached via a ring carbon atom or via a ring nitrogen atom and has up to two heteroatoms from the group consisting of N and/or O, which may be substituted up to two times, independently of one another, by (C₁-C₄)-alkyl, which for its part may be substituted by hydroxyl methoxy or ethoxy, by carboxyl, amino, hydroxyl methoxy, ethoxy or an oxo group,
R¹ represents hydrogen, methyl or represents ethyl which may be substituted by hydroxyl or amino,
and
R² represents (C₁-C₄)-alkyl which may be mono- to disubstituted, independently of one another, by hydroxyl, amino, (C₁-C₄)-alkoxy, mono- or di-(C₁-C₄)-alkylamino, or represents (C₅-C₇)-cycloalkyl
or
R¹ and R² together with the nitrogen atom to which they are attached form a pyrrolidine, piperidine, piperazine, or morpholine ring which may be mono- or disubstituted, independently of one another, by (C₁-C₄)-alkyl, which for its part is optionally substituted by hydroxyl or amino, by amino, mono or di-(C₁-C₄)-alkylamino, hydroxyl, (C₁-C₄)-alkoxy, oxo, carboxyl, carbamoyl or by (C₁-C₄)-alkylcarbonyl.
and its pharmaceutically acceptable salts, solvates, hydrates and hydrates of the salts.

3. Compounds according to Claim 1 of the formula (Ia)
in which
A represents phenyl which may be substituted by fluorine,
Y represents CH or N,
E represents a bond or represents a group of the formula *-C(O)-NH-,
in which * denotes the point of attachment to the group R³,
R³ represents amino or mono-(C₁-C₄)-alkylamino which may be substituted in the alkyl group by hydroxyl or amino,
represents (C₁-C₄)-alkyl which is optionally substituted by hydroxyl, amino, mono- or dimethylamino,
or
represents pyrrolidine, piperazine or piperidine which may be substituted up to two times, independently of one another, by methyl, ethyl, n-propyl, isopropyl, n-butyl, sec-butyl, isobutyl or tert-butyl, which for their part may be substituted by hydroxyl, by amino or hydroxyl,
R¹ and R² together with the nitrogen atom to which they are attached form a piperidine, piperazine or morpholine ring which may be mono- or disubstituted, independently of one another, by methyl, ethyl, n-propyl or isopropyl, which for their part are optionally substituted by hydroxyl or amino, by amino, hydroxyl or oxo,
and its pharmaceutically acceptable salts, solvates, hydrates and hydrates of the salts.

4. Process for preparing the compounds of the formula (I), as defined in Claim 1, **characterized in that**
compounds of the formula (II) 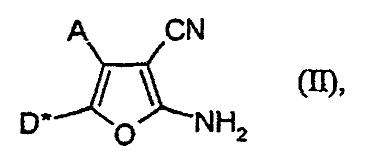 in which D* represents D or an unsubstituted phenyl ring and A and D are as defined in Claim 1
are either
[A] initially reacted with formic acid in acetic anhydride to give compounds of the formula (III) 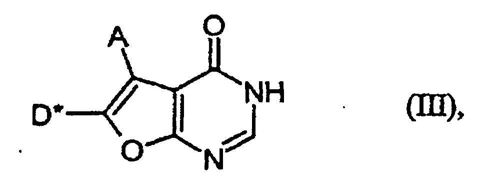 in which D* represents D or represents an unsubstituted phenyl ring and A and D are as defined in Claim 1,
then converted with phosphoryl chloride into compounds of the formula (IV) 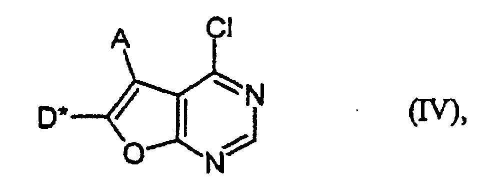 in which D* represents D or represents an unsubstituted phenyl ring and A and D are as defined in Claim 1,
if D* represents an unsubstituted phenyl ring, this phenyl ring is then nitrated, and are finally reacted with compounds of the formula (V) 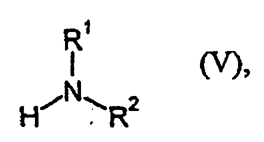 in which R¹ and R² are as defined in Claim 1,
to compounds of the formula (I)
or
[B] initially converted with triethyl orthoformate into compounds of the formula (VI) 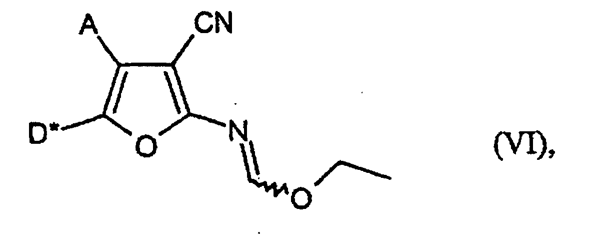 in which D* represents D or represents an unsubstituted phenyl ring and A and D are as defined in Claim 1,
and then reacted with compounds of the formula (VII) 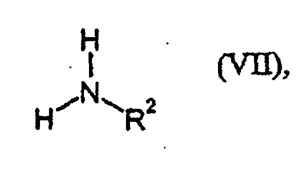 in which R² is as defined in Claim 1,
and then reacted directly or, if D* represents an unsubstituted phenyl ring, with a base by subsequent nitration of this phenyl ring, to give compounds of the formula (I),
where the resulting compounds of the formula (1) can, if appropriate, subsequently be subjected to further derivatizations which can be carried out by customary methods.

5. Compounds of the formula (I) as defined in Claim 1 for the prophylaxis and/or treatment of disorders.

6. Pharmaceuticals, comprising at least one compound of the formula (I) as defined in Claim 1 and at least one further auxiliary.

7. Pharmaceuticals, comprising at least one compound of the formula (I) as defined in Claim 1 and at least one further active compound.

8. Use of compounds of the formula (I) as defined in Claim 1 for preparing pharmaceuticals for the prophylaxis and/or treatment of ischemia-related peripheral and cardiovascular disorders.

9. Use of compounds of the formula (I) as defined in Claim 1 for preparing pharmaceuticals for the acute and chronic treatment of ischemic disorders of the cardiovascular system, such as, for example, coronary heart disease, of stable and unstable angina pectoris, of peripheral and arterial occlusion diseases, of thrombotic vascular occlusions, of myocardial infarction and of reperfusion damage.

10. Use of compounds of the formula (I) as defined in Claim 1 for preparing pharmaceuticals for treating acute and/or chronic pain and neurodegenerative disorders.

## Revendications

1. Composés de formule (I) 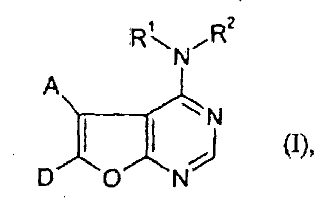 dans laquelle
A est un reste phényle ou un reste hétéroaryle pentagonal ou hexagonal ayant jusqu'à trois hétéroatomes de la série N, O et/ou S, qui peuvent être substitués chacun jusqu'à trois fois indépendamment par des substituants du groupe halogéno, hydroxy, alkoxy en C₁ à C₆, trifluorométhyle, trifluorométhoxy, amino, carboxyle et (alkoxy en C₁ à C₆)-carbonyle,
ou un groupe de formule 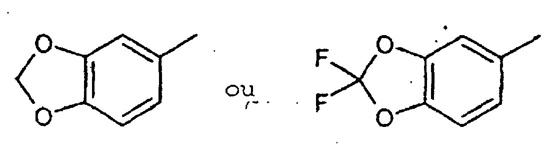
D représente un groupe de formule
R³-E-G-
dans laquelle
G est un reste phénylène ou un reste hétéroarylène pentagonal ou hexagonal ayant jusqu'à trois hétéroatomes de la série N, O et/ou S, qui peuvent être substitués chacun jusqu'à deux fois indépendamment par des substituants du groupe halogène, trifluorométhyle, trifluorométhoxy, alkoxy en C₁ à C₆, amino, nitro et carboxyle,
E représente une liaison, un groupe carbonyle, un groupe sulfonyle ou un groupe de formule *-C(O)-NR⁴ - ou *-SO₂-NR⁴-,
dans laquelle le signe * désigne la position de liaison au groupe R³, et R⁴ représente l'hydrogène ou un reste alkyle en C₁ à C₆,
et
R³ est un halogène, un reste trifluorométhyle, un groupe hydroxy, un reste alkoxy en C₁ à C₆ éventuellement substitué par un radical hydroxy ou amino, un reste trifluorométhoxy, un groupe nitro, carboxyle, ou un groupe de formule H-C(O)-NR⁴ -,
dans laquelle R⁴ a la définition indiquée ci-dessus,
un reste alkyle en C₁ à C₆, qui peut être substitué une à deux fois indépendamment par des substituants choisis dans le groupe halogène, trifluorométhyle, hydroxy, alkoxy en C₁ à C₆, amino, mono- ou di-(alkyle en C₁ à C₆)-amino, acylamino en C₁ à C₆, (alkoxy en C₁ à C₆)-carbonylamino, amidino, guanidino, carboxyle, (alkoxy en C₁ à C₆) -carbonyle, aryle en C₆ à C₁₀, aryloxy en C₆ à C₁₀ et hétéroaryle pentagonal à décagonal ayant jusqu'à trois hétéroatomes de la série N, O et/ou S, les restes aryle, aryloxy et hétéroaryle pouvant eux-mêmes être substitués chacun une ou deux fois indépendamment par un radical halogène, hydroxy, amino, alkyle en C₁ à C₄, alkoxy en C₁ à C₆, cyano ou nitro,
un reste cycloalkyle en C₃ à C₇, qui peut être substitué par un radical phényle, ou une à quatre fois par un radical alkyle en C₁ à C₄,
un reste aryle en C₆ à C₁₀ qui peut être substitué une ou deux fois indépendamment par des substituants choisis dans le groupe halogéno, trifluorométhyle, alkyle en C₁ à C₆, hydroxy, alkoxy en C₁ à C₆, alcanoyle en C₁ à C₆, cyano, nitro, amino, mono- et di-(alkyle en C₁ à C₆)-amino, acylamino en C₁ à C₆, carboxyle, (alkoxy en C₁ à C₆)-carbonyle et hétéroaryle pentagonal ou hexagonal ayant jusqu'à deux hétéroatomes de la série N, O et/ou S,
un reste hétérocyclyle tétragonal à heptagonal saturé ou partiellement insaturé ayant jusqu'à trois hétéroatomes de la série N, O et/ou S, lié par l'intermédiaire d'un atome de carbone du noyau ou par l'intermédiaire d'un atome d'azote du noyau, qui peut être substitué jusqu'à trois fois indépendamment par un radical alkyle en C₁ à C₆ qui peut lui-même être substitué par un radical hydroxy, alkoxy en C₁ à C₄ ou phényle, un radical (alkoxy en C₁ à C₆) -carbonyle, (alkoxy en C₁ à C₆) -carbonylamino, alcanoyle en C₁ à C₆, (alkyle en C₁ à C₆) -carbonylamino, 1,2-dioxyéthylène, carboxyle, amino, hydroxy, alkoxy en C₁ à C₆ ou un groupe oxo,
un reste hétéroaryle pentagonal à décagonal ayant jusqu'à trois hétéroatomes de la série N, O et/ou S, lié par l'intermédiaire d'un atome de carbone du noyau ou par l'intermédiaire de l'atome d'azote du noyau, qui peut lui-même éventuellement être substitué une ou deux fois indépendamment par des substituants choisis dans le groupe halogène, nitro, amino, hydroxy, alkyle en C₁ à C₆, cycloalkyle en C₃ à C₆, phényle, benzyle et hétéroaryle pentagonal ayant jusqu'à deux hétéroatomes de la série N, O et/ou S,
ou bien
un groupe de formule -NR⁵R⁶,
dans laquelle
R⁵ et R⁶ représentent, indépendamment l'un de l'autre, l'hydrogène, un reste alkyle en C₁ à C₆ qui peut être substitué par un radical hydroxy, amino, alkoxy en C₁ à C₄, mono- ou di-(alkyle en C₁ à C₄)-amino ou phényle, un reste cycloalkyle en C₃ à C₇ qui peut être substitué une ou deux fois indépendamment par un radical hydroxy, amino, alkoxy en C₁ à C₄, mono- ou di-(alkyle en C₁ à C₄)-amino ou alkyle en C₁ à C₄, un reste aryle en C₆ à C₁₀ qui peut être substitué une ou deux fois indépendamment par un radical hydroxy, halogéno, amino, alkoxy en C₁ à C₄ ou nitro, ou un reste hétérocyclyle pentagonal ou hexagonal ou un reste hétéroaryle pentagonal ou hexagonal ayant chacun jusqu'à deux hétéroatomes de la série N, O et/ou S,
ou bien
D représente un groupe de formule 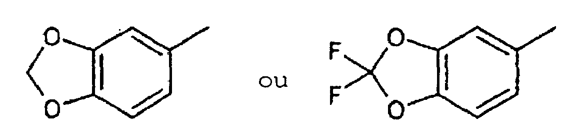
R¹ représente l'hydrogène, un reste cycloalkyle en C₃ à C₆ ou un reste alkyle en C₁ à C₆ qui peut être substitué une ou deux fois indépendamment par un radical hydroxy, alkoxy en C₁ à C₆, amino, mono- ou di-(alkyle en C₁ à C₆) -amino,
et
R² représente un reste alkyle en C₁ à C₆, qui peut être substitué une ou deux fois indépendamment par des substituants choisis dans le groupe trifluorométhyle, hydroxy, alkoxy en C₁ à C₆, amino, mono- ou di-(alkyle en C₁ à C₆)-amino, phénylamino, carboxyle, (alkoxy en C₁ à C₆)-carbonyle, cycloalkyle en C₃ à C₇, phényle qui est lui-même éventuellement substitué une ou deux fois par un radical alkoxy en C₁ à C₄, hétérocyclyle pentagonal ou hexagonal ayant jusqu'à deux hétéroatomes de la série N, O et/ou S et hétéroaryle pentagonal ou hexagonal ayant jusqu'à deux hétéroatomes de la série N, O et/ou S, qui est lui-même éventuellement substitué par un radical alkyle en C₁ à C₄ ou hydroxy-(alkyle en C₁ à C₄),
un reste aryle en C₆ à C₁₀ qui peut être substitué par un radical hydroxy, alkoxy en C₁ à C₆, amino, mono- ou di-(alkyle en C₁ à C₆) -amino, un reste hétéroaryle pentagonal ou hexagonal ayant jusqu'à trois hétéroatomes de la série N, O et/ou S, qui peut être substitué par un radical alkyle en C₁ à C₆, trifluorométhyle, halogéno, hydroxy, alkoxy en C₁ à C₆, trifluorométhoxy, amino, mono- ou di-(alkyle en C₁ à C₆)-amino,
un reste hétérocyclyle pentagonal ou hexagonal ayant jusqu'à deux hétéroatomes de la série N, O et/ou S, qui peut être substitué par un radical benzyle ou une à quatre fois par un radical alkyle en C₁ à C₄,
ou bien
un reste cycloalkyle en C₄ à C₈, qui peut être substitué une ou deux fois indépendamment par un radical hydroxy, amino, alkyle en C₁ à C₆, alkoxy en C₁ à C₆ , mono- ou di-(alkyle en C₁ à C₆) -amino ou par un groupe de formule R⁷-C(O)-NH- ou R⁷-SO₂-NH-,
où
R⁷ représente un reste alkoxy en C₁ à C₆, phényle, benzyle, phénylamino, mono- ou di-(alkyle en C₁ à C₆)-amino, qui sont eux-mêmes éventuellement substitués dans le groupe alkyle par un radical (alkoxy en C₁ à C₄)-carbonyle ou carboxyle, ou un reste cycloalkylamino en C₄ à C₇ qui est lui-même éventuellement substitué dans le groupe cycloalkyle par un radical alkyle en C₁ à C₄, un reste alkyle en C₁ à C₆ qui peut être substitué par un radical hydroxy, alkoxy en C₁ à C₆, amino, mono- ou di- (alkyle en C₁ à C₆) -amino, acylamino en C₁ à C₆ ou par un radical hétérocylyle pentagonal ou hexagonal ayant jusqu'à deux hétéroatomes de la série N, O et/ou S,
un reste hétérocyclyle pentagonal ou hexagonal ayant jusqu'à deux hétéroatomes de la série N, O et/ou S, lié par l'intermédiaire d'un atome de carbone du noyau ou par l'intermédiaire d'un atome d'azote du noyau, qui est éventuellement substitué par un groupe oxo,
ou bien
un reste hétéroaryle pentagonal ou hexagonal ayant jusqu'à trois hétéroatomes de la série N, O et/ou S, qui est éventuellement substitué une ou deux fois par un radical alkyle en C₁ à C₄,
ou bien
R¹ et R² forment, conjointement avec l'atome d'azote auquel ils sont liés, un hétérocycle de 4 à 11 chaînons, monocyclique, bicyclique ou spirocyclique, qui peut contenir jusqu'à deux autres hétéroatomes de la série N, O et/ou S et qui peut être substitué une à quatre fois, indépendamment, par des substituants choisis dans le groupe amino, mono- ou di-(alkyle en C₁ à C₆)-amino, hydroxy, alkoxy en C₁ à C₆, oxo, carboxyle, carbamoyle, (alkoxy en C₁ à C₆) -carbonyle, (alkoxy en C₁ à C₆) -carbonylamino, alcanoyle en C₁ à C₆, (alkyle en C₁ à C₆) -carbonylamino, alkyle en C₁ à C₆, qui est lui-même éventuellement substitué par un radical hydroxy, alkoxy en C₁ à C₆, amino, mono- ou di-(alkyle en C₁ à C₆)-amino, phényle ou par un hétérocycle pentagonal ou hexagonal ayant jusqu'à deux hétéroatomes de la série N, O et/ou S, phényle qui est lui-même éventuellement substitué par un halogène, cycloalkyle en C₃ à C₇, pyridyle, thiényle et hétérocyclyle pentagonal ou hexagonal ayant jusqu'à deux hétéroatomes de la série N, O et/ou S,
ainsi que leurs sels, leurs produits de solvatation, leurs hydrates et les hydrates des sels, acceptables du point de vue pharmaceutique.

2. Composés suivant la revendication 1, de formule (Ia) 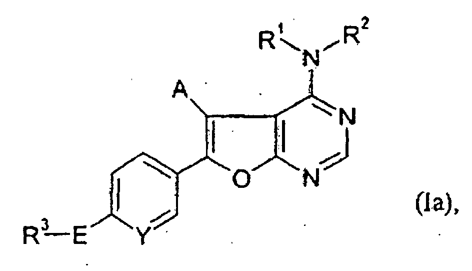 dans laquelle
A est un reste phényle qui peut être substitué par un radical fluoro, chloro, bromo ou méthoxy,
Y représente CH ou N,
E est une liaison, un reste carbonyle ou un groupe de formule *-C(O)-NH-,
dans laquelle le signe * désigne la position de liaison au groupe R₃,
R³ est un groupe hydroxy, méthoxy, amino ou un reste mono-(alkyle en C₁ à C₄)-amino qui peut être substitué dans le groupe alkyle par un radical hydroxy ou amino, un reste alkyle en C₁ à C₄ qui est éventuellement substitué par un radical hydroxy, méthoxy, éthoxy, amino, mono- ou di- (méthylamino) , (alkoxy en C₁ à C₄)-carbonylamino, acétamido, carboxyle ou (alkoxy en C₁ à C₄ ) - carbonyle,
ou bien
un hétérocycle tétragonal à hexagonal saturé ayant jusqu'à deux hétéroatomes de la série N et/ou O, lié par l'intermédiaire d'un atome de carbone du noyau ou par l'intermédiaire d'un atome d'azote du noyau, qui peut être substitué jusqu'à deux fois indépendamment par un radical alkyle en C₁ à C₄, qui peut lui-même être substitué par un radical hydroxy, méthoxy ou éthoxy, par un radical carboxyle, amino, hydroxy, méthoxy, éthoxy ou par un groupe oxo,
R¹ représente l'hydrogène, un reste méthyle ou éthyle, qui peut être substitué par un radical hydroxy ou amino.
et
R² représente un reste alkyle en C₁ à C₄ qui peut être substitué une ou deux fois indépendamment par un radical hydroxy, amino, alkoxy en C₁ à C₄, mono- ou di-(alkyle en C₁ à C₄) -amino, ou un reste cycloalkyle en C₅ à C₇,
ou bien
R¹ et R² forment conjointement avec l'atome d'azote auquel ils sont liés un noyau pyrrolidine, pipéridine, pipérazine ou morpholine, qui peut être substitué une ou deux fois indépendamment par un radical alkyle en C₁ à C₄, lui-même éventuellement substitué par un radical hydroxy ou amino, par un radical amino, mono- ou di-(alkyle en C₁ à C₄)-amino, hydroxy, alkoxy en C₁ à C₄, oxo, carboxyle, carbamoyle ou par un radical (alkoxy en C₁ à C₄)-carbonyle,
ainsi que leurs sels, leurs produits de solvatation, leurs hydrates et les hydrates des sels, acceptables du point de vue pharmaceutique.

3. Composés suivant la revendication 1, de formule (Ia)
dans laquelle
A représente un reste phényle qui peut être substitué par du fluor,
Y représente CH ou N,
E est une liaison ou un groupe de formule *-C(O)-NH-,
dans laquelle le signe * désigne la position de liaison au groupe R³,
R³ est un groupe amino ou un reste mono-(alkyle en C₁ à C₄)-amino, qui peut être substitué dans le groupe alkyle par un radical hydroxy ou amino,
un reste alkyle en C₁ à C₄ qui est éventuellement substitué par un radical hydroxy, amino, mono- ou di-(méthylamino),
ou bien
des restes pyrrolidine, pipérazine ou pipéridine qui peuvent eux-mêmes être substitués jusqu'à deux fois indépendamment par des radicaux méthyle, éthyle, n-propyle, isopropyle, n-butyle, sec.-butyle, isobutyle ou tertiobutyle pouvant eux-mêmes être substitués par un radical hydroxy, par un groupe amino ou un groupe hydroxy,
R¹ et R² forment conjointement avec l'atome d'azote auquel ils sont liés un noyau pipéridine, pipérazine ou morpholine qui peut être substitué une ou deux fois indépendamment par des radicaux méthyle, éthyle, n-propyle ou isopropyle eux-mêmes éventuellement substitués par un radical hydroxy ou amino, par un radical amino, hydroxy ou oxo,
ainsi que leurs sels, leurs produits de solvatation, leurs hydrates et les hydrates de leurs sels, acceptables du point de vue pharmaceutique.

4. Procédés de production des composés de formule (I) telle que définie dans la revendication 1, **caractérisés en ce qu'**on fait réagir des composés de formule (II) 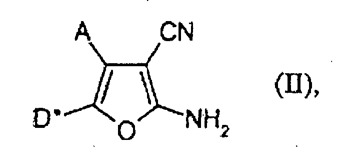 dans laquelle D* représente D ou un noyau phényle non substitué et A et D ont la définition indiquée dans la revendication 1,
soit
[A] tout d'abord avec l'acide formique dans l'acétanhydride pour obtenir des composés de formule (III) 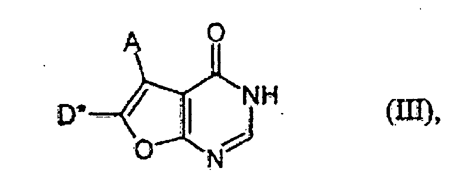 dans laquelle D* représente D ou un noyau phényle non substitué et A et D ont la définition indiquée dans la revendication 1,
que l'on transforme ensuite avec le chlorure de phosphoryle en composés de formule (IV) 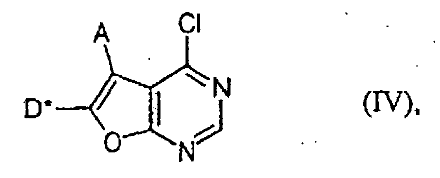 dans laquelle D* représente D ou un noyau phényle non substitué et A et D ont la définition indiquée dans la revendication 1,
puis, au cas où D* représente un noyau phényle non substitué, on nitre ce noyau phényle, et on conduit finalement la réaction avec des composés de formule (V), 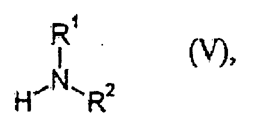 dans laquelle R¹ et R² ont la définition indiquée dans la revendication 1,
pour obtenir des composés de formule (I)
soit
[B] tout d'abord avec l'ester triéthylique de l'acide orthoformique pour les transformer en composés de formule (VI) 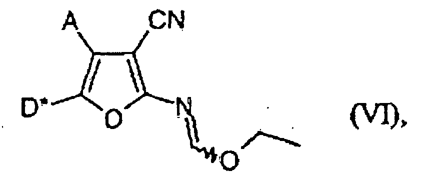 dans laquelle D* représente D ou un noyau phényle non substitué et A et D ont- la définition indiquée dans la revendication 1,
que l'on fait ensuite réagir avec des composés de formule (VII) 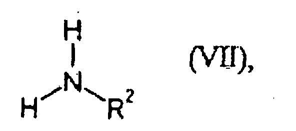 dans laquelle R² a la définition indiquée dans la revendication 1,
puis on conduit la réaction avec une base, directement ou, au cas où D* représente un noyau phényle non substitué, par nitration subséquente de ce noyau phényle, pour obtenir des composés de formule (I),
les composés de formule (I) ainsi obtenus pouvant ensuite, le cas échéant être soumis à d'autres dérivatisations, qui peuvent être conduites par des procédés classiques.

5. Composés de formule (I) tels que définis dans la revendication 1, destinés à la prophylaxie et/ou au traitement de maladies.

6. Médicament contenant au moins un composé de formule (I) tel que défini dans la revendication 1 et au moins une autre substance auxiliaire.

7. Médicament contenant au moins un composé de formule (I) tel que défini dans la revendication 1 et au moins une autre substance active.

8. Utilisation de composés de formule (I) tels que définis dans la revendication 1, pour la préparation de médicaments destinés à la prophylaxie et/ou au traitement de maladies vasculaires périphériques et cardiaques à cause ischémique.

9. Utilisation de composés de formule (I) tels que définis dans la revendication 1, pour la préparation de médicaments destinés au traitement aigu et chronique de maladies ischémiques du système cardiovasculaire, par exemple de l'insuffisance coronarienne, de l'angine de poitrine stable et instable, de maladies périphériques et artérielles oblitérantes, d'occlusions vasculaires thrombotiques, de l'infarctus du myocarde et de lésions de reperfusion.

10. Utilisation de composés de formule (I) tels que définis dans la revendication 1, pour la préparation de médicaments destinés au traitement de douleurs aiguës et/où chroniques ainsi que de maladies neurodégénératives.
